(19) Europäisches Patentamt
European Patent Office
Office européen des brevets

(11) EP 2 594 631 A1

(12) EUROPEAN PATENT APPLICATION

(43) Date of publication:
**22.05.2013 Bulletin 2013/21**

(21) Application number: **12180052.8**

(22) Date of filing: **05.04.2006**

(51) Int Cl.:
***C12M 1/00*** (2006.01) ***C12M 1/34*** (2006.01)
***C12M 3/00*** (2006.01) ***C12Q 1/68*** (2006.01)
***G01N 15/06*** (2006.01) ***G01N 33/00*** (2006.01)
***G01N 33/48*** (2006.01) ***B82Y 5/00*** (2011.01)
***B82Y 10/00*** (2011.01) ***G01N 33/543*** (2006.01)
***G01N 33/574*** (2006.01) *B01L 3/00* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority:
**05.04.2005 US 668415 P**
**29.07.2005 US 703833 P**
**29.12.2005 US 323946**
**29.12.2005 US 322790**
**29.12.2005 US 324041**
**29.12.2005 US 323945**
**29.12.2005 US 323962**

(62) Document number(s) of the earlier application(s) in accordance with Art. 76 EPC:
**06749394.0 / 1 874 920**

(71) Applicants:
- **Cellpoint Diagnostics**
  **Watertown, MA 02472 (US)**
- **Living Microsystems**
  **Watertown MA 02472 (US)**
- **The General Hospital Corporation**
  **Boston, MA 02114 (US)**

(72) Inventors:
- **Fuchs, Martin**
  **Uxbridge, MA 01569 (US)**
- **Toner, Mehmet**
  **Wellesley, MA 02481 (US)**
- **Wang, Ying-xin**
  **Rockville, MD (US)**
- **Huang, Yi-Shuian**
  **Roslindale, MA 02131 (US)**
- **Huang, Lotien Richard**
  **Chestnut Hill, MA 02467-2129 (US)**
- **Krueger, Neil X.**
  **Roslindale, MA 02131 (US)**

(74) Representative: **Vossius & Partner**
**Siebertstrasse 4**
**81675 München (DE)**

Remarks:
•This application was filed on 10-08-2012 as a divisional application to the application mentioned under INID code 62.
•Claims filed after the date of filing of the application/ after the date of receipt of the divisaional application (Rule 68(4) EPC).

# (54) Devices and method for detecting circulating tumor cells and other particles

(57) The invention features devices and methods for detecting, enriching, and analyzing circulating tumor cells and other particles. The invention further features methods of diagnosing a condition, e.g., cancer, in a subject by analyzing a cellular sample from the subject.

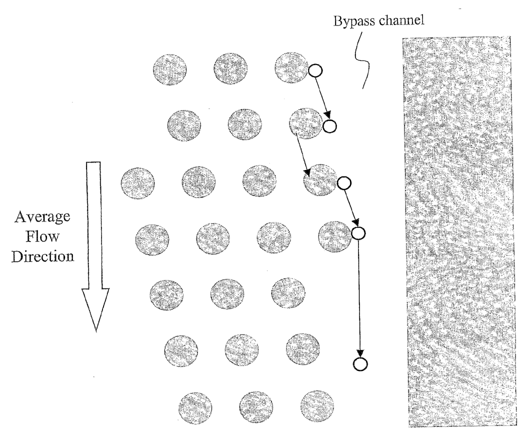


Fig. 9

EP 2 594 631 A1

SEQUENCE LISTING

<110> CELLPOINT DIAGNOSTICS
LIVING MICROSYSTEMS
THE GENERAL HOSPITAL CORPORATION

<120> Devices and Methods for Enrichment and Alteration of Circulating Tumor Cells and Other Particles

<130> N2777 EP/1 S3

<140> EP12 18 0052.8
<141> 2006-04-05

<150> PCT/US2006/012778
<151> 2006-04-05

<150> US 60/668,415
<151> 2005-04-05

<150> US 11/323,962
<151> 2005-12-29

<150> US 11/323,946
<151> 2005-12-29

<150> US 11/323,945
<151> 2005-12-29

<150> US 11/322,790
<151> 2005-12-29

<150> US 11/324,041
<151> 2005-12-29

<150> US 60/703,833
<151> 2005-07-29

<160> 686

<170> BiSSAP 1.0

<210> 1
<211> 17
<212> DNA
<213> Artificial Sequence

<220>
<221> source
<222> 1..17
<223> /mol_type="DNA"
/note="synthetic"
/organism="Artificial Sequence"

<400> 1
ttgctgctgg tggtggc 17

<210> 2
<211> 20
<212> DNA
<213> Artificial Sequence

```
<220>
<221> source
<222> 1..20
<223> /mol_type="DNA"
      /note="synthetic"
      /organism="Artificial Sequence"

<400> 2
cagggattcc gtcatatggc                                                   20


<210> 3
<211> 18
<212> DNA
<213> Artificial Sequence

<220>
<221> source
<222> 1..18
<223> /mol_type="DNA"
      /note="synthetic"
      /organism="Artificial Sequence"

<400> 3
gatcggcctc ttcatgcg                                                     18


<210> 4
<211> 22
<212> DNA
<213> Artificial Sequence

<220>
<221> source
<222> 1..22
<223> /mol_type="DNA"
      /note="synthetic"
      /organism="Artificial Sequence"

<400> 4
gatccaaagg tcatcaactc cc                                                22


<210> 5
<211> 18
<212> DNA
<213> Artificial Sequence

<220>
<221> source
<222> 1..18
<223> /mol_type="DNA"
      /note="Synthetic"
      /organism="Artificial Sequence"

<400> 5
gctgtccaac gaatgggc                                                     18


<210> 6
<211> 20
<212> DNA
<213> Artificial Sequence
```

<220>
<221> source
<222> 1..20
<223> /mol_type="DNA"
/note="synthetic"
/organism="Artificial Sequence"

<400> 6
ggcgttctcc tttctccagg 20

<210> 7
<211> 19
<212> DNA
<213> Artificial Sequence

<220>
<221> source
<222> 1..19
<223> /mol_type="DNA"
/note="synthetic"
/organism="Artificial Sequence"

<400> 7
atgcactggg ccaggtctt 19

<210> 8
<211> 21
<212> DNA
<213> Artificial Sequence

<220>
<221> source
<222> 1..21
<223> /mol_type="DNA"
/note="synthetic"
/organism="Artificial Sequence"

<400> 8
cgatggtaca tatgggtggc t 21

<210> 9
<211> 19
<212> DNA
<213> Artificial Sequence

<220>
<221> source
<222> 1..19
<223> /mol_type="DNA"
/note="synthetic"
/organism="Artificial Sequence"

<400> 9
aggctgtcca acgaatggg 19

<210> 10
<211> 19
<212> DNA

<213> Artificial Sequence

<220>
<221> source
<222> 1..19
<223> /mol_type="DNA"
/note="synthetic"
/organism="Artificial Sequence"

<400> 10
ctgagggagg cgttctcct 19

<210> 11
<211> 22
<212> DNA
<213> Artificial Sequence

<220>
<221> source
<222> 1..22
<223> /mol_type="DNA"
/note="synthetic"
/organism="Artificial Sequence"

<400> 11
tcagagcctg tgtttctacc aa 22

<210> 12
<211> 22
<212> DNA
<213> Artificial Sequence

<220>
<221> source
<222> 1..22
<223> /mol_type="DNA"
/note="synthetic"
/organism="Artificial Sequence"

<400> 12
tggtctcaca ggaccactga tt 22

<210> 13
<211> 20
<212> DNA
<213> Artificial Sequence

<220>
<221> source
<222> 1..20
<223> /mol_type="DNA"
/note="synthetic"
/organism="Artificial Sequence"

<400> 13
tccaaatgag ctggcaagtg 20

<210> 14
<211> 23

<212> DNA
<213> Artificial Sequence

<220>
<221> source
<222> 1..23
<223> /mol_type="DNA"
/note="synthetic"
/organism="Artificial Sequence"

<400> 14
tcccaaacac tcagtgaaac aaa 23

<210> 15
<211> 24
<212> DNA
<213> Artificial Sequence

<220>
<221> source
<222> 1..24
<223> /mol_type="DNA"
/note="synthetic"
/organism="Artificial Sequence"

<400> 15
aaataatcag tgtgattcgt ggag 24

<210> 16
<211> 22
<212> DNA
<213> Artificial Sequence

<220>
<221> source
<222> 1..22
<223> /mol_type="DNA"
/note="synthetic"
/organism="Artificial Sequence"

<400> 16
gaggccagtg ctgtctctaa gg 22

<210> 17
<211> 20
<212> DNA
<213> Artificial Sequence

<220>
<221> source
<222> 1..20
<223> /mol_type="DNA"
/note="synthetic"
/organism="Artificial Sequence"

<400> 17
gtgcatcgct ggtaacatcc 20

<210> 18

<211> 20
<212> DNA
<213> Artificial Sequence

<220>
<221> source
<222> 1..20
<223> /mol_type="DNA"
/note="synthetic"
/organism="Artificial Sequence"

<400> 18
tgtggagatg agcagggtct 20

<210> 19
<211> 21
<212> DNA
<213> Artificial Sequence

<220>
<221> source
<222> 1..21
<223> /mol_type="DNA"
/note="synthetic"
/organism="Artificial Sequence"

<400> 19
acttcacagc cctgcgtaaa c 21

<210> 20
<211> 21
<212> DNA
<213> Artificial Sequence

<220>
<221> source
<222> 1..21
<223> /mol_type="DNA"
/note="synthetic"
/organism="Artificial Sequence"

<400> 20
atgggacagg cactgatttg t 21

<210> 21
<211> 20
<212> DNA
<213> Artificial Sequence

<220>
<221> source
<222> 1..20
<223> /mol_type="DNA"
/note="synthetic"
/organism="Artificial Sequence"

<400> 21
atcgcattca tgcgtcttca 20

<210> 22
<211> 20
<212> DNA
<213> Artificial Sequence

<220>
<221> source
<222> 1..20
<223> /mol_type="DNA"
/note="synthetic"
/organism="Artificial Sequence"

<400> 22
atccccatgg caaactcttg 20

<210> 23
<211> 22
<212> DNA
<213> Artificial Sequence

<220>
<221> source
<222> 1..22
<223> /mol_type="DNA"
/note="synthetic"
/organism="Artificial Sequence"

<400> 23
gcagcgggtt acatcttctt tc 22

<210> 24
<211> 22
<212> DNA
<213> Artificial Sequence

<220>
<221> source
<222> 1..22
<223> /mol_type="DNA"
/note="synthetic"
/organism="Artificial Sequence"

<400> 24
cagctctggc tcacactacc ag 22

<210> 25
<211> 22
<212> DNA
<213> Artificial Sequence

<220>
<221> source
<222> 1..22
<223> /mol_type="DNA"
/note="synthetic"
/organism="Artificial Sequence"

<400> 25
gcagcgggtt acatcttctt tc 22

<210> 26
<211> 19
<212> DNA
<213> Artificial Sequence

<220>
<221> source
<222> 1..19
<223> /mol_type="DNA"
/note="synthetic"
/organism="Artificial Sequence"

<400> 26
catcctcccc tgcatgtgt 19

<210> 27
<211> 20
<212> DNA
<213> Artificial Sequence

<220>
<221> source
<222> 1..20
<223> /mol_type="DNA"
/note="synthetic"
/organism="Artificial Sequence"

<400> 27
ccgcagcatg tcaagatcac 20

<210> 28
<211> 25
<212> DNA
<213> Artificial Sequence

<220>
<221> source
<222> 1..25
<223> /mol_type="DNA"
/note="synthetic"
/organism="Artificial Sequence"

<400> 28
tccttctgca tggtattctt tctct 25

<210> 29
<211> 14
<212> DNA
<213> Artificial Sequence

<220>
<221> source
<222> 1..14
<223> /mol_type="DNA"
/note="synthetic"
/organism="Artificial Sequence"

<400> 29
tttgggctgg ccaa 14

<210> 30
<211> 14
<212> DNA
<213> Artificial Sequence

<220>
<221> source
<222> 1..14
<223> /mol_type="DNA"
/note="synthetic"
/organism="Artificial Sequence"

<400> 30
ttttgggcgg gcca 14

<210> 31
<211> 17
<212> DNA
<213> Artificial Sequence

<220>
<221> source
<222> 1..17
<223> /mol_type="DNA"
/note="synthetic"
/organism="Artificial Sequence"

<400> 31
atggccagcg tggacaa 17

<210> 32
<211> 23
<212> DNA
<213> Artificial Sequence

<220>
<221> source
<222> 1..23
<223> /mol_type="DNA"
/note="synthetic"
/organism="Artificial Sequence"

<400> 32
agcaggtact gggagccaat att 23

<210> 33
<211> 16
<212> DNA
<213> Artificial Sequence

<220>
<221> source
<222> 1..16
<223> /mol_type="DNA"
/note="synthetic"
/organism="Artificial Sequence"

<400> 33

atgagctgcg tgatga 16

<210> 34
<211> 16
<212> DNA
<213> Artificial Sequence

<220>
<221> source
<222> 1..16
<223> /mol_type="DNA"
/note="synthetic"
/organism="Artificial Sequence"

<400> 34
atgagctgca tgatga 16

<210> 35
<211> 22
<212> DNA
<213> Artificial Sequence

<220>
<221> source
<222> 1..22
<223> /mol_type="DNA"
/note="synthetic"
/organism="Artificial Sequence"

<400> 35
gcctcttaca cccagtggag aa 22

<210> 36
<211> 18
<212> DNA
<213> Artificial Sequence

<220>
<221> source
<222> 1..18
<223> /mol_type="DNA"
/note="synthetic"
/organism="Artificial Sequence"

<400> 36
gcctgtgcca gggacctt 18

<210> 37
<211> 14
<212> DNA
<213> Artificial Sequence

<220>
<221> source
<222> 1..14
<223> /mol_type="DNA"
/note="synthetic"
/organism="Artificial Sequence"

<400> 37
accggagccc agca 14

<210> 38
<211> 14
<212> DNA
<213> Artificial Sequence

<220>
<221> source
<222> 1..14
<223> /mol_type="DNA"
/note="synthetic"
/organism="Artificial Sequence"

<400> 38
accggagctc agca 14

<210> 39
<211> 14
<212> DNA
<213> Artificial Sequence

<220>
<221> source
<222> 1..14
<223> /mol_type="DNA"
/note="synthetic"
/organism="Artificial Sequence"

<400> 39
accggagcac agca 14

<210> 40
<211> 24
<212> DNA
<213> Artificial Sequence

<220>
<221> source
<222> 1..24
<223> /mol_type="DNA"
/note="synthetic"
/organism="Artificial Sequence"

<400> 40
acagcagggt cttctctgtt tcag 24

<210> 41
<211> 21
<212> DNA
<213> Artificial Sequence

<220>
<221> source
<222> 1..21
<223> /mol_type="DNA"
/note="synthetic"
/organism="Artificial Sequence"

```
<400> 41
atcttgacat gctgcggtgt t                                                 21


<210> 42
<211> 14
<212> DNA
<213> Artificial Sequence

<220>
<221> source
<222> 1..14
<223> /mol_type="DNA"
      /note="synthetic"
      /organism="Artificial Sequence"

<400> 42
ttggtgcacc gcga                                                         14


<210> 43
<211> 14
<212> DNA
<213> Artificial Sequence

<220>
<221> source
<222> 1..14
<223> /mol_type="DNA"
      /note="synthetic"
      /organism="Artificial Sequence"

<400> 43
tggtgctccg cgac                                                         14


<210> 44
<211> 19
<212> DNA
<213> Artificial Sequence

<220>
<221> source
<222> 1..19
<223> /mol_type="DNA"
      /note="synthetic"
      /organism="Artificial Sequence"

<400> 44
agtcaggacc catgcacgg                                                    19


<210> 45
<211> 20
<212> DNA
<213> Artificial Sequence

<220>
<221> source
<222> 1..20
<223> /mol_type="DNA"
      /note="synthetic"
```

/organism="Artificial Sequence"

<400> 45
acccaagatg cagcagtgtg 20

<210> 46
<211> 21
<212> DNA
<213> Artificial Sequence

<220>
<221> source
<222> 1..21
<223> /mol_type="DNA"
/note="synthetic"
/organism="Artificial Sequence"

<400> 46
gatgtcctcc ttgttctact c 21

<210> 47
<211> 22
<212> DNA
<213> Artificial Sequence

<220>
<221> source
<222> 1..22
<223> /mol_type="DNA"
/note="synthetic"
/organism="Artificial Sequence"

<400> 47
tacagggaat aatcgagcat gc 22

<210> 48
<211> 22
<212> DNA
<213> Artificial Sequence

<220>
<221> source
<222> 1..22
<223> /mol_type="DNA"
/note="synthetic"
/organism="Artificial Sequence"

<400> 48
gaagggaaat agcaaatgga ca 22

<210> 49
<211> 20
<212> DNA
<213> Artificial Sequence

<220>
<221> source
<222> 1..20
<223> /mol_type="DNA"

/note="synthetic"
/organism="Artificial Sequence"

<400> 49
cgatggagtc caagttctgg 20

<210> 50
<211> 20
<212> DNA
<213> Artificial Sequence

<220>
<221> source
<222> 1..20
<223> /mol_type="DNA"
/note="synthetic"
/organism="Artificial Sequence"

<400> 50
agcacttaca gctctggcca 20

<210> 51
<211> 22
<212> DNA
<213> Artificial Sequence

<220>
<221> source
<222> 1..22
<223> /mol_type="DNA"
/note="synthetic"
/organism="Artificial Sequence"

<400> 51
gactgaacat aactgtaggc tg 22

<210> 52
<211> 20
<212> DNA
<213> Artificial Sequence

<220>
<221> source
<222> 1..20
<223> /mol_type="DNA"
/note="Synthetic"
/organism="Artificial Sequence"

<400> 52
gttcggcacg gtgtataagg 20

<210> 53
<211> 20
<212> DNA
<213> Artificial Sequence

<220>
<221> source
<222> 1..20

<223> /mol_type="DNA"
/note="Synthetic"
/organism="Artificial Sequence"

<400> 53
ctggccatca cgtaggcttc 20

<210> 54
<400> 54
000

<210> 55
<400> 55
000

<210> 56
<400> 56
000

<210> 57
<400> 57
000

<210> 58
<400> 58
000

<210> 59
<400> 59
000

<210> 60
<400> 60
000

<210> 61
<400> 61
000

<210> 62
<400> 62
000

<210> 63
<400> 63
000

<210> 64
<400> 64
000

<210> 65
<400> 65
000

<210> 66
<400> 66
000

<210> 67
<400> 67
000

<210> 68
<400> 68
000

<210> 69
<400> 69
000

<210> 70
<400> 70
000

<210> 71
<400> 71
000

<210> 72
<400> 72
000

<210> 73
<400> 73
000

<210> 74
<400> 74
000

<210> 75
<400> 75
000

<210> 76
<400> 76
000

<210> 77
<400> 77
000

<210> 78
<400> 78
000

<210> 79
<400> 79
000

<210> 80
<400> 80
000

<210> 81
<400> 81
000

<210> 82
<400> 82
000

<210> 83
<400> 83

000

<210> 84
<400> 84
000

<210> 85
<400> 85
000

<210> 86
<400> 86
000

<210> 87
<400> 87
000

<210> 88
<400> 88
000

<210> 89
<400> 89
000

<210> 90
<400> 90
000

<210> 91
<400> 91
000

<210> 92
<400> 92
000

<210> 93
<400> 93
000

<210> 94
<400> 94
000

<210> 95
<400> 95
000

<210> 96
<400> 96
000

<210> 97
<400> 97
000

<210> 98
<400> 98
000

<210> 99

<400> 99
000

<210> 100
<400> 100
000

<210> 101
<211> 21
<212> DNA
<213> Artificial Sequence

<220>
<221> source
<222> 1..21
<223> /mol_type="DNA"
/note="synthetic"
/organism="Artificial Sequence"

<400> 101
tggatcccag aaggtgagaa a 21

<210> 102
<211> 24
<212> DNA
<213> Artificial Sequence

<220>
<221> source
<222> 1..24
<223> /mol_type="DNA"
/note="synthetic"
/organism="Artificial Sequence"

<400> 102
agcagaaact cacatcgagg attt 24

<210> 103
<211> 19
<212> DNA
<213> Artificial Sequence

<220>
<221> source
<222> 1..19
<223> /mol_type="DNA"
/note="synthetic"
/organism="Artificial Sequence"

<400> 103
aaggaattaa gagaagcaa 19

<210> 104
<211> 16
<212> DNA
<213> Artificial Sequence

<220>
<221> source
<222> 1..16

<223> /mol_type="DNA"
/note="synthetic"
/organism="Artificial Sequence"

<400> 104
ctatcaaaac atctcc 16

<210> 105
<211> 16
<212> DNA
<213> Artificial Sequence

<220>
<221> source
<222> 1..16
<223> /mol_type="DNA"
/note="synthetic"
/organism="Artificial Sequence"

<400> 105
ctatcaagac atctcc 16

<210> 106
<400> 106
000

<210> 107
<400> 107
000

<210> 108
<400> 108
000

<210> 109
<400> 109
000

<210> 110
<400> 110
000

<210> 111
<400> 111
000

<210> 112
<400> 112
000

<210> 113
<400> 113
000

<210> 114
<400> 114
000

<210> 115
<400> 115
000

<210> 116
<400> 116
000

<210> 117
<400> 117
000

<210> 118
<400> 118
000

<210> 119
<400> 119
000

<210> 120
<400> 120
000

<210> 121
<400> 121
000

<210> 122
<400> 122
000

<210> 123
<400> 123
000

<210> 124
<400> 124
000

<210> 125
<400> 125
000

<210> 126
<400> 126
000

<210> 127
<400> 127
000

<210> 128
<400> 128
000

<210> 129
<400> 129
000

<210> 130
<400> 130
000

<210> 131
<400> 131

000

<210> 132
<400> 132
000

<210> 133
<400> 133
000

<210> 134
<400> 134
000

<210> 135
<400> 135
000

<210> 136
<400> 136
000

<210> 137
<400> 137
000

<210> 138
<400> 138
000

<210> 139
<400> 139
000

<210> 140
<400> 140
000

<210> 141
<400> 141
000

<210> 142
<400> 142
000

<210> 143
<400> 143
000

<210> 144
<400> 144
000

<210> 145
<400> 145
000

<210> 146
<400> 146
000

<210> 147

<400> 147
000

<210> 148
<400> 148
000

<210> 149
<400> 149
000

<210> 150
<400> 150
000

<210> 151
<400> 151
000

<210> 152
<400> 152
000

<210> 153
<400> 153
000

<210> 154
<400> 154
000

<210> 155
<400> 155
000

<210> 156
<400> 156
000

<210> 157
<400> 157
000

<210> 158
<400> 158
000

<210> 159
<400> 159
000

<210> 160
<400> 160
000

<210> 161
<400> 161
000

<210> 162
<400> 162
000

<210> 163
<400> 163
000

<210> 164
<400> 164
000

<210> 165
<400> 165
000

<210> 166
<400> 166
000

<210> 167
<400> 167
000

<210> 168
<400> 168
000

<210> 169
<400> 169
000

<210> 170
<400> 170
000

<210> 171
<400> 171
000

<210> 172
<400> 172
000

<210> 173
<400> 173
000

<210> 174
<400> 174
000

<210> 175
<400> 175
000

<210> 176
<400> 176
000

<210> 177
<400> 177
000

<210> 178
<400> 178
000

<210> 179
<400> 179
000

<210> 180
<400> 180
000

<210> 181
<400> 181
000

<210> 182
<400> 182
000

<210> 183
<400> 183
000

<210> 184
<400> 184
000

<210> 185
<400> 185
000

<210> 186
<400> 186
000

<210> 187
<400> 187
000

<210> 188
<400> 188
000

<210> 189
<400> 189
000

<210> 190
<400> 190
000

<210> 191
<400> 191
000

<210> 192
<400> 192
000

<210> 193
<400> 193
000

<210> 194
<400> 194

000

<210> 195
<400> 195
000

<210> 196
<400> 196
000

<210> 197
<400> 197
000

<210> 198
<400> 198
000

<210> 199
<400> 199
000

<210> 200
<400> 200
000

<210> 201
<400> 201
000

<210> 202
<400> 202
000

<210> 203
<400> 203
000

<210> 204
<400> 204
000

<210> 205
<400> 205
000

<210> 206
<400> 206
000

<210> 207
<400> 207
000

<210> 208
<400> 208
000

<210> 209
<400> 209
000

<210> 210

<400> 210
000

<210> 211
<400> 211
000

<210> 212
<400> 212
000

<210> 213
<211> 22
<212> DNA
<213> Artificial Sequence

<220>
<221> source
<222> 1..22
<223> /mol_type="DNA"
/note="synthetic"
/organism="Artificial Sequence"

<400> 213
catttcccct aatccttttc ca 22

<210> 214
<211> 22
<212> DNA
<213> Artificial Sequence

<220>
<221> source
<222> 1..22
<223> /mol_type="DNA"
/note="synthetic"
/organism="Artificial Sequence"

<400> 214
gtgatcccag atttaggcct tc 22

<210> 215
<211> 22
<212> DNA
<213> Artificial Sequence

<220>
<221> source
<222> 1..22
<223> /mol_type="DNA"
/note="synthetic"
/organism="Artificial Sequence"

<400> 215
gcctctcgtg gtttgttttg tc 22

<210> 216
<211> 22
<212> DNA
<213> Artificial Sequence

<220>
<221> source
<222> 1..22
<223> /mol_type="DNA"
/note="synthetic"
/organism="Artificial Sequence"

<400> 216
cccagggtag ggtccaataa tc 22

<210> 217
<211> 22
<212> DNA
<213> Artificial Sequence

<220>
<221> source
<222> 1..22
<223> /mol_type="DNA"
/note="synthetic"
/organism="Artificial Sequence"

<400> 217
cttcctggtg gaggtgactg at 22

<210> 218
<211> 22
<212> DNA
<213> Artificial Sequence

<220>
<221> source
<222> 1..22
<223> /mol_type="DNA"
/note="synthetic"
/organism="Artificial Sequence"

<400> 218
caggcatagt gtgtgatggt ca 22

<210> 219
<211> 23
<212> DNA
<213> Artificial Sequence

<220>
<221> source
<222> 1..23
<223> /mol_type="DNA"
/note="synthetic"
/organism="Artificial Sequence"

<400> 219
tcacgataca cattctcaga tcc 23

<210> 220
<211> 22
<212> DNA

<213> Artificial Sequence

<220>
<221> source
<222> 1..22
<223> /mol_type="DNA"
/note="synthetic"
/organism="Artificial Sequence"

<400> 220
gaagatctcc cagaggagga tg 22

<210> 221
<211> 21
<212> DNA
<213> Artificial Sequence

<220>
<221> source
<222> 1..21
<223> /mol_type="DNA"
/note="synthetic"
/organism="Artificial Sequence"

<400> 221
cgtaacgtgc tgttgaccaa t 21

<210> 222
<211> 22
<212> DNA
<213> Artificial Sequence

<220>
<221> source
<222> 1..22
<223> /mol_type="DNA"
/note="synthetic"
/organism="Artificial Sequence"

<400> 222
aaacgaggga agagccagaa ag 22

<210> 223
<211> 22
<212> DNA
<213> Artificial Sequence

<220>
<221> source
<222> 1..22
<223> /mol_type="DNA"
/note="synthetic"
/organism="Artificial Sequence"

<400> 223
tggggagcac aataaaagaa ga 22

<210> 224
<211> 22

<212> DNA
<213> Artificial Sequence

<220>
<221> source
<222> 1..22
<223> /mol_type="DNA"
/note="synthetic"
/organism="Artificial Sequence"

<400> 224
actcttggct cctggattct tg 22

<210> 225
<211> 22
<212> DNA
<213> Artificial Sequence

<220>
<221> source
<222> 1..22
<223> /mol_type="DNA"
/note="synthetic"
/organism="Artificial Sequence"

<400> 225
ggaagtcagt gtgcagggaa ta 22

<210> 226
<211> 22
<212> DNA
<213> Artificial Sequence

<220>
<221> source
<222> 1..22
<223> /mol_type="DNA"
/note="synthetic"
/organism="Artificial Sequence"

<400> 226
ttttagcaga aataggcaag ca 22

<210> 227
<211> 24
<212> DNA
<213> Artificial Sequence

<220>
<221> source
<222> 1..24
<223> /mol_type="DNA"
/note="synthetic"
/organism="Artificial Sequence"

<400> 227
tggtaatcct aaacacaatg caga 24

<210> 228

<211> 22
<212> DNA
<213> Artificial Sequence

<220>
<221> source
<222> 1..22
<223> /mol_type="DNA"
/note="synthetic"
/organism="Artificial Sequence"

<400> 228
ctgggcaaca cagtgagatc ct 22

<210> 229
<211> 23
<212> DNA
<213> Artificial Sequence

<220>
<221> source
<222> 1..23
<223> /mol_type="DNA"
/note="synthetic"
/organism="Artificial Sequence"

<400> 229
tcacaaattt ctttgctgtg tcc 23

<210> 230
<211> 23
<212> DNA
<213> Artificial Sequence

<220>
<221> source
<222> 1..23
<223> /mol_type="DNA"
/note="synthetic"
/organism="Artificial Sequence"

<400> 230
catggaactc cagattagcc tgt 23

<210> 231
<211> 22
<212> DNA
<213> Artificial Sequence

<220>
<221> source
<222> 1..22
<223> /mol_type="DNA"
/note="synthetic"
/organism="Artificial Sequence"

<400> 231
gattgttgca gatcgtggac at 22

<210> 232
<211> 22
<212> DNA
<213> Artificial Sequence

<220>
<221> source
<222> 1..22
<223> /mol_type="DNA"
/note="synthetic"
/organism="Artificial Sequence"

<400> 232
cgcttaaatc ttcccattcc ag 22

<210> 233
<211> 22
<212> DNA
<213> Artificial Sequence

<220>
<221> source
<222> 1..22
<223> /mol_type="DNA"
/note="synthetic"
/organism="Artificial Sequence"

<400> 233
ctccatggca ccatcattaa ca 22

<210> 234
<211> 22
<212> DNA
<213> Artificial Sequence

<220>
<221> source
<222> 1..22
<223> /mol_type="DNA"
/note="synthetic"
/organism="Artificial Sequence"

<400> 234
ctcaggacac aagtgctctg ct 22

<210> 235
<211> 24
<212> DNA
<213> Artificial Sequence

<220>
<221> source
<222> 1..24
<223> /mol_type="DNA"
/note="synthetic"
/organism="Artificial Sequence"

<400> 235
gcagttcatg gttcatcttc tttt 24

<210> 236
<211> 22
<212> DNA
<213> Artificial Sequence

<220>
<221> source
<222> 1..22
<223> /mol_type="DNA"
/note="synthetic"
/organism="Artificial Sequence"

<400> 236
caaaatagcc caccctggat ta 22

<210> 237
<211> 21
<212> DNA
<213> Artificial Sequence

<220>
<221> source
<222> 1..21
<223> /mol_type="DNA"
/note="synthetic"
/organism="Artificial Sequence"

<400> 237
ctttctgcat tgcccaagat g 21

<210> 238
<211> 22
<212> DNA
<213> Artificial Sequence

<220>
<221> source
<222> 1..22
<223> /mol_type="DNA"
/note="synthetic"
/organism="Artificial Sequence"

<400> 238
caaggtctca gtgagtggtg ga 22

<210> 239
<211> 23
<212> DNA
<213> Artificial Sequence

<220>
<221> source
<222> 1..23
<223> /mol_type="DNA"
/note="synthetic"
/organism="Artificial Sequence"

<400> 239
gagaagggtc tttctgactc tgc 23

<210> 240
<211> 22
<212> DNA
<213> Artificial Sequence

<220>
<221> source
<222> 1..22
<223> /mol_type="DNA"
/note="synthetic"
/organism="Artificial Sequence"

<400> 240
caggtgtttc tcctgtgagg tg 22

<210> 241
<211> 22
<212> DNA
<213> Artificial Sequence

<220>
<221> source
<222> 1..22
<223> /mol_type="DNA"
/note="synthetic"
/organism="Artificial Sequence"

<400> 241
cacattgcgg cctagaatgt ta 22

<210> 242
<211> 20
<212> DNA
<213> Artificial Sequence

<220>
<221> source
<222> 1..20
<223> /mol_type="DNA"
/note="synthetic"
/organism="Artificial Sequence"

<400> 242
accccgtcac aaccttcagt 20

<210> 243
<211> 22
<212> DNA
<213> Artificial Sequence

<220>
<221> source
<222> 1..22
<223> /mol_type="DNA"
/note="synthetic"
/organism="Artificial Sequence"

<400> 243

gccgtagccc caaagtgtac ta 22

<210> 244
<211> 22
<212> DNA
<213> Artificial Sequence

<220>
<221> source
<222> 1..22
<223> /mol_type="DNA"
/note="synthetic"
/organism="Artificial Sequence"

<400> 244
tcagctcaaa cctgtgattt cc 22

<210> 245
<211> 24
<212> DNA
<213> Artificial Sequence

<220>
<221> source
<222> 1..24
<223> /mol_type="DNA"
/note="synthetic"
/organism="Artificial Sequence"

<400> 245
ctcactctcc ataaatgcta cgaa 24

<210> 246
<211> 22
<212> DNA
<213> Artificial Sequence

<220>
<221> source
<222> 1..22
<223> /mol_type="DNA"
/note="synthetic"
/organism="Artificial Sequence"

<400> 246
gacttaacgt gtccccttt gc 22

<210> 247
<211> 22
<212> DNA
<213> Artificial Sequence

<220>
<221> source
<222> 1..22
<223> /mol_type="DNA"
/note="synthetic"
/organism="Artificial Sequence"

<400> 247
gcctcttcgg ggtaatcaga ta 22

<210> 248
<211> 22
<212> DNA
<213> Artificial Sequence

<220>
<221> source
<222> 1..22
<223> /mol_type="DNA"
/note="synthetic"
/organism="Artificial Sequence"

<400> 248
gaagtctgtg gtttagcgga ca 22

<210> 249
<211> 22
<212> DNA
<213> Artificial Sequence

<220>
<221> source
<222> 1..22
<223> /mol_type="DNA"
/note="synthetic"
/organism="Artificial Sequence"

<400> 249
atcttttgcc tggaggaact tt 22

<210> 250
<211> 22
<212> DNA
<213> Artificial Sequence

<220>
<221> source
<222> 1..22
<223> /mol_type="DNA"
/note="synthetic"
/organism="Artificial Sequence"

<400> 250
cagggtaaat tcatcccatt ga 22

<210> 251
<211> 22
<212> DNA
<213> Artificial Sequence

<220>
<221> source
<222> 1..22
<223> /mol_type="DNA"
/note="synthetic"
/organism="Artificial Sequence"

<400> 251
cagcagccag cacaactact tt 22

<210> 252
<211> 23
<212> DNA
<213> Artificial Sequence

<220>
<221> source
<222> 1..23
<223> /mol_type="DNA"
/note="synthetic"
/organism="Artificial Sequence"

<400> 252
ttggctagat gaaccattga tga 23

<210> 253
<211> 24
<212> DNA
<213> Artificial Sequence

<220>
<221> source
<222> 1..24
<223> /mol_type="DNA"
/note="synthetic"
/organism="Artificial Sequence"

<400> 253
tgaatgaagc tcctgtgttt actc 24

<210> 254
<211> 22
<212> DNA
<213> Artificial Sequence

<220>
<221> source
<222> 1..22
<223> /mol_type="DNA"
/note="synthetic"
/organism="Artificial Sequence"

<400> 254
atgttcatcg caggctaatg tg 22

<210> 255
<211> 24
<212> DNA
<213> Artificial Sequence

<220>
<221> source
<222> 1..24
<223> /mol_type="DNA"
/note="synthetic"

/organism="Artificial Sequence"

<400> 255
aaaacaggga gaacttctaa gcaa 24

<210> 256
<211> 21
<212> DNA
<213> Artificial Sequence

<220>
<221> source
<222> 1..21
<223> /mol_type="DNA"
/note="synthetic"
/organism="Artificial Sequence"

<400> 256
catggcagag tcattcccac t 21

<210> 257
<211> 22
<212> DNA
<213> Artificial Sequence

<220>
<221> source
<222> 1..22
<223> /mol_type="DNA"
/note="synthetic"
/organism="Artificial Sequence"

<400> 257
caatgctaga acaacgcctg tc 22

<210> 258
<211> 22
<212> DNA
<213> Artificial Sequence

<220>
<221> source
<222> 1..22
<223> /mol_type="DNA"
/note="synthetic"
/organism="Artificial Sequence"

<400> 258
tccctccact gaggacaaag tt 22

<210> 259
<211> 22
<212> DNA
<213> Artificial Sequence

<220>
<221> source
<222> 1..22
<223> /mol_type="DNA"

/note="synthetic"
/organism="Artificial Sequence"

<400> 259
gggagagctt gagaaagttg ga 22

<210> 260
<211> 22
<212> DNA
<213> Artificial Sequence

<220>
<221> source
<222> 1..22
<223> /mol_type="DNA"
/note="synthetic"
/organism="Artificial Sequence"

<400> 260
atttcctcgg atggatgtac ca 22

<210> 261
<211> 22
<212> DNA
<213> Artificial Sequence

<220>
<221> source
<222> 1..22
<223> /mol_type="DNA"
/note="synthetic"
/organism="Artificial Sequence"

<400> 261
tcagagcctg tgtttctacc aa 22

<210> 262
<211> 22
<212> DNA
<213> Artificial Sequence

<220>
<221> source
<222> 1..22
<223> /mol_type="DNA"
/note="synthetic"
/organism="Artificial Sequence"

<400> 262
tggtctcaca ggaccactga tt 22

<210> 263
<211> 24
<212> DNA
<213> Artificial Sequence

<220>
<221> source
<222> 1..24

<223> /mol_type="DNA"
/note="synthetic"
/organism="Artificial Sequence"

<400> 263
aaataatcag tgtgattcgt ggag 24

<210> 264
<211> 22
<212> DNA
<213> Artificial Sequence

<220>
<221> source
<222> 1..22
<223> /mol_type="DNA"
/note="synthetic"
/organism="Artificial Sequence"

<400> 264
gaggccagtg ctgtctctaa gg 22

<210> 265
<211> 21
<212> DNA
<213> Artificial Sequence

<220>
<221> source
<222> 1..21
<223> /mol_type="DNA"
/note="synthetic"
/organism="Artificial Sequence"

<400> 265
acttcacagc cctgcgtaaa c 21

<210> 266
<211> 21
<212> DNA
<213> Artificial Sequence

<220>
<221> source
<222> 1..21
<223> /mol_type="DNA"
/note="synthetic"
/organism="Artificial Sequence"

<400> 266
atgggacagg cactgatttg t 21

<210> 267
<211> 22
<212> DNA
<213> Artificial Sequence

<220>
<221> source

<222> 1..22
<223> /mol_type="DNA"
/note="synthetic"
/organism="Artificial Sequence"

<400> 267
gcagcgggtt acatcttctt tc 22

<210> 268
<211> 22
<212> DNA
<213> Artificial Sequence

<220>
<221> source
<222> 1..22
<223> /mol_type="DNA"
/note="synthetic"
/organism="Artificial Sequence"

<400> 268
cagctctggc tcacactacc ag 22

<210> 269
<211> 22
<212> DNA
<213> Artificial Sequence

<220>
<221> source
<222> 1..22
<223> /mol_type="DNA"
/note="synthetic"
/organism="Artificial Sequence"

<400> 269
cctgaactcc gtcagactga aa 22

<210> 270
<211> 20
<212> DNA
<213> Artificial Sequence

<220>
<221> source
<222> 1..20
<223> /mol_type="DNA"
/note="synthetic"
/organism="Artificial Sequence"

<400> 270
gcagctggac tcgatttcct 20

<210> 271
<211> 23
<212> DNA
<213> Artificial Sequence

<220>

<221> source
<222> 1..23
<223> /mol_type="DNA"
/note="synthetic"
/organism="Artificial Sequence"

<400> 271
ccttacagca atcctgtgaa aca 23

<210> 272
<211> 22
<212> DNA
<213> Artificial Sequence

<220>
<221> source
<222> 1..22
<223> /mol_type="DNA"
/note="synthetic"
/organism="Artificial Sequence"

<400> 272
tgcccaatga gtcaagaagt gt 22

<210> 273
<211> 22
<212> DNA
<213> Artificial Sequence

<220>
<221> source
<222> 1..22
<223> /mol_type="DNA"
/note="synthetic"
/organism="Artificial Sequence"

<400> 273
atgtacagtg ctggcatggt ct 22

<210> 274
<211> 22
<212> DNA
<213> Artificial Sequence

<220>
<221> source
<222> 1..22
<223> /mol_type="DNA"
/note="synthetic"
/organism="Artificial Sequence"

<400> 274
cactcacgga tgctgcttag tt 22

<210> 275
<211> 21
<212> DNA
<213> Artificial Sequence

<220>
<221> source
<222> 1..21
<223> /mol_type="DNA"
/note="synthetic"
/organism="Artificial Sequence"

<400> 275
taaggcaccc acatcatgtc a 21

<210> 276
<211> 24
<212> DNA
<213> Artificial Sequence

<220>
<221> source
<222> 1..24
<223> /mol_type="DNA"
/note="synthetic"
/organism="Artificial Sequence"

<400> 276
tggacctaaa aggcttacaa tcaa 24

<210> 277
<211> 24
<212> DNA
<213> Artificial Sequence

<220>
<221> source
<222> 1..24
<223> /mol_type="DNA"
/note="synthetic"
/organism="Artificial Sequence"

<400> 277
gccttttagg tccactatgg aatg 24

<210> 278
<211> 22
<212> DNA
<213> Artificial Sequence

<220>
<221> source
<222> 1..22
<223> /mol_type="DNA"
/note="synthetic"
/organism="Artificial Sequence"

<400> 278
ccaggcgatg ctactactgg tc 22

<210> 279
<211> 22
<212> DNA
<213> Artificial Sequence

<220>
<221> source
<222> 1..22
<223> /mol_type="DNA"
/note="synthetic"
/organism="Artificial Sequence"

<400> 279
tcatagcaca cctccctcac tg 22

<210> 280
<211> 22
<212> DNA
<213> Artificial Sequence

<220>
<221> source
<222> 1..22
<223> /mol_type="DNA"
/note="synthetic"
/organism="Artificial Sequence"

<400> 280
acacaacaaa gagcttgtgc ag 22

<210> 281
<211> 24
<212> DNA
<213> Artificial Sequence

<220>
<221> source
<222> 1..24
<223> /mol_type="DNA"
/note="synthetic"
/organism="Artificial Sequence"

<400> 281
ccattacttt gagaaggaca ggaa 24

<210> 282
<211> 22
<212> DNA
<213> Artificial Sequence

<220>
<221> source
<222> 1..22
<223> /mol_type="DNA"
/note="synthetic"
/organism="Artificial Sequence"

<400> 282
tattcttgct ggatgcgttt ct 22

<210> 283
<211> 21
<212> DNA

<213> Artificial Sequence

<220>
<221> source
<222> 1..21
<223> /mol_type="DNA"
/note="synthetic"
/organism="Artificial Sequence"

<400> 283
aggagggcag aggactagct g 21

<210> 284
<211> 20
<212> DNA
<213> Artificial Sequence

<220>
<221> source
<222> 1..20
<223> /mol_type="DNA"
/note="synthetic"
/organism="Artificial Sequence"

<400> 284
ggcaatgtga atgtgcactg 20

<210> 285
<211> 20
<212> DNA
<213> Artificial Sequence

<220>
<221> source
<222> 1..20
<223> /mol_type="DNA"
/note="synthetic"
/organism="Artificial Sequence"

<400> 285
cttgaacctg ggaggtggag 20

<210> 286
<211> 22
<212> DNA
<213> Artificial Sequence

<220>
<221> source
<222> 1..22
<223> /mol_type="DNA"
/note="synthetic"
/organism="Artificial Sequence"

<400> 286
atcagggtgg gaggagtaaa ga 22

<210> 287
<211> 22

<212> DNA
<213> Artificial Sequence

<220>
<221> source
<222> 1..22
<223> /mol_type="DNA"
/note="synthetic"
/organism="Artificial Sequence"

<400> 287
cccacttacc tctcacctgt gc 22

<210> 288
<211> 22
<212> DNA
<213> Artificial Sequence

<220>
<221> source
<222> 1..22
<223> /mol_type="DNA"
/note="synthetic"
/organism="Artificial Sequence"

<400> 288
gtgaacttcc ggtaggaaat gg 22

<210> 289
<211> 20
<212> DNA
<213> Artificial Sequence

<220>
<221> source
<222> 1..20
<223> /mol_type="DNA"
/note="synthetic"
/organism="Artificial Sequence"

<400> 289
aggggacctc aagggagaag 20

<210> 290
<211> 22
<212> DNA
<213> Artificial Sequence

<220>
<221> source
<222> 1..22
<223> /mol_type="DNA"
/note="synthetic"
/organism="Artificial Sequence"

<400> 290
agatcatgcc agtgaactcc ag 22

<210> 291

<211> 22
<212> DNA
<213> Artificial Sequence

<220>
<221> source
<222> 1..22
<223> /mol_type="DNA"
/note="synthetic"
/organism="Artificial Sequence"

<400> 291
ggaccaggaa agtccttgct tt 22

<210> 292
<211> 22
<212> DNA
<213> Artificial Sequence

<220>
<221> source
<222> 1..22
<223> /mol_type="DNA"
/note="synthetic"
/organism="Artificial Sequence"

<400> 292
ggtggggaac attaaactga gg 22

<210> 293
<211> 22
<212> DNA
<213> Artificial Sequence

<220>
<221> source
<222> 1..22
<223> /mol_type="DNA"
/note="synthetic"
/organism="Artificial Sequence"

<400> 293
gcttcaggtt gttttgttgc ag 22

<210> 294
<211> 22
<212> DNA
<213> Artificial Sequence

<220>
<221> source
<222> 1..22
<223> /mol_type="DNA"
/note="synthetic"
/organism="Artificial Sequence"

<400> 294
acccttgctt gagggaaata tg 22

<210> 295
<211> 22
<212> DNA
<213> Artificial Sequence

<220>
<221> source
<222> 1..22
<223> /mol_type="DNA"
/note="synthetic"
/organism="Artificial Sequence"

<400> 295
cccagctcct agggtacagt ct 22

<210> 296
<211> 23
<212> DNA
<213> Artificial Sequence

<220>
<221> source
<222> 1..23
<223> /mol_type="DNA"
/note="synthetic"
/organism="Artificial Sequence"

<400> 296
cagtcagctt caaaatccct ctt 23

<210> 297
<211> 24
<212> DNA
<213> Artificial Sequence

<220>
<221> source
<222> 1..24
<223> /mol_type="DNA"
/note="synthetic"
/organism="Artificial Sequence"

<400> 297
tcacttccct gtgagtaaag aaaa 24

<210> 298
<211> 23
<212> DNA
<213> Artificial Sequence

<220>
<221> source
<222> 1..23
<223> /mol_type="DNA"
/note="synthetic"
/organism="Artificial Sequence"

<400> 298
ggccatttaa ttcttgtcct tga 23

<210> 299
<211> 23
<212> DNA
<213> Artificial Sequence

<220>
<221> source
<222> 1..23
<223> /mol_type="DNA"
/note="synthetic"
/organism="Artificial Sequence"

<400> 299
tggacttgtg caaactcaaa ctg 23

<210> 300
<211> 23
<212> DNA
<213> Artificial Sequence

<220>
<221> source
<222> 1..23
<223> /mol_type="DNA"
/note="synthetic"
/organism="Artificial Sequence"

<400> 300
tcccaatata gggcagtcat gtt 23

<210> 301
<211> 23
<212> DNA
<213> Artificial Sequence

<220>
<221> source
<222> 1..23
<223> /mol_type="DNA"
/note="synthetic"
/organism="Artificial Sequence"

<400> 301
tctcaatcag ttgagttgcc ttg 23

<210> 302
<211> 22
<212> DNA
<213> Artificial Sequence

<220>
<221> source
<222> 1..22
<223> /mol_type="DNA"
/note="synthetic"
/organism="Artificial Sequence"

<400> 302
agctgtgcaa gtgtggaaac at 22

<210> 303
<211> 22
<212> DNA
<213> Artificial Sequence

<220>
<221> source
<222> 1..22
<223> /mol_type="DNA"
/note="synthetic"
/organism="Artificial Sequence"

<400> 303
gctgtgaggg taaatgagac ca 22

<210> 304
<211> 22
<212> DNA
<213> Artificial Sequence

<220>
<221> source
<222> 1..22
<223> /mol_type="DNA"
/note="synthetic"
/organism="Artificial Sequence"

<400> 304
gtctcctggt gagtgactgt gg 22

<210> 305
<211> 20
<212> DNA
<213> Artificial Sequence

<220>
<221> source
<222> 1..20
<223> /mol_type="DNA"
/note="synthetic"
/organism="Artificial Sequence"

<400> 305
ccttccttcg tctccacagc 20

<210> 306
<211> 21
<212> DNA
<213> Artificial Sequence

<220>
<221> source
<222> 1..21
<223> /mol_type="DNA"
/note="synthetic"
/organism="Artificial Sequence"

<400> 306

gtccttgtgc caacagtcga g 21

<210> 307
<211> 22
<212> DNA
<213> Artificial Sequence

<220>
<221> source
<222> 1..22
<223> /mol_type="DNA"
/note="synthetic"
/organism="Artificial Sequence"

<400> 307
gcttggcaag gagaagagaa ca 22

<210> 308
<211> 23
<212> DNA
<213> Artificial Sequence

<220>
<221> source
<222> 1..23
<223> /mol_type="DNA"
/note="synthetic"
/organism="Artificial Sequence"

<400> 308
gcttgctttc ttgcttgaac aac 23

<210> 309
<211> 22
<212> DNA
<213> Artificial Sequence

<220>
<221> source
<222> 1..22
<223> /mol_type="DNA"
/note="synthetic"
/organism="Artificial Sequence"

<400> 309
gctggtcacc ttgagcttct ct 22

<210> 310
<211> 21
<212> DNA
<213> Artificial Sequence

<220>
<221> source
<222> 1..21
<223> /mol_type="DNA"
/note="synthetic"
/organism="Artificial Sequence"

<400> 310
ccatgctggg ctctttgatt a 21

<210> 311
<211> 22
<212> DNA
<213> Artificial Sequence

<220>
<221> source
<222> 1..22
<223> /mol_type="DNA"
/note="synthetic"
/organism="Artificial Sequence"

<400> 311
caccactctg aagttggcct ct 22

<210> 312
<211> 21
<212> DNA
<213> Artificial Sequence

<220>
<221> source
<222> 1..21
<223> /mol_type="DNA"
/note="synthetic"
/organism="Artificial Sequence"

<400> 312
atggctctgc acatttgttc c 21

<210> 313
<211> 22
<212> DNA
<213> Artificial Sequence

<220>
<221> source
<222> 1..22
<223> /mol_type="DNA"
/note="synthetic"
/organism="Artificial Sequence"

<400> 313
cagagtggga aaaggcactt ca 22

<210> 314
<211> 22
<212> DNA
<213> Artificial Sequence

<220>
<221> source
<222> 1..22
<223> /mol_type="DNA"
/note="synthetic"
/organism="Artificial Sequence"

<400> 314
ccagagtcct gtgcagacat tc 22

<210> 315
<211> 22
<212> DNA
<213> Artificial Sequence

<220>
<221> source
<222> 1..22
<223> /mol_type="DNA"
/note="synthetic"
/organism="Artificial Sequence"

<400> 315
atggggatta actgggatgt tg 22

<210> 316
<211> 23
<212> DNA
<213> Artificial Sequence

<220>
<221> source
<222> 1..23
<223> /mol_type="DNA"
/note="synthetic"
/organism="Artificial Sequence"

<400> 316
cgtagctcca gacatcacta gca 23

<210> 317
<211> 22
<212> DNA
<213> Artificial Sequence

<220>
<221> source
<222> 1..22
<223> /mol_type="DNA"
/note="synthetic"
/organism="Artificial Sequence"

<400> 317
gcaacctggt ctgcaaagtc tc 22

<210> 318
<211> 20
<212> DNA
<213> Artificial Sequence

<220>
<221> source
<222> 1..20
<223> /mol_type="DNA"
/note="synthetic"

/organism="Artificial Sequence"

<400> 318
acccagcagt ccagcatgag 20

<210> 319
<400> 319
000

<210> 320
<400> 320
000

<210> 321
<400> 321
000

<210> 322
<400> 322
000

<210> 323
<400> 323
000

<210> 324
<400> 324
000

<210> 325
<400> 325
000

<210> 326
<400> 326
000

<210> 327
<400> 327
000

<210> 328
<400> 328
000

<210> 329
<400> 329
000

<210> 330
<400> 330
000

<210> 331
<400> 331
000

<210> 332
<400> 332
000

<210> 333

<400> 333
000

<210> 334
<400> 334
000

<210> 335
<400> 335
000

<210> 336
<400> 336
000

<210> 337
<400> 337
000

<210> 338
<400> 338
000

<210> 339
<400> 339
000

<210> 340
<400> 340
000

<210> 341
<400> 341
000

<210> 342
<400> 342
000

<210> 343
<400> 343
000

<210> 344
<400> 344
000

<210> 345
<400> 345
000

<210> 346
<400> 346
000

<210> 347
<400> 347
000

<210> 348
<400> 348
000

<210> 349
<400> 349
000

<210> 350
<400> 350
000

<210> 351
<400> 351
000

<210> 352
<400> 352
000

<210> 353
<400> 353
000

<210> 354
<400> 354
000

<210> 355
<400> 355
000

<210> 356
<400> 356
000

<210> 357
<400> 357
000

<210> 358
<400> 358
000

<210> 359
<400> 359
000

<210> 360
<400> 360
000

<210> 361
<400> 361
000

<210> 362
<400> 362
000

<210> 363
<400> 363
000

<210> 364
<400> 364
000

<210> 365
<400> 365
000

<210> 366
<400> 366
000

<210> 367
<400> 367
000

<210> 368
<400> 368
000

<210> 369
<400> 369
000

<210> 370
<400> 370
000

<210> 371
<400> 371
000

<210> 372
<400> 372
000

<210> 373
<400> 373
000

<210> 374
<400> 374
000

<210> 375
<400> 375
000

<210> 376
<400> 376
000

<210> 377
<400> 377
000

<210> 378
<400> 378
000

<210> 379
<400> 379
000

<210> 380
<400> 380

000

<210> 381
<400> 381
000

<210> 382
<400> 382
000

<210> 383
<400> 383
000

<210> 384
<400> 384
000

<210> 385
<400> 385
000

<210> 386
<400> 386
000

<210> 387
<400> 387
000

<210> 388
<400> 388
000

<210> 389
<400> 389
000

<210> 390
<400> 390
000

<210> 391
<400> 391
000

<210> 392
<400> 392
000

<210> 393
<400> 393
000

<210> 394
<400> 394
000

<210> 395
<400> 395
000

<210> 396

<400> 396
000

<210> 397
<400> 397
000

<210> 398
<400> 398
000

<210> 399
<400> 399
000

<210> 400
<400> 400
000

<210> 401
<400> 401
000

<210> 402
<400> 402
000

<210> 403
<400> 403
000

<210> 404
<400> 404
000

<210> 405
<400> 405
000

<210> 406
<400> 406
000

<210> 407
<400> 407
000

<210> 408
<400> 408
000

<210> 409
<400> 409
000

<210> 410
<400> 410
000

<210> 411
<400> 411
000

<210> 412
<400> 412
000

<210> 413
<400> 413
000

<210> 414
<400> 414
000

<210> 415
<400> 415
000

<210> 416
<400> 416
000

<210> 417
<400> 417
000

<210> 418
<400> 418
000

<210> 419
<400> 419
000

<210> 420
<400> 420
000

<210> 421
<400> 421
000

<210> 422
<400> 422
000

<210> 423
<400> 423
000

<210> 424
<400> 424
000

<210> 425
<211> 3878
<212> DNA
<213> Artificial Sequence

<220>
<221> source
<222> 1..3878
<223> /mol_type="DNA"
/note="Synthetic"
/organism="Artificial Sequence"

<400> 425
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380

actccttcac acatactcct cctctggatc cacaggaact ggatattctg aaaaccgtaa 1440

aggaaatcac agggtttttg ctgattcagg cttggcctga aaacaggacg gacctccatg 1500

cctttgagaa cctagaaatc atacgcggca ggaccaagca acatggtcag ttttctcttg 1560

cagtcgtcag cctgaacata acatccttgg gattacgctc cctcaaggag ataagtgatg 1620

gagatgtgat aatttcagga aacaaaaatt tgtgctatgc aaatacaata aactggaaaa 1680

aactgtttgg gacctccggt cagaaaacca aaattataag caacagaggt gaaaacagct 1740

gcaaggccac aggccaggtc tgccatgcct tgtgctcccc cgagggctgc tggggcccgg 1800

agcccaggga ctgcgtctct tgccggaatg tcagccgagg cagggaatgc gtggacaagt 1860

gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctga 2400
gctccggtgc gttcggcacg gtgtataagg gactctggat cccagaaggt gagaaagtta 2460
aaattcccgt cgctatcaag gaattaagag aagcaacatc tccgaaagcc aacaaggaaa 2520
tcctcgatga agcctagctg atggccagcg tggacaaccc ccacgtgtgc cgcctgctgg 2580
gcatctgcct cacctccacc gtgcagctca tcacgcagct catgcccttc ggctgcctcc 2640
tggactatgt ccgggaacac aaagacaata ttggctccca gtacctgctc aactggtgtg 2700
tgcagatcgc aaagggcatg aactacttgg aggaccgtcg cttggtgcac cgcgacctgg 2760
cagccaggaa cgtactggtg aaaacaccgc agcatgtcaa gatcacagat tttgggctgg 2820
ccaaactgct gggtgcggaa gagaaagaat accatgcaga aggaggcaaa gtgcctatca 2880
agtggatggc attggaatca attttacaca gaatctatac ccaccagagt gatgtctgga 2940
gctacggggt gactgtttgg gagttgatga cctttggatc caagccatat gacggaatcc 3000
ctgccagcga gatctcctcc atcctggaga aaggagaacg cctccctcag ccacccatat 3060
gtaccatcga tgtctacatg atcatggtca agtgctggat gatagacgca gatagtcgcc 3120
caaagttccg tgagttgatc atcgaattct ccaaaatggc ccgagacccc cagcgctacc 3180
ttgtcattca gggggatgaa agaatgcatt tgccaagtcc tacagactcc aacttctacc 3240
gtgccctgat ggatgaagaa gacatggacg acgtggtgga tgccgacgag tacctcatcc 3300
cacagcaggg cttcttcagc agcccctcca cgtcacggac tcccctcctg agctctctga 3360
gtgcaaccag caacaattcc accgtggctt gcattgatag aaatgggctg caaagctgtc 3420
ccatcaagga agacagcttc ttgcagcgat acagctcaga ccccacaggc gccttgactg 3480
aggacagcat agacgacacc ttcctcccag tgcctgaata cataaaccag tccgttccca 3540
aaaggcccgc tggctctgtg cagaatcctg tctatcacaa tcagcctctg aaccccgcgc 3600
ccagcagaga cccacactac caggaccccc acagcactgc agtgggcaac cccgagtatc 3660
tcaacactgt ccagcccacc tgtgtcaaca gcacattcga cagccctgcc cactgggccc 3720

agaaaggcag ccaccaaatt agcctggaca accctgacta ccagcaggac ttctttccca 3780

aggaagccaa gccaaatggc atctttaagg gctccacagc tgaaaatgca gaatacctaa 3840

gggtcgcgcc acaaagcagt gaatttattg gagcatga 3878

<210> 426
<211> 3878
<212> DNA
<213> Artificial Sequence

<220>
<221> source
<222> 1..3878
<223> /mol_type="DNA"
/note="Synthetic"
/organism="Artificial Sequence"

<400> 426
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactcccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

```
tcaaaaactg cacctccatc agtggcgatc tccacatcct gccggtggca tttaggggtg     1380
actccttcac acatactcct cctctggatc cacaggaact ggatattctg aaaaccgtaa     1440
aggaaatcac agggtttttg ctgattcagg cttggcctga aaacaggacg gacctccatg     1500
cctttgagaa cctagaaatc atacgcggca ggaccaagca acatggtcag ttttctcttg     1560
cagtcgtcag cctgaacata acatccttgg gattacgctc cctcaaggag ataagtgatg     1620
gagatgtgat aatttcagga aacaaaaatt tgtgctatgc aaatacaata aactggaaaa     1680
aactgtttgg gacctccggt cagaaaacca aaattataag caacagaggt gaaaacagct     1740
gcaaggccac aggccaggtc tgccatgcct tgtgctcccc cgagggctgc tggggcccgg     1800
agcccaggga ctgcgtctct tgccggaatg tcagccgagg cagggaatgc gtggacaagt     1860
gcaaccttct ggagggtgag ccaagggagt ttgtggagaa ctctgagtgc atacagtgcc     1920
acccagagtg cctgcctcag gccatgaaca tcacctgcac aggacgggga ccagacaact     1980
gtatccagtg tgcccactac attgacggcc cccactgcgt caagacctgc ccggcaggag     2040
tcatgggaga aaacaacacc ctggtctgga agtacgcaga cgccggccat gtgtgccacc     2100
tgtgccatcc aaactgcacc tacggatgca ctgggccagg tcttgaaggc tgtccaacga     2160
atgggcctaa gatcccgtcc atcgccactg ggatggtggg ggccctcctc ttgctgctgg     2220
tggtggccct ggggatcggc ctcttcatgc gaaggcacca catcgttcgg aagcgcacgc     2280
tgcggaggct gctgcaggag agggagcttg tggagcctct tacacccagt ggagaagctc     2340
ccaaccaagc tctcttgagg atcttgaagg aaactgaatt caaaaagatc aaagtgctga     2400
gctccggtgc gttcggcacg gtgtataagg gactctggat cccagaaggt gagaaagtta     2460
aaattcccgt cgctatcaag gaattaagag aagcaacatc tccgaaagcc aacaaggaaa     2520
tcctcgatga agcctagctg atggccagcg tggacaaccc ccacgtgtgc cgcctgctgg     2580
gcatctgcct cacctccacc gtgcagctca tcacgcagct catgcccttc ggctgcctcc     2640
tggactatgt ccgggaacac aaagacaata ttggctccca gtacctgctc aactggtgtg     2700
tgcagatcgc aaagggcatg aactacttgg aggaccgtcg cttggtgcac cgcgacctgg     2760
cagccaggaa cgtactggtg aaaacaccgc agcatgtcaa gatcacagat tttgggctgg     2820
ccaaactgct gggtgcggaa gagaaagaat accatgcaga aggaggcaaa gtgcctatca     2880
agtggatggc attggaatca attttacaca gaatctatac ccaccagagt gatgtctgga     2940
gctacggggt gactgtttgg gagttgatga cctttggatc caagccatat gacggaatcc     3000
ctgccagcga gatctcctcc atcctggaga aaggagaacg cctccctcag ccacccatat     3060
gtaccatcga tgtctacatg atcatggtca agtgctggat gatagacgca gatagtcgcc     3120
caaagttccg tgagttgatc atcgaattct ccaaaatggc ccgagacccc cagcgctacc     3180
```

ttgtcattca gggggatgaa agaatgcatt tgccaagtcc tacagactcc aacttctacc 3240

gtgccctgat ggatgaagaa gacatggacg acgtggtgga tgccgacgag tacctcatcc 3300

cacagcaggg cttcttcagc agcccctcca cgtcacggac tcccctcctg agctctctga 3360

gtgcaaccag caacaattcc accgtggctt gcattgatag aaatgggctg caaagctgtc 3420

ccatcaagga agacagcttc ttgcagcgat acagctcaga ccccacaggc gccttgactg 3480

aggacagcat agacgacacc ttcctcccag tgcctgaata cataaaccag tccgttccca 3540

aaaggcccgc tggctctgtg cagaatcctg tctatcacaa tcagcctctg aaccccgcgc 3600

ccagcagaga cccacactac caggaccccc acagcactgc agtgggcaac cccgagtatc 3660

tcaacactgt ccagcccacc tgtgtcaaca gcacattcga cagccctgcc cactgggccc 3720

agaaaggcag ccaccaaatt agcctggaca accctgacta ccagcaggac ttctttccca 3780

aggaagccaa gccaaatggc atctttaagg gctccacagc tgaaaatgca gaatacctaa 3840

gggtcgcgcc acaaagcagt gaatttattg gagcatga 3878

<210> 427
<211> 3863
<212> DNA
<213> Artificial Sequence

<220>
<221> source
<222> 1..3863
<223> /mol_type="DNA"
/note="synthetic"
/organism="Artificial Sequence"

<400> 427
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactccccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaaa acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640

aacacaaaga caatattggc tcccagtacc tgctcaactg gtgtgtgcag atcgcaaagg 2700

gcatgaacta cttggaggac cgtcgcttgg tgcaccgcga cctggcagcc aggaacgtac 2760

tggtgaaaac accgcagcat gtcaagatca cagattttgg gctggccaaa ctgctgggtg 2820

cggaagagaa agaataccat gcagaaggag gcaaagtgcc tatcaagtgg atggcattgg 2880

aatcaatttt acacagaatc tatacccacc agagtgatgt ctggagctac ggggtgactg 2940

tttgggagtt gatgaccttt ggatccaagc catatgacgg aatccctgcc agcgagatct 3000

cctccatcct ggagaaagga gaacgcctcc ctcagccacc catatgtacc atcgatgtct 3060

acatgatcat ggtcaagtgc tggatgatag acgcagatag tcgcccaaag ttccgtgagt 3120

tgatcatcga attctccaaa atggcccgag acccccagcg ctaccttgtc attcaggggg 3180

atgaaagaat gcatttgcca agtcctacag actccaactt ctaccgtgcc ctgatggatg 3240

aagaagacat ggacgacgtg gtggatgccg acgagtacct catcccacag cagggcttct 3300

tcagcagccc ctccacgtca cggactcccc tcctgagctc tctgagtgca accagcaaca 3360

attccaccgt ggcttgcatt gatagaaatg ggctgcaaag ctgtcccatc aaggaagaca 3420

gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 428
<211> 3863
<212> DNA
<213> Artificial Sequence

<220>
<221> source
<222> 1..3863
<223> /mol_type="DNA"
/note="synthetic"
/organism="Artificial Sequence"

<400> 428
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300
gcccggcgag tcgggctctg gaggaaaaga aagtttgcca aggcacgagt aacaagctca 360
cgcagttggg cacttttgaa gatcattttc tcagcctcca gaggatgttc aataactgtg 420
aggtggtcct tgggaatttg gaaattacct atgtgcagag gaattatgat ctttccttct 480
taaagaccat ccaggaggtg gctggttatg tcctcattgc cctcaacaca gtggagcgaa 540
ttcctttgga aaacctgcag atcatcagag gaaatatgta ctacgaaaat tcctatgcct 600
tagcagtctt atctaactat gatgcaaata aaaccggact gaaggagctg cccatgagaa 660
atttacagga aatcctgcat ggcgccgtgc ggttcagcaa caaccctgcc ctgtgcaacg 720
tggagagcat ccagtggcgg gacatagtca gcagtgactt tctcagcaac atgtcgatgg 780
acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactccccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100

tgtgccatcc aaactgcacc tacggatgca ctgggccagg tcttgaaggc tgtccaacga 2160

atgggcctaa gatcccgtcc atcgccactg ggatggtggg ggccctcctc ttgctgctgg 2220

tggtggccct ggggatcggc ctcttcatgc gaaggcacca catcgttcgg aagcgcacgc 2280

tgcggaggct gctgcaggag agggagcttg tggagcctct tacacccagt ggagaagctc 2340

ccaaccaagc tctcttgagg atcttgaagg aaactgaatt caaaaagatc aaagtgctgg 2400

gctccggtgc gttcggcacg gtgtataagg gactctggat cccagaaggt gagaaagtta 2460

aaattcccgt cgctatcaaa acatctccga aagccaacaa ggaaatcctc gatgaagcct 2520

agctgatggc cagcgtggac aacccccacg tgtgccgcct gctgggcatc tgcctcacct 2580

ccaccgtgca gctcatcacg cagctcatgc ccttcggctg cctcctggac tatgtccggg 2640

aacacaaaga caatattggc tcccagtacc tgctcaactg gtgtgtgcag atcgcaaagg 2700

gcatgaacta cttggaggac cgtcgcttgg tgcaccgcga cctggcagcc aggaacgtac 2760

tggtgaaaac accgcagcat gtcaagatca cagattttgg gctggccaaa ctgctgggtg 2820

cggaagagaa agaataccat gcagaaggag gcaaagtgcc tatcaagtgg atggcattgg 2880

aatcaatttt acacagaatc tatacccacc agagtgatgt ctggagctac ggggtgactg 2940

tttgggagtt gatgaccttt ggatccaagc catatgacgg aatccctgcc agcgagatct 3000

cctccatcct ggagaaagga gaacgcctcc ctcagccacc catatgtacc atcgatgtct 3060

acatgatcat ggtcaagtgc tggatgatag acgcagatag tcgcccaaag ttccgtgagt 3120

tgatcatcga attctccaaa atggcccgag acccccagcg ctaccttgtc attcaggggg 3180

atgaaagaat gcatttgcca agtcctacag actccaactt ctaccgtgcc ctgatggatg 3240

aagaagacat ggacgacgtg gtggatgccg acgagtacct catcccacag cagggcttct 3300

tcagcagccc ctccacgtca cggactcccc tcctgagctc tctgagtgca accagcaaca 3360

attccaccgt ggcttgcatt gatagaaatg ggctgcaaag ctgtcccatc aaggaagaca 3420

gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 429
<211> 3863

<212> DNA
<213> Artificial Sequence

<220>
<221> source
<222> 1..3863
<223> /mol_type="DNA"
/note="synthetic"
/organism="Artificial Sequence"

<400> 429
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380

actccttcac acatactcct cctctggatc cacaggaact ggatattctg aaaaccgtaa 1440

aggaaatcac agggtttttg ctgattcagg cttggcctga aaacaggacg gacctccatg 1500

cctttgagaa cctagaaatc atacgcggca ggaccaagca acatggtcag ttttctcttg 1560

cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaaa acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940
tttgggagtt gatgaccttt ggatccaagc catatgacgg aatccctgcc agcgagatct 3000
cctccatcct ggagaaagga gaacgcctcc ctcagccacc catatgtacc atcgatgtct 3060
acatgatcat ggtcaagtgc tggatgatag acgcagatag tcgcccaaag ttccgtgagt 3120
tgatcatcga attctccaaa atggcccgag acccccagcg ctaccttgtc attcaggggg 3180
atgaaagaat gcatttgcca agtcctacag actccaactt ctaccgtgcc ctgatggatg 3240
aagaagacat ggacgacgtg gtggatgccg acgagtacct catcccacag cagggcttct 3300
tcagcagccc ctccacgtca cggactcccc tcctgagctc tctgagtgca accagcaaca 3360
attccaccgt ggcttgcatt gatagaaatg ggctgcaaag ctgtcccatc aaggaagaca 3420
gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 430
<211> 3863
<212> DNA
<213> Artificial Sequence

<220>
<221> source
<222> 1..3863
<223> /mol_type="DNA"
/note="synthetic"
/organism="Artificial Sequence"

<400> 430
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaaa acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940

tttgggagtt gatgaccttt ggatccaagc catatgacgg aatccctgcc agcgagatct 3000

cctccatcct ggagaaagga gaacgcctcc ctcagccacc catatgtacc atcgatgtct 3060

acatgatcat ggtcaagtgc tggatgatag acgcagatag tcgcccaaag ttccgtgagt 3120

tgatcatcga attctccaaa atggcccgag accccccagcg ctaccttgtc attcaggggg 3180

atgaaagaat gcatttgcca agtcctacag actccaactt ctaccgtgcc ctgatggatg 3240

aagaagacat ggacgacgtg gtggatgccg acgagtacct catcccacag cagggcttct 3300

tcagcagccc ctccacgtca cggactcccc tcctgagctc tctgagtgca accagcaaca 3360

attccaccgt ggcttgcatt gatagaaatg ggctgcaaag ctgtcccatc aaggaagaca 3420

gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 431
<211> 3863
<212> DNA
<213> Artificial Sequence

<220>
<221> source
<222> 1..3863
<223> /mol_type="DNA"
/note="synthetic"
/organism="Artificial Sequence"

<400> 431
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540
ttcctttgga aaacctgcag atcatcagag gaaatatgta ctacgaaaat tcctatgcct 600
tagcagtctt atctaactat gatgcaaata aaaccggact gaaggagctg cccatgagaa 660
atttacagga aatcctgcat ggcgccgtgc ggttcagcaa caaccctgcc ctgtgcaacg 720
tggagagcat ccagtggcgg gacatagtca gcagtgactt tctcagcaac atgtcgatgg 780
acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactccccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400

```
gctccggtgc gttcggcacg gtgtataagg gactctggat cccagaaggt gagaaagtta      2460
aaattcccgt cgctatcaaa acatctccga aagccaacaa ggaaatcctc gatgaagcct      2520
agctgatggc cagcgtggac aacccccacg tgtgccgcct gctgggcatc tgcctcacct      2580
ccaccgtgca gctcatcacg cagctcatgc ccttcggctg cctcctggac tatgtccggg      2640
aacacaaaga caatattggc tcccagtacc tgctcaactg gtgtgtgcag atcgcaaagg      2700
gcatgaacta cttggaggac cgtcgcttgg tgcaccgcga cctggcagcc aggaacgtac      2760
tggtgaaaac accgcagcat gtcaagatca cagattttgg gctggccaaa ctgctgggtg      2820
cggaagagaa agaataccat gcagaaggag gcaaagtgcc tatcaagtgg atggcattgg      2880
aatcaatttt acacagaatc tatacccacc agagtgatgt ctggagctac ggggtgactg      2940
tttgggagtt gatgaccttt ggatccaagc catatgacgg aatccctgcc agcgagatct      3000
cctccatcct ggagaaagga gaacgcctcc ctcagccacc catatgtacc atcgatgtct      3060
acatgatcat ggtcaagtgc tggatgatag acgcagatag tcgcccaaag ttccgtgagt      3120
tgatcatcga attctccaaa atggcccgag acccccagcg ctaccttgtc attcaggggg      3180
atgaaagaat gcatttgcca agtcctacag actccaactt ctaccgtgcc ctgatggatg      3240
aagaagacat ggacgacgtg gtggatgccg acgagtacct catcccacag cagggcttct      3300
tcagcagccc ctccacgtca cggactcccc tcctgagctc tctgagtgca accagcaaca      3360
attccaccgt ggcttgcatt gatagaaatg ggctgcaaag ctgtcccatc aaggaagaca      3420
gcttcttgca gcgatacagc tcagacccca caggcgcctt gactgaggac agcatagacg      3480
acaccttcct cccagtgcct gaatacataa accagtccgt tcccaaaagg cccgctggct      3540
ctgtgcagaa tcctgtctat cacaatcagc ctctgaaccc cgcgcccagc agagacccac      3600
actaccagga cccccacagc actgcagtgg gcaaccccga gtatctcaac actgtccagc      3660
ccacctgtgt caacagcaca ttcgacagcc ctgcccactg ggcccagaaa ggcagccacc      3720
aaattagcct ggacaaccct gactaccagc aggacttctt tcccaaggaa gccaagccaa      3780
atggcatctt taagggctcc acagctgaaa atgcagaata cctaagggtc gcgccacaaa      3840
gcagtgaatt tattggagca tga                                             3863
```

<210> 432
<211> 3863
<212> DNA
<213> Artificial Sequence

<220>
<221> source
<222> 1..3863
<223> /mol_type="DNA"
/note="synthetic"
/organism="Artificial Sequence"

<400> 432
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380

actccttcac acatactcct cctctggatc cacaggaact ggatattctg aaaaccgtaa 1440

aggaaatcac agggtttttg ctgattcagg cttggcctga aaacaggacg gacctccatg 1500

cctttgagaa cctagaaatc atacgcggca ggaccaagca acatggtcag ttttctcttg 1560

cagtcgtcag cctgaacata acatccttgg gattacgctc cctcaaggag ataagtgatg 1620

gagatgtgat aatttcagga aacaaaaatt tgtgctatgc aaatacaata aactggaaaa 1680

aactgtttgg gacctccggt cagaaaacca aaattataag caacagaggt gaaaacagct 1740

gcaaggccac aggccaggtc tgccatgcct tgtgctcccc cgagggctgc tggggcccgg 1800

agcccaggga ctgcgtctct tgccggaatg tcagccgagg cagggaatgc gtggacaagt 1860

gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaaa acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940
tttgggagtt gatgaccttt ggatccaagc catatgacgg aatccctgcc agcgagatct 3000
cctccatcct ggagaaagga gaacgcctcc ctcagccacc catatgtacc atcgatgtct 3060
acatgatcat ggtcaagtgc tggatgatag acgcagatag tcgcccaaag ttccgtgagt 3120
tgatcatcga attctccaaa atggcccgag acccccagcg ctaccttgtc attcaggggg 3180
atgaaagaat gcatttgcca agtcctacag actccaactt ctaccgtgcc ctgatggatg 3240
aagaagacat ggacgacgtg gtggatgccg acgagtacct catcccacag cagggcttct 3300
tcagcagccc ctccacgtca cggactcccc tcctgagctc tctgagtgca accagcaaca 3360
attccaccgt ggcttgcatt gatagaaatg ggctgcaaag ctgtcccatc aaggaagaca 3420
gcttcttgca gcgatacagc tcagacccca caggcgcctt gactgaggac agcatagacg 3480
acaccttcct cccagtgcct gaatacataa accagtccgt tcccaaaagg cccgctggct 3540
ctgtgcagaa tcctgtctat cacaatcagc ctctgaaccc cgcgcccagc agagacccac 3600
actaccagga cccccacagc actgcagtgg gcaaccccga gtatctcaac actgtccagc 3660
ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 433
<211> 3863
<212> DNA
<213> Artificial Sequence

<220>
<221> source
<222> 1..3863
<223> /mol_type="DNA"
/note="synthetic"
/organism="Artificial Sequence"

<400> 433
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaaa acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940
tttgggagtt gatgaccttt ggatccaagc catatgacgg aatccctgcc agcgagatct 3000
cctccatcct ggagaaagga gaacgcctcc ctcagccacc catatgtacc atcgatgtct 3060
acatgatcat ggtcaagtgc tggatgatag acgcagatag tcgcccaaag ttccgtgagt 3120
tgatcatcga attctccaaa atggcccgag acccccagcg ctaccttgtc attcaggggg 3180

atgaaagaat gcatttgcca agtcctacag actccaactt ctaccgtgcc ctgatggatg 3240

aagaagacat ggacgacgtg gtggatgccg acgagtacct catcccacag cagggcttct 3300

tcagcagccc ctccacgtca cggactcccc tcctgagctc tctgagtgca accagcaaca 3360

attccaccgt ggcttgcatt gatagaaatg ggctgcaaag ctgtcccatc aaggaagaca 3420

gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 434
<211> 3863
<212> DNA
<213> Artificial Sequence

<220>
<221> source
<222> 1..3863
<223> /mol_type="DNA"
/note="synthetic"
/organism="Artificial Sequence"

<400> 434
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactccccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640

aacacaaaga caatattggc tcccagtacc tgctcaactg gtgtgtgcag atcgcaaagg 2700

gcatgaacta cttggaggac cgtcgcttgg tgcaccgcga cctggcagcc aggaacgtac 2760

tggtgaaaac accgcagcat gtcaagatca cagattttgg gctggccaaa ctgctgggtg 2820

cggaagagaa agaataccat gcagaaggag gcaaagtgcc tatcaagtgg atggcattgg 2880

aatcaatttt acacagaatc tatacccacc agagtgatgt ctggagctac ggggtgactg 2940

tttgggagtt gatgaccttt ggatccaagc catatgacgg aatccctgcc agcgagatct 3000

cctccatcct ggagaaagga gaacgcctcc ctcagccacc catatgtacc atcgatgtct 3060

acatgatcat ggtcaagtgc tggatgatag acgcagatag tcgcccaaag ttccgtgagt 3120

tgatcatcga attctccaaa atggcccgag acccccagcg ctaccttgtc attcaggggg 3180

atgaaagaat gcatttgcca agtcctacag actccaactt ctaccgtgcc ctgatggatg 3240

aagaagacat ggacgacgtg gtggatgccg acgagtacct catcccacag cagggcttct 3300

tcagcagccc ctccacgtca cggactcccc tcctgagctc tctgagtgca accagcaaca 3360

attccaccgt ggcttgcatt gatagaaatg ggctgcaaag ctgtcccatc aaggaagaca 3420

gcttcttgca gcgatacagc tcagacccca caggcgcctt gactgaggac agcatagacg 3480

acaccttcct cccagtgcct gaatacataa accagtccgt tcccaaaagg cccgctggct 3540

ctgtgcagaa tcctgtctat cacaatcagc ctctgaaccc cgcgcccagc agagacccac 3600

actaccagga cccccacagc actgcagtgg gcaaccccga gtatctcaac actgtccagc 3660

ccacctgtgt caacagcaca ttcgacagcc ctgcccactg ggcccagaaa ggcagccacc 3720

aaattagcct ggacaaccct gactaccagc aggacttctt tcccaaggaa gccaagccaa 3780

atggcatctt taagggctcc acagctgaaa atgcagaata cctaagggtc gcgccacaaa 3840

gcagtgaatt tattggagca tga 3863

<210> 435
<211> 3863
<212> DNA
<213> Artificial Sequence

<220>
<221> source
<222> 1..3863
<223> /mol_type="DNA"
/note="synthetic"
/organism="Artificial Sequence"

<400> 435
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

```
cagcgatgcg accctccggg acggccgggg cagcgctcct ggcgctgctg gctgcgctct      300
gcccggcgag tcgggctctg gaggaaaaga aagtttgcca aggcacgagt aacaagctca      360
cgcagttggg cacttttgaa gatcattttc tcagcctcca gaggatgttc aataactgtg      420
aggtggtcct tgggaatttg gaaattacct atgtgcagag gaattatgat ctttccttct      480
taaagaccat ccaggaggtg gctggttatg tcctcattgc cctcaacaca gtggagcgaa      540
ttcctttgga aaacctgcag atcatcagag gaaatatgta ctacgaaaat tcctatgcct      600
tagcagtctt atctaactat gatgcaaata aaaccggact gaaggagctg cccatgagaa      660
atttacagga aatcctgcat ggcgccgtgc ggttcagcaa caaccctgcc ctgtgcaacg      720
tggagagcat ccagtggcgg gacatagtca gcagtgactt tctcagcaac atgtcgatgg      780
acttccagaa ccacctgggc agctgccaaa agtgtgatcc aagctgtccc aatgggagct      840
gctggggtgc aggagaggag aactgccaga aactgaccaa aatcatctgt gcccagcagt      900
gctccgggcg ctgccgtggc aactccccca gtgactgctg ccacaaccag tgtgctgcag      960
gctgcacagg cccccgggag agcgactgcc tggtctgccg caaattccga gacgaagcca     1020
cgtgcaagga cacctgcccc ccactcatga tctacaaccc caccacgtac cagatggatg     1080
tgaaccccga gggcaaatac agctttggtg ccacctgcgt gaagaagtgt ccccgtaatt     1140
atgtggtgac agatcacggc tcgtgcgtcc gagcctgtgg ggccgacagc tatgagatgg     1200
aggaagacgg cgtccgcaag tgtaagaagt gcgaagggcc ttgccgcaaa gtgtgtaacg     1260
gaataggtat tggtgaattt aaagactcac tctccataaa tgctacgaat attaaacact     1320
tcaaaaactg cacctccatc agtggcgatc tccacatcct gccggtggca tttagggggtg    1380
actccttcac acatactcct cctctggatc cacaggaact ggatattctg aaaaccgtaa     1440
aggaaatcac agggtttttg ctgattcagg cttggcctga aaacaggacg gacctccatg     1500
cctttgagaa cctagaaatc atacgcggca ggaccaagca acatggtcag ttttctcttg     1560
cagtcgtcag cctgaacata acatccttgg gattacgctc cctcaaggag ataagtgatg     1620
gagatgtgat aatttcagga aacaaaaatt tgtgctatgc aaatacaata aactggaaaa     1680
aactgtttgg gacctccggt cagaaaacca aaattataag caacagaggt gaaaacagct     1740
gcaaggccac aggccaggtc tgccatgcct tgtgctcccc cgagggctgc tggggcccgg     1800
agcccaggga ctgcgtctct tgccggaatg tcagccgagg cagggaatgc gtggacaagt     1860
gcaaccttct ggagggtgag ccaagggagt ttgtggagaa ctctgagtgc atacagtgcc     1920
acccagagtg cctgcctcag gccatgaaca tcacctgcac aggacgggga ccagacaact     1980
gtatccagtg tgcccactac attgacggcc cccactgcgt caagacctgc ccggcaggag     2040
tcatgggaga aaacaacacc ctggtctgga agtacgcaga cgccggccat gtgtgccacc     2100
```

tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940
tttgggagtt gatgaccttt ggatccaagc catatgacgg aatccctgcc agcgagatct 3000
cctccatcct ggagaaagga gaacgcctcc ctcagccacc catatgtacc atcgatgtct 3060
acatgatcat ggtcaagtgc tggatgatag acgcagatag tcgcccaaag ttccgtgagt 3120
tgatcatcga attctccaaa atggcccgag acccccagcg ctaccttgtc attcaggggg 3180
atgaaagaat gcatttgcca agtcctacag actccaactt ctaccgtgcc ctgatggatg 3240
aagaagacat ggacgacgtg gtggatgccg acgagtacct catcccacag cagggcttct 3300
tcagcagccc ctccacgtca cggactcccc tcctgagctc tctgagtgca accagcaaca 3360
attccaccgt ggcttgcatt gatagaaatg ggctgcaaag ctgtcccatc aaggaagaca 3420
gcttcttgca gcgatacagc tcagacccca caggcgcctt gactgaggac agcatagacg 3480
acaccttcct cccagtgcct gaatacataa accagtccgt tcccaaaagg cccgctggct 3540
ctgtgcagaa tcctgtctat cacaatcagc ctctgaaccc cgcgcccagc agagacccac 3600
actaccagga cccccacagc actgcagtgg gcaaccccga gtatctcaac actgtccagc 3660
ccacctgtgt caacagcaca ttcgacagcc ctgcccactg ggcccagaaa ggcagccacc 3720
aaattagcct ggacaaccct gactaccagc aggacttctt tcccaaggaa gccaagccaa 3780
atggcatctt taagggctcc acagctgaaa atgcagaata cctaagggtc gcgccacaaa 3840
gcagtgaatt tattggagca tga 3863

<210> 436
<211> 3863

<212> DNA
<213> Artificial Sequence

<220>
<221> source
<222> 1..3863
<223> /mol_type="DNA"
/note="synthetic"
/organism="Artificial Sequence"

<400> 436
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380

actccttcac acatactcct cctctggatc cacaggaact ggatattctg aaaaccgtaa 1440

aggaaatcac agggtttttg ctgattcagg cttggcctga aaacaggacg gacctccatg 1500

cctttgagaa cctagaaatc atacgcggca ggaccaagca acatggtcag ttttctcttg 1560

cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag acatctccga aagccaacaa ggaaatcctc gatgaagcct 2520
agctgatggc cagcgtggac aacccccacg tgtgccgcct gctgggcatc tgcctcacct 2580
ccaccgtgca gctcatcacg cagctcatgc ccttcggctg cctcctggac tatgtccggg 2640
aacacaaaga caatattggc tcccagtacc tgctcaactg gtgtgtgcag atcgcaaagg 2700
gcatgaacta cttggaggac cgtcgcttgg tgcaccgcga cctggcagcc aggaacgtac 2760
tggtgaaaac accgcagcat gtcaagatca cagattttgg gctggccaaa ctgctgggtg 2820
cggaagagaa agaataccat gcagaaggag gcaaagtgcc tatcaagtgg atggcattgg 2880
aatcaatttt acacagaatc tatacccacc agagtgatgt ctggagctac ggggtgactg 2940
tttgggagtt gatgaccttt ggatccaagc catatgacgg aatccctgcc agcgagatct 3000
cctccatcct ggagaaagga gaacgcctcc ctcagccacc catatgtacc atcgatgtct 3060
acatgatcat ggtcaagtgc tggatgatag acgcagatag tcgcccaaag ttccgtgagt 3120
tgatcatcga attctccaaa atggcccgag acccccagcg ctaccttgtc attcaggggg 3180
atgaaagaat gcatttgcca agtcctacag actccaactt ctaccgtgcc ctgatggatg 3240
aagaagacat ggacgacgtg gtggatgccg acgagtacct catcccacag cagggcttct 3300
tcagcagccc ctccacgtca cggactcccc tcctgagctc tctgagtgca accagcaaca 3360
attccaccgt ggcttgcatt gatagaaatg ggctgcaaag ctgtcccatc aaggaagaca 3420
gcttcttgca gcgatacagc tcagacccca caggcgcctt gactgaggac agcatagacg 3480

```
acaccttcct cccagtgcct gaatacataa accagtccgt tcccaaaagg cccgctggct     3540

ctgtgcagaa tcctgtctat cacaatcagc ctctgaaccc cgcgcccagc agagacccac     3600

actaccagga cccccacagc actgcagtgg gcaaccccga gtatctcaac actgtccagc     3660

ccacctgtgt caacagcaca ttcgacagcc ctgcccactg ggcccagaaa ggcagccacc     3720

aaattagcct ggacaaccct gactaccagc aggacttctt tcccaaggaa gccaagccaa     3780

atggcatctt taagggctcc acagctgaaa atgcagaata cctaagggtc gcgccacaaa     3840

gcagtgaatt tattggagca tga                                             3863
```

<210> 437
<211> 3854
<212> DNA
<213> Artificial Sequence

<220>
<221> source
<222> 1..3854
<223> /mol_type="DNA"
/note="synthetic"
/organism="Artificial Sequence"

<400> 437

```
ccccgcgcag cgcggccgca gcagcctccg ccccccgcac ggtgtgaccg cccgacgcgc       60

ccgaggcggc cggactcccg agctagcccc ggcggccgcc gccgcccaga ccggacgaca      120

ggccacctcg tcggcgtccg cccgagtccc cgcctcgccg ccaacgccac aaccaccgcg      180

cacggccccc tgactccgtc cagtattgat cgggagagcc ggagcgagct cttcggggag      240

cagcgatgcg accctccggg acggccgggg cagcgctcct ggcgctgctg gctgcgctct      300

gcccggcgag tcgggctctg gaggaaaaga aagtttgcca aggcacgagt aacaagctca      360

cgcagttggg cacttttgaa gatcattttc tcagcctcca gaggatgttc aataactgtg      420

aggtggtcct tgggaatttg gaaattacct atgtgcagag gaattatgat ctttccttct      480

taaagaccat ccaggaggtg gctggttatg tcctcattgc cctcaacaca gtggagcgaa      540

ttcctttgga aaacctgcag atcatcagag gaaatatgta ctacgaaaat tcctatgcct      600

tagcagtctt atctaactat gatgcaaata aaaccggact gaaggagctg cccatgagaa      660

atttacagga aatcctgcat ggcgccgtgc ggttcagcaa caaccctgcc ctgtgcaacg      720

tggagagcat ccagtggcgg gacatagtca gcagtgactt tctcagcaac atgtcgatgg      780

acttccagaa ccacctgggc agctgccaaa agtgtgatcc aagctgtccc aatgggagct      840

gctggggtgc aggagaggag aactgccaga aactgaccaa aatcatctgt gcccagcagt      900

gctccgggcg ctgccgtggc aactccccca gtgactgctg ccacaaccag tgtgctgcag      960

gctgcacagg cccccgggag agcgactgcc tggtctgccg caaattccga gacgaagcca     1020
```

cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag gaattaagag aagcaacact cgatgaagcc tagctgatgg 2520
ccagcgtgga caacccccac gtgtgccgcc tgctgggcat ctgcctcacc tccaccgtgc 2580
agctcatcac gcagctcatg cccttcggct gcctcctgga ctatgtccgg gaacacaaag 2640
acaatattgg ctcccagtac ctgctcaact ggtgtgtgca gatcgcaaag ggcatgaact 2700
acttggagga ccgtcgcttg gtgcaccgcg acctggcagc caggaacgta ctggtgaaaa 2760
caccgcagca tgtcaagatc acagattttg ggctggccaa actgctgggt gcggaagaga 2820
aagaatacca tgcagaagga ggcaaagtgc ctatcaagtg gatggcattg gaatcaattt 2880
tacacagaat ctatacccac cagagtgatg tctggagcta cggggtgact gtttgggagt 2940

tgatgacctt tggatccaag ccatatgacg gaatccctgc cagcgagatc tcctccatcc 3000

tggagaaagg agaacgcctc cctcagccac ccatatgtac catcgatgtc tacatgatca 3060

tggtcaagtg ctggatgata gacgcagata gtcgcccaaa gttccgtgag ttgatcatcg 3120

aattctccaa aatggcccga gacccccagc gctaccttgt cattcagggg gatgaaagaa 3180

tgcatttgcc aagtcctaca gactccaact tctaccgtgc cctgatggat gaagaagaca 3240

tggacgacgt ggtggatgcc gacgagtacc tcatcccaca gcagggcttc ttcagcagcc 3300

cctccacgtc acggactccc ctcctgagct ctctgagtgc aaccagcaac aattccaccg 3360

tggcttgcat tgatagaaat gggctgcaaa gctgtcccat caaggaagac agcttcttgc 3420

agcgatacag ctcagacccc acaggcgcct tgactgagga cagcatagac gacaccttcc 3480

tcccagtgcc tgaatacata aaccagtccg ttcccaaaag gcccgctggc tctgtgcaga 3540

atcctgtcta tcacaatcag cctctgaacc ccgcgcccag cagagaccca cactaccagg 3600

acccccacag cactgcagtg ggcaaccccg agtatctcaa cactgtccag cccacctgtg 3660

tcaacagcac attcgacagc cctgcccact gggcccagaa aggcagccac caaattagcc 3720

tggacaaccc tgactaccag caggacttct ttcccaagga agccaagcca aatggcatct 3780

ttaagggctc cacagctgaa aatgcagaat acctaagggt cgcgccacaa agcagtgaat 3840

ttattggagc atga 3854

<210> 438
<211> 3878
<212> DNA
<213> Artificial Sequence

<220>
<221> source
<222> 1..3878
<223> /mol_type="DNA"
/note="synthetic"
/organism="Artificial Sequence"

<400> 438
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540
ttcctttgga aaacctgcag atcatcagag gaaatatgta ctacgaaaat tcctatgcct 600
tagcagtctt atctaactat gatgcaaata aaaccggact gaaggagctg cccatgagaa 660
atttacagga aatcctgcat ggcgccgtgc ggttcagcaa caaccctgcc ctgtgcaacg 720
tggagagcat ccagtggcgg gacatagtca gcagtgactt tctcagcaac atgtcgatgg 780
acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactccccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400

gctccggtgc gttcggcacg gtgtataagg gactctggat cccagaaggt gagaaagtta 2460
aaattcccgt cgctatcaag gaattaagag aagcaacatc tccgaaagcc aacaaggaaa 2520
tcctcgatga agcctagctg atggccagcg tggacaaccc ccacgtgtgc cgcctgctgg 2580
gcatctgcct cacctccacc gtgcagctca tcacgcagct catgcccttc ggctgcctcc 2640
tggactatgt ccgggaacac aaagacaata ttggctccca gtacctgctc aactggtgtg 2700
tgcagatcgc aaagggcatg aactacttgg aggaccgtcg cttggtgcac cgcgacctgg 2760
cagccaggaa cgtactggtg aaaacaccgc agcatgtcaa gatcacagat tttgggcggg 2820
ccaaactgct gggtgcggaa gagaaagaat accatgcaga aggaggcaaa gtgcctatca 2880
agtggatggc attggaatca attttacaca gaatctatac ccaccagagt gatgtctgga 2940
gctacggggt gactgtttgg gagttgatga cctttggatc caagccatat gacggaatcc 3000
ctgccagcga gatctcctcc atcctggaga aaggagaacg cctccctcag ccacccatat 3060
gtaccatcga tgtctacatg atcatggtca agtgctggat gatagacgca gatagtcgcc 3120
caaagttccg tgagttgatc atcgaattct ccaaaatggc ccgagacccc cagcgctacc 3180
ttgtcattca gggggatgaa agaatgcatt tgccaagtcc tacagactcc aacttctacc 3240
gtgccctgat ggatgaagaa gacatggacg acgtggtgga tgccgacgag tacctcatcc 3300
cacagcaggg cttcttcagc agcccctcca cgtcacggac tcccctcctg agctctctga 3360
gtgcaaccag caacaattcc accgtggctt gcattgatag aaatgggctg caaagctgtc 3420
ccatcaagga agacagcttc ttgcagcgat acagctcaga ccccacaggc gccttgactg 3480
aggacagcat agacgacacc ttcctcccag tgcctgaata cataaaccag tccgttccca 3540
aaaggcccgc tggctctgtg cagaatcctg tctatcacaa tcagcctctg aaccccgcgc 3600
ccagcagaga cccacactac caggaccccc acagcactgc agtgggcaac cccgagtatc 3660
tcaacactgt ccagcccacc tgtgtcaaca gcacattcga cagccctgcc cactgggccc 3720
agaaaggcag ccaccaaatt agcctggaca accctgacta ccagcaggac ttctttccca 3780
aggaagccaa gccaaatggc atctttaagg gctccacagc tgaaaatgca gaatacctaa 3840
gggtcgcgcc acaaagcagt gaatttattg gagcatga 3878

<210> 439
<211> 3878
<212> DNA
<213> Artificial Sequence

<220>
<221> source
<222> 1..3878
<223> /mol_type="DNA"
/note="synthetic"
/organism="Artificial Sequence"

<400> 439
ccccgcgcag cgcggccgca gcagcctccg ccccccgcac ggtgtgaccg cccgacgcgc 60
ccgaggcggc cggactcccg agctagcccc ggcggccgcc gccgcccaga ccggacgaca 120
ggccacctcg tcggcgtccg cccgagtccc cgcctcgccg ccaacgccac aaccaccgcg 180
cacggccccc tgactccgtc cagtattgat cgggagagcc ggagcgagct cttcggggag 240
cagcgatgcg accctccggg acggccgggg cagcgctcct ggcgctgctg gctgcgctct 300
gcccggcgag tcgggctctg gaggaaaaga aagtttgcca aggcacgagt aacaagctca 360
cgcagttggg cacttttgaa gatcattttc tcagcctcca gaggatgttc aataactgtg 420
aggtggtcct tgggaatttg gaaattacct atgtgcagag gaattatgat ctttccttct 480
taaagaccat ccaggaggtg gctggttatg tcctcattgc cctcaacaca gtggagcgaa 540
ttcctttgga aaacctgcag atcatcagag gaaatatgta ctacgaaaat tcctatgcct 600
tagcagtctt atctaactat gatgcaaata aaaccggact gaaggagctg cccatgagaa 660
atttacagga aatcctgcat ggcgccgtgc ggttcagcaa caaccctgcc ctgtgcaacg 720
tggagagcat ccagtggcgg gacatagtca gcagtgactt tctcagcaac atgtcgatgg 780
acttccagaa ccacctgggc agctgccaaa agtgtgatcc aagctgtccc aatgggagct 840
gctggggtgc aggagaggag aactgccaga aactgaccaa aatcatctgt gcccagcagt 900
gctccgggcg ctgccgtggc aactcccca gtgactgctg ccacaaccag tgtgctgcag 960
gctgcacagg cccccgggag agcgactgcc tggtctgccg caaattccga gacgaagcca 1020
cgtgcaagga cacctgcccc ccactcatga tctacaaccc caccacgtac cagatggatg 1080
tgaaccccga gggcaaatac agctttggtg ccacctgcgt gaagaagtgt ccccgtaatt 1140
atgtggtgac agatcacggc tcgtgcgtcc gagcctgtgg ggccgacagc tatgagatgg 1200
aggaagacgg cgtccgcaag tgtaagaagt gcgaagggcc ttgccgcaaa gtgtgtaacg 1260
gaataggtat tggtgaattt aaagactcac tctccataaa tgctacgaat attaaacact 1320
tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860

gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag gaattaagag aagcaacatc tccgaaagcc aacaaggaaa 2520
tcctcgatga agcctacgtg atggccagcg tggacaaccc ccacgtgtgc cgcctgctgg 2580
gcatctgcct cacctccacc gtgcagctca tcacgcagct catgcccttc ggctgcctcc 2640
tggactatgt ccgggaacac aaagacaata ttggctccca gtacctgctc aactggtgtg 2700
tgcagatcgc aaagggcatg aactacttgg aggaccgtcg cttggtgcac cgcgacctgg 2760
cagccaggaa cgtactggtg aaaacaccgc agcatgtcaa gatcacagat tttgggcggg 2820
ccaaactgct gggtgcggaa gagaaagaat accatgcaga aggaggcaaa gtgcctatca 2880
agtggatggc attggaatca attttacaca gaatctatac ccaccagagt gatgtctgga 2940
gctacggggt gactgtttgg gagttgatga cctttggatc caagccatat gacggaatcc 3000
ctgccagcga gatctcctcc atcctggaga aaggagaacg cctccctcag ccacccatat 3060
gtaccatcga tgtctacatg atcatggtca agtgctggat gatagacgca gatagtcgcc 3120
caaagttccg tgagttgatc atcgaattct ccaaaatggc ccgagacccc cagcgctacc 3180
ttgtcattca ggggggatgaa agaatgcatt tgccaagtcc tacagactcc aacttctacc 3240
gtgccctgat ggatgaagaa gacatggacg acgtggtgga tgccgacgag tacctcatcc 3300
cacagcaggg cttcttcagc agcccctcca cgtcacggac tcccctcctg agctctctga 3360
gtgcaaccag caacaattcc accgtggctt gcattgatag aaatgggctg caaagctgtc 3420
ccatcaagga agacagcttc ttgcagcgat acagctcaga ccccacaggc gccttgactg 3480
aggacagcat agacgacacc ttcctcccag tgcctgaata cataaaccag tccgttccca 3540
aaaggcccgc tggctctgtg cagaatcctg tctatcacaa tcagcctctg aaccccgcgc 3600
ccagcagaga cccacactac caggaccccc acagcactgc agtgggcaac cccgagtatc 3660
tcaacactgt ccagcccacc tgtgtcaaca gcacattcga cagccctgcc cactgggccc 3720

agaaaggcag ccaccaaatt agcctggaca accctgacta ccagcaggac ttctttccca 3780

aggaagccaa gccaaatggc atctttaagg gctccacagc tgaaaatgca gaatacctaa 3840

gggtcgcgcc acaaagcagt gaatttattg gagcatga 3878

<210> 440
<211> 3878
<212> DNA
<213> Artificial Sequence

<220>
<221> source
<222> 1..3878
<223> /mol_type="DNA"
/note="synthetic"
/organism="Artificial Sequence"

<400> 440
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380
actccttcac acatactcct cctctggatc cacaggaact ggatattctg aaaaccgtaa 1440
aggaaatcac agggtttttg ctgattcagg cttggcctga aaacaggacg gacctccatg 1500
cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag gaattaagag aagcaacatc tccgaaagcc aacaaggaaa 2520
tcctcgatga agcctagctg atggccagcg tggacaaccc ccacgtgtgc cgcctgctgg 2580
gcatctgcct cacctccacc gtgcagctca tcacgcagct catgcccttc ggctgcctcc 2640
tggactatgt ccgggaacac aaagacaata ttggctccca gtacctgctc aactggtgtg 2700
tgcagatcgc aaagggcatg aactacttgg aggaccgtcg cttggtgcac cgcgacctgg 2760
cagccaggaa cgtactggtg aaaacaccgc agcatgtcaa gatcacagat tttgggcggg 2820
ccaaactgct gggtgcggaa gagaaagaat accatgcaga aggaggcaaa gtgcctatca 2880
agtggatggc attggaatca attttacaca gaatctatac ccaccagagt gatgtctgga 2940
gctacggggt gactgtttgg gagttgatga cctttggatc caagccatat gacggaatcc 3000
ctgccagcga gatctcctcc atcctggaga aaggagaacg cctccctcag ccacccatat 3060
gtaccatcga tgtctacatg atcatggtca agtgctggat gatagacgca gatagtcgcc 3120
caaagttccg tgagttgatc atcgaattct ccaaaatggc ccgagacccc cagcgctacc 3180

ttgtcattca gggggatgaa agaatgcatt tgccaagtcc tacagactcc aacttctacc 3240

gtgccctgat ggatgaagaa gacatggacg acgtggtgga tgccgacgag tacctcatcc 3300

cacagcaggg cttcttcagc agcccctcca cgtcacggac tcccctcctg agctctctga 3360

gtgcaaccag caacaattcc accgtggctt gcattgatag aaatgggctg caaagctgtc 3420

ccatcaagga agacagcttc ttgcagcgat acagctcaga ccccacaggc gccttgactg 3480

aggacagcat agacgacacc ttcctcccag tgcctgaata cataaaccag tccgttccca 3540

aaaggcccgc tggctctgtg cagaatcctg tctatcacaa tcagcctctg aacccgcgc 3600

ccagcagaga cccacactac caggaccccc acagcactgc agtgggcaac cccgagtatc 3660

tcaacactgt ccagcccacc tgtgtcaaca gcacattcga cagccctgcc cactgggccc 3720

agaaaggcag ccaccaaatt agcctggaca accctgacta ccagcaggac ttctttccca 3780

aggaagccaa gccaaatggc atctttaagg gctccacagc tgaaaatgca gaatacctaa 3840

gggtcgcgcc acaaagcagt gaatttattg gagcatga 3878

<210> 441
<400> 441
000

<210> 442
<400> 442
000

<210> 443
<400> 443
000

<210> 444
<400> 444
000

<210> 445
<400> 445
000

<210> 446
<400> 446
000

<210> 447
<211> 36
<212> DNA
<213> Artificial Sequence

<220>
<221> source
<222> 1..36
<223> /mol_type="DNA"
/note="Synthetic"
/organism="Artificial Sequence"

<400> 447
tgtaaaacga cggccagtcg cccagaccgg acgaca 36

<210> 448
<211> 39
<212> DNA
<213> Artificial Sequence

<220>
<221> source
<222> 1..39
<223> /mol_type="DNA"
/note="Synthetic"
/organism="Artificial Sequence"

<400> 448
caggaaacag ctatgaccag ggcaatgagg acataacca 39

<210> 449
<211> 38
<212> DNA
<213> Artificial Sequence

<220>
<221> source
<222> 1..38
<223> /mol_type="DNA"
/note="Synthetic"
/organism="Artificial Sequence"

<400> 449
tgtaaaacga cggccagtgg tggtccttgg gaatttgg 38

<210> 450
<211> 40
<212> DNA
<213> Artificial Sequence

<220>
<221> source
<222> 1..40
<223> /mol_type="DNA"
/note="Synthetic"
/organism="Artificial Sequence"

<400> 450
caggaaacag ctatgacccc atcgacatgt tgctgagaaa 40

<210> 451
<211> 38
<212> DNA
<213> Artificial Sequence

<220>
<221> source
<222> 1..38
<223> /mol_type="DNA"
/note="Synthetic"
/organism="Artificial Sequence"

<400> 451

tgtaaaacga cggccagtga aggagctgcc catgagaa 38

<210> 452
<211> 38
<212> DNA
<213> Artificial Sequence

<220>
<221> source
<222> 1..38
<223> /mol_type="DNA"
/note="Synthetic"
/organism="Artificial Sequence"

<400> 452
caggaaacag ctatgacccg tggcttcgtc tcggaatt 38

<210> 453
<211> 40
<212> DNA
<213> Artificial Sequence

<220>
<221> source
<222> 1..40
<223> /mol_type="DNA"
/note="Synthetic"
/organism="Artificial Sequence"

<400> 453
tgtaaaacga cggccagtga aactgaccaa aatcatctgt 40

<210> 454
<211> 40
<212> DNA
<213> Artificial Sequence

<220>
<221> source
<222> 1..40
<223> /mol_type="DNA"
/note="Synthetic"
/organism="Artificial Sequence"

<400> 454
caggaaacag ctatgaccta cctattccgt tacacacttt 40

<210> 455
<211> 40
<212> DNA
<213> Artificial Sequence

<220>
<221> source
<222> 1..40
<223> /mol_type="DNA"
/note="Synthetic"
/organism="Artificial Sequence"

<400> 455
tgtaaaacga cggccagtcc gtaattatgt ggtgacagat 40

<210> 456
<211> 40
<212> DNA
<213> Artificial Sequence

<220>
<221> source
<222> 1..40
<223> /mol_type="DNA"
/note="Synthetic"
/organism="Artificial Sequence"

<400> 456
caggaaacag ctatgaccgc gtatgatttc taggttctca 40

<210> 457
<211> 41
<212> DNA
<213> Artificial Sequence

<220>
<221> source
<222> 1..41
<223> /mol_type="DNA"
/note="Synthetic"
/organism="Artificial Sequence"

<400> 457
tgtaaaacga cggccagtct gaaaaccgta aaggaaatca c 41

<210> 458
<211> 37
<212> DNA
<213> Artificial Sequence

<220>
<221> source
<222> 1..37
<223> /mol_type="DNA"
/note="Synthetic"
/organism="Artificial Sequence"

<400> 458
caggaaacag ctatgacccc tgcctcggct gacattc 37

<210> 459
<211> 40
<212> DNA
<213> Artificial Sequence

<220>
<221> source
<222> 1..40
<223> /mol_type="DNA"
/note="Synthetic"
/organism="Artificial Sequence"

<400> 459
tgtaaaacga cggccagtta agcaacagag gtgaaaacag 40

<210> 460
<211> 40
<212> DNA
<213> Artificial Sequence

<220>
<221> source
<222> 1..40
<223> /mol_type="DNA"
/note="Synthetic"
/organism="Artificial Sequence"

<400> 460
caggaaacag ctatgaccgg tgttgttttc tcccatgact 40

<210> 461
<211> 38
<212> DNA
<213> Artificial Sequence

<220>
<221> source
<222> 1..38
<223> /mol_type="DNA"
/note="Synthetic"
/organism="Artificial Sequence"

<400> 461
tgtaaaacga cggccagtgg accagacaac tgtatcca 38

<210> 462
<211> 40
<212> DNA
<213> Artificial Sequence

<220>
<221> source
<222> 1..40
<223> /mol_type="DNA"
/note="Synthetic"
/organism="Artificial Sequence"

<400> 462
caggaaacag ctatgacctt ccttcaagat cctcaagaga 40

<210> 463
<211> 38
<212> DNA
<213> Artificial Sequence

<220>
<221> source
<222> 1..38
<223> /mol_type="DNA"
/note="Synthetic"

/organism="Artificial Sequence"

<400> 463
tgtaaaacga cggccagtga tcggcctctt catgcgaa 38

<210> 464
<211> 38
<212> DNA
<213> Artificial Sequence

<220>
<221> source
<222> 1..38
<223> /mol_type="DNA"
/note="Synthetic"
/organism="Artificial Sequence"

<400> 464
caggaaacag ctatgaccac ggtggaggtg aggcagat 38

<210> 465
<211> 39
<212> DNA
<213> Artificial Sequence

<220>
<221> source
<222> 1..39
<223> /mol_type="DNA"
/note="Synthetic"
/organism="Artificial Sequence"

<400> 465
tgtaaaacga cggccagtcg aaagccaaca aggaaatcc 39

<210> 466
<211> 40
<212> DNA
<213> Artificial Sequence

<220>
<221> source
<222> 1..40
<223> /mol_type="DNA"
/note="Synthetic"
/organism="Artificial Sequence"

<400> 466
caggaaacag ctatgaccat tccaatgcca tccacttgat 40

<210> 467
<211> 39
<212> DNA
<213> Artificial Sequence

<220>
<221> source
<222> 1..39
<223> /mol_type="DNA"

/note="Synthetic"
/organism="Artificial Sequence"

<400> 467
tgtaaaacga cggccagtaa caccgcagca tgtcaagat 39

<210> 468
<211> 39
<212> DNA
<213> Artificial Sequence

<220>
<221> source
<222> 1..39
<223> /mol_type="DNA"
/note="Synthetic"
/organism="Artificial Sequence"

<400> 468
caggaaacag ctatgaccct cgggccattt tggagaatt 39

<210> 469
<211> 39
<212> DNA
<213> Artificial Sequence

<220>
<221> source
<222> 1..39
<223> /mol_type="DNA"
/note="Synthetic"
/organism="Artificial Sequence"

<400> 469
tgtaaaacga cggccagttc agccacccat atgtaccat 39

<210> 470
<211> 39
<212> DNA
<213> Artificial Sequence

<220>
<221> source
<222> 1..39
<223> /mol_type="DNA"
/note="Synthetic"
/organism="Artificial Sequence"

<400> 470
caggaaacag ctatgaccgc tttgcagccc atttctatc 39

<210> 471
<211> 38
<212> DNA
<213> Artificial Sequence

<220>
<221> source
<222> 1..38

<223> /mol_type="DNA"
/note="Synthetic"
/organism="Artificial Sequence"

<400> 471
tgtaaaacga cggccagtac agcagggctt cttcagca 38

<210> 472
<211> 38
<212> DNA
<213> Artificial Sequence

<220>
<221> source
<222> 1..38
<223> /mol_type="DNA"
/note="Synthetic"
/organism="Artificial Sequence"

<400> 472
caggaaacag ctatgacctg acacaggtgg gctggaca 38

<210> 473
<211> 40
<212> DNA
<213> Artificial Sequence

<220>
<221> source
<222> 1..40
<223> /mol_type="DNA"
/note="Synthetic"
/organism="Artificial Sequence"

<400> 473
tgtaaaacga cggccagtga atcctgtcta tcacaatcag 40

<210> 474
<211> 40
<212> DNA
<213> Artificial Sequence

<220>
<221> source
<222> 1..40
<223> /mol_type="DNA"
/note="Synthetic"
/organism="Artificial Sequence"

<400> 474
caggaaacag ctatgaccgg tatcgaaaga gtctggattt 40

<210> 475
<211> 38
<212> DNA
<213> Artificial Sequence

<220>
<221> source

<222> 1..38
<223> /mol_type="DNA"
/note="Synthetic"
/organism="Artificial Sequence"

<400> 475
tgtaaaacga cggccagtgc tccacagctg aaaatgca 38

<210> 476
<211> 39
<212> DNA
<213> Artificial Sequence

<220>
<221> source
<222> 1..39
<223> /mol_type="DNA"
/note="Synthetic"
/organism="Artificial Sequence"

<400> 476
caggaaacag ctatgaccac gttgcaaaac cagtctgtg 39

<210> 477
<400> 477
000

<210> 478
<400> 478
000

<210> 479
<400> 479
000

<210> 480
<400> 480
000

<210> 481
<400> 481
000

<210> 482
<400> 482
000

<210> 483
<400> 483
000

<210> 484
<400> 484
000

<210> 485
<400> 485
000

<210> 486
<400> 486

000

<210> 487
<400> 487
000

<210> 488
<400> 488
000

<210> 489
<400> 489
000

<210> 490
<400> 490
000

<210> 491
<400> 491
000

<210> 492
<400> 492
000

<210> 493
<211> 54
<212> DNA
<213> Artificial Sequence

<220>
<221> source
<222> 1..54
<223> /mol_type="DNA"
/note="synthetic"
/organism="Artificial Sequence"

<400> 493
aaaattcccg tcgctatcaa ggaattaaga gaagcaacat ctccgaaagc caac 54

<210> 494
<211> 24
<212> DNA
<213> Artificial Sequence

<220>
<221> source
<222> 1..24
<223> /mol_type="DNA"
/note="synthetic"
/organism="Artificial Sequence"

<400> 494
tttgggctgg ccaaactgct gggt 24

<210> 495
<211> 38
<212> DNA
<213> Artificial Sequence

<220>
<221> source
<222> 1..38
<223> /mol_type="DNA"
/note="synthetic"
/organism="Artificial Sequence"

<400> 495
aaaattcccg tcgctatcaa aacatctccg aaagcaac 38

<210> 496
<211> 24
<212> DNA
<213> Artificial Sequence

<220>
<221> source
<222> 1..24
<223> /mol_type="DNA"
/note="synthetic"
/organism="Artificial Sequence"

<400> 496
tttgggctgg ccaaactgct gggt 24

<210> 497
<211> 42
<212> DNA
<213> Artificial Sequence

<220>
<221> source
<222> 1..42
<223> /mol_type="DNA"
/note="synthetic"
/organism="Artificial Sequence"

<400> 497
aaaattcccg tcgctatcaa ggaatcatct ccgaaagcca ac 42

<210> 498
<211> 24
<212> DNA
<213> Artificial Sequence

<220>
<221> source
<222> 1..24
<223> /mol_type="DNA"
/note="synthetic"
/organism="Artificial Sequence"

<400> 498
tttgggctgg ccaaactgct gggt 24

<210> 499
<211> 36
<212> DNA
<213> Artificial Sequence

<220>
<221> source
<222> 1..36
<223> /mol_type="DNA"
/note="synthetic"
/organism="Artificial Sequence"

<400> 499
aaaattcccg tcgctatcaa ggaatcgaaa gccaac 36

<210> 500
<211> 24
<212> DNA
<213> Artificial Sequence

<220>
<221> source
<222> 1..24
<223> /mol_type="DNA"
/note="synthetic"
/organism="Artificial Sequence"

<400> 500
tttgggctgg ccaaactgct gggt 24

<210> 501
<211> 54
<212> DNA
<213> Artificial Sequence

<220>
<221> source
<222> 1..54
<223> /mol_type="DNA"
/note="synthetic"
/organism="Artificial Sequence"

<400> 501
aaaattcccg tcgctatcaa ggaattaaga gaagcaacat ctccgaaagc caac 54

<210> 502
<211> 24
<212> DNA
<213> Artificial Sequence

<220>
<221> source
<222> 1..24
<223> /mol_type="DNA"
/note="synthetic"
/organism="Artificial Sequence"

<400> 502
tttgggcggg ccaaactgct gggt 24

<210> 503
<211> 54
<212> DNA

<213> Artificial Sequence

<220>
<221> source
<222> 1..54
<223> /mol_type="DNA"
/note="synthetic"
/organism="Artificial Sequence"

<400> 503
aaaattcccg tcgctatcaa ggaattaaga gaagcaacat ctccgaaagc caac 54

<210> 504
<211> 24
<212> DNA
<213> Artificial Sequence

<220>
<221> source
<222> 1..24
<223> /mol_type="DNA"
/note="synthetic"
/organism="Artificial Sequence"

<400> 504
tttgggctgg ccaaacagct gggt 24

<210> 505
<211> 25
<212> DNA
<213> Artificial Sequence

<220>
<221> source
<222> 1..25
<223> /mol_type="DNA"
/note="synthetic"
/organism="Artificial Sequence"

<400> 505
gcaatatcag ccttaggtgc ggctc 25

<210> 506
<211> 26
<212> DNA
<213> Artificial Sequence

<220>
<221> source
<222> 1..26
<223> /mol_type="DNA"
/note="synthetic"
/organism="Artificial Sequence"

<400> 506
catagaaagt gaacatttag gatgtg 26

<210> 507
<211> 24

<212> DNA
<213> Artificial Sequence

<220>
<221> source
<222> 1..24
<223> /mol_type="DNA"
/note="synthetic"
/organism="Artificial Sequence"

<400> 507
ctaacgttcg ccagccataa gtcc 24

<210> 508
<211> 26
<212> DNA
<213> Artificial Sequence

<220>
<221> source
<222> 1..26
<223> /mol_type="DNA"
/note="synthetic"
/organism="Artificial Sequence"

<400> 508
gctgcgagct cacccagaat gtctgg 26

<210> 509
<211> 18
<212> PRT
<213> Artificial Sequence

<220>
<221> SOURCE
<222> 1..18
<223> /mol_type="protein"
/note="synthetic"
/organism="Artificial Sequence"

<400> 509
Lys Ile Pro Val Ala Ile Lys Glu Leu Arg Glu Ala Thr Ser Pro Lys
1 5 10 15
Ala Asn

<210> 510
<211> 8
<212> PRT
<213> Artificial Sequence

<220>
<221> SOURCE
<222> 1..8
<223> /mol_type="protein"
/note="synthetic"
/organism="Artificial Sequence"

<400> 510
Phe Gly Leu Ala Lys Leu Leu Gly
1 5

<210> 511
<211> 3878
<212> DNA
<213> Artificial Sequence

<220>
<221> source
<222> 1..3878
<223> /mol_type="DNA"
/note="Synthetic"
/organism="Artificial Sequence"

<400> 511
ccccgcgcag cgcggccgca gcagcctccg ccccccgcac ggtgtgaccg cccgacgcgc 60

ccgaggcggc cggactcccg agctagcccc ggcggccgcc gccgcccaga ccggacgaca 120

ggccacctcg tcggcgtccg cccgagtccc cgcctcgccg ccaacgccac aaccaccgcg 180

cacggccccc tgactccgtc cagtattgat cgggagagcc ggagcgagct cttcggggag 240

cagcgatgcg accctccggg acggccgggg cagcgctcct ggcgctgctg gctgcgctct 300

gcccggcgag tcgggctctg gaggaaaaga aagtttgcca aggcacgagt aacaagctca 360

cgcagttggg cacttttgaa gatcattttc tcagcctcca gaggatgttc aataactgtg 420

aggtggtcct tgggaatttg gaaattacct atgtgcagag gaattatgat ctttccttct 480

taaagaccat ccaggaggtg gctggttatg tcctcattgc cctcaacaca gtggagcgaa 540

ttcctttgga aaacctgcag atcatcagag gaaatatgta ctacgaaaat tcctatgcct 600

tagcagtctt atctaactat gatgcaaata aaaccggact gaaggagctg cccatgagaa 660

atttacagga aatcctgcat ggcgccgtgc ggttcagcaa caaccctgcc ctgtgcaacg 720

tggagagcat ccagtggcgg gacatagtca gcagtgactt tctcagcaac atgtcgatgg 780

acttccagaa ccacctgggc agctgccaaa agtgtgatcc aagctgtccc aatgggagct 840

gctggggtgc aggagaggag aactgccaga aactgaccaa aatcatctgt gcccagcagt 900

gctccgggcg ctgccgtggc aactccccca gtgactgctg ccacaaccag tgtgctgcag 960

gctgcacagg cccccgggag agcgactgcc tggtctgccg caaattccga gacgaagcca 1020

cgtgcaagga cacctgcccc ccactcatga tctacaaccc caccacgtac cagatggatg 1080

tgaaccccga gggcaaatac agctttggtg ccacctgcgt gaagaagtgt ccccgtaatt 1140

atgtggtgac agatcacggc tcgtgcgtcc gagcctgtgg ggccgacagc tatgagatgg 1200

aggaagacgg cgtccgcaag tgtaagaagt gcgaagggcc ttgccgcaaa gtgtgtaacg 1260

gaataggtat tggtgaattt aaagactcac tctccataaa tgctacgaat attaaacact 1320

tcaaaaactg cacctccatc agtggcgatc tccacatcct gccggtggca tttaggggtg 1380

actccttcac acatactcct cctctggatc cacaggaact ggatattctg aaaaccgtaa 1440

aggaaatcac agggtttttg ctgattcagg cttggcctga aaacaggacg gacctccatg 1500

cctttgagaa cctagaaatc atacgcggca ggaccaagca acatggtcag ttttctcttg 1560
cagtcgtcag cctgaacata acatccttgg gattacgctc cctcaaggag ataagtgatg 1620
gagatgtgat aatttcagga aacaaaaatt tgtgctatgc aaatacaata aactggaaaa 1680
aactgtttgg gacctccggt cagaaaacca aaattataag caacagaggt gaaaacagct 1740
gcaaggccac aggccaggtc tgccatgcct tgtgctcccc cgagggctgc tggggcccgg 1800
agcccaggga ctgcgtctct tgccggaatg tcagccgagg cagggaatgc gtggacaagt 1860
gcaaccttct ggagggtgag ccaagggagt ttgtggagaa ctctgagtgc atacagtgcc 1920
acccagagtg cctgcctcag gccatgaaca tcacctgcac aggacgggga ccagacaact 1980
gtatccagtg tgcccactac attgacggcc cccactgcgt caagacctgc ccggcaggag 2040
tcatgggaga aaacaacacc ctggtctgga agtacgcaga cgccggccat gtgtgccacc 2100
tgtgccatcc aaactgcacc tacggatgca ctgggccagg tcttgaaggc tgtccaacga 2160
atgggcctaa gatcccgtcc atcgccactg ggatggtggg ggccctcctc ttgctgctgg 2220
tggtggccct ggggatcggc ctcttcatgc gaaggcacca catcgttcgg aagcgcacgc 2280
tgcggaggct gctgcaggag agggagcttg tggagcctct tacacccagt ggagaagctc 2340
ccaaccaagc tctcttgagg atcttgaagg aaactgaatt caaaaagatc aaagtgctgg 2400
gctccggtgc gttcggcacg gtgtataagg gactctggat cccagaaggt gagaaagtta 2460
aaattcccgt cgctatcaag gaattaagag aagcaacatc tccgaaagcc aacaaggaaa 2520
tcctcgatga agcctagctg atggccagcg tggacaaccc ccacgtgtgc cgcctgctgg 2580
gcatctgcct cacctccacc gtgcagctca tcacgcagct catgcccttc ggctgcctcc 2640
tggactatgt ccgggaacac aaagacaata ttggctccca gtacctgctc aactggtgtg 2700
tgcagatcgc aaagggcatg aactacttgg aggaccgtcg cttggtgcac cgcgacctgg 2760
cagccaggaa cgtactggtg aaaacaccgc agcatgtcaa gatcacagat tttgggctgg 2820
ccaaactgct gggtgcggaa gagaaagaat accatgcaga aggaggcaaa gtgcctatca 2880
agtggatggc attggaatca attttacaca gaatctatac ccaccagagt gatgtctgga 2940
gctacggggt gactgtttgg gagttgatga cctttggatc caagccatat gacggaatcc 3000
ctgccagcga gatctcctcc atcctggaga aaggagaacg cctccctcag ccacccatat 3060
gtaccatcga tgtctacatg atcatggtca agtgctggat gatagacgca gatagtcgcc 3120
caaagttccg tgagttgatc atcgaattct ccaaaatggc ccgagacccc cagcgctacc 3180
ttgtcattca gggggatgaa agaatgcatt tgccaagtcc tacagactcc aacttctacc 3240
gtgccctgat ggatgaagaa gacatggacg acgtggtgga tgccgacgag tacctcatcc 3300
cacagcaggg cttcttcagc agcccctcca cgtcacggac tcccctcctg agctctctga 3360

gtgcaaccag caacaattcc accgtggctt gcattgatag aaatgggctg caaagctgtc 3420

ccatcaagga agacagcttc ttgcagcgat acagctcaga ccccacaggc gccttgactg 3480

aggacagcat agacgacacc ttcctcccag tgcctgaata cataaaccag tccgttccca 3540

aaaggcccgc tggctctgtg cagaatcctg tctatcacaa tcagcctctg aaccccgcgc 3600

ccagcagaga cccacactac caggaccccc acagcactgc agtgggcaac cccgagtatc 3660

tcaacactgt ccagcccacc tgtgtcaaca gcacattcga cagccctgcc cactgggccc 3720

agaaaggcag ccaccaaatt agcctggaca accctgacta ccagcaggac ttctttccca 3780

aggaagccaa gccaaatggc atctttaagg gctccacagc tgaaaatgca gaatacctaa 3840

gggtcgcgcc acaaagcagt gaatttattg gagcatga 3878

<210> 512
<211> 1210
<212> PRT
<213> Artificial Sequence

<220>
<221> SOURCE
<222> 1..1210
<223> /mol_type="protein"
/note="synthetic"
/organism="Artificial Sequence"

<400> 512
Met Arg Pro Ser Gly Thr Ala Gly Ala Ala Leu Leu Ala Leu Leu Ala
1 5 10 15
Ala Leu Cys Pro Ala Ser Arg Ala Leu Glu Glu Lys Lys Val Cys Gln
20 25 30
Gly Thr Ser Asn Lys Leu Thr Gln Leu Gly Thr Phe Glu Asp His Phe
35 40 45
Leu Ser Leu Gln Arg Met Phe Asn Asn Cys Glu Val Val Leu Gly Asn
50 55 60
Leu Glu Ile Thr Tyr Val Gln Arg Asn Tyr Asp Leu Ser Phe Leu Lys
65 70 75 80
Thr Tyr Gln Glu Val Ala Gly Tyr Val Leu Ile Ala Leu Asn Thr Val
85 90 95
Glu Arg Ile Pro Leu Glu Asn Leu Gln Ile Ile Arg Gly Asn Met Tyr
100 105 110
Tyr Glu Asn Ser Tyr Ala Leu Ala Val Leu Ser Asn Tyr Asp Ala Asn
115 120 125
Lys Thr Gly Leu Lys Glu Leu Pro Met Arg Asn Leu Gln Glu Ile Leu
130 135 140
His Gly Ala Val Arg Phe Ser Asn Asn Pro Ala Leu Cys Asn Val Glu
145 150 155 160
Ser Ile Gln Trp Arg Asp Ile Val Ser Ser Asp Phe Leu Ser Asn Met
165 170 175
Ser Met Asp Phe Gln Asn His Leu Gly Ser Cys Gln Lys Cys Asp Pro
180 185 190
Ser Cys Pro Asn Gly Ser Cys Trp Gly Ala Gly Glu Glu Asn Cys Gln
195 200 205
Lys Leu Thr Lys Ile Ile Cys Ala Gln Gln Cys Ser Gly Arg Cys Arg
210 215 220
Gly Lys Ser Pro Ser Asp Cys Cys His Asn Gln Cys Ala Ala Gly Cys
225 230 235 240
Thr Gly Pro Arg Glu Ser Asp Cys Leu Val Cys Arg Lys Phe Arg Asp

```
                245                 250                 255
Glu Ala Thr Cys Lys Asp Thr Cys Pro Pro Leu Met Leu Tyr Asn Pro
            260                 265                 270
Thr Thr Tyr Gln Met Asp Val Asn Pro Glu Gly Lys Tyr Ser Phe Gly
        275                 280                 285
Ala Thr Cys Val Lys Lys Cys Pro Arg Asn Tyr Val Val Thr Asp His
    290                 295                 300
Gly Ser Cys Val Arg Ala Cys Gly Ala Asp Ser Tyr Glu Met Glu Glu
305                 310                 315                 320
Asp Gly Val Arg Lys Cys Lys Lys Cys Glu Gly Pro Cys Arg Lys Val
                325                 330                 335
Cys Asn Gly Ile Gly Ile Gly Glu Phe Lys Asp Ser Leu Ser Ile Asn
            340                 345                 350
Ala Thr Asn Ile Lys His Phe Lys Asn Cys Thr Ser Ile Ser Gly Asp
        355                 360                 365
Leu His Ile Leu Pro Val Ala Phe Arg Gly Asp Ser Phe Thr His Thr
    370                 375                 380
Pro Pro Leu Asp Pro Gln Glu Leu Asp Ile Leu Lys Thr Val Lys Glu
385                 390                 395                 400
Ile Thr Gly Phe Leu Leu Ile Gln Ala Trp Pro Glu Asn Arg Thr Asp
                405                 410                 415
Leu His Ala Phe Glu Asn Leu Glu Ile Ile Arg Gly Arg Thr Lys Gln
            420                 425                 430
His Gly Gln Phe Ser Leu Ala Val Val Ser Leu Asn Ile Thr Ser Leu
        435                 440                 445
Gly Leu Arg Ser Leu Lys Glu Ile Ser Asp Gly Asp Val Ile Ile Ser
    450                 455                 460
Gly Asn Lys Asn Leu Cys Tyr Ala Asn Thr Ile Asn Trp Lys Lys Leu
465                 470                 475                 480
Phe Gly Thr Ser Gly Gln Lys Thr Lys Ile Ile Ser Asn Arg Gly Glu
                485                 490                 495
Asn Ser Cys Lys Ala Thr Gly Gln Val Cys His Ala Leu Cys Ser Pro
            500                 505                 510
Glu Gly Cys Trp Gly Pro Glu Pro Arg Asp Cys Val Ser Cys Arg Asn
        515                 520                 525
Val Ser Arg Gly Arg Glu Cys Val Asp Lys Cys Asn Leu Leu Glu Gly
    530                 535                 540
Glu Pro Arg Glu Phe Val Glu Asn Ser Glu Cys Ile Gln Cys His Pro
545                 550                 555                 560
Glu Cys Leu Pro Gln Ala Met Asn Ile Thr Cys Thr Gly Arg Gly Pro
                565                 570                 575
Asp Asn Cys Ile Gln Cys Ala His Tyr Ile Asp Gly Pro His Cys Val
            580                 585                 590
Lys Thr Cys Pro Ala Gly Val Met Gly Glu Asn Asn Thr Leu Val Trp
        595                 600                 605
Lys Tyr Ala Asp Ala Gly His Val Cys His Leu Cys His Pro Asn Cys
    610                 615                 620
Thr Tyr Gly Cys Thr Gly Pro Gly Leu Glu Gly Cys Pro Thr Asn Gly
625                 630                 635                 640
Pro Lys Ile Pro Ser Ile Ala Thr Gly Met Val Gly Ala Leu Leu Leu
                645                 650                 655
Leu Leu Val Val Ala Leu Gly Ile Gly Leu Phe Met Arg Arg Arg His
            660                 665                 670
Ile Val Arg Lys Arg Thr Leu Arg Arg Leu Leu Gln Glu Arg Glu Leu
        675                 680                 685
Val Glu Pro Leu Thr Pro Ser Gly Glu Ala Pro Asn Gln Ala Leu Leu
    690                 695                 700
Arg Ile Leu Lys Glu Thr Glu Phe Lys Lys Ile Lys Val Leu Gly Ser
705                 710                 715                 720
Gly Ala Phe Gly Thr Val Tyr Lys Gly Leu Trp Ile Pro Glu Gly Glu
                725                 730                 735
Lys Val Lys Ile Pro Val Ala Ile Lys Glu Leu Arg Glu Ala Thr Ser
            740                 745                 750
```

```
Pro Lys Ala Asn Lys Glu Ile Leu Asp Glu Ala Tyr Val Met Ala Ser
        755                 760                 765
Val Asp Asn Pro His Val Cys Arg Leu Leu Gly Ile Cys Leu Thr Ser
    770                 775                 780
Thr Val Gln Leu Ile Thr Gln Leu Met Pro Phe Gly Cys Leu Leu Asp
785                 790                 795                 800
Tyr Val Arg Glu His Lys Asp Asn Ile Gly Ser Gln Tyr Leu Leu Asn
                805                 810                 815
Trp Cys Val Gln Ile Ala Lys Gly Met Asn Tyr Leu Glu Asp Arg Arg
            820                 825                 830
Leu Val His Arg Asp Leu Ala Ala Arg Asn Val Leu Val Lys Thr Pro
        835                 840                 845
Gln His Val Lys Ile Thr Asp Phe Gly Leu Ala Lys Leu Leu Gly Ala
    850                 855                 860
Glu Glu Lys Glu Tyr His Ala Glu Gly Gly Lys Val Pro Ile Lys Trp
865                 870                 875                 880
Met Ala Leu Glu Ser Ile Leu His Arg Ile Tyr Thr His Gln Ser Asp
                885                 890                 895
Val Trp Ser Tyr Gly Val Thr Val Trp Glu Leu Met Thr Phe Gly Ser
            900                 905                 910
Lys Pro Tyr Asp Gly Ile Pro Ala Ser Glu Ile Ser Ser Ile Leu Glu
        915                 920                 925
Lys Gly Glu Arg Leu Pro Gln Pro Pro Ile Cys Thr Ile Asp Val Tyr
    930                 935                 940
Met Ile Met Val Lys Cys Trp Met Ile Asp Ala Asp Ser Arg Pro Lys
945                 950                 955                 960
Phe Arg Glu Leu Ile Ile Glu Phe Ser Lys Met Ala Arg Asp Pro Gln
                965                 970                 975
Arg Tyr Leu Val Ile Gln Gly Asp Glu Arg Met His Leu Pro Ser Pro
            980                 985                 990
Thr Asp Ser Asn Phe Tyr Arg Ala Leu Met Asp Glu Glu Asp Met Asp
        995                 1000                1005
Asp Val Val Asp Ala Asp Glu Tyr Leu Ile Pro Gln Gln Gly Phe Phe
    1010                1015                1020
Ser Ser Pro Ser Thr Ser Arg Thr Pro Leu Leu Ser Ser Leu Ser Ala
1025                1030                1035                1040
Thr Ser Asn Asn Ser Thr Val Ala Cys Ile Asp Arg Asn Gly Leu Gln
                1045                1050                1055
Ser Cys Pro Ile Lys Glu Asp Ser Phe Leu Gln Arg Tyr Ser Ser Asp
            1060                1065                1070
Pro Thr Gly Ala Leu Thr Glu Asp Ser Ile Asp Asp Thr Phe Leu Pro
        1075                1080                1085
Val Pro Glu Tyr Ile Asn Gln Ser Val Pro Lys Arg Pro Ala Gly Ser
    1090                1095                1100
Val Gln Asn Pro Val Tyr His Asn Gln Pro Leu Asn Pro Ala Pro Ser
1105                1110                1115                1120
Arg Asp Pro His Tyr Gln Asp Pro His Ser Thr Ala Val Gly Asn Pro
                1125                1130                1135
Glu Tyr Leu Asn Thr Val Gln Pro Thr Cys Val Asn Ser Thr Phe Asp
            1140                1145                1150
Ser Pro Ala His Trp Ala Gln Lys Gly Ser His Gln Ile Ser Leu Asp
        1155                1160                1165
Asn Pro Asp Tyr Gln Gln Asp Phe Phe Pro Lys Glu Ala Lys Pro Asn
    1170                1175                1180
Gly Ile Phe Lys Gly Ser Thr Ala Glu Asn Ala Glu Tyr Leu Arg Val
1185                1190                1195                1200
Ala Pro Gln Ser Ser Glu Phe Ile Gly Ala
                1205                1210
```

<210> 513
<211> 25
<212> DNA
<213> Artificial Sequence

<220>
<221> source
<222> 1..25
<223> /mol_type="DNA"
/note="synthetic"
/organism="Artificial Sequence"

<400> 513
cagatttggc tcgacctgga catag 25

<210> 514
<211> 22
<212> DNA
<213> Artificial Sequence

<220>
<221> source
<222> 1..22
<223> /mol_type="DNA"
/note="synthetic"
/organism="Artificial Sequence"

<400> 514
cagctgatct caaggaaaca gg 22

<210> 515
<211> 24
<212> DNA
<213> Artificial Sequence

<220>
<221> source
<222> 1..24
<223> /mol_type="DNA"
/note="synthetic"
/organism="Artificial Sequence"

<400> 515
gtattatcag tcactaaagc tcac 24

<210> 516
<211> 22
<212> DNA
<213> Artificial Sequence

<220>
<221> source
<222> 1..22
<223> /mol_type="DNA"
/note="synthetic"
/organism="Artificial Sequence"

<400> 516
cacacttcaa gtggaattct gc 22

<210> 517
<211> 25
<212> DNA

<213> Artificial Sequence

<220>
<221> source
<222> 1..25
<223> /mol_type="DNA"
/note="synthetic"
/organism="Artificial Sequence"

<400> 517
ctcgtgtgca ttagggttca actgg 25

<210> 518
<211> 26
<212> DNA
<213> Artificial Sequence

<220>
<221> source
<222> 1..26
<223> /mol_type="DNA"
/note="synthetic"
/organism="Artificial Sequence"

<400> 518
ccttctccga ggtggaattg agtgac 26

<210> 519
<211> 26
<212> DNA
<213> Artificial Sequence

<220>
<221> source
<222> 1..26
<223> /mol_type="DNA"
/note="synthetic"
/organism="Artificial Sequence"

<400> 519
gctaattgcg ggactcttgt tcgcac 26

<210> 520
<211> 22
<212> DNA
<213> Artificial Sequence

<220>
<221> source
<222> 1..22
<223> /mol_type="DNA"
/note="synthetic"
/organism="Artificial Sequence"

<400> 520
tacatgcttt tctagtggtc ag 22

<210> 521
<211> 25

<212> DNA
<213> Artificial Sequence

<220>
<221> source
<222> 1..25
<223> /mol_type="DNA"
/note="synthetic"
/organism="Artificial Sequence"

<400> 521
ggtctcaagt gattctacaa accag 25

<210> 522
<211> 24
<212> DNA
<213> Artificial Sequence

<220>
<221> source
<222> 1..24
<223> /mol_type="DNA"
/note="synthetic"
/organism="Artificial Sequence"

<400> 522
ccttcaccta ctggttcaca tctg 24

<210> 523
<211> 26
<212> DNA
<213> Artificial Sequence

<220>
<221> source
<222> 1..26
<223> /mol_type="DNA"
/note="synthetic"
/organism="Artificial Sequence"

<400> 523
catggtttga cttagtttga atgtgg 26

<210> 524
<211> 27
<212> DNA
<213> Artificial Sequence

<220>
<221> source
<222> 1..27
<223> /mol_type="DNA"
/note="synthetic"
/organism="Artificial Sequence"

<400> 524
ggatactaaa gatactttgt caccagg 27

<210> 525

<211> 26
<212> DNA
<213> Artificial Sequence

<220>
<221> source
<222> 1..26
<223> /mol_type="DNA"
/note="synthetic"
/organism="Artificial Sequence"

<400> 525
gaacactagg ctgcaaagac agtaac 26

<210> 526
<211> 24
<212> DNA
<213> Artificial Sequence

<220>
<221> source
<222> 1..24
<223> /mol_type="DNA"
/note="synthetic"
/organism="Artificial Sequence"

<400> 526
ccaagcaagg caaacacatc cacc 24

<210> 527
<211> 23
<212> DNA
<213> Artificial Sequence

<220>
<221> source
<222> 1..23
<223> /mol_type="DNA"
/note="synthetic"
/organism="Artificial Sequence"

<400> 527
ggaggatgga gcctttccat cac 23

<210> 528
<211> 24
<212> DNA
<213> Artificial Sequence

<220>
<221> source
<222> 1..24
<223> /mol_type="DNA"
/note="synthetic"
/organism="Artificial Sequence"

<400> 528
gaagaggaag atgtgttcct ttgg 24

<210> 529
<211> 24
<212> DNA
<213> Artificial Sequence

<220>
<221> source
<222> 1..24
<223> /mol_type="DNA"
/note="synthetic"
/organism="Artificial Sequence"

<400> 529
gaatgaagga tgatgtggca gtgg 24

<210> 530
<211> 21
<212> DNA
<213> Artificial Sequence

<220>
<221> source
<222> 1..21
<223> /mol_type="DNA"
/note="synthetic"
/organism="Artificial Sequence"

<400> 530
caaaacatca gccattaacg g 21

<210> 531
<211> 26
<212> DNA
<213> Artificial Sequence

<220>
<221> source
<222> 1..26
<223> /mol_type="DNA"
/note="synthetic"
/organism="Artificial Sequence"

<400> 531
ccacttactg ttcatataat acagag 26

<210> 532
<211> 25
<212> DNA
<213> Artificial Sequence

<220>
<221> source
<222> 1..25
<223> /mol_type="DNA"
/note="synthetic"
/organism="Artificial Sequence"

<400> 532
catgtgagat agcatttggg aatgc 25

<210> 533
<211> 26
<212> DNA
<213> Artificial Sequence

<220>
<221> source
<222> 1..26
<223> /mol_type="DNA"
/note="Synthetic"
/organism="Artificial Sequence"

<400> 533
catgacctac catcattgga aagcag 26

<210> 534
<211> 21
<212> DNA
<213> Artificial Sequence

<220>
<221> source
<222> 1..21
<223> /mol_type="DNA"
/note="Synthetic"
/organism="Artificial Sequence"

<400> 534
gtaatttcac agttaggaat c 21

<210> 535
<211> 25
<212> DNA
<213> Artificial Sequence

<220>
<221> source
<222> 1..25
<223> /mol_type="DNA"
/note="Synthetic"
/organism="Artificial Sequence"

<400> 535
gtcacccaag gtcatggagc acagg 25

<210> 536
<211> 23
<212> DNA
<213> Artificial Sequence

<220>
<221> source
<222> 1..23
<223> /mol_type="DNA"
/note="Synthetic"
/organism="Artificial Sequence"

<400> 536
cagaatgcct gtaaagctat aac 23

<210> 537
<211> 24
<212> DNA
<213> Artificial Sequence

<220>
<221> source
<222> 1..24
<223> /mol_type="DNA"
/note="Synthetic"
/organism="Artificial Sequence"

<400> 537
gtcctggagt cccaactcct tgac 24

<210> 538
<211> 25
<212> DNA
<213> Artificial Sequence

<220>
<221> source
<222> 1..25
<223> /mol_type="DNA"
/note="Synthetic"
/organism="Artificial Sequence"

<400> 538
ggaagtggct ctgatggccg tcctg 25

<210> 539
<211> 27
<212> DNA
<213> Artificial Sequence

<220>
<221> source
<222> 1..27
<223> /mol_type="DNA"
/note="Synthetic"
/organism="Artificial Sequence"

<400> 539
ccactcacac acactaaata ttttaag 27

<210> 540
<211> 27
<212> DNA
<213> Artificial Sequence

<220>
<221> source
<222> 1..27
<223> /mol_type="DNA"
/note="Synthetic"
/organism="Artificial Sequence"

<400> 540

gaccaaaaca ccttaagtaa ctgactc 27

<210> 541
<211> 24
<212> DNA
<213> Artificial Sequence

<220>
<221> source
<222> 1..24
<223> /mol_type="DNA"
/note="Synthetic"
/organism="Artificial Sequence"

<400> 541
ccaatccaac atccagacac atag 24

<210> 542
<211> 24
<212> DNA
<213> Artificial Sequence

<220>
<221> source
<222> 1..24
<223> /mol_type="DNA"
/note="Synthetic"
/organism="Artificial Sequence"

<400> 542
ccagagccat agaaacttga tcag 24

<210> 543
<211> 29
<212> DNA
<213> Artificial Sequence

<220>
<221> source
<222> 1..29
<223> /mol_type="DNA"
/note="Synthetic"
/organism="Artificial Sequence"

<400> 543
gtatggacta tggcacttca attgcatgg 29

<210> 544
<211> 28
<212> DNA
<213> Artificial Sequence

<220>
<221> source
<222> 1..28
<223> /mol_type="DNA"
/note="Synthetic"
/organism="Artificial Sequence"

<400> 544
ccagagaaca tggcaaccag cacaggac 28

<210> 545
<211> 25
<212> DNA
<213> Artificial Sequence

<220>
<221> source
<222> 1..25
<223> /mol_type="DNA"
/note="Synthetic"
/organism="Artificial Sequence"

<400> 545
caaatgagct ggcaagtgcc gtgtc 25

<210> 546
<211> 25
<212> DNA
<213> Artificial Sequence

<220>
<221> source
<222> 1..25
<223> /mol_type="DNA"
/note="Synthetic"
/organism="Artificial Sequence"

<400> 546
gagtttccca aacactcagt gaaac 25

<210> 547
<211> 25
<212> DNA
<213> Artificial Sequence

<220>
<221> source
<222> 1..25
<223> /mol_type="DNA"
/note="Synthetic"
/organism="Artificial Sequence"

<400> 547
gcaatatcag ccttaggtgc ggctc 25

<210> 548
<211> 26
<212> DNA
<213> Artificial Sequence

<220>
<221> source
<222> 1..26
<223> /mol_type="DNA"
/note="Synthetic"
/organism="Artificial Sequence"

<400> 548
catagaaagt gaacatttag gatgtg 26

<210> 549
<211> 24
<212> DNA
<213> Artificial Sequence

<220>
<221> source
<222> 1..24
<223> /mol_type="DNA"
/note="Synthetic"
/organism="Artificial Sequence"

<400> 549
ccatgagtac gtattttgaa actc 24

<210> 550
<211> 24
<212> DNA
<213> Artificial Sequence

<220>
<221> source
<222> 1..24
<223> /mol_type="DNA"
/note="Synthetic"
/organism="Artificial Sequence"

<400> 550
catatcccca tggcaaactc ttgc 24

<210> 551
<211> 24
<212> DNA
<213> Artificial Sequence

<220>
<221> source
<222> 1..24
<223> /mol_type="DNA"
/note="Synthetic"
/organism="Artificial Sequence"

<400> 551
ctaacgttcg ccagccataa gtcc 24

<210> 552
<211> 26
<212> DNA
<213> Artificial Sequence

<220>
<221> source
<222> 1..26
<223> /mol_type="DNA"
/note="Synthetic"

/organism="Artificial Sequence"

<400> 552
gctgcgagct cacccagaat gtctgg 26

<210> 553
<211> 25
<212> DNA
<213> Artificial Sequence

<220>
<221> source
<222> 1..25
<223> /mol_type="DNA"
/note="Synthetic"
/organism="Artificial Sequence"

<400> 553
gacgggtcct ggggtgatct ggctc 25

<210> 554
<400> 554
000

<210> 555
<211> 24
<212> DNA
<213> Artificial Sequence

<220>
<221> source
<222> 1..24
<223> /mol_type="DNA"
/note="Synthetic"
/organism="Artificial Sequence"

<400> 555
caggactaca gaaatgtagg tttc 24

<210> 556
<211> 26
<212> DNA
<213> Artificial Sequence

<220>
<221> source
<222> 1..26
<223> /mol_type="DNA"
/note="Synthetic"
/organism="Artificial Sequence"

<400> 556
gtgcctgcct taagtaatgt gatgac 26

<210> 557
<211> 25
<212> DNA
<213> Artificial Sequence

<220>
<221> source
<222> 1..25
<223> /mol_type="DNA"
/note="Synthetic"
/organism="Artificial Sequence"

<400> 557
gactggaagt gtcgcatcac caatg 25

<210> 558
<211> 25
<212> DNA
<213> Artificial Sequence

<220>
<221> source
<222> 1..25
<223> /mol_type="DNA"
/note="Synthetic"
/organism="Artificial Sequence"

<400> 558
ggtttaataa tgcgatctgg gacac 25

<210> 559
<211> 25
<212> DNA
<213> Artificial Sequence

<220>
<221> source
<222> 1..25
<223> /mol_type="DNA"
/note="Synthetic"
/organism="Artificial Sequence"

<400> 559
gcagctataa tttagagaac caagg 25

<210> 560
<211> 21
<212> DNA
<213> Artificial Sequence

<220>
<221> source
<222> 1..21
<223> /mol_type="DNA"
/note="Synthetic"
/organism="Artificial Sequence"

<400> 560
aaaattgact tcatttccat g 21

<210> 561
<211> 22
<212> DNA
<213> Artificial Sequence

<220>
<221> source
<222> 1..22
<223> /mol_type="DNA"
/note="Synthetic"
/organism="Artificial Sequence"

<400> 561
cctagttgct ctaaaactaa cg 22

<210> 562
<211> 24
<212> DNA
<213> Artificial Sequence

<220>
<221> source
<222> 1..24
<223> /mol_type="DNA"
/note="Synthetic"
/organism="Artificial Sequence"

<400> 562
ctgtgaggcg tgacagccgt gcag 24

<210> 563
<211> 25
<212> DNA
<213> Artificial Sequence

<220>
<221> source
<222> 1..25
<223> /mol_type="DNA"
/note="Synthetic"
/organism="Artificial Sequence"

<400> 563
caacctacta atcagaacca gcatc 25

<210> 564
<211> 24
<212> DNA
<213> Artificial Sequence

<220>
<221> source
<222> 1..24
<223> /mol_type="DNA"
/note="Synthetic"
/organism="Artificial Sequence"

<400> 564
ccttcactgt gtctgcaaat ctgc 24

<210> 565
<211> 24
<212> DNA

<213> Artificial Sequence

<220>
<221> source
<222> 1..24
<223> /mol_type="DNA"
/note="Synthetic"
/organism="Artificial Sequence"

<400> 565
cctgtcataa gtctccttgt tgag 24

<210> 566
<211> 25
<212> DNA
<213> Artificial Sequence

<220>
<221> source
<222> 1..25
<223> /mol_type="DNA"
/note="Synthetic"
/organism="Artificial Sequence"

<400> 566
cagtctgtgg gtctaagagc taatg 25

<210> 567
<211> 24
<212> DNA
<213> Artificial Sequence

<220>
<221> source
<222> 1..24
<223> /mol_type="DNA"
/note="Synthetic"
/organism="Artificial Sequence"

<400> 567
caggaatggg tgagtctctg tgtg 24

<210> 568
<211> 23
<212> DNA
<213> Artificial Sequence

<220>
<221> source
<222> 1..23
<223> /mol_type="DNA"
/note="Synthetic"
/organism="Artificial Sequence"

<400> 568
gtggaattct gcccaggcct ttc 23

<210> 569
<211> 25

<212> DNA
<213> Artificial Sequence

<220>
<221> source
<222> 1..25
<223> /mol_type="DNA"
/note="Synthetic"
/organism="Artificial Sequence"

<400> 569
gattctacaa accagccagc caaac 25

<210> 570
<211> 24
<212> DNA
<213> Artificial Sequence

<220>
<221> source
<222> 1..24
<223> /mol_type="DNA"
/note="Synthetic"
/organism="Artificial Sequence"

<400> 570
cctactggtt cacatctgac cctg 24

<210> 571
<211> 24
<212> DNA
<213> Artificial Sequence

<220>
<221> source
<222> 1..24
<223> /mol_type="DNA"
/note="Synthetic"
/organism="Artificial Sequence"

<400> 571
gtttgaatgt ggtttcgttg gaag 24

<210> 572
<211> 27
<212> DNA
<213> Artificial Sequence

<220>
<221> source
<222> 1..27
<223> /mol_type="DNA"
/note="Synthetic"
/organism="Artificial Sequence"

<400> 572
ctttgtcacc aggcagaggg caatatc 27

<210> 573

<211> 23
<212> DNA
<213> Artificial Sequence

<220>
<221> source
<222> 1..23
<223> /mol_type="DNA"
/note="Synthetic"
/organism="Artificial Sequence"

<400> 573
gacagtaact tgggctttct gac 23

<210> 574
<211> 23
<212> DNA
<213> Artificial Sequence

<220>
<221> source
<222> 1..23
<223> /mol_type="DNA"
/note="Synthetic"
/organism="Artificial Sequence"

<400> 574
catccaccca aagactctcc aag 23

<210> 575
<211> 25
<212> DNA
<213> Artificial Sequence

<220>
<221> source
<222> 1..25
<223> /mol_type="DNA"
/note="Synthetic"
/organism="Artificial Sequence"

<400> 575
ctgttcatat aatacagagt ccctg 25

<210> 576
<211> 22
<212> DNA
<213> Artificial Sequence

<220>
<221> source
<222> 1..22
<223> /mol_type="DNA"
/note="Synthetic"
/organism="Artificial Sequence"

<400> 576
gagagatgca ggagctctgt gc 22

<210> 577
<211> 25
<212> DNA
<213> Artificial Sequence

<220>
<221> source
<222> 1..25
<223> /mol_type="DNA"
/note="Synthetic"
/organism="Artificial Sequence"

<400> 577
gcagtttgta gtcaatcaaa ggtgg 25

<210> 578
<211> 22
<212> DNA
<213> Artificial Sequence

<220>
<221> source
<222> 1..22
<223> /mol_type="DNA"
/note="Synthetic"
/organism="Artificial Sequence"

<400> 578
gtaatttaaa tgggaatagc cc 22

<210> 579
<211> 24
<212> DNA
<213> Artificial Sequence

<220>
<221> source
<222> 1..24
<223> /mol_type="DNA"
/note="Synthetic"
/organism="Artificial Sequence"

<400> 579
caactccttg accattacct caag 24

<210> 580
<211> 23
<212> DNA
<213> Artificial Sequence

<220>
<221> source
<222> 1..23
<223> /mol_type="DNA"
/note="Synthetic"
/organism="Artificial Sequence"

<400> 580
gatggccgtc ctgcccacac agg 23

<210> 581
<211> 21
<212> DNA
<213> Artificial Sequence

<220>
<221> source
<222> 1..21
<223> /mol_type="DNA"
/note="Synthetic"
/organism="Artificial Sequence"

<400> 581
gagtagttta gcatatattg c 21

<210> 582
<211> 23
<212> DNA
<213> Artificial Sequence

<220>
<221> source
<222> 1..23
<223> /mol_type="DNA"
/note="Synthetic"
/organism="Artificial Sequence"

<400> 582
gacagtcaga aatgcaggaa agc 23

<210> 583
<211> 26
<212> DNA
<213> Artificial Sequence

<220>
<221> source
<222> 1..26
<223> /mol_type="DNA"
/note="Synthetic"
/organism="Artificial Sequence"

<400> 583
caagtgccgt gtcctggcac ccaagc 26

<210> 584
<211> 24
<212> DNA
<213> Artificial Sequence

<220>
<221> source
<222> 1..24
<223> /mol_type="DNA"
/note="Synthetic"
/organism="Artificial Sequence"

<400> 584
ccaaacactc agtgaaacaa agag 24

<210> 585
<211> 21
<212> DNA
<213> Artificial Sequence

<220>
<221> source
<222> 1..21
<223> /mol_type="DNA"
/note="Synthetic"
/organism="Artificial Sequence"

<400> 585
ccttaggtgc ggctccacag c 21

<210> 586
<211> 22
<212> DNA
<213> Artificial Sequence

<220>
<221> source
<222> 1..22
<223> /mol_type="DNA"
/note="Synthetic"
/organism="Artificial Sequence"

<400> 586
catttaggat gtggagatga gc 22

<210> 587
<211> 23
<212> DNA
<213> Artificial Sequence

<220>
<221> source
<222> 1..23
<223> /mol_type="DNA"
/note="Synthetic"
/organism="Artificial Sequence"

<400> 587
gaaactcaag atcgcattca tgc 23

<210> 588
<211> 23
<212> DNA
<213> Artificial Sequence

<220>
<221> source
<222> 1..23
<223> /mol_type="DNA"
/note="Synthetic"
/organism="Artificial Sequence"

<400> 588

gcaaactctt gctatcccag gag 23

<210> 589
<211> 22
<212> DNA
<213> Artificial Sequence

<220>
<221> source
<222> 1..22
<223> /mol_type="DNA"
/note="Synthetic"
/organism="Artificial Sequence"

<400> 589
cagccataag tcctcgacgt gg 22

<210> 590
<211> 24
<212> DNA
<213> Artificial Sequence

<220>
<221> source
<222> 1..24
<223> /mol_type="DNA"
/note="Synthetic"
/organism="Artificial Sequence"

<400> 590
catcctcccc tgcatgtgtt aaac 24

<210> 591
<211> 24
<212> DNA
<213> Artificial Sequence

<220>
<221> source
<222> 1..24
<223> /mol_type="DNA"
/note="Synthetic"
/organism="Artificial Sequence"

<400> 591
gtaggtttct aaacatcaag aaac 24

<210> 592
<211> 24
<212> DNA
<213> Artificial Sequence

<220>
<221> source
<222> 1..24
<223> /mol_type="DNA"
/note="Synthetic"
/organism="Artificial Sequence"

<400> 592
gtgatgacat ttctccaggg atgc 24

<210> 593
<211> 23
<212> DNA
<213> Artificial Sequence

<220>
<221> source
<222> 1..23
<223> /mol_type="DNA"
/note="Synthetic"
/organism="Artificial Sequence"

<400> 593
catcaccaat gccttcttta agc 23

<210> 594
<211> 24
<212> DNA
<213> Artificial Sequence

<220>
<221> source
<222> 1..24
<223> /mol_type="DNA"
/note="Synthetic"
/organism="Artificial Sequence"

<400> 594
gctggagggt ttaataatgc gatc 24

<210> 595
<211> 24
<212> DNA
<213> Artificial Sequence

<220>
<221> source
<222> 1..24
<223> /mol_type="DNA"
/note="Synthetic"
/organism="Artificial Sequence"

<400> 595
gcaaacacac aggcacctgc tggc 24

<210> 596
<211> 27
<212> DNA
<213> Artificial Sequence

<220>
<221> source
<222> 1..27
<223> /mol_type="DNA"
/note="Synthetic"
/organism="Artificial Sequence"

<400> 596
catttccatg tgagtttcac tagatgg 27

<210> 597
<211> 24
<212> DNA
<213> Artificial Sequence

<220>
<221> source
<222> 1..24
<223> /mol_type="DNA"
/note="Synthetic"
/organism="Artificial Sequence"

<400> 597
gaccggacga caggccacct cgtc 24

<210> 598
<211> 25
<212> DNA
<213> Artificial Sequence

<220>
<221> source
<222> 1..25
<223> /mol_type="DNA"
/note="Synthetic"
/organism="Artificial Sequence"

<400> 598
gaagaacgaa acgtcccgtt cctcc 25

<210> 599
<211> 23
<212> DNA
<213> Artificial Sequence

<220>
<221> source
<222> 1..23
<223> /mol_type="DNA"
/note="Synthetic"
/organism="Artificial Sequence"

<400> 599
gttgagcact cgtgtgcatt agg 23

<210> 600
<211> 22
<212> DNA
<213> Artificial Sequence

<220>
<221> source
<222> 1..22
<223> /mol_type="DNA"
/note="Synthetic"

/organism="Artificial Sequence"

<400> 600
ctcagtgcac gtgtactggg ta 22

<210> 601
<211> 35
<212> DNA
<213> Artificial Sequence

<220>
<221> source
<222> 1..35
<223> /mol_type="DNA"
/note="Synthetic"
/organism="Artificial Sequence"

<400> 601
gttcactggg ctaattgcgg gactcttgtt cgcac 35

<210> 602
<211> 28
<212> DNA
<213> Artificial Sequence

<220>
<221> source
<222> 1..28
<223> /mol_type="DNA"
/note="Synthetic"
/organism="Artificial Sequence"

<400> 602
ggtaaataca tgcttttcta gtggtcag 28

<210> 603
<211> 23
<212> DNA
<213> Artificial Sequence

<220>
<221> source
<222> 1..23
<223> /mol_type="DNA"
/note="Synthetic"
/organism="Artificial Sequence"

<400> 603
ggaggatgga gcctttccat cac 23

<210> 604
<211> 24
<212> DNA
<213> Artificial Sequence

<220>
<221> source
<222> 1..24
<223> /mol_type="DNA"

/note="Synthetic"
/organism="Artificial Sequence"

<400> 604
gaagaggaag atgtgttcct ttgg 24

<210> 605
<211> 24
<212> DNA
<213> Artificial Sequence

<220>
<221> source
<222> 1..24
<223> /mol_type="DNA"
/note="Synthetic"
/organism="Artificial Sequence"

<400> 605
gaatgaagga tgatgtggca gtgg 24

<210> 606
<211> 25
<212> DNA
<213> Artificial Sequence

<220>
<221> source
<222> 1..25
<223> /mol_type="DNA"
/note="Synthetic"
/organism="Artificial Sequence"

<400> 606
gtatgtgtga aggagtcact gaaac 25

<210> 607
<211> 22
<212> DNA
<213> Artificial Sequence

<220>
<221> source
<222> 1..22
<223> /mol_type="DNA"
/note="Synthetic"
/organism="Artificial Sequence"

<400> 607
ggtgagtcac aggttcagtt gg 22

<210> 608
<211> 21
<212> DNA
<213> Artificial Sequence

<220>
<221> source
<222> 1..21

<223> /mol_type="DNA"
/note="Synthetic"
/organism="Artificial Sequence"

<400> 608
caaaacatca gccattaacg g 21

<210> 609
<211> 22
<212> DNA
<213> Artificial Sequence

<220>
<221> source
<222> 1..22
<223> /mol_type="DNA"
/note="Synthetic"
/organism="Artificial Sequence"

<400> 609
gtagccagca tgtctgtgtc ac 22

<210> 610
<211> 23
<212> DNA
<213> Artificial Sequence

<220>
<221> source
<222> 1..23
<223> /mol_type="DNA"
/note="Synthetic"
/organism="Artificial Sequence"

<400> 610
cagaatgcct gtaaagctat aac 23

<210> 611
<211> 23
<212> DNA
<213> Artificial Sequence

<220>
<221> source
<222> 1..23
<223> /mol_type="DNA"
/note="Synthetic"
/organism="Artificial Sequence"

<400> 611
catttggctt tccccactca cac 23

<210> 612
<211> 27
<212> DNA
<213> Artificial Sequence

<220>
<221> source

<222> 1..27
<223> /mol_type="DNA"
/note="Synthetic"
/organism="Artificial Sequence"

<400> 612
gaccaaaaca ccttaagtaa ctgactc 27

<210> 613
<211> 25
<212> DNA
<213> Artificial Sequence

<220>
<221> source
<222> 1..25
<223> /mol_type="DNA"
/note="Synthetic"
/organism="Artificial Sequence"

<400> 613
gaagctacat agtgtctcac tttcc 25

<210> 614
<211> 25
<212> DNA
<213> Artificial Sequence

<220>
<221> source
<222> 1..25
<223> /mol_type="DNA"
/note="Synthetic"
/organism="Artificial Sequence"

<400> 614
cacaactgct aatggcccgt tctcg 25

<210> 615
<211> 23
<212> DNA
<213> Artificial Sequence

<220>
<221> source
<222> 1..23
<223> /mol_type="DNA"
/note="Synthetic"
/organism="Artificial Sequence"

<400> 615
gctcctgctc cctgtcataa gtc 23

<210> 616
<211> 25
<212> DNA
<213> Artificial Sequence

<220>

<221> source
<222> 1..25
<223> /mol_type="DNA"
/note="Synthetic"
/organism="Artificial Sequence"

<400> 616
gaagtcctgc tggtagtcag ggttg 25

<210> 617
<211> 24
<212> DNA
<213> Artificial Sequence

<220>
<221> source
<222> 1..24
<223> /mol_type="DNA"
/note="Synthetic"
/organism="Artificial Sequence"

<400> 617
ctgcagtggg caaccccgag tatc 24

<210> 618
<211> 25
<212> DNA
<213> Artificial Sequence

<220>
<221> source
<222> 1..25
<223> /mol_type="DNA"
/note="Synthetic"
/organism="Artificial Sequence"

<400> 618
cagtctgtgg gtctaagagc taatg 25

<210> 619
<211> 22
<212> DNA
<213> Artificial Sequence

<220>
<221> source
<222> 1..22
<223> /mol_type="DNA"
/note="Synthetic"
/organism="Artificial Sequence"

<400> 619
gacaggccac ctcgtcggcg tc 22

<210> 620
<211> 22
<212> DNA
<213> Artificial Sequence

<220>
<221> source
<222> 1..22
<223> /mol_type="DNA"
/note="Synthetic"
/organism="Artificial Sequence"

<400> 620
cagctgatct caaggaaaca gg 22

<210> 621
<211> 25
<212> DNA
<213> Artificial Sequence

<220>
<221> source
<222> 1..25
<223> /mol_type="DNA"
/note="Synthetic"
/organism="Artificial Sequence"

<400> 621
ctcgtgtgca ttagggttca actgg 25

<210> 622
<211> 26
<212> DNA
<213> Artificial Sequence

<220>
<221> source
<222> 1..26
<223> /mol_type="DNA"
/note="Synthetic"
/organism="Artificial Sequence"

<400> 622
ccttctccga ggtggaattg agtgac 26

<210> 623
<211> 26
<212> DNA
<213> Artificial Sequence

<220>
<221> source
<222> 1..26
<223> /mol_type="DNA"
/note="Synthetic"
/organism="Artificial Sequence"

<400> 623
gctaattgcg ggactcttgt tcgcac 26

<210> 624
<211> 22
<212> DNA
<213> Artificial Sequence

<220>
<221> source
<222> 1..22
<223> /mol_type="DNA"
/note="Synthetic"
/organism="Artificial Sequence"

<400> 624
tacatgcttt tctagtggtc ag 22

<210> 625
<211> 23
<212> DNA
<213> Artificial Sequence

<220>
<221> source
<222> 1..23
<223> /mol_type="DNA"
/note="Synthetic"
/organism="Artificial Sequence"

<400> 625
cctttccatc acccctcaag agg 23

<210> 626
<211> 25
<212> DNA
<213> Artificial Sequence

<220>
<221> source
<222> 1..25
<223> /mol_type="DNA"
/note="Synthetic"
/organism="Artificial Sequence"

<400> 626
gatgtgttcc tttggaggtg gcatg 25

<210> 627
<211> 24
<212> DNA
<213> Artificial Sequence

<220>
<221> source
<222> 1..24
<223> /mol_type="DNA"
/note="Synthetic"
/organism="Artificial Sequence"

<400> 627
gatgtggcag tggcggttcc ggtg 24

<210> 628
<211> 24
<212> DNA

<213> Artificial Sequence

<220>
<221> source
<222> 1..24
<223> /mol_type="DNA"
/note="Synthetic"
/organism="Artificial Sequence"

<400> 628
ggagtcactg aaacaaacaa cagg 24

<210> 629
<211> 21
<212> DNA
<213> Artificial Sequence

<220>
<221> source
<222> 1..21
<223> /mol_type="DNA"
/note="Synthetic"
/organism="Artificial Sequence"

<400> 629
ggttcagttg cttgtataaa g 21

<210> 630
<211> 24
<212> DNA
<213> Artificial Sequence

<220>
<221> source
<222> 1..24
<223> /mol_type="DNA"
/note="Synthetic"
/organism="Artificial Sequence"

<400> 630
ccattaacgg taaaatttca gaag 24

<210> 631
<211> 20
<212> DNA
<213> Artificial Sequence

<220>
<221> source
<222> 1..20
<223> /mol_type="DNA"
/note="Synthetic"
/organism="Artificial Sequence"

<400> 631
ccaaggtcat ggagcacagg 20

<210> 632
<211> 25

<212> DNA
<213> Artificial Sequence

<220>
<221> source
<222> 1..25
<223> /mol_type="DNA"
/note="Synthetic"
/organism="Artificial Sequence"

<400> 632
ctgtaaagct ataacaacaa cctgg 25

<210> 633
<211> 27
<212> DNA
<213> Artificial Sequence

<220>
<221> source
<222> 1..27
<223> /mol_type="DNA"
/note="Synthetic"
/organism="Artificial Sequence"

<400> 633
ccactcacac acactaaata ttttaag 27

<210> 634
<211> 24
<212> DNA
<213> Artificial Sequence

<220>
<221> source
<222> 1..24
<223> /mol_type="DNA"
/note="Synthetic"
/organism="Artificial Sequence"

<400> 634
gtaactgact caaatacaaa ccac 24

<210> 635
<211> 25
<212> DNA
<213> Artificial Sequence

<220>
<221> source
<222> 1..25
<223> /mol_type="DNA"
/note="Synthetic"
/organism="Artificial Sequence"

<400> 635
gaagctacat agtgtctcac tttcc 25

<210> 636

<211> 25
<212> DNA
<213> Artificial Sequence

<220>
<221> source
<222> 1..25
<223> /mol_type="DNA"
/note="Synthetic"
/organism="Artificial Sequence"

<400> 636
cacaactgct aatggcccgt tctcg 25

<210> 637
<211> 24
<212> DNA
<213> Artificial Sequence

<220>
<221> source
<222> 1..24
<223> /mol_type="DNA"
/note="Synthetic"
/organism="Artificial Sequence"

<400> 637
cctgtcataa gtctccttgt tgag 24

<210> 638
<211> 20
<212> DNA
<213> Artificial Sequence

<220>
<221> source
<222> 1..20
<223> /mol_type="DNA"
/note="Synthetic"
/organism="Artificial Sequence"

<400> 638
ggtagtcagg gttgtccagg 20

<210> 639
<211> 23
<212> DNA
<213> Artificial Sequence

<220>
<221> source
<222> 1..23
<223> /mol_type="DNA"
/note="Synthetic"
/organism="Artificial Sequence"

<400> 639
cgagtatctc aacactgtcc agc 23

<210> 640
<211> 25
<212> DNA
<213> Artificial Sequence

<220>
<221> source
<222> 1..25
<223> /mol_type="DNA"
/note="Synthetic"
/organism="Artificial Sequence"

<400> 640
ctaagagcta atgcgggcat ggctg 25

<210> 641
<400> 641
000

<210> 642
<400> 642
000

<210> 643
<400> 643
000

<210> 644
<400> 644
000

<210> 645
<211> 18
<212> DNA
<213> Artificial Sequence

<220>
<221> source
<222> 1..18
<223> /mol_type="DNA"
/note="synthetic"
/organism="Artificial Sequence"

<400> 645
tgtaaaacga cggccagt 18

<210> 646
<211> 22
<212> DNA
<213> Artificial Sequence

<220>
<221> source
<222> 1..22
<223> /mol_type="DNA"
/note="synthetic"
/organism="Artificial Sequence"

<400> 646
tggtctcaca ggaccactga tt 22

<210> 647
<211> 22
<212> DNA
<213> Artificial Sequence

<220>
<221> source
<222> 1..22
<223> /mol_type="DNA"
/note="synthetic"
/organism="Artificial Sequence"

<400> 647
gaggccagtg ctgtctctaa gg 22

<210> 648
<211> 21
<212> DNA
<213> Artificial Sequence

<220>
<221> source
<222> 1..21
<223> /mol_type="DNA"
/note="synthetic"
/organism="Artificial Sequence"

<400> 648
atgggacagg cactgatttg t 21

<210> 649
<211> 22
<212> DNA
<213> Artificial Sequence

<220>
<221> source
<222> 1..22
<223> /mol_type="DNA"
/note="synthetic"
/organism="Artificial Sequence"

<400> 649
cagctctggc tcacactacc ag 22

<210> 650
<211> 20
<212> DNA
<213> Artificial Sequence

<220>
<221> source
<222> 1..20
<223> /mol_type="DNA"
/note="synthetic"
/organism="Artificial Sequence"

<400> 650
gcagctggac tcgatttcct 20

<210> 651
<211> 22
<212> DNA
<213> Artificial Sequence

<220>
<221> source
<222> 1..22
<223> /mol_type="DNA"
/note="synthetic"
/organism="Artificial Sequence"

<400> 651
tgcccaatga gtcaagaagt gt 22

<210> 652
<211> 22
<212> DNA
<213> Artificial Sequence

<220>
<221> source
<222> 1..22
<223> /mol_type="DNA"
/note="synthetic"
/organism="Artificial Sequence"

<400> 652
cactcacgga tgctgcttag tt 22

<210> 653
<211> 22
<212> DNA
<213> Artificial Sequence

<220>
<221> source
<222> 1..22
<223> /mol_type="DNA"
/note="synthetic"
/organism="Artificial Sequence"

<400> 653
tcagagcctg tgtttctacc aa 22

<210> 654
<211> 24
<212> DNA
<213> Artificial Sequence

<220>
<221> source
<222> 1..24
<223> /mol_type="DNA"
/note="synthetic"
/organism="Artificial Sequence"

<400> 654

```
aaataatcag tgtgattcgt ggag                                              24


<210> 655
<211> 21
<212> DNA
<213> Artificial Sequence

<220>
<221> source
<222> 1..21
<223> /mol_type="DNA"
      /note="synthetic"
      /organism="Artificial Sequence"

<400> 655
acttcacagc cctgcgtaaa c                                                 21


<210> 656
<211> 22
<212> DNA
<213> Artificial Sequence

<220>
<221> source
<222> 1..22
<223> /mol_type="DNA"
      /note="synthetic"
      /organism="Artificial Sequence"

<400> 656
gcagcgggtt acatcttctt tc                                                22


<210> 657
<211> 22
<212> DNA
<213> Artificial Sequence

<220>
<221> source
<222> 1..22
<223> /mol_type="DNA"
      /note="synthetic"
      /organism="Artificial Sequence"

<400> 657
cctgaactcc gtcagactga aa                                                22


<210> 658
<211> 23
<212> DNA
<213> Artificial Sequence

<220>
<221> source
<222> 1..23
<223> /mol_type="DNA"
      /note="synthetic"
      /organism="Artificial Sequence"
```

<400> 658
ccttacagca atcctgtgaa aca 23

<210> 659
<211> 22
<212> DNA
<213> Artificial Sequence

<220>
<221> source
<222> 1..22
<223> /mol_type="DNA"
/note="synthetic"
/organism="Artificial Sequence"

<400> 659
atgtacagtg ctggcatggt ct 22

<210> 660
<211> 20
<212> DNA
<213> Artificial Sequence

<220>
<221> source
<222> 1..20
<223> /mol_type="DNA"
/note="synthetic"
/organism="Artificial Sequence"

<400> 660
tccaaatgag ctggcaagtg 20

<210> 661
<211> 20
<212> DNA
<213> Artificial Sequence

<220>
<221> source
<222> 1..20
<223> /mol_type="DNA"
/note="synthetic"
/organism="Artificial Sequence"

<400> 661
gtgcatcgct ggtaacatcc 20

<210> 662
<211> 20
<212> DNA
<213> Artificial Sequence

<220>
<221> source
<222> 1..20
<223> /mol_type="DNA"
/note="synthetic"
/organism="Artificial Sequence"

<400> 662
atcgcattca tgcgtcttca 20

<210> 663
<211> 19
<212> DNA
<213> Artificial Sequence

<220>
<221> source
<222> 1..19
<223> /mol_type="DNA"
/note="synthetic"
/organism="Artificial Sequence"

<400> 663
gctcagagcc tggcatgaa 19

<210> 664
<211> 20
<212> DNA
<213> Artificial Sequence

<220>
<221> source
<222> 1..20
<223> /mol_type="DNA"
/note="synthetic"
/organism="Artificial Sequence"

<400> 664
tggctcgtct gtgtgtgtca 20

<210> 665
<211> 20
<212> DNA
<213> Artificial Sequence

<220>
<221> source
<222> 1..20
<223> /mol_type="DNA"
/note="synthetic"
/organism="Artificial Sequence"

<400> 665
tgaagcaaat tgcccaagac 20

<210> 666
<211> 20
<212> DNA
<213> Artificial Sequence

<220>
<221> source
<222> 1..20
<223> /mol_type="DNA"
/note="synthetic"

/organism="Artificial Sequence"

<400> 666
aagtgtcgca tcaccaatgc 20

<210> 667
<211> 23
<212> DNA
<213> Artificial Sequence

<220>
<221> source
<222> 1..23
<223> /mol_type="DNA"
/note="synthetic"
/organism="Artificial Sequence"

<400> 667
tcccaaacac tcagtgaaac aaa 23

<210> 668
<211> 20
<212> DNA
<213> Artificial Sequence

<220>
<221> source
<222> 1..20
<223> /mol_type="DNA"
/note="synthetic"
/organism="Artificial Sequence"

<400> 668
tgtggagatg agcagggtct 20

<210> 669
<211> 20
<212> DNA
<213> Artificial Sequence

<220>
<221> source
<222> 1..20
<223> /mol_type="DNA"
/note="synthetic"
/organism="Artificial Sequence"

<400> 669
atccccatgg caaactcttg 20

<210> 670
<211> 19
<212> DNA
<213> Artificial Sequence

<220>
<221> source
<222> 1..19
<223> /mol_type="DNA"

/note="synthetic"
/organism="Artificial Sequence"

<400> 670
catcctcccc tgcatgtgt 19

<210> 671
<211> 25
<212> DNA
<213> Artificial Sequence

<220>
<221> source
<222> 1..25
<223> /mol_type="DNA"
/note="synthetic"
/organism="Artificial Sequence"

<400> 671
cgaaagaaaa tacttgcatg tcaga 25

<210> 672
<211> 20
<212> DNA
<213> Artificial Sequence

<220>
<221> source
<222> 1..20
<223> /mol_type="DNA"
/note="synthetic"
/organism="Artificial Sequence"

<400> 672
tgacatttct ccagggatgc 20

<210> 673
<211> 19
<212> DNA
<213> Artificial Sequence

<220>
<221> source
<222> 1..19
<223> /mol_type="DNA"
/note="synthetic"
/organism="Artificial Sequence"

<400> 673
atgcgatctg ggacacagg 19

<210> 674
<211> 16
<212> DNA
<213> Artificial Sequence

<220>
<221> source
<222> 1..16

<223> /mol_type="DNA"
/note="synthetic"
/organism="Artificial Sequence"

<400> 674
aacagctatg accatg 16

<210> 675
<211> 26
<212> DNA
<213> Artificial Sequence

<220>
<221> source
<222> 1..26
<223> /mol_type="DNA"
/note="synthetic"
/organism="Artificial Sequence"

<400> 675
caagtgccgt gtcctggcag ccaagc 26

<210> 676
<211> 24
<212> DNA
<213> Artificial Sequence

<220>
<221> source
<222> 1..24
<223> /mol_type="DNA"
/note="synthetic"
/organism="Artificial Sequence"

<400> 676
ccaaacactc agtgaaacaa agag 24

<210> 677
<211> 25
<212> DNA
<213> Artificial Sequence

<220>
<221> source
<222> 1..25
<223> /mol_type="DNA"
/note="synthetic"
/organism="Artificial Sequence"

<400> 677
gcacccaagc ccatgccgtg gctgc 25

<210> 678
<211> 26
<212> DNA
<213> Artificial Sequence

<220>
<221> source

<222> 1..26
<223> /mol_type="DNA"
/note="synthetic"
/organism="Artificial Sequence"

<400> 678
gaaacaaaga gtaaagtaga tgatgg 26

<210> 679
<211> 24
<212> DNA
<213> Artificial Sequence

<220>
<221> source
<222> 1..24
<223> /mol_type="DNA"
/note="synthetic"
/organism="Artificial Sequence"

<400> 679
caccttcaca atataccctc catg 24

<210> 680
<211> 22
<212> DNA
<213> Artificial Sequence

<220>
<221> source
<222> 1..22
<223> /mol_type="DNA"
/note="synthetic"
/organism="Artificial Sequence"

<400> 680
gacagccgtg cagggaaaaa cc 22

<210> 681
<211> 24
<212> DNA
<213> Artificial Sequence

<220>
<221> source
<222> 1..24
<223> /mol_type="DNA"
/note="synthetic"
/organism="Artificial Sequence"

<400> 681
gaaccagcat ctcaaggaga tctc 24

<210> 682
<211> 24
<212> DNA
<213> Artificial Sequence

<220>

<221> source
<222> 1..24
<223> /mol_type="DNA"
/note="synthetic"
/organism="Artificial Sequence"

<400> 682
gagcacctgg cttggacact ggag 24

<210> 683
<211> 26
<212> DNA
<213> Artificial Sequence

<220>
<221> source
<222> 1..26
<223> /mol_type="DNA"
/note="synthetic"
/organism="Artificial Sequence"

<400> 683
gagcagccct gaactccgtc agactg 26

<210> 684
<211> 26
<212> DNA
<213> Artificial Sequence

<220>
<221> source
<222> 1..26
<223> /mol_type="DNA"
/note="synthetic"
/organism="Artificial Sequence"

<400> 684
ctcagtacaa tagatagaca gcaatg 26

<210> 685
<211> 25
<212> DNA
<213> Artificial Sequence

<220>
<221> source
<222> 1..25
<223> /mol_type="DNA"
/note="synthetic"
/organism="Artificial Sequence"

<400> 685
gacgggtcct ggggtgatct ggctc 25

<210> 686
<211> 26
<212> DNA
<213> Artificial Sequence

```
<220>
<221> source
<222> 1..26
<223> /mol_type="DNA"
      /note="synthetic"
      /organism="Artificial Sequence"

<400> 686
ctcagtacaa tagatagaca gcaatg                                          26
```

## Description

## BACKGROUND OF THE INVENTION

The invention relates to the fields of medical diagnostics and microfluidics.

Cancer is a disease marked by the uncontrolled proliferation of abnormal cells. In normal tissue, cells divide and organize within the tissue in response to signals from surrounding cells. Cancer cells do not respond in the same way to these signals, causing them to proliferate and, in many organs, form a tumor. As the growth of a tumor continues, genetic alterations may accumulate, manifesting as a more aggressive growth phenotype of the cancer cells. If left untreated, metastasis, the spread of cancer cells to distant areas of the body by way of the lymph system or bloodstream, may ensue. Metastasis results in the formation of secondary tumors at multiple sites, damaging healthy tissue. Most cancer death is caused by such secondary tumors.

Despite decades of advances in cancer diagnosis and therapy, many cancers continue to go undetected until late in their development. As one example, most early-stage lung cancers are asymptomatic and are not detected in time for curative treatment, resulting in an overall five-year survival rate for patients with lung cancer of less than 15%. However, in those instances in which lung cancer is detected and treated at an early stage, the prognosis is much more favorable.

Therefore, there exists a need to develop new methods for detecting cancer at earlier stages in the development of the disease.

## SUMMARY OF THE INVENTION

The invention features a method of detecting cancer cells in a cellular sample by:

a) introducing the cellular sample into a device including a channel having a structure that directs the cancer cells in a first direction to produce a first output sample enriched in the cancer cells and one or more second cells in a second direction to produce a second output sample enriched in the second cells; and
b) detecting the presence or absence of the cancer cells in the first output sample.

The structure can include an array of obstacles that form a network of gaps. The obstacles can be capable of selectively capturing the cancer cells. The channel may include an array of obstacles forming a network of gaps, wherein fluid flows through the gaps such that the fluid is divided unequally into a major flux and a minor flux. The cellular sample is, for example, a blood sample or fraction thereof.

Step b) can include reacting the first output sample with an antibody to a marker, e.g., selected from Table 1, for the cancer cells. Step b) can also include determining the number of cells in the first output sample, e.g., by determining the total amount of DNA in the first output sample. Alternatively, step b) includes determining the number of the cancer cells in the first output sample and optionally determining the number of endothelial cells in the cellular sample, e.g., to determine the ratio of the cancer cells to the endothelial cells. Step b) may include detecting a mutation in DNA or RNA in the first output sample, e.g., in a gene encoding a polypeptide listed in Table 1. Step b) may also include analyzing protein phosphorylation, protein glycosylation, DNA methylation, microRNA levels, or cell morphology in the first output sample. Step b) may also include detecting mitochondrial DNA, telomerase, or a nuclear matrix protein in the first output sample; detecting one or more mitochondrial abnormalities in the first output sample; detecting the presence or absence of perinuclear compartments in a cell of the first output sample; or performing gene expression analysis, in-cell PCR, or fluorescence in-situ hybridization of the first output sample. Gene expression analysis may be used to determine the tissue or tissues of origin of the cancer cells and may be performed on a single cancer cell.

The cellular sample can include one or more progenitor endothelial cells, wherein at least one progenitor endothelial cell is in the first output sample.

The device may further include a continuous flow device having a first inlet, a first outlet, and a second outlet, wherein the cellular sample is applied to the first inlet, the first output sample flows out of the first outlet, and the second output sample flows out of the second outlet. The device may further include a second inlet, and wherein a second fluid, e.g., a buffer, a lysis reagent, a nucleic acid amplification reagent, an osmolarity regulating reagent, a labeling reagent, a preservative, or a fixing reagent, is applied to the second inlet.

The detecting may include hyperspectral imaging of the first output sample. Prior to or concurrently with step a), the cellular sample may also contacted with a labeling reagent that preferentially labels the cancer cells. The labeling reagent may include beads, wherein the hydrodynamic size of a labeled cancer cell is at least 10% greater than the hydrodynamic size of the cancer cell in the absence of the label.

The invention further features a method of detecting cancer cells in a cellular sample by:

a) enriching one or more of the cancer cells from the cellular sample without using magnetic particles or without using an antibody or fragment thereof, wherein the enriching is based on cell size, shape, or deformability; and
b) determining the number of the enriched cancer cells.

The method may further include:

i) enriching one or more endothelial cells from the cellular sample; and
ii) determining the number of the enriched endothelial cells, e.g., to determine the ratio of the cancer cells to the endothelial cells.

Step b) includes, for example, counting the enriched cancer cells or determining the total amount of DNA in the enriched cancer cells. Steps a) and b) can be repeated with a second cellular sample, e.g., taken from the same subject as the first.

The invention also features a method for diagnosing a condition in a subject by:

a) introducing a cellular sample from the subject into a device including a channel having a structure that directs one or more cancer cells in a first direction to produce a first output sample enriched in the cancer cells and one or more second cells in a second direction to produce a second output sample enriched in the second cells;
b) detecting the presence or absence of the cancer cells in the first output sample; and
c) diagnosing the presence or absence of the condition based on the results of step b).

The method can further include imaging, e.g., by computed axial tomography, positron emission tomography, or magnetic resonance imaging, a portion of the subject prior to the diagnosis, wherein the diagnosis is further based on the results of the imaging.

The method may also include:

i) determining the number of endothelial cells, e.g., progenitor endothelial cells, in the cellular sample; and
ii) determining the ratio of the cancer cells to the endothelial cells, wherein the diagnosis is further based on the ratio.

The condition is, for example, a hematological condition, an inflammatory condition, an ischemic condition, a neoplastic condition, infection, trauma, endometriosis, or kidney failure.

The invention further features a method for diagnosing a condition in a subject; the method includes the steps of:

a) introducing a cellular sample from the subject into a device including a channel having a structure that directs one or more first cells in a first direction to produce a first output sample enriched in the first cells and one or more second cells in a second direction to produce a second output sample enriched in the second cells;
b) analyzing the first output sample; and
c) diagnosing the presence or absence of the condition based on the results of step b).

The channel can include an array of obstacles forming a network of gaps, configured such that fluid flows through said gaps such that said fluid is divided unequally into a major flux and a minor flux.

The device can be configured to direct cells having a hydrodynamic size greater than 12 microns, preferably greater than 14 microns, in the first direction, and cells having a hydrodynamic size less than or equal to 12 microns in the second direction.

The device can also be configured to direct cells having a hydrodynamic size greater than or equal to 5 microns and less than or equal to 10 microns in the first direction, and cells having a hydrodynamic size greater than 10 microns in the second direction.

The device can also be configured to direct cells having a hydrodynamic size greater than or equal to 4 microns and less than or equal to 8 microns in the first direction, and cells having a hydrodynamic size greater than 8 microns in the second direction.

In a preferred embodiment, the first output sample makes up at least 90% of the first cells in said cellular sample.

In some embodiments, steps a) through c) are repeated one or more times with additional cellular samples from the subject, and cellular samples are obtained at regular intervals such as one day, two days, three days, one week, two weeks, one month, two months, three months, six months, or one year.

In other embodiments, the first output sample is enriched in the first cells relative to the cellular sample by a factor of at least 1,000.

The condition that is diagnosed can be a hematological condition, an inflammatory condition, an ischemic condition, a neoplastic condition, infection, trauma, endometriosis, or kidney failure. The neoplastic condition can be selected from the group consisting of acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, or Wilm's tumor.

In some embodiments, the cellular sample is less than 50 mL in volume.

For the diagnosis of cancer, step b) can involve detecting the presence or absence of a cancer biomarker in the first output sample; the cancer biomarker is a polypeptide selected from Table 1, or a nucleic acid encoding the polypeptide. Cancer-associated nucleic acid biomarkers can be genomic DNA, mRNA, or microRNA. Step b) of the method can also involve analyzing the expression pattern of a nucleic acid associated with cancer; the nucleic acid can be genomic DNA, mRNA, or microRNA. The cancer-associated nucleic acid can be one that contains a mutation and encodes a polypeptide selected from Table 1, e.g., EpCAM, E-Cadherin, Mucin-1, Cytokeratin 8, EGFR, or leukocyte associated receptor (LAR).

In the method, step b) can further involve contacting the first output sample with a device having a surface with one or more binding moieties that selectively bind one or more cells from the first output sample, such as epithelial or neoplastic cells that are associated with acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, or Wilm's tumor.

In some embodiments, the cancer is not thyroid cancer.

The binding moieties can be a polypeptide such as an antibody (e.g., monoclonal) or fragment thereof that binds to, e.g., EpCAM.

In other embodiments:

The structure includes an array of obstacles that form a network of gaps, and the obstacles are capable of selectively capturing said first cells; and

The gaps between the obstacles are more than 15 microns, more than 20 microns, or less than 60 microns.

The cellular sample can be blood, sweat, tears, ear flow, sputum, lymph, bone marrow suspension, urine, saliva, semen, vaginal flow, cerebrospinal fluid, brain fluid, ascites, milk, secretions of the respiratory, intestinal or genitourinary tract, amniotic fluid, or a water sample.

In other embodiments:

Step b) involves analyzing the size distribution of the first output sample; and
Step b) comprises determining the number of the first cells.

The invention also features a method for identifying a cell pattern associated with a condition of interest; the method includes the steps of:

a) obtaining a cellular sample from each of a plurality of control subjects and a plurality of case subjects having the condition of interest;
b) enriching by size, from each cellular sample, cells having a hydrodynamic size greater than 12 microns;
c) analyzing cells enriched in step b); and

d) performing an association study using the results obtained in step c).

The invention also features a method for diagnosing a condition in a subject; the method includes the steps of:

a) providing a cell pattern associated with the condition;
b) obtaining a cellular sample from the subject;
c) enriching by size, from the cellular sample, cells having a hydrodynamic size greater than 12 microns;
d) analyzing cells enriched in step c); and
e) diagnosing the presence or absence of the condition in the subject based on the cell pattern of step a) together with the analysis of step d).

In this method step c) involves detecting RNA levels in the cells enriched in step b), such as mRNA or microRNA.
Preferably the method employs at least 50 case subjects and at least 50 control subjects.
In one embodiment of the method, step c) involves determining the number of cells enriched in step b). This can be accomplished using a cellular characteristic such as impedance, light absorption, light scattering, or capacitance.
In another embodiment, step c) involves analyzing the size distribution of the cells enriched in step b). This can be accomplished using a microscope, a cell counter, a magnet, a biocavity laser, a mass spectrometer, a PCR device, an RT-PCR device, a matrix, a microarray, or a hyperspectral imaging system in order to determine size distribution.
The cellular sample can be blood, sweat, tears, ear flow, sputum, lymph, bone marrow suspension, urine, saliva, semen, vaginal flow, cerebrospinal fluid, brain fluid, ascites, milk, secretions of the respiratory, intestinal or genitourinary tract, amniotic fluid, or a water sample.
In another embodiment, step c) involves determining the tissue or tissues of origin of cells enriched in step b).
In other embodiments, step c) involves identifying, from the cells enriched in step b), one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.
In other embodiments, step e) involves contacting the cells enriched in step b) with one or more binding moieties that selectively bind the first cells, e.g. a polypeptide such as an antibody (e.g., monoclonal) or fragment thereof, e.g., anti-Ber-Ep4, anti-EpCAM, anti-E-Cadherin, anti-Mucin-1, anti-Cytokeratin 8, or anti-CD34+.
In another embodiment, the device is configured to direct the cells having a hydrodynamic size greater than 12 microns in a first direction, and cells having a hydrodynamic size less than or equal to 12 microns in a second direction.
In another embodiment, the device selectively captures the cells enriched in step b).
The invention also features a method for determining the efficacy of a drug treatment administered to a subject; the method includes the steps of:

a) obtaining a first cellular sample from the subject before the treatment;
b) introducing the first cellular sample into a device including a channel having a structure that directs one or more first cells in a first direction to produce a first output sample enriched in the first cells and one or more second cells in a second direction to produce a second output sample enriched in the second cells;
c) analyzing the first output sample;
d) obtaining a second cellular sample from the subject concurrently with or subsequent to the drug treatment;
e) repeating steps b) and c) for the second cellular sample; and
f) comparing the results of step c) for the first cellular sample and the second cellular sample,

wherein the comparison determines efficacy of the drug treatment.
In some embodiments, the device is configured to direct cells having a hydrodynamic size greater than 12 microns in the first direction, and cells having a hydrodynamic size less than or equal to 12 microns in the second direction.
In some embodiments the device is configured to direct cells having a hydrodynamic size greater than or equal to 6 microns and less than or equal to 12 microns in the first direction, and cells having a hydrodynamic size less than 6 microns or cells having a hydrodynamic size greater than 12 microns in the second direction.
Step c) can involve detecting RNA levels in the first cells from the first cellular sample or the second cellular sample; the RNA can be mRNA or microRNA.
Step c) can also involve determining the number of first cells from the first cellular sample or the second cellular sample.
Step d) can involve identifying, from the first cells from the first cellular sample or the second cellular sample, one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.
The invention also features a method of determining the efficacy of a therapy for a condition in a patient by performing an enrichment step by size of cells from a body fluid sample from the patient by directing cells having a hydrodynamic size greater than a predetermined size in a direction different than other smaller components in the sample, and performing an evaluation of the cells greater than the predetermined size as an indication of the efficacy of the therapy. The condition is, for example, an inflammatory condition, an ischemic condition, an infection, a trauma, endometriosis, or a neoplastic condition, such as lung, breast or prostate cancer. The body fluid is, for example, blood, sweat, tears, lymph, ear flow, sputum, bone marrow suspension, urine, saliva, semen, vaginal flow, cerebrospinal fluid, brain fluid, ascites, milk, secretions of the respiratory, intestinal, and urogenital tracts, amniotic fluid, or a body tissue extract. The evaluation may include identifying the number and/or type of the cells or determining the total amount of DNA in the cells. Alternatively, the evaluation is selected from the group consisting of determining the number of endothelial cells, determining the ratio of cancer cells to endothelial cells, detecting a mutation in DNA or RNA, analyzing SNPs, determining the morphology of the cells, detecting a marker of the cells (e.g., by reacting the cells with a specific binding agent which is specific for the marker), and detecting the activity of the cells. The evaluation may also include identifying a pattern of the cells. The method may further include multiple enrichment and evaluation steps, wherein the results of the evaluation steps are compared as an indication of the efficacy of the therapy. The multiple enrichment and evaluation steps are typically separated by an interval of time, e.g., daily, weekly, biweekly, bimonthly, monthly, quarterly, biyearly, or yearly. The method may further include providing therapy to the patient during at least one the interval of time. The enrichment step is preferably performed with a device, e.g., a microfluidic device, including an array of obstacles.

The invention also features a method of screening for therapeutic agents for a condition in a patient by performing an enrichment step by size of cells from a body fluid sample from the patient by directing cells, e.g., cancer cells, having a hydrodynamic size greater than a predetermined size in a direction different than other smaller components in the sample, performing a first evaluation of the cells greater than the predetermined size, contacting the cells with at least one therapeutic agent, performing a second evaluation of the cells, and comparing the first and second evaluations of the cells as a screen for a therapeutic agent. The cells may be cultured prior to contacting with the therapeutic agent. The method may also include contacting separate samples of the enriched cells with different therapeutic agents and comparing the evaluations of the cells after the contacting. The enrichment step is preferably performed with a device comprising an array of obstacles. The method may further include treating the patient with the most effective therapeutic agent identified in the screening. The evaluation may include identifying the number of cells or a pattern of the cells. Desirably, some of the cells are preserved. Some of the preserved cells may then be contacted with a therapeutic agent and evaluated as an indication of the efficacy of the therapeutic agent. The condition is, for example, an inflammatory condition, an ischemic condition, an infection, a trauma, endometriosis, or a neoplastic condition, such as lung, breast or prostate cancer. The body fluid is, for example, blood, sweat, tears, lymph, ear flow, sputum, bone marrow suspension, urine, saliva, semen, vaginal flow, cerebrospinal fluid, brain fluid, ascites, milk, secretions of the respiratory, intestinal, and urogenital tracts, amniotic fluid, or a body tissue extract.

The invention also features a method of determining an effective therapeutic agent for a condition by performing an enrichment step by size of cells from a body tissue sample by directing cells having a hydrodynamic size greater than a predetermined size in a direction different than other smaller components in the sample, collecting cells of the predetermined size, evaluating characteristics of the cells, preserving the cells in an archive, and identifying an effective therapeutic agent for the condition from the characteristics of the cells. The condition is, for example, an inflammatory condition, an ischemic condition, an infection, a trauma, endometriosis, or a neoplastic condition, such as lung, breast or prostate cancer. The method may further include analyzing the cells for genotype or SNP determination.

The invention further features a device for processing a cellular sample; the device includes:

a) a channel including a structure that directs one or more first cells in a first direction to produce a first output sample enriched in the first cells and one or more second cells in a second direction to produce a second output sample enriched in the second cells, wherein the device is configured either:

i) to direct cells having a hydrodynamic size greater than 12 microns in the first direction, and cells having a hydrodynamic size less than or equal to 12 microns in the second direction; or

ii) to direct cells having a hydrodynamic size greater than or equal to 6 microns and less than or equal to 12 microns in the first direction, and cells having a hydrodynamic size less than 6 microns or cells having a hydrodynamic size greater than 12 microns in the second direction; and

b) a detection module for analyzing the first output sample or the second output sample, wherein the detection module is fluidically coupled to the channel.

The channel can include an array of obstacles forming a network of gaps, wherein fluid flows through the gaps such that the fluid is divided unequally into a major flux and a minor flux.

The device can be configured to direct cells having a hydrodynamic size greater than or equal to 6 microns and less than or equal to 12 microns in the first direction, and cells having a hydrodynamic size less than 6 microns or cells having a hydrodynamic size greater than 12 microns in the second direction.

Alternatively, the device can be configured to direct cells having a hydrodynamic size greater than or equal to 8 microns and less than or equal to 10 microns in the direction, and cells having a hydrodynamic size less than 8 microns or cells having a hydrodynamic size greater than 10 microns in the second direction.

The detection module of the device can be adapted to identify a marker associated with cancer in the first cells. The detection module can include an antibody that specifically binds the first cells, e.g., an antibody that specifically binds one or more markers selected from Table 1. The detection module can be configured to detect one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

The detection module can include a microscope, a cell counter, a magnet, a biocavity laser, a mass spectrometer, a PCR device, an RT-PCR device, a matrix, a microarray, or a hyperspectral imaging system.

In another aspect, the invention features a device for processing a cellular sample; the device includes:

a) a channel including a structure that directs one or more cancer cells in a first direction to produce a first output sample enriched in the cancer cells and one or more second cells in a second direction to produce a second output sample enriched in the second cells; and

b) a capture module for capturing cancer cells or the second cells, wherein the capture module is fluidically coupled to the channel, and wherein the capture module includes one or more binding moieties that selectively bind cancer cells or second cells.

The structure can include an array of obstacles that form a network of gaps. The binding moieties can be ones that specifically bind one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens, and the obstacles can include the binding moieties. The device can be configured to direct cells having a hydrodynamic size greater than 12, 14, or 16 microns in the first direction, and can further include a cell counting module fluidically coupled to the capture module. The binding moieties can include a polypeptide such as an antibody (which can be monoclonal), e.g., one which binds to EpCAM.

In another aspect, the invention features a device for processing a cellular sample; the device includes a channel having a structure that directs one or more first cells in a first direction to produce a first output sample enriched in the first cells and one or more second cells in a second direction to produce a second output sample enriched in the second cells, wherein the structure includes an array of obstacles that form a network of gaps, and wherein at least some of the obstacles include monoclonal anti-EpCAM antibodies or fragments thereof that selectively bind first cells or second cells.

In another aspect, the invention features a device for processing a cellular sample; the device includes:

a) an enrichment module that is capable of enriching cells in the cellular sample based on size; and

b) a cell counting module for determining the number of cells enriched by the enrichment module, wherein the cell counting module is fluidically coupled to the enrichment module.

The enrichment module can include a channel including a structure that directs one or more first cells (e.g., cancer cells) in a first direction to produce a first output sample enriched in the first cells and one or more second cells in a second direction to produce a second output sample enriched in the second cells.

The device can be configured to direct cells having a hydrodynamic size greater than 12 microns in the first direction, and cells having a hydrodynamic size less than or equal to 12 microns in the second direction. Alternatively, the device can be configured to direct cells having a hydrodynamic size greater than or equal to 6 microns and less than or equal to 12 microns in the first direction, and cells having a hydrodynamic size less than 6 microns or cells having a hydrodynamic size greater than 12 microns in the second direction. The structure of the device can include an array of obstacles that form a network of gaps. The cell counting module can utilize impedance, optics, or capacitance to determine the number of cells in the first output sample or the second output sample.

The device can further include a detector adapted to visualize the first output sample or the second output sample; the detector is fluidically coupled to the capture module.

The invention further features a device for processing a cellular sample; the device includes a channel including a structure that directs one or more first cells in a first direction to produce a first output sample enriched in the first cells and one or more second cells in a second direction to produce a second output sample enriched in the second cells, wherein the device is capable of processing at least 20 mL, and preferably at least 50 mL of fluid per hour.

The structure can include an array of obstacles that form a network of gaps, which can be between 20 and 100 microns in size.

The channel can include an array of obstacles forming a network of gaps, so that fluid flows through the gaps such that the fluid is divided unequally into a major flux and a minor flux.

The array of obstacles can be a staggered two-dimensional array of obstacles, or the array can include a plurality of rows, each successive row being offset by less than half of the period of the previous row. The device can further include one or more additional arrays of obstacles in series or in parallel with the first array of obstacles.

The first cells can have a larger average hydrodynamic size than the second cells.

The cellular sample can be blood or a fraction thereof.

The device can be configured to direct cells having a hydrodynamic size greater than 12 microns, 14 microns, or 16 microns in the first direction.

The device can include a continuous flow device having a first inlet, a first outlet, and a second outlet, wherein the cellular sample is applied to the first inlet, the first output sample flows out of the first outlet, and the second output sample flows out of the second outlet.

The device can be capable of producing a first output sample enriched in the first cells, wherein the volume of the first output sample is smaller than the volume of the cellular sample.

The device can be configured such that the first output sample includes at least 80% of the first cells in the cellular sample, and such that the second output sample includes less than 20% of the first cells in the cellular sample.

When the device provides continuous flow, it can include a second inlet, to which a second fluid is applied.

The first cells can be epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

The device can further include a detector module fluidically coupled to the channel; the detector module can include a microscope, a cell counter, a magnet, a biocavity laser, a mass spectrometer, a PCR device, an RT-PCR device, a matrix, a microarray, or a hyperspectral imaging system, and it can detect a label that selectively binds the first cells.

The device can be adapted for implantation in a subject, e.g., in or near the circulatory system of a subject.

In another aspect, the invention features a system that is capable of being fluidically coupled to the circulatory system of a subject; the system includes a device for processing a cellular sample and includes a channel having a structure that directs one or more first cells in a first direction to produce a first output sample enriched in the first cells and one or more second cells in a second direction to produce a second output sample enriched in the second cells. The system can be fluidically coupled to the circulatory system through tubing or an arteriovenous shunt, and can be capable of removing one or more analytes from the circulatory system. The system can be adapted for continuous blood flow through the device and can be disposable.

In another aspect, the invention features a method for depleting an analyte from a cellular sample; the method includes introducing the cellular sample into a device for processing a cellular sample; the device includes a channel having a structure that directs one or more first cells in a first direction to produce a first output sample enriched in the first cells and one or more second cells in a second direction to produce a second output sample enriched in the second cells, wherein the first output sample or the second output sample is depleted in the analyte relative to the cellular sample. The cellular sample can be blood, sweat, tears, ear flow, sputum, lymph, bone marrow suspension, urine, saliva, semen, vaginal flow, cerebrospinal fluid, brain fluid, ascites, milk, secretions of the respiratory, intestinal, or genitourinary tract, amniotic fluid, or a water sample.

The cellular sample can be taken from a subject afflicted with a hematological condition, an inflammatory condition, an ischemic condition, a neoplastic condition, infection, trauma, endometriosis, or kidney failure. The neoplastic condition can be acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, or Wilm's tumor.

The invention also features a method for diagnosing a condition in a subject; the method includes the steps of:

a) introducing a cellular sample from the subject into a device for processing a cellular sample, which includes a channel having a structure that directs one or more first cells in a first direction to produce a first output sample enriched in the first cells and one or more second cells in a second direction to produce a second output sample enriched in the second cells, wherein the device is capable of processing at least 20 mL of fluid per hour;

b) analyzing the first output sample; and

c) diagnosing the presence or absence of the condition based on the results of step b).

Step b) can involve analyzing the cells of the first output sample for one or more of the characteristics of adhesion, migration, binding, morphology, division, level of gene expression, or presence of a somatic mutation.

Alternatively, step b) can involve detecting the presence or absence of one or more markers selected from Table 1, detecting the presence or absence of a mutation in a nucleic acid that encodes one or more markers selected from Table 1, detecting the presence or absence of a deletion in a nucleic acid that encodes one or more markers selected from Table 1, detecting the level of expression of one or more markers selected from Table 1, or detecting the level of microRNA in the first output sample.

Alternatively, step b) can involve determining the number of the first cells in the first output sample.

The method can be used to detect a hematological condition, an inflammatory condition, an ischemic condition, a neoplastic condition, infection, trauma, endometriosis, or kidney failure.

The invention further features a device for processing a cellular sample. The device includes a channel having an array of obstacles forming a network of gaps, wherein fluid flows through the gaps such that the fluid is divided unequally into a major flux and a minor flux, and wherein the array is configured to direct epithelial cells in a direction not parallel to the average direction of flow in the array.

In preferred embodiments of the device:

The device further includes two inlets; the device further includes two outlets; two such devices are fluidically coupled; the gaps are sized to direct epithelial cells to one of the outlets; the gaps are sized to direct epithelial cells from the fluid flowing into one inlet to the fluid flowing into another inlet; and the obstacles are composed of a polymer, silicon, glass, or fused silica.

The device can be used to produce an enriched sample of epithelial cells, e.g., cancer cells such as cancer stem cells, by introducing a cellular sample into the device so that, as the cellular sample flows through the device, epithelial cells present in the cellular sample are directed in a direction not parallel to the average flow direction of the cellular sample and another component of the cellular sample is directed along the average flow direction, thereby producing a sample enriched in epithelial cells relative to said other component.

The component can include red blood cells, platelets, leukocytes, or endothelial cells, which can range from 8 $\mu$m to 30 $\mu$m in diameter. The epithelial cells can be capable of undergoing DNA analysis, RNA analysis, protein analysis, or metabolome analysis; one analytical method is in-cell PCR. Cells can also be analyzed by determining the level of cytokeratin mRNA present.

In practicing the method, the volume of the enriched sample can be substantially smaller than the volume of the cellular sample, resulting in concentration of epithelial cells.

A lysis buffer can be co-introduced into the device, and the enriched sample can contain components of epithelial cells. Also, an exchange buffer can be co-introduced into the device, and the enriched sample can contain exchange buffer.

The method can further include contacting the enriched sample with a labeling reagent that preferentially labels epithelial cells.

The method can also include quantifying the number of cells in the enriched sample, e.g., in a cell volume greater than 500 fL in the enriched sample.

In other embodiments of the method: the device determines the number of cells of diameter greater than 14 $\mu$m in said enriched sample; the shear stress on each of the cells inside the device is below 10 dynes per square centimeter at all times; and the cellular sample is contacted with a labeling reagent that preferentially labels epithelial cells, wherein the labeling reagent is first combined with the cellular sample either prior to introduction of the cellular sample to the device or following introduction of the cellular sample to the device; the labeling reagent can be a particulate labeling reagent, and labeled cells can be quantified. The labeling reagent can be quantum dots, and the labeled cells can be analyzed using 2-photon excitation. The method can further include making a bulk measurement of the enriched sample. The labeling reagent bound to cells can be separated from labeling reagent that remains unbound to said cells. The labeling reagent can be co-introduced into the device, resulting in labeling of cells within the device.

The labeling reagent can include a quantum dot, an antibody, a phage, an aptamer, a fluorophore, an enzyme, or a bead which can include, e.g., an affinity reagent or polystyrene, can be neutrally buoyant, or can include an antibody. The labeling reagent can increase the size of said cells, e.g., by at least 10%, by at least 100%, or by at least 1000%.

The device can also be used to quantify epithelial cells by:

a. providing a cellular sample;

b. introducing the cellular sample into the device to produce a sample enriched in epithelial cells; and

c. quantifying the epithelial cells.

The device can also be used in a diagnostic method including the steps of:

a. introducing a cellular sample from a patient into the device to produce a sample enriched in cells larger than normal blood cells; and
b. determining the number of cells larger than normal blood cells present in the sample to determine a disease state.

An alternative diagnostic method includes the steps of:

a. introducing a cellular sample from a patient into the device to produce a sample enriched in cells larger than normal blood cells; and
b. performing DNA analysis, RNA analysis, proteome analysis, or metabolome analysis on the enriched sample in order to determine a disease state.

The DNA analysis can involve determining the presence and identity, or the absence, of one or more mutations in the epidermal growth factor receptor gene; this method can include the steps of:

i. isolating genomic DNA from the enriched sample; and
ii. amplifying one or more of exons 18,19, 20, and 21 of the epidermal growth factor receptor gene; this method can further include the steps of:
iii. amplifying subregions of the amplification products corresponding to known epidermal growth factor receptor mutations, resulting in further amplification products;
iv. sequencing the further amplification products; and
v. comparing the resulting sequences to the wild-type sequence of the epidermal growth factor receptor gene and a list of known mutations of the epidermal growth factor receptor gene.

In another aspect, the invention features a device for processing a cellular sample. The device includes:

a. a channel having an array of obstacles forming a network of gaps, wherein fluid flows through the gaps such that the fluid is divided unequally into a major flux and a minor flux so that the average direction of movement of cells larger than a critical size is not parallel to the average direction of fluidic flow;
b. an outlet disposed to collect cells larger than normal blood cells; and
c. a cell detector that counts cells.

The invention further features a device for processing a cellular sample; the device includes a channel including a ceiling (which can be transparent) positioned over an array of obstacles that form a network of gaps; the device is configured to allow cells having a diameter of less than or equal to a critical size to flow through the network of gaps, and to capture cells having a diameter of greater than said critical size between the ceiling and the obstacles.
The critical size can be between 5 and 20 microns, e.g., 12 or 14 microns.
The cells having a diameter greater than said critical size can include one or more rare cells such as epithelial cells, cancer cells, bone marrow cells, fetal cells, progenitor cells, stem cells, foam cells, mesenchymal cells; immune system cells, endothelial cells, endometrial cells, connective tissue cells, trophoblasts, bacteria, fungi, or pathogens. The device can be used to detect the presence or absence of cells having a diameter of greater than the critical size between the ceiling and the obstacles. When the ceiling is transparent, detection can be achieved using a microscope.
In some methods of using the device, the method can include introducing a buffer, a lysis reagent, a nucleic acid amplification reagent, an osmolarity regulating reagent, a labeling reagent, a preservative, or a fixing reagent into the device subsequent to introducing the cellular sample. The osmolarity regulating reagent can include a hypertonic solution which causes the cells having a diameter of greater than the critical size between the ceiling and the obstacles to be released.
The invention also features a business method of identifying therapeutic agents for the treatment of a condition in a patient by, in exchange for a fee, performing an enrichment step by size of cells from a body fluid sample from the patient by directing cells having a hydrodynamic size greater than a predetermined size in a direction different than other smaller components in the sample, performing an evaluation of the cells greater than the predetermined size as a screen for a therapeutic agent. The method is, for example, performed in a CLIA lab, e.g., that is licensed to perform the method steps. The method may further include multiple enrichment and evaluations steps. The cells may be contacted with the therapeutic agent between at least one enrichment and evaluation step. The cells may be cultured prior to the evaluation step. At least some of the cells greater than the predetermined size may also be stored.

The invention further features a business method of determining the efficacy of the therapeutic treatment of a patient with a condition by, in exchange for a fee, performing an enrichment step by size of cells from a body fluid sample from the patient by directing cells having a hydrodynamic size greater than a predetermined size in a direction different than other smaller components in the sample, and evaluating the cells greater than the predetermined size as an indication of the efficacy of the therapeutic treatment. The method may also include multiple enrichment and evaluating steps over the course of the therapeutic treatment. The method is, for example, performed in a CLIA lab, e.g., that is licensed to perform the method steps. The CLIA lab may also be contracted by a health care provider to perform the method. The method may further include the CLIA lab providing a report to the health care provider setting forth the results. The cells are, for example, contacted with a therapeutic agent between at least one enrichment and evaluation step.

The invention also features a business method of determining an effective therapeutic agent for the treatment of a condition in a patient by performing an enrichment step by size of cells from a body fluid sample from the patient by directing cells having a hydrodynamic size greater than a predetermined size in a direction different than other smaller components in the sample, collecting the cells greater than the predetermined size, evaluating characteristics of the cells, storing the cells in an archive, and identifying an effective therapeutic agent for the condition from the characteristics of the cells. The characteristics include categorization of the cells by genotype or SNPs. The characteristics may be correlated to a particular therapeutic agent effective for the condition, e.g., an inflammatory condition, an ischemic condition, an infection, a trauma, endometriosis, or a neoplastic condition (such as lung, breast or prostate cancer). The method may be performed in a CLIA lab, e.g., that is licensed to perform the method. The method may further include providing a report on the effective therapeutic agent for treating the condition, e.g., wherein the report is provided to a health care professional, a laboratory, or a pharmaceutical company.

The invention also features a business method of determining the likelihood of cancer, e.g., breast, prostate, or lung, reoccurring in a patient with cancer by, in exchange for a fee, performing an enrichment step by size of cells from a body tissue sample from the patient by directing cells having a hydrodynamic size greater than a predetermined size in a direction different than other smaller components in the sample, and evaluating the cells greater than the predetermined size as an indication of the likelihood of cancer reoccurrence. The body tissue sample is, for example, blood, bone marrow suspension, a body fluid, or a body tissue extract. The method may include multiple enrichment and evaluating steps, which may be separated by intervals of time. The patient may be treated for the cancer after at least one of the enrichment and evaluating steps. Moreover, the changes in the cells over the multiple evaluating steps are an indication of the likelihood of cancer reoccurrence. The evaluating may include determining the number of the cells or the activity of the cells. The method may also include providing a report about the results of the evaluating, e.g., to a health care professional. The enrichment step is performed, for example, with a device comprising an array of obstacles.

The invention further features a method for determining the likelihood of effectiveness of an epidermal growth factor receptor (EGFR) targeting treatment in a human patient affected with cancer. The method comprises detecting the presence or absence of at least one nucleic acid variant in the kinase domain of the erbB1 gene of the patient relative to the wild type erbB1 gene. The presence of at least one variant is indicative of the effectiveness of an EGFR targeting treatment. Preferably, the nucleic acid variant increases the kinase activity of the EGFR. The patient can then be treated with an EGFR targeting treatment. In one embodiment of the present invention, the EGFR targeting treatment is a tyrosine kinase inhibitor, such as an anilinoquinazoline, which may be a synthetic anilinoquinazoline. Often, the synthetic anilinoquinazoline is either gefitinib or erlotinib.

In another embodiment, the EGFR targeting treatment is an irreversible EGFR inhibitor, including 4-dimethylamino-but-2-enoic acid [4-(3-chloro-4-fluoro phenylamino)-3-cyano-7-ethoxy-quinolin-6-yl]-amide ("EKB-569," sometimes also referred to as "EKI-569"; see, e.g., International Publication No. WO 2005/018677 and Torrance et al., Nature Medicine, vol. 6, No. 9, Sept. 2000, p. 1024) and/or HKI-272 or HKI-357 (Wyeth; see Greenberger et al., Proc. 11th NCI EORTC-AACR Symposium on New Drugs in Cancer Therapy, Clinical Cancer Res. Vol. 6 Supplement, Nov. 2000, ISSN 1078-0432; in Rabindran et al., Cancer Res. 64: 3958-3965 (2004); Holbro and Hynes, Ann. Rev. Pharm. Tox. 44: 195-217 (2004); Tsou et al., J. Med. Chem. 2005, 48, 1107-1131; and Tejpar et al., J. Clin. Oncol. ASCO Annual Meeting Proc. Vol. 22, No. 14S: 3579 (2004)). In one embodiment of the present invention, the EGFR is obtained from a biological sample from a patient with or at risk for developing cancer. The variant in the kinase domain of EGFR (or the erbB1 gene) affects the conformational structure of the ATP-binding pocket. Preferably, the variant in the kinase domain of EGFR is an in frame deletion or a substitution in exon 18, 19, 20, or 21.

In some embodiments, the mutations and the nucleic acid variant of the erbB1 gene is a substitution of a thymine for a guanine or an adenine for a guanine at nucleotide 2155 of SEQ ID NO: 511; a deletion of nucleotides 2235 to 2249, 2240 to 2251, 2240 to 2257, 2236 to 2250, 2254 to 2277, or 2236 to 2244 of SEQ ID NO: 511; an insertion of nucleotides guanine, guanine, and thymine (GGT) after nucleotide 2316 and before nucleotide 2317 of SEQ ID NO: 511; or a substitution of a guanine for a thymine at nucleotide 2573 or an adenine for a thymine at nucleotide 2 5 82 of SEQ ID NO: 511.

In another embodiment, the detection of the presence or absence or at least one variant provides for contacting EGFR nucleic acid containing a variant site with at least one nucleic acid probe. The probe preferentially hybridizes with a nucleic acid sequence including a variant site and containing complementary nucleotide bases at the variant site under selective hybridization conditions. Hybridization can be detected with a detectable label.

In yet another embodiment, the detection of the presence or absence of at least one variant comprises sequencing at least one nucleic acid sequence and comparing the obtained sequence with the known erbB1 nucleic acid sequence.

In a preferred embodiment, the detection of the presence or absence of at least one nucleic acid variant comprises performing a polymerase chain reaction (PCR). The erbB1 nucleic acid sequence containing the hypothetical variant is amplified and the nucleotide sequence of the amplified nucleic acid is determined. Determining the nucleotide sequence of the amplified nucleic acid comprises sequencing at least one nucleic acid segment. Alternatively, amplification products can analyzed by using any method capable of separating the amplification products according to their size, including automated and manual gel electrophoresis and the like.

Alternatively, the detection of the presence or absence of at least one variant comprises determining the haplotype of a plurality of variants in a gene.

In another embodiment, the presence or absence of an EGFR variant can be detected by analyzing the erbB 1 gene product (protein). In this embodiment, a probe that specifically binds to a variant EGFR is utilized. In a preferred embodiment, the probe is an antibody that preferentially binds to a variant EGFR. The presence of a variant EGFR predicts the likelihood of effectiveness of an EGFR targeting treatment. Alternatively, the probe may be an antibody fragment, chimeric antibody, humanized antibody, or aptamer.

The present invention further provides a probe which specifically binds under selective binding conditions to a nucleic acid sequence comprising at least one nucleic acid variant in the EGFR gene (erbB1). In one embodiment, the variant is a mutation in the kinase domain of erbB1 that confers a structural change in the ATP-binding pocket.

The probe of the present invention may comprise a nucleic acid sequence of about 500 nucleotide bases, preferably about 100 nucleotides bases, and most preferably about 50 or about 25 nucleotide bases or fewer in length. The probe may be composed of DNA, RNA, or peptide nucleic acid (PNA). Furthermore, the probe may contain a detectable label, such as, for example, a fluorescent or enzymatic label.

The present invention additionally provides a method to determine the likelihood of effectiveness of an epidermal growth factor receptor (EGFR) targeting treatment in a patient affected with cancer. The method comprises determining the kinase activity of the EGFR in a biological sample from a patient. An increase in kinase activity following stimulation with an EGFR ligand, compared to a normal control, indicates that the EGFR targeting treatment is likely to be effective.

In yet another embodiment, the present invention discloses a method for selecting a compound that inhibits the catalytic kinase activity of a variant epidermal growth factor receptor (EGFR). As a first step, a variant EGFR is contacted with a potential compound. The resultant kinase activity of the variant EGFR is then detected and a compound is selected that inhibits the kinase activity of the variant EGFR. In one embodiment, the variant EGFR is contained within a cell.

The method can also be used to select a compound that inhibits the kinase activity of a variant EGFR having a secondary mutation in the kinase domain that confers resistance to a TKI, e.g., gefitinib or erlotinib.

In another embodiment, a method for predicting the acquisition of secondary mutations (or selecting for mutations) in the kinase domain of the erbB1 gene is disclosed. A cell expressing a variant form of the erbB1 gene is contacted with an effective, yet sub-lethal dose of a tyrosine kinase inhibitor. Cells that are resistant to a growth arrest effect of the tyrosine kinase inhibitor are selected and the erbB1 nucleic acid is analyzed for the presence of additional mutations in the erbB1 kinase domain. In one embodiment, the cell is in vitro. In another embodiment, the cell is obtained from a transgenic animal. In one embodiment, the transgenic animal is a mouse. In this mouse model, cells to be studied are obtained from a tumor biopsy. Cells containing a secondary mutation in the erbB1 kinase domain selected by the present invention can be used in the above methods to select a compound that inhibits the kinase activity of the variant EGFR having a secondary mutation in the kinase domain.

In an alternative embodiment for predicting the acquisition of secondary mutations in the kinase domain of the erbB1 gene, cells expressing a variant form of the erbB1 gene are first contacted with an effective amount of a mutagenizing agent. The mutagenizing is, for example, ethyl methanesulfonate (EMS), N-ethyl-N-nitrosourea (ENU), N-methyl-N-nitrosourea (MNU), phocarbaxine hydrochloride (Prc), methyl methanesulfonate (MeMS), chlorambucil (Chi), melphalan, porcarbazine hydrochloride, cyclophosphamide (Cp), diethyl sulfate (Et2SO4), acrylamide monomer (AA), triethylene melamin (TEM), nitrogen mustard, vincristine, dimethymitrosamine, N-methyl-N'-nitro-Nitrosoguanidine (MNNG), 7,12 dimethylbenz(a)anthracene (DMBA), ethylene oxide, hexamethylphosphoramide, bisulfan, or ethyl methanesulforate (EtMs). The cell is then contacted with an effective, yet sub-lethal dose of a tyrosine kinase inhibitor. Cells that are resistant to a growth arrest effect of the tyrosine kinase inhibitor are selected and the erbB1 nucleic acid is analyzed for the presence of additional mutations in the erbB1 kinase domain.

In one embodiment of the present application, the presence of EGFR mutations can be determined using immunological techniques well known in the art, e.g., antibody techniques such as immunohistochemistry, immunocytochemistry, FACS scanning, immunoblotting, radioimmunoassays, western blotting, immunoprecipitation, enzyme-linked immunosorbent assays (ELISA), and derivative techniques that make use of antibodies directed against activated downstream targets of EGFR. Examples of such targets include, for example, phosphorylated STAT3, phosphorylated STAT5, and phosphorylated Akt. Using phospho-specific antibodies, the activation status of STAT3, STAT5, and Akt can be determined. Activation of STAT3, STAT5, and Akt are useful as a diagnostic indicator of activating EGFR mutations. In one embodiment, the presence of activated (phosphorylated) STAT5, STAT3, or Akt indicates that an EGFR targeting treatment is likely to be effective.

The invention further provides a method of screening for variants in the kinase domain of the erbB1 gene in a test biological sample by immunohistochemical or immunocytochemical methods.

Antibodies, polyclonal or monoclonal, can be purchased from a variety of commercial suppliers, or may be manufactured using well-known methods, e.g., as described in Harlow et al., Antibodies: A Laboratory Manual, 2nd Ed; Cold Spring Harbor Laboratory Press, Cold Spring Harbor, N.Y. (1988). In general, examples of antibodies useful in the present invention include anti-phospho-STAT3, anti-phospho-STAT5, and anti-phospho-Akt antibodies. Such antibodies can be purchased, for example, from Upstate Biotechnology (Lake Placid, NY), New England Biolabs (Beverly, MA), NeoMarkers (Fremont, CA). Biological samples appropriate for such detection assays include, but are not limited to, cells, tissue biopsy, whole blood, plasma, serum, sputum, cerebrospinal fluid, breast aspirates, pleural fluid, urine and the like. For direct labeling techniques, a labeled antibody is utilized. For indirect labeling techniques, the sample is further reacted with a labeled substance. Immunological methods of the present invention are advantageous because they require only small quantities of biological material. Such methods may be done at the cellular level and thereby necessitate a minimum of one cell.

Preferably, several cells are obtained from a patient affected with or at risk for developing cancer and assayed according to the methods of the present invention.

An agent for detecting mutant EGFR protein is an antibody capable of binding to mutant EGFR protein, preferably an antibody with a detectable label. Antibodies can be polyclonal, or more preferably, monoclonal. An intact antibody, or a fragment thereof (e.g., Fab or F(ab)2), can be used. Direct labeling of the probe or antibody may be accomplished by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin.

In another embodiment, the methods further involve obtaining a control biological sample from a control subject, contacting the control sample with a compound or agent capable of detecting mutant EGFR protein, mRNA, or genomic DNA, such that the presence of mutant EGFR protein, mRNA, or genomic DNA is detected in the biological sample, and comparing the presence of mutant EGFR protein, mRNA, or genomic DNA in the control sample with the presence of mutant EGFR protein, mRNA, or genomic DNA in the test sample.

In a different embodiment, the diagnostic assay is for mutant EGFR activity. In a specific embodiment, the mutant EGFR activity is a tyrosine kinase activity. One such diagnostic assay is for detecting EGFR-mediated phosphorylation of at least one EGFR substrate. Levels of EGFR activity can be assayed for, e.g., various mutant EGFR polypeptides, various tissues containing mutant EGFR, biopsies from cancer tissues suspected of having at least one mutant EGFR, and the like.

Comparisons of the levels of EGFR activity in these various cells, tissues, or extracts of the same, can optionally be made. In one embodiment, high levels of EGFR activity in cancerous tissue is diagnostic for cancers that may be susceptible to treatments with one or more tyrosine kinase inhibitor. In related embodiments, EGFR activity levels can be determined between treated and untreated biopsy samples, cell lines, transgenic animals, or extracts from any of these, to determine the effect of a given treatment on mutant EGFR activity as compared to an untreated control.

In one embodiment, the invention provides a method for selecting a treatment for a patient affected by or at risk for developing cancer by determining the presence or absence of at least one kinase activity increasing nucleic acid variance in the kinase domain of the erbB1 gene. In another embodiment, the variant is a plurality of variants, whereby a plurality may include variants from one, two, three or more gene loci.

In certain embodiments, the presence of the at least one variant is indicative that the treatment will be effective or otherwise beneficial (or more likely to be beneficial) in the patient. Stating that the treatment will be effective means that the probability of beneficial therapeutic effect is greater than in a person not having the appropriate presence of the particular kinase activity increasing nucleic acid variant(s) in the kinase domain of the erbB1 gene.

Also included are kits for the detection of the EGFR mutations. These kits include the separation device comprising a channel comprising a structure which directs first cells of one size in a first direction into a first output and second cells in a second direction into a second output. Optionally, the kits include a capture device comprising specific binding moieties which specifically bind the first cells. The kit also comprises degenerate primers to amplify a target nucleic acid in the kinase domain of the erbB1 gene. The kit may alternatively also comprise buffers, enzymes, and containers for performing the amplification and analysis of the amplification products. The kit may also be a component of a screening, diagnostic or prognostic kit comprising other tools such as DNA microarrays. Often, the kit also provides one or more control templates, such as nucleic acids isolated from normal tissue sample, and/or a series of samples representing different variants in the kinase domain of the erbB1 gene.

In one embodiment, the kit provides two or more primer pairs, each pair capable of amplifying a different region of the erbB1 gene (each region a site of potential variance) thereby providing a kit for analysis of expression of several gene variants in a biological sample in one reaction or several parallel reactions.

Any of the devices of the invention may be used together with a set of instructions for the device.

By "approximately equal" in the context of length, size, area, or other measurements is meant equal to within 10%, 5%, 4%, 3%, 2%, or even 1%.

By "biological particle" is meant any species of biological origin that is insoluble in aqueous media. Examples include cells, particulate cell components, viruses, and complexes including proteins, lipids, nucleic acids, and carbohydrates.

By "biological sample" is meant any sample of biological origin or containing, or potentially containing, biological particles. Preferred biological samples are cellular samples.

By "blood component" is meant any component of whole blood, including host red blood cells, white blood cells, platelets, or epithelial cells, in particular, CTCs. Blood components also include the components of plasma, e.g., proteins, lipids, nucleic acids, and carbohydrates, and any other cells that may be present in blood, e.g., because of current or past pregnancy, organ transplant, infection, injury, or disease.

By "cellular sample" is meant a sample containing cells or components thereof. Such samples include naturally occurring fluids (e.g., blood, sweat, tears, ear flow, sputum, lymph, bone marrow suspension, urine, saliva, semen, vaginal flow, cerebrospinal fluid, cervical lavage, brain fluid, ascites, milk, secretions of the respiratory, intestinal or genitourinary tract, amniotic fluid, and water samples) and fluids into which cells have been introduced (e.g., culture media and liquefied tissue samples). The term also includes a lysate.

By "channel" is meant a gap through which fluid may flow. A channel may be a capillary, a conduit, or a strip of hydrophilic pattern on an otherwise hydrophobic surface wherein aqueous fluids are confined.

By "circulating tumor cell" (CTC) is meant a cancer cell that is exfoliated from a solid tumor of a subject and is found in the subject's circulating blood.

By "component" of cell is meant any component of a cell that may be at least partially isolated upon lysis of the cell. Cellular components may be organelles (e.g., nuclei, perinuclear compartments, nuclear membranes, mitochondria, chloroplasts, or cell membranes), polymers or molecular complexes (e.g., lipids, polysaccharides, proteins (membrane, trans-membrane, or cytosolic), nucleic acids (native, therapeutic, or pathogenic), viral particles, or ribosomes), or other molecules (e.g., hormones, ions, cofactors, or drugs).

By "component" of a cellular sample is meant a subset of cells, or components thereof, contained within the sample.

By "density" in reference to an array of obstacles is meant the number of obstacles per unit of area, or alternatively the percentage of volume occupied by such obstacles. Array density is increased either by placing obstacles closer together or by increasing the size of obstacles relative to the gaps between obstacles.

By "enriched sample" is meant a sample containing components that has been processed to increase the relative population of components of interest relative to other components typically present in a sample. For example, samples may be enriched by increasing the relative population of cells of interest by at least 10%, 25%, 50%, 75%, 100% or by a factor of at least 1,000, 10,000, 100,000, 1,000,000, 10,000,000, or even 100,000,000.

The terms "ErbB1," "epidermal growth factor receptor," and "EGFR" are used interchangeably herein and refer to native sequence EGFR as disclosed, for example, in Carpenter et al., Ann. Rev. Biochem. 56:881-914 (1987), including variants thereof (e.g., a deletion mutant EGFR as in Humphrey et al. PNAS (USA) 87:4207-4211 (1990)). ErbBl refers to the gene encoding the EGFR protein product.

By "exchange buffer" in the context of a cellular sample is meant a medium distinct from the medium in which the cellular sample is originally suspended, and into which one or more components of the cellular sample are to be exchanged.

By "flow-extracting boundary" is meant a boundary designed to remove fluid from an array.

By "flow-feeding boundary" is meant a boundary designed to add fluid to an array.

By "gap" is meant an opening through which fluids or particles may flow. For example, a gap may be a capillary, a space between two obstacles wherein fluids may flow, or a hydrophilic pattern on an otherwise hydrophobic surface wherein aqueous fluids are confined. In a preferred embodiment of the invention, the network of gaps is defined by an array of obstacles. In this embodiment, the gaps are the spaces between adjacent obstacles. In a preferred embodiment, the network of gaps is constructed with an array of obstacles on the surface of a substrate.

By "hydrodynamic size" is meant the effective size of a particle when interacting with a flow, obstacles, or other particles. It is used as a general term for particle volume, shape, and deformability in the flow.

By "hyperspectral" in reference to an imaging process or method is meant the acquisition of an image at five or more wavelengths or bands of wavelengths.

By "intracellular activation" is meant activation of second messenger pathways leading to transcription factor activation, or activation of kinases or other metabolic pathways. Intracellular activation through modulation of external cell membrane antigens may also lead to changes in receptor trafficking.

By "labeling reagent" is meant a reagent that is capable of binding to an analyte, being internalized or otherwise absorbed, and being detected, e.g., through shape, morphology, color, fluorescence, luminescence, phosphorescence, absorbance, magnetic properties, or radioactive emission.

By "microfluidic" is meant having at least one dimension of less than 1 mm.

By "microstructure" in reference to a surface is meant the microscopic structure of a surface that includes one or more individual features measuring less than 1 mm in at least one dimension. Exemplary microfeatures are micro-obstacles, micro-posts, micro-grooves, micro-fins, and micro-corrugation.

By "obstacle" is meant an impediment to flow in a channel, e.g., a protrusion from one surface. For example, an obstacle may refer to a post outstanding on a base substrate or a hydrophobic barrier for aqueous fluids. In some embodiments, the obstacle may be partially permeable. For example, an obstacle may be a post made of porous material, wherein the pores allow penetration of an aqueous component but are too small for the particles being separated to enter.

By "shrinking reagent" is meant a reagent that decreases the hydrodynamic size of a particle. Shrinking reagents may act by decreasing the volume, increasing the deformability, or changing the shape of a particle.

By "swelling reagent" is meant a reagent that increases the hydrodynamic size of a particle. Swelling reagents may act by increasing the volume, reducing the deformability, or changing the shape of a particle.

Other features and advantages will be apparent from the following description and the claims.

## BRIEF DESCRIPTION OF THE DRAWINGS

Figures 1A-1E are schematic depictions of an array that separates cells based on lateral displacement: (A) illustrates the lateral displacement of subsequent rows; (B) illustrates how fluid flowing through a gap is divided unequally around obstacles in subsequent rows; (C) illustrates how a particle with a hydrodynamic size above the critical size is displaced laterally in the device; (D) illustrates an array of cylindrical obstacles; and (E) illustrates an array of elliptical obstacles.

Figure 2 is a schematic description illustrating the unequal division of the flux through a gap around obstacles in subsequent rows.

Figure 3 is a schematic depiction of how the critical size depends on the flow profile, which is parabolic in this example.

Figure 4 is an illustration of how shape affects the movement of particles through a device.

Figure 5 is an illustration of how deformability affects the movement of particles through a device.

Figure 6 is a schematic depiction of lateral displacement. Particles having a hydrodynamic size above the critical size move to the edge of the array, while particles having a hydrodynamic size below the critical size pass through the device without lateral displacement.

Figure 7 is a schematic depiction of a three stage device.

Figure 8 is a schematic depiction of the maximum size and cut-off size for the device of Figure 7.

Figure 9 is a schematic depiction of a bypass channel.

Figure 10 is a schematic depiction of a bypass channel.

Figure 11 is a schematic depiction of a three stage device having a common bypass channel.

Figure 12 is a schematic depiction of a three stage, duplex device having a common bypass channel.

Figure 13 is a schematic depiction of a three stage device having a common bypass channel, where the flow through the device is substantially constant.

Figure 14 is a schematic depiction of a three stage, duplex device having a common bypass channel, where the flow through the device is substantially constant.

Figure 15 is a schematic depiction of a three stage device having a common bypass channel, where the fluidic resistance in the bypass channel and the adjacent stage are substantially constant.

Figure 16 is a schematic depiction of a three stage, duplex device having a common bypass channel, where the fluidic resistance in the bypass channel and the adjacent stage are substantially constant.

Figure 17 is a schematic depiction of a three stage device having two, separate bypass channels.

Figure 18 is a schematic depiction of a three stage device having two, separate bypass channels, which are in arbitrary configuration.

Figure 19 is a schematic depiction of a three stage, duplex device having three, separate bypass channels.

Figure 20 is a schematic depiction of a three stage device having two, separate bypass channels, wherein the flow through each stage is substantially constant.

Figure 21 is a schematic depiction of a three stage, duplex device having three, separate bypass channels, wherein the flow through each stage is substantially constant.

Figure 22 is a schematic depiction of a flow-extracting boundary.

Figure 23 is a schematic depiction of a flow-feeding boundary.

Figure 24 is a schematic depiction of a flow-feeding boundary, including a bypass channel.

Figure 25 is a schematic depiction of two flow-feeding boundaries flanking a central bypass channel.

Figure 26 is a schematic depiction of a device having four channels that act as on-chip flow resistors.

Figures 27 and 28 are schematic depictions of the effect of on-chip resistors on the relative width of two fluids flowing in a device.

Figure 29 is a schematic depiction of a duplex device having a common inlet for the two outer regions.

Figure 30A is a schematic depiction of a multiple arrays on a device.

Figure 30B is a schematic depiction of multiple arrays with common inlets and product outlets on a device.

Figure 31 is a schematic depiction of a multi-stage device with a small footprint.

Figure 32 is a schematic depiction of blood passing through a device.

Figure 33A is a graph of cell count versus hydrodynamic size for a microfluidic separation of normal whole blood.

Figure 33B is a graph of cell count versus hydrodynamic size for a microfluidic separation of whole blood including a population of circulating tumor cells (CTCs). Figure 33C is the graph of Figure 33B, additionally showing a size cutoff that excludes most native blood cells. Figure 33D is the graph of Figure 33C, additionally showing a population of cells larger than the size cutoff and indicative of a disease state.

Figures 34A-34D are schematic depictions of moving a particle from a sample to a buffer in a single stage (A), three stage (B), duplex (C), or three stage duplex (D) device.

Figure 35A is a schematic depiction of a two stage device employed to move a particle from blood to a buffer to produce three products. Figure 35B is a schematic graph of the maximum size and cut off size of the two stages. Figure 35C is a schematic graph of the composition of the three products.

Figure 36 is a schematic depiction of a two stage device for alteration, where each stage has a bypass channel.

Figure 37 is a schematic depiction of the use of fluidic channels to connect two stages in a device.

Figure 38 is a schematic depiction of the use of fluidic channels to connect two stages in a device, wherein the two stages are configured as a small footprint array.

Figure 39A is a schematic depiction of a two stage device having a bypass channel that accepts output from both stages. Figure 39B is a schematic graph of the size range of product achievable with this device.

Figure 40 is a schematic depiction of a two stage device for alteration having bypass channels that flank each stage and empty into the same outlet.

Figure 41 is a schematic depiction of a device for the sequential movement and alteration of particles.

Figure 42A is a schematic depiction of a device of the invention and its operation. Figure 42B is an illustration of the device of Figure 42A and a further-schematized representation of this device.

Figures 43A and 43B are schematic depictions of two distinct configurations for joining two devices together. In Figure 43A, a cascade configuration is shown, in which outlet 1 of one device is joined to a sample inlet of a second device. In Figure 43B, a bandpass configuration is shown, in which outlet 2 of one device is joined to a sample inlet of a second device.

Figure 44 is a schematic depiction of an enhanced method of size separation in which target cells are labeled with immunoaffinity beads.

Figure 45 is a schematic depiction of a method for performing size fractionation and for separating free labeling reagents, e.g., antibodies, from bound labeling reagents by using a device of the invention.

Figure 46 is a schematic depiction of a method shown in Figure 45. In this case, non-target cells may copurify with target cells, but these non-target cells do not interfere with quantification of target cells.

Figure 47 is a schematic depiction of a method for enriching large cells from a mixture and producing a concentrated sample of these cells.

Figure 48 is a schematic depiction of a method for lysing cells inside a device of the invention and separating whole cells from organelles and other cellular components.

Figure 49 is a schematic depiction of two devices arrayed in a cascade configuration and used for performing size fractionation and for separating free labeling reagent from bound labeling reagent by using a device of the invention.

Figure 50 is a schematic depiction of two devices arrayed in a cascade configuration and used for performing size fractionation and for separating free labeling reagent from bound labeling reagent by using a device of the invention. In this figure, phage is utilized for binding and detection rather than antibodies.

Figure 51 is a schematic depiction of two devices arrayed in a bandpass configuration.

Figure 52 is a graph of cell count versus hydrodynamic size for a microfluidic separation of normal whole blood.

Figure 53 is a set of histograms from input, product, and waste samples generated with a Coulter "A$^C$-T diff" clinical blood analyzer. The x-axis depicts cell volume in femtomoles.

Figure 54 is a pair of representative micrographs from product and waste streams of fetal blood processed with a cell enrichment module, showing clear separation of nucleated cells and red blood cells.

Figure 55 is a pair of images showing cells fixed on a cell enrichment module with paraformaldehyde and observed by fluorescence microscopy. Target cells are bound to the obstacles and floor of the capture module.

Figure 56 is a schematic depiction of a method of the invention. This method features isolating and counting large cells within a cellular sample, wherein the count is indicative of a patient's disease state, and subsequently further analyzing the large cell subpopulation.

Figure 57A is a design for a preferred embodiment of the invention.

Figure 57B is a table of design parameters corresponding to Figure 57A. Figure 57C is a mask design of a chip of the invention.

Figure 58 is a schematic depiction of a method of detecting epidermal growth factor receptor (EGFR) mutations in CTCs in blood.

Figure 59 is a schematic depiction of a process for generating EGFR sequencing templates. EGFR mRNA is reverse transcribed to make cDNA; next, two PCR amplifications are performed sequentially.

Figure 60 is a schematic depiction of an allele-specific TaqMan 5' Nuclease Real Time PCR assay used to amplify EGFR subregions specific to particular mutations of interest.

Figure 61 is a set of sequencing charts showing the detection of several EGFR mutations (shaded regions) above the background level of fluorescence.

Figure 62A is an image of an agarose gel showing that EpCAM and EGFR are expressed in tumor cells but not in leukocytes. BCKDK is expressed in both types of cells, while CD45 is expressed only in leukocytes. Figure 62B is a graph and table showing a Pharmagene XpressWay™ profile of EGFR mRNA expression. Expression levels are profiled in 72 tissues via quantitative RT-PCR, and > 10,000 copies per cell are detected in almost every tissue profiled except for blood. The table shows quantitation of mRNA for tissues #1-4 from the graph.

Figure 63 is a pair of images of agarose gels showing the results of a two sets of PCR assays. In the first set (left), PCR is performed on EGFR input RNA at various concentrations. In the second set of assays, samples from the first set of PCR reactions are amplified with nested primers.

Figure 64A is an image of an agarose gel showing the results of a set of PCR assays in which NCI-H1975 RNA is mixed with various quantities of peripheral blood mononuclear cell (PBMC) RNA and reverse transcribed prior to PCR. Spurious amplification bands are seen at the highest dilution. Figure 64B is an image of an agarose gel showing the results of a set of PCR assays in which the samples shown in Figure 64A are further amplified using nested primers. No spurious amplification bands are produced, even at the highest dilution.

Figure 65 is an image of an agarose gel showing the results of a set of PCR assays. In the associated experiment, whole blood spiked with H1650 cells was run on two devices of the invention, and cDNA was synthesized from the resulting enriched samples. PCR using EGFR and CD45 primers was performed. Both wild type (138 bp) and mutant (123 bp) EGFR bands are visible in the lanes showing EGFR amplifications.

Figure 66A is a schematic depiction of an array of the invention containing staggered subarrays. Figure 66B is a schematic depiction contrasting a regular array with a staggered array. Figure 66C is a schematic depiction showing the flow and capture of cells in a staggered array. Figure 66D is a schematic depiction showing a device containing an outlet port surrounded by a region of narrowed flow paths. Figure 66E is a schematic depiction of a device that is structured in the depth dimension to create narrowed flow paths. Figure 66F is a schematic depiction of the device of Figure 66E, showing captured cells. Figure 66G is a set of microscope views showing stained H1650 cells captured in the narrow flow regions of a device of the invention.

Figure 67A is a chart and inset showing the size distribution of several cellular samples, including white blood cells and various cancer cell lines, as measured by a Beckman Coulter Z2 counting device. The main chart uses a logarithmic scale for the volume axis, while the inset uses a linear scale to better represent the distribution of white blood cells. Figure 67B is a chart showing the size distribution of several cancer cell lines. Figure 67C is the chart of Figure 67B, further showing three exemplary size cutoffs.

Figure 68A is a schematic depiction of a capture device of the invention that features a functionalized microscope slide on the bottom of a sample chamber. Figure 68B is a schematic depiction of a method of rocking cells in the capture device in order to keep the cells tumbling and prevent sedimentation. Figure 68C is a schematic depiction of a method of rotating the capture device as an alternative to rocking. Figure 68D is a schematic depiction of a capture device that includes two additional fluid chambers, which may be alternately filled and emptied in order to cause fluid motion inside the main chamber of the device. Figure 68E is a schematic depiction of a microscope slide with multiple, spatially patterned capture functionalities on the surface.

Figure 69A is a schematic depiction of a centrifugation device of the invention, shown both at rest and in operation. Figure 69B is a schematic depiction of a cell binding to a functionalized surface in a gravitational field (top) and a centrifugal field (bottom). Figure 69C is a schematic depiction of the device of Figure 69A in which the chambers are inverted during the spin. Figure 69D is a schematic depiction of the device of Figure 69C, further showing a second functionalized surface for the capture of contaminating cells. Figure 69E is a schematic depiction of a centrifugal device in which the functionalized slide is inclined at an angle during the spin. Figure 69F is a pair of charts showing spin speed versus operating time, including periods that may be optimized: "spin up" (1), "spin time" (2), "spin down" (3), and rest time (4). Figure 69G is a schematic depiction of a centrifugal device that includes a functionalized microstructure surface.

Figure 70A is an image of an enrichment device showing the flow paths of a small cell (left) and a large cell (right). The small cell may be seen to have very little interaction with the obstacles and flows essentially in the average flow direction, while the large cell contacts each obstacle along its path and is directed laterally through the array. Figure 70B is a schematic depiction of a device of the invention containing a regular array of obstacles. Figure 70C is a schematic depiction of a device of the invention that includes multiple arrays in which the direction of deflection, the gap size, and/or the distance between obstacles is varied throughout the device, while the critical size is kept constant.

Figure 71 is a listing of wild type epidermal growth factor receptor (EGFR) nucleic acid (SEQ ID NO: 511) and amino acid (SEQ ID NO: 512) sequences.

Figure 72A-E are chromatographic sequencing profiles showing portions of the sequences of EGFR variants.

Figures are not necessarily to scale.

## DETAILED DESCRIPTION OF THE INVENTION

The invention features devices and methods for detecting, enriching, and analyzing circulating tumor cells (CTCs) and other particles. The invention further features methods of diagnosing a condition in a subject, e.g., cancer, by analyzing a cellular sample from the subject. In some embodiments, devices of the invention include arrays of obstacles that allow displacement of CTCs or other fluid components.

While this application focuses primarily on the detection, enrichment, and analysis of CTCs or epithelial cells, the devices and methods of the invention are useful for processing a wide range of other cells and particles, e.g., red blood cells, white blood cells, fetal cells, stem cells (e.g., undifferentiated), bone marrow cells, progenitor cells, foam cells, mesenchymal cells, endothelial cells, endometrial cells, trophoblasts, cancer cells, immune system cells (host or graft), connective tissue cells, bacteria, fungi, cellular pathogens (e.g., bacterial or protozoa), cellular organelles and other cellular components (e.g., mitochondria and nuclei), and viruses.

Exemplary devices and methods of the invention are described in detail below.

### Circulating tumor cells (CTCs)

Epithelial cells that are exfoliated from solid tumors have been found in very low concentrations in the circulation of patients with advanced cancers of the breast, colon, liver, ovary, prostate, and lung, and the presence or relative number of these cells in blood has been correlated with overall prognosis and response to therapy. These CTCs may be an early indicator of tumor expansion or inetastasis before the appearance of clinical symptoms.

CTCs typically have a short half-life of approximately one day, and their presence generally indicates a recent influx from a proliferating tumor. Therefore, CTCs are part of a dynamic process that may reflect the current clinical status of patient disease and therapeutic response. Enumeration and characterization of CTCs, using the devices and methods of the invention, is useful in assessing cancer prognosis and in monitoring therapeutic efficacy for early detection of treatment failure that may lead to disease relapse. In addition, CTC analysis according to the invention enables the detection of early relapse in presymptomatic patients who have completed a course of therapy.

CTCs are generally larger than most blood cells (see, e.g., Fig. 33B). Therefore, one useful approach for analyzing CTCs in blood is to enrich cells based on size, resulting in a cell population enriched in CTCs. This cell population may then be subjected to further processing or analysis. Other methods of enrichment of CTCs are also possible using the invention. Devices and methods for enriching, enumerating, and analyzing CTCs are described below.

### Device

In general, the devices include one or more arrays of obstacles that allow lateral displacement of CTCs and other components of fluids, thereby offering mechanisms of enriching or otherwise processing such components. Prior art devices that differ from those the present invention, but which, like those of the invention, employ obstacles for this purpose, are described, e.g., in Huang et al. Science 304, 987-990 (2004) and U.S. Publication No. 20040144651. The devices of the invention for separating particles according to size typically employ an array of a network of gaps, wherein a fluid passing through a gap is divided unequally into subsequent gaps, even though the gaps may be identical in dimensions. The method uses a flow that carries cells to be separated through the array of gaps. The flow is aligned at a small angle (flow angle) with respect to a line-of-sight of the array. Cells having a hydrodynamic size larger than a critical size migrate along the line-of-sight, i.e., laterally, through the array, whereas those having a hydrodynamic size smaller than the critical size follow the average flow direction. Flow in the device occurs under laminar flow conditions. Devices of the invention are optionally configured as continuous-flow devices.

The critical size is a function of several design parameters. With reference to the obstacle array in Figs. 1A-1C, each row of obstacles is shifted horizontally with respect to the previous row by $\Delta\lambda$, where $\lambda$ is the center-to-center distance between the obstacles (Fig. 1A). The parameter $\Delta\lambda/\lambda$ (the "bifurcation ratio," $\varepsilon$) determines the ratio of flow bifurcated to the left of the next obstacle. In Figs. 1A-1C, $\varepsilon$is 1/3, for the convenience of illustration, In general, if the flux through a gap between two obstacles is $\phi$, the minor flux is $\varepsilon\phi$, and the major flux is $(1-\varepsilon)\phi$ (Fig. 2). In this example, the flux through a gap is divided essentially into thirds (Fig. 1B). While each of the three fluxes through a gap weaves around the array of obstacles, the average direction of each flux is in the overall direction of flow. Fig. 1C illustrates the movement of particles sized above the critical size through the array. Such particles move with the major flux, being transferred sequentially to the major flux passing through each gap.

Referring to Fig. 2, the critical size is approximately $2R_{critical}$, where $R_{critical}$ is the distance between the stagnant flow line and the obstacle. If the center of mass of a particle, e.g., a cell, falls outside $R_{critical}$, the particle would follow the major flux and move laterally through the array. $R_{critical}$ may be determined if the flow profile across the gap is known (Fig. 3); it is the thickness of the layer of fluids that would make up the minor flux. For a given gap size, d, $R_{critical}$ may be tailored based on the bifurcation ratio, $\varepsilon$. In general, the smaller s, the smaller $R_{critical}$.

In an array for lateral displacement, particles of different shapes behave as if they have different sizes (Fig. 4). For example, lymphocytes are spheres of ~5 $\mu$m diameter, and erythrocytes are biconcave disks of ~7 $\mu$m diameter, and ~1.5 $\mu$m thick. The long axis of erythrocytes (diameter) is larger than that of the lymphocytes, but the short axis (thickness) is smaller.If erythrocytes align their long axes to a flow when driven through an array of obstacles by the flow, their hydrodynamic size is effectively their thickness (~1.5 $\mu$m), which is smaller than lymphocytes. When an erythrocyte is driven through an array of obstacles by a hydrodynamic flow, it tends to align its long axis to the flow and behave like a ~1.5 $\mu$m-wide particle, which is effectively "smaller" than lymphocytes. The method and device may therefore separate cells according to their shapes, although the volumes of the cells could be the same. In addition, particles having different deformability behave as if they have different sizes (Fig. 5). For example, two particles having the same unreformed shape may be separated by lateral displacement, as the cell with the greater deformability may deform when it comes into contact with an obstacle in the array and change shape. Thus, separation in the device may be achieved based on any parameter that affects hydrodynamic size including the physical dimensions, the shape, and the deformability of the particle.

Referring to Fig. 6, feeding a mixture of particles, e.g., cells, of different hydrodynamic sizes from the top of the array and collecting the particles at the bottom, as shown schematically, produces two outputs, the product containing cells larger than the critical size, $2R_{critical}$ and waste containing cells smaller than the critical size. Although labeled "waste" in Fig. 6, particles below the critical size may be collected while the particles above the critical size are discarded. Both types of outputs may also be desirably collected, e.g., when fractionating a sample into two or more sub-samples. Cells larger than the gap size will get trapped inside the array. Therefore, an array has a working size range. Cells have to be larger than a cut-off size ($2R_{critical}$) and smaller than a maximum pass-through size (array gap size) to be directed into the major flux. The "size range" of an array is defined as the ratio of maximum pass-through size to cut-off size.

In some cases, the gaps between obstacles are more than 15 microns, more than 20 microns, or less than 60 microns in size. In other cases, the gaps are between 20 and 100 microns in size.

In certain embodiments, a device of the invention may contain obstacles that include binding moieties, e.g., monoclonal anti-EpCAM antibodies or fragments thereof, that selectively bind to particular cell types, e.g., cells of epithelial origin, e.g., tumor cells. All of the obstacles of the device may include these binding moieties; alternatively, only a subset of the obstacles include them. Devices may also include additional modules that are fluidically coupled, e.g., a cell counting module or a detection module. For example, the detection module may be configured to visualize an output sample of the device. In addition, devices of the invention may be configured to direct cells in a selected size range in one direction, and other cells in a second direction. For example, the device may be configured to enrich cells having a hydrodynamic size greater than 12 microns, 14 microns, 16 microns, 18 microns, or even 20 microns from smaller cells in the sample. Alternatively, the device may enrich cells having a hydrodynamic size greater than or equal to 6 microns and less than or equal to 12 microns, e.g., cells having a hydrodynamic size greater than or equal to 8 microns and less than or equal to 10 microns, from other cells. The device may also enrich cells having a hydrodynamic size greater than or equal to 5 microns and less than or equal to 10 microns from cells having a hydrodynamic size greater than 10 microns; alternatively, it may enrich cells having a hydrodynamic size greater than or equal to 4 microns and less than or equal to 8 microns from cells having a hydrodynamic size greater than 8 microns. In general, the device may be configured to separate two groups of cells, where the first group has a larger average hydrodynamic size than the second group.

In some embodiments, devices of the invention may process more than 20 mL of fluid per hour, or even 50 mL of fluid per hour.

As described above, a device of the invention typically contains an array of obstacles that form a network of gaps. For example, such a device may include a staggered two-dimensional array of obstacles, e.g., such that each successive row is offset by less than half of the period of the previous row. The device may also include a second staggered two-dimensional array of obstacles, which is optionally oriented in a different direction than the first array. In this case, the first array may be situated upstream of the second array, and the second array may have a higher density than the first array. Multiple arrays may be configured in this manner, such that each additional array has an equal or higher density than any array upstream of the additional array.

Devices of the invention may be adapted for implantation in a subject. For example, such a device may be adapted for placement in or near the circulatory system of a subject in order to be able to process blood samples. Such devices may be part of an implantable system of the invention that is fluidically coupled to the circulatory system of a subject, e.g., through tubing or an arteriovenous shunt. In some cases, systems of the invention that include implantable devices, e.g., disposable systems, may remove one or more analytes, components, or materials from the circulatory system. These systems may be adapted for continuous blood flow through the device.

**Samples mobilization devices**

The invention additionally encompasses devices for cell enrichment, e.g., enrichment of CTCs, that employ sample mobilization. A sample mobilization device gives rise to movement of cells, or other components of a fluid sample, relative to features, e.g., obstacles, of the device. For example, one device of the invention includes a receptacle that may hold a cellular sample, a detachably attached lid configured to fit within the receptacle that includes a functionalized lid surface including one or more capture moieties that selectively capture cells of interest, and an sample mobilizer coupled to either the receptacle or the lid. Optionally, the receptacle has a functionalized surface including one or more capture moieties that selectively capture a second cell type. The lid surface may have any shape, e.g., square, rectangular, or circular. The device may be manufactured using any materials known in the art, e.g., glass, silicon, or plastic. In some cases, the lid surface or receptacle surface includes a microstructure, e.g., a micro-obstacle, a micro-corrugation, a micro-groove, or a micro-fin. The capture moieties may include one or more antibodies that specifically bind to a particular cell type, and these antibodies may be configured in an array. As with other devices of the invention, the antibodies may specifically bind to any of a wide variety of cells, e.g., leukocytes or epithelial cells. Preferably, the antibodies are able to bind specifically to CTCs. Furthermore, the antibodies may specifically bind a cell surface cancer marker, e.g., EpCAM, E-Cadherin, Mucin-1, Cytokeratin 8, epidermal growth factor receptor (EGFR), and leukocyte associated receptor (LAR), or a marker selected from Table 1. In some cases, the lid of a sample mobilization device may be designed to fit into the receptacle at a nonorthogonal angle with respect to a wall of the receptacle. The receptacle may be designed to hold any desirable amount of sample, e.g., 10 mL or 50 mL.

# Table 1

| |
|---|
| 2AR |
| A DISINTEGRIN |
| ACTIVATOR OF THYROID AND RETINOIC ACID RECEPTOR (ACTR) |
| ADAM 11 |
| ADIPOGENESIS INHIBITORY FACTOR (ADIF) |
| ALPHA 6 INTEGRIN SUBUNIT |
| ALPHA V INTEGRIN SUBUNIT |
| ALPHA-CATENIN |
| AMPLIFIED IN BREAST CANCER 1 (AIB1) |
| AMPLIFIED IN BREAST CANCER 3 (AIB3) |
| AMPLIFIED IN BREAST CANCER 4 (AIB4) |
| AMYLOID PRECURSOR PROTEIN SECRETASE (APPS) |
| AP-2 GAMMA |
| APPS |
| ATP-BINDING CASSETTE TRANSPORTER (ABCT) |
| PLACENTA-SPECIFIC (ABCP) |
| ATP-BINDING CASSETTE SUBFAMILY C MEMBER (ABCC1) |
| BAG-1 |
| BASIGIN (BSG) |
| BCEI |
| B-CELL DIFFERENTIATION FACTOR (BCDF) |
| B-CELL LEUKEMIA 2 (BCL-2) |
| B-CELL STIMULATORY FACTOR-2 (BSF-2) |
| BCL-1 |
| BCL-2-ASSOCIATED X PROTEIN (BAX) |
| BCRP |
| BETA 1 INTEGRIN SUBUNIT |
| BETA 3 INTEGRIN SUBUNIT |
| BETA 5 INTEGRIN SUBUNIT |
| BETA-2 INTERFERON |
| BETA-CATENIN |

| |
|---|
| BETA-CATENIN |
| BONE SIALOPROTEIN (BSP) |
| BREAST CANCER ESTROGEN-INDUCIBLE SEQUENCE (BCEI) |
| BREAST CANCER RESISTANCE PROTEIN (BCRP) |
| BREAST CANCER TYPE 1 (BRCA1) |
| BREAST CANCER TYPE 2 (BRCA2) |
| BREAST CARCINOMA AMPLIFIED SEQUENCE 2 (BCAS2) |
| CADHERIN |
| EPITHELIAL CADHERIN-11 |
| CADHERIN-ASSOCIATED PROTEIN |
| CALCITONIN RECEPTOR (CTR) |
| CALCIUM PLACENTAL PROTEIN (CAPL) |
| CALCYCLIN |
| CALLA |
| CAM5 |
| CAPL |
| CARCINOEMBRYONIC ANTIGEN (CEA) |
| CATENIN |
| ALPHA 1 |
| CATHEPSIN B |
| CATHEPSIN D |
| CATHEPSIN K |
| CATHEPSIN L2 |
| CATHEPSIN O |
| CATHEPSIN O1 |
| CATHEPSIN V |
| CD10 |
| CD146 |
| CD147 |
| CD24 |
| CD29 |
| CD44 |
| CD51 |
| CD54 |
| CD61 |
| CD66e |

| |
|---|
| CD82 |
| CD87 |
| CD9 |
| CEA |
| CELLULAR RETINOL-BINDING PROTEIN 1 (CRBP1) |
| c-ERBB-2 |
| CK7 |
| CK8 |
| CK18 |
| CK19 |
| CK20 |
| CLAUDIN-7 |
| c-MET |
| COLLAGENASE |
| FIBROBLAST |
| COLLAGENASE |
| INTERSTITIAL |
| COLLAGENASE-3 |
| COMMON ACUTE LYMPHOCYTIC LEUKEMIA ANTIGEN (CALLA) |
| CONNEXIN 26 (Cx26) |
| CONNEXIN 43 (Cx43) |
| CORTACTIN |
| COX-2 |
| CTLA-8 |
| CTR |
| CTSD |
| CYCLIN D1 |
| CYCLOOXYGENASE-2 |
| CYTOKERATIN 18 |
| CYTOKERATIN 19 |
| CYTOKERATIN 8 |
| CYTOTOXIC T-LYMPHOCYTE-ASSOCIATED SERINE ESTERASE 8 (CTLA-8) |
| DIFFERENTIATION-INHIBITING ACTIVITY (DIA) |
| DNA AMPLIFIED IN MAMMARY CARCINOMA 1 (DAM1) |
| DNA TOPOISOMERASE II ALPHA |
| DR-NM23 |
| E-CADHERIN |
| EMMPRIN |

| |
|---|
| EMS1 |
| ENDOTHELIAL CELL GROWTH FACTOR (ECGR) |
| PLATELET-DERIVED (PD-ECGF) |
| ENKEPHALINASE |
| EPIDERMAL GROWTH FACTOR RECEPTOR (EGFR) |
| EPISIALIN |
| EPITHELIAL MEMBRANE ANTIGEN (EMA) |
| ER-ALPHA |
| ERBB2 |
| ERBB4 |
| ER-BETA |
| ERF-1 |
| ERYTHROID-POTENTIATING ACTIVITY (EPA) |
| ESR1 |
| ESTROGEN RECEPTOR-ALPHA |
| ESTROGEN RECEPTOR-BETA |
| ETS-1 |
| EXTRACELLULAR MATRIX METALLOPROTEINASE INDUCER (EMMPRIN) |
| FIBRONECTIN RECEPTOR |
| BETA POLYPEPTIDE (FNRB) |
| FIBRONECTIN RECEPTOR BETA SUBUNIT (FNRB) |
| FLK-1 |
| GA15.3 |
| GA733.2 |
| GALECTIN-3 |
| GAMMA-CATENIN |
| GAP JUNCTION PROTEIN (26 kDa) |
| GAP JUNCTION PROTEIN (43 kDa) |
| GAP JUNCTION PROTEIN ALPHA-1 (GJA1) |
| GAP JUNCTION PROTEIN BETA-2 (GJB2) |
| GCP1 |
| GELATINASE A |
| GELATINASE B |

| |
|---|
| GELATINASE (72 kDa) |
| GELATINASE (92 kDa) |
| GLIOSTATIN |
| GLUCOCORTICOID RECEPTOR INTERACTING PROTEIN 1 (GRIP1) |
| GLUTATHIONE S-TRANSFERASE p |
| GM-CSF |
| GRANULOCYTE CHEMOTACTIC PROTEIN 1 (GCP1) |
| GRANULOCYTE-MACROPHAGE-COLONY STIMULATING FACTOR |
| GROWTH FACTOR RECEPTOR BOUND-7 (GRB-7) |
| GSTp |
| HAP |
| HEAT-SHOCK COGNATE PROTEIN 70 (HSC70) |
| HEAT-STABLE ANTIGEN |
| HEPATOCYTE GROWTH FACTOR (HGF) |
| HEPATOCYTE GROWTH FACTOR RECEPTOR (HGFR) |
| HEPATOCYTE-STIMULATING FACTOR III (HSF III) |
| HER-2 |
| HER2/NEU |
| HERMES ANTIGEN |
| HET |
| HHM |
| HUMORAL HYPERCALCEMIA OF MALIGNANCY (HHM) |
| ICERE-1 |
| INT-1 |
| INTERCELLULAR ADHESION MOLECULE-1 (ICAM-1) |
| INTERFERON-GAMMA-INDUCING FACTOR (IGIF) |
| INTERLEUKIN-1 ALPHA (IL-1A) |
| INTERLEUKIN-1 BETA (IL-1B) |
| INTERLEUKIN-11 (IL-11) |
| INTERLEUKIN-17 (IL-17) |

| |
|---|
| INTERLEUKIN-18 (IL-18) |
| INTERLEUKIN-6 (IL-6) |
| INTERLEUKIN-8 (IL-8) |
| INVERSELY CORRELATED WITH ESTROGEN RECEPTOR EXPRESSION-1 (ICERE-1) |
| KAI1 |
| KDR |
| KERATIN 8 |
| KERATIN 18 |
| KERATIN 19 |
| KISS-1 |
| LEUKEMIA INHIBITORY FACTOR (LIF) |
| LIF |
| LOST IN INFLAMMATORY BREAST CANCER (LIBC) |
| LOT ("LOST ON TRANSFORMATION") |
| LYMPHOCYTE HOMING RECEPTOR |
| MACROPHAGE-COLONY STIMULATING FACTOR |
| MAGE-3 |
| MAMMAGLOBIN |
| MASPIN |
| MC56 |
| M-CSF |
| MDC |
| MDNCF |
| MDR |
| MELANOMA CELL ADHESION MOLECULE (MCAM) |
| MEMBRANE METALLOENDOPEPTIDASE (MME) |
| MEMBRANE-ASSOCIATED NEUTRAL ENDOPEPTIDASE (NEP) |
| CYSTEINE-RICH PROTEIN (MDC) |
| METASTASIN (MTS-1) |
| MLN64 |
| MMP1 |
| MMP2 |
| MMP3 |
| MMP7 |
| MMP9 |

| |
|---|
| MMP11 |
| MMP13 |
| MMP14 |
| MMP15 |
| MMP16 |
| MMP17 |
| MOESIN |
| MONOCYTE ARGININE-SERPIN |
| MONOCYTE-DERIVED NEUTROPHIL CHEMOTACTIC FACTOR |
| MONOCYTE-DERIVED PLASMINOGEN ACTIVATOR INHIBITOR |
| MTS-1 |
| MUC-1 |
| MUC18 |
| MUCIN LIKE CANCER ASSOCIATED ANTIGEN (MCA) |
| MUCIN |
| MUC-1 |
| MULTIDRUG RESISTANCE PROTEIN 1 (MDR, MDR1) |
| MULTIDRUG RESISTANCE RELATED PROTEIN-1 (MRP, MRP-1) |
| N-CADHERIN |
| NEP |
| NEU |
| NEUTRAL ENDOPEPTIDASE |
| NEUTROPHIL-ACTIVATING PEPTIDE 1 (NAP1) |
| NM23-H1 |
| NM23-H2 |
| NME1 |
| NME2 |
| NUCLEAR RECEPTOR COACTIVATOR-1 (NCoA-1) |
| NUCLEAR RECEPTOR COACTIVATOR-2 (NCoA-2) |
| NUCLEAR RECEPTOR COACTIVATOR-3 (NCoA-3) |
| NUCLEOSIDE DIPHOSPHATE KINASE A (NDPKA) |
| NUCLEOSIDE DIPHOSPHATE KINASE B (NDPKB) |

| |
|---|
| ONCOSTATIN M (OSM) |
| ORNITHINE DECARBOXYLASE (ODC) |
| OSTEOCLAST DIFFERENTIATION FACTOR (ODF) |
| OSTEOCLAST DIFFERENTIATION FACTOR RECEPTOR (ODFR) |
| OSTEONECTIN (OSN, ON) |
| OSTEOPONTIN (OPN) |
| OXYTOCIN RECEPTOR (OXTR) |
| p27/kip1 |
| p300/CBP COINTEGRATOR ASSOCIATE PROTEIN (p/CIP) |
| p53 |
| p9Ka |
| PAI-1 |
| PAI-2 |
| PARATHYROID ADENOMATOSIS 1 (PRAD1) |
| PARATHYROID HORMONE-LIKE HORMONE (PTHLH) |
| PARATHYROID HORMONE-RELATED PEPTIDE (PTHrP) |
| P-CADHERIN |
| PD-ECGF |
| PDGF-β |
| PEANUT-REACTIVE URINARY MUCIN (PUM) |
| P-GLYCOPROTEIN (P-GP) |
| PGP-1 |
| PHGS-2 |
| PHS-2 |
| PIP |
| PLAKOGLOBIN |
| PLASMINOGEN ACTIVATOR INHIBITOR (TYPE 1) |
| PLASMINOGEN ACTIVATOR INHIBITOR (TYPE 2) |
| PLASMINOGEN ACTIVATOR (TISSUE-TYPE) |
| PLASMINOGEN ACTIVATOR (UROKINASE-TYPE) |

| |
|---|
| PLATELET GLYCOPROTEIN IIIa (GP3A) |
| PLAU |
| PLEOMORPHIC ADENOMA GENE-LIKE 1 (PLAGL1) |
| POLYMORPHIC EPITHELIAL MUCIN (PEM) |
| PRAD1 |
| PROGESTERONE RECEPTOR (PgR) |
| PROGESTERONE RESISTANCE |
| PROSTAGLANDIN ENDOPEROXIDE SYNTHASE-2 |
| PROSTAGLANDIN G/H SYNTHASE-2 |
| PROSTAGLANDIN H SYNTHASE-2 |
| pS2 |
| PS6K |
| PSORIASIN |
| PTHLH |
| PTHrP |
| RAD51 |
| RAD52 |
| RAD54 |
| RAP46 |
| RECEPTOR-ASSOCIATED COACTIVATOR 3 (RAC3) |
| REPRESSOR OF ESTROGEN RECEPTOR ACTIVITY (REA) |
| S100A4 |
| S100A6 |
| S100A7 |
| S6K |
| SART-1 |
| SCAFFOLD ATTACHMENT FACTOR B (SAF-B) |
| SCATTER FACTOR (SF) |
| SECRETED PHOSPHOPROTEIN-1 (SPP-1) |
| SECRETED PROTEIN |
| ACIDIC AND RICH IN CYSTEINE (SPARC) |
| STANNICALCIN |
| STEROID RECEPTOR COACTIVATOR-1 (SRC-1) |

| |
|---|
| STEROID RECEPTOR COACTIVATOR-2 (SRC-2) |
| STEROID RECEPTOR COACTIVATOR-3 (SRC-3) |
| STEROID RECEPTOR RNA ACTIVATOR (SRA) |
| STROMELYSIN-1 |
| STROMELYSIN-3 |
| TENASCIN-C (TN-C) |
| TESTES-SPECIFIC PROTEASE 50 |
| THROMBOSPONDIN I |
| THROMBOSPONDIN II |
| THYMIDINE PHOSPHORYLASE (TP) |
| THYROID HORMONE RECEPTOR ACTIVATOR MOLECULE 1 (TRAM-1) |
| TIGHT JUNCTION PROTEIN 1 (TJP1) |
| TIMP1 |
| TIMP2 |
| TIMP3 |
| TIMP4 |
| TISSUE-TYPE PLASMINOGEN ACTIVATOR |
| TN-C |
| TP53 |
| tPA |
| TRANSCRIPTIONAL INTERMEDIARY FACTOR 2 (TIF2) |
| TREFOIL FACTOR 1 (TFF1) |
| TSG101 |
| TSP-1 |
| TSP1 |
| TSP-2 |
| TSP2 |
| TSP50 |
| TUMOR CELL COLLAGENASE STIMULATING FACTOR (TCSF) |
| TUMOR-ASSOCIATED EPITHELIAL MUCIN |
| uPA |
| uPAR |
| UROKINASE |

| |
|---|
| UROKINASE-TYPE PLASMINOGEN ACTIVATOR |
| UROKINASE-TYPE PLASMINOGEN ACTIVATOR RECEPTOR (uPAR) |
| UVOMORULIN |
| VASCULAR ENDOTHELIAL GROWTH FACTOR |
| VASCULAR ENDOTHELIAL GROWTH FACTOR RECEPTOR-2 (VEGFR2) |
| VASCULAR ENDOTHELIAL GROWTH FACTOR-A |
| VASCULAR PERMEABILITY FACTOR |
| VEGFR2 |
| VERY LATE T-CELL ANTIGEN BETA (VLA-BETA) |
| VIMENTIN |
| VITRONECTIN RECEPTOR ALPHA POLYPEPTIDE (VNRA) |
| VITRONECTIN RECEPTOR |
| VON WILLEBRAND FACTOR |
| VPF |
| VWF |
| WNT-1 |
| ZAC |
| ZO-1 |
| ZONULA OCCLUDENS-1 |

Any sample mobilization component may be used in the device. For example, the sample mobilizer may include a mechanical rocker or a sonicator. Alternatively, it may be adapted to provide centrifugal force to the receptacle and lid. A centrifugal sample mobilizer may be used to mobilize sample components, e.g., cells, within a fluid sample, e.g., a fluid sample having a free surface. A centrifugal sample mobilizer may also be used to drive cell rolling along the lid surface. In one example, a centrifugal sample mobilizer may include an axle that rotates the receptacle; in some embodiments, the centrifugal force generated by operating the device is capable of driving the lid into a nonorthogonal angle with respect to the axle.

Another sample mobilization component that may be used in the device utilizes two fluidically coupled chambers, each of which has a surface in contact with the internal space of the receptacle. In such a device, which utilizes pressure-driven flow, each chamber is filled with a fluid, e.g., air, and when one chamber is compressed, a portion of the fluid therein enters the other chamber, increasing its volume. By placing these chambers in contact with a cellular sample in the receptacle and altering their volumes, e.g., squeezing the chambers in alternation, the sample is mobilized.

**Uses of Devices of the Invention**

The invention features improved devices for the enrichment of CTCs and other particles, including bacteria, viruses, fungi, cells, cellular components, viruses, nucleic acids, proteins, and protein complexes, according to size. The devices may be used to effect various manipulations on particles in a sample. Such manipulations include enrichment or concentration of a particle, including size based fractionation, or alteration of the particle itself or the fluid carrying the particle. Preferably, the devices are employed to enrich CTCs or other rare particles from a heterogeneous mixture or to alter a rare particle, e.g., by exchanging the liquid in the suspension or by contacting a particle with a reagent. Such devices allow for a high degree of enrichment, with limited stress on cells, e.g., reduced mechanical lysis or intracellular activation of cells.

**Array Design**

*Single-stage array.* In one embodiment, a single stage contains an array of obstacles, e.g., cylindrical obstacles (Fig. 1D), forming a network of gaps. In certain embodiments, the array has a maximum pass-through size that is several times larger than the cut-off size, e.g., when enriching CTCs from other cells in a blood sample. This result may be achieved using a combination of a large gap size d and a small bifurcation ratio $\varepsilon$. In preferred embodiments, the ε is at most 1/2, e.g., at most 1/3, 1/10, 1/30, 1/100, 1/300, or 1/1000. In such embodiments, the obstacle shape may affect the flow profile in the gap, e.g., such that fluid flowing through the gaps is unevenly distributed around the obstacles; however, the obstacles may be compressed in the flow direction, in order to make the array short (Fig. 1E). Single stage arrays may include bypass channels as described herein.

*Multiple-stage arrays.* In another embodiment, multiple stages are employed to enrich particles over a wide size range. An exemplary device is shown in Fig. 7. The device shown has three stages, but any number of stages may be employed. Typically, the cut-off size in the first stage is larger than the cut-off in the second stage, and the first stage cut-off size is smaller than the maximum pass-through size of the second stage (Fig. 8). The same is true for the following stages. The first stage will deflect (and remove) particles, e.g., that would cause clogging in the second stage, before they reach the second stage. Similarly, the second stage will deflect (and remove) particles that would cause clogging in the third stage, before they reach the third stage. In general, an array may have as many stages as desired, connected either serially or in parallel.

As described, in a multiple-stage array, large particles, e.g., cells, that could cause clogging downstream are deflected first, and these deflected particles need to bypass the downstream stages to avoid clogging. Thus, devices of the invention may include bypass channels that remove output from an array. Although described here in terms of removing particles above the critical size, bypass channels may also be employed to remove output from any portion of the array.

Different designs for bypass channels are as follows.

Single bypass channels. In this design, all stages share one bypass channel, or there is only one stage. The physical boundary of the bypass channel may be defined by the array boundary on one side and a sidewall on the other (Figs. 9-11). Single bypass channels may also be employed with duplex arrays (Fig. 12).

Single bypass channels may also be designed, in conjunction with an array to maintain constant flux through a device (Fig. 13). The bypass channel has varying width designed maintain constant flux through all the stages, so that the flow in the channel does not interfere with the flow in the arrays. Such a design may also be employed with an array duplex (Fig. 14). Single bypass channels may also be designed in conjunction with the array in order to maintain substantially constant fluidic resistance through all stages (Fig. 15). Such a design may also be employed with an array duplex (Fig. 16.)

Multiple bypass channels. In this design (Fig. 17), each stage has its own bypass channel, and the channels are separated from each other by sidewalls. Large particles, e.g., cells are deflected into the major flux to the lower right corner of the first stage and then into in the bypass channel (bypass channel 1 in Fig. 17). Smaller cells that would not cause clogging in the second stage proceed to the second stage, and cells above the critical size of the second stage are deflected to the lower right corner of the second stage and into in another bypass channel (bypass channel 2 in Fig. 17). This design may be repeated for as many stages as desired. In this embodiment, the bypass channels are not fluidically connected, allowing for collection or other manipulation of multiple fractions. The bypass channels do not need to be straight or be physically parallel to each other (Fig. 18). Multiple bypass channels may also be employed with duplex arrays (Fig. 19).

Multiple bypass channels may be designed, in conjunction with an array to maintain constant flux through a device (Fig. 20). In this example, bypass channels are designed to remove an amount of flow so the flow in the array is not perturbed, i.e., substantially constant. Such a design may also be employed with an array duplex (Fig. 21). In this design, the center bypass channel may be shared between the two arrays in the duplex.

*Optimal Boundary Design.* If the array were infinitely large, the flow distribution would be the same at every gap. The flux $\phi$ going through a gap would be the same, and the minor flux would be $\varepsilon\phi$ for every gap. In practice, the boundaries of the array perturb this infinite flow pattern. Portions of the boundaries of arrays may be designed to generate the flow pattern of an infinite array. Boundaries may be flow-feeding, i.e., the boundary injects fluid into the array or flow-extracting, i.e., the boundary extracts fluid from the array.

A preferred flow-extracting boundary widens gradually to extract $\varepsilon\phi$ (represented by arrows in Fig. 22) from each gap at the boundary ($d$ = 24 μm, $\varepsilon$ = 1/60). For example, the distance between the array and the sidewall gradually increases to allow for the addition of $\varepsilon\phi$ from each gap to the boundary. The flow pattern inside this array is not affected by the bypass channel because of the boundary design.

A preferred flow-feeding boundary narrows gradually to feed exactly $\varepsilon\phi$ (represented by arrows in Fig. 23) into each gap at the boundary ($d$ = 24 μm, $\varepsilon$ = 1/60). For example, the distance between the array and the sidewall gradually decreases to allow for the removal of $\varepsilon\phi$ to each gap from the boundary. Again, the flow pattern inside this array is not affected by the bypass channel because of the boundary design.

A flow-feeding boundary may also be as wide as or wider than the gaps of an array (Fig. 24) ($d$ = 24 μm, $\varepsilon$ = 1/60). A wide boundary may be desired if the boundary serves as a bypass channel, e.g., to allow for collection of particles. A boundary may be employed that uses part of its entire flow to feed the array and feeds $\varepsilon\phi$ into each gap at the boundary (represented by arrows in Fig. 24).

Fig. 25 shows a single bypass channel in a duplex array ($\varepsilon$= 1/10, $d$ = 8 μm). The bypass channel includes two flow-feeding boundaries. The flux across the dashed line 1 in the bypass channel is $\Phi$bypass. A flow $\phi$ joins $\Phi$bypass from a gap to the left of the dashed line. The shapes of the obstacles at the boundaries are adjusted so that the flows going into the arrays are $\varepsilon\phi$) at each gap at the boundaries. The flux at dashed line 2 is again $\Phi$bypass.

In some cases, arrays of the invention may include a plurality of rows of obstacles, each successive row being offset by less than half of the period of the previous row, such that at least 50%, 60%, 70%, 80%, 90%, 95%, or even 99% of gaps between obstacles each has a length approximately equal to a first length parameter, and at most 50%, 40%, 30%, 20%, 14%, 5%, or even 1%, respectively, of gaps between obstacles each has a length approximately equal to a second length parameter shorter than the first length parameter. Gaps having a length approximately equal to the second length parameter may be distributed throughout the array either uniformly or non-uniformly. The second length parameter may be sized to capture a cell of interest larger than a predetermined size from a cellular sample. The first length parameter is longer than the second length parameter, e.g., by a factor of 1.1, 1.5, 2, 3, 5, 10, 20, 50, or even 100. Exemplary distances for the first length parameter are in the range of 30 to 100 microns, and exemplary distances for the second length parameter are in the range of 10 to 50 microns.

Optionally, each obstacle of an array of the invention has approximately the same size; alternatively, at least 50%, 60%, 70%, 80%, 90%, 95%, or even 99% of the obstacles have approximately the same size. In some cases, at least 50%, 60%, 70%, 80%, 90%, 95%, or even 99% of the gaps between obstacles in each row each has a length approximately equal to a first length parameter, and up to 50%, 40%, 30%, 20%, 10%, 5%, or even 1%, respectively, of the gaps between obstacles in each row each has a length approximately equal to a second length parameter, which may be shorter than the first length parameter.

In some arrays, a subset of the obstacles, e.g., 50%, 40%, 30%, 20%, 10%, 5%, or even 1%, are unaligned with the centers of the remaining obstacles in their row. Unaligned obstacles may be distributed throughout the array either uniformly or non-uniformly.

Arrays of the invention may have obstacles with different cross-sections; for example, 50%, 60%, 70%, 80%, 90%, 95%, or even 99% of the obstacles may each have a cross-sectional area approximately equal to a first area parameter, and 50%, 40%, 30%, 20%, 10%, 5%, or even 1%, respectively, of the obstacles may each have a cross-sectional area approximately equal to a second area parameter. Optionally, the second area parameter is larger than the first area parameter. In addition, at least one obstacle having a cross-sectional area approximately equal to the first area parameter or second area parameter may have an asymmetrical cross-section.

Arrays of the invention may also include a first subarray of obstacles and a second subarray of obstacles, such that each of the subarrays includes a gap between two obstacles in that subarray, and such that the array includes an interface between the first subarray and the second subarray including a restricted gap that is smaller than the gap between two obstacles in either subarray. The subarrays may be arranged in a two-dimensional configuration; furthermore, they may be staggered, either periodically or uniformly. Each subarray may contain any number of obstacles, e.g., between 2 and 200, between 3 and 50, or between 6 and 20. Exemplary diameters for subarray obstacles are, e.g., in the range of 25 to 200 microns. In general, the gap between two obstacles in an array of the invention may be, e.g., at least 20, 40, 60, 80, or 100 microns; in the case of the restricted gap described above, this gap may be, e.g., at most 100, 80, 60, 40, or 20 microns. Other gap lengths are also possible.

Arrays of the invention may be coupled to a substrate, e.g., plastic, and may include a microfluidic gap. Arrays may additionally be coupled to one or more binding moieties, e.g., binding moieties described herein, that selectively bind to cells of interest. Arrays may also be inside a receptacle, e.g., a receptacle coupled to a transparent cover.

In another embodiment, a two-dimensional array of obstacles forms a network of gaps, such that the array of obstacles includes a plurality of rows distributed on a surface to create fluid flow paths through the device, wherein at least 50%, 60%, 70%, 80%, 90%, 95%, or even 99% of the flow paths each has a width approximately equal to a first width parameter, and at most 50%, 40%, 30%, 20%, 10%, 5%, or even 1%, respectively, of the flow paths each has a width approximately equal to a second, smaller width parameter. Such an array may be used, e.g., to enrich an analyte from a fluid sample. Flow paths each having a width approximately equal to the second width parameter may be distributed throughout the device either uniformly or non-unifonnly, and the second width parameter may be sized to capture the desired analyte within the flow paths that are approximately of the second width parameter. Optionally, the array includes an inlet and an outlet. Optionally, in arrays that include outlets, a region of obstacles with flow path widths equal to or smaller than the second width surrounds the outlet. Such devices may, e.g., have three two-dimensional arrays fluidly connected in series, such that the percentage of the flow paths of the second width increases in the direction of flow of fluid through the device.

Arrays may be coupled to other elements to form devices of the invention. For example, an array may be fluidically coupled to a sample reservoir, a detector, or other elements or modules disclosed herein. Arrays may also function as devices without the need for additional elements or modules. In addition, arrays of the invention may be two-dimensional arrays, or they may adopt another geometry.

Any of the arrays described herein may be used in conjunction with any of the devices or methods of the invention.

**Device design**

**On-chip flow resistor for defining and stabilizing flow**

Devices of the invention may also employ fluidic resistors to define and stabilize flows within an array and to also define the flows collected from the array. Fig. 26 shows a schematic of planar device; a sample, e.g., blood containing CTCs, inlet channel, a buffer inlet channel, a waste outlet channel, and a product outlet channel are each connected to an array. The inlets and outlets act as flow resistors. Fig. 26 also shows the corresponding fluidic resistances of these different device components.

**Flow definition within the array**

Figs. 27 and 28 show the currents and corresponding widths of the sample and buffer flows within the array when the device has a constant depth and is operated with a given pressure drop. The flow is determined by the pressure drop divided by the resistance. In this particular device, $I_{blood}$ and $I_{buffer}$ are equivalent, and this determines equivalent widths of the blood and buffer streams in the array.

**Definition of collection fraction**

By controlling the relative resistance of the product and waste outlet channels, one may modulate the collection tolerance for each fraction. For example, in this particular set of schematics, when $R_{product}$ is greater than $R_{waste}$, a more concentrated product fraction will result at the expense of a potentially increased loss to and dilution of waste fraction. Conversely, when $R_{product}$ is less than $R_{waste}$, a more dilute and higher yield product fraction will be collected at the expense of potential contamination from the waste stream.

**Multiplexed arrays**

The invention features multiplexed arrays. Putting multiple arrays on one device increases sample-processing throughput of CTCs or other cells of interest and allows for parallel processing of multiple samples or portions of the sample for different fractions or manipulations. Multiplexing is further desirable for preparative devices. The simplest multiplex device includes two devices attached in series, i.e., a cascade. For example, the output from the major flux of one device may be coupled to the input of a second device. Alternatively, the output from the minor flux of one device may be coupled to the input of the second device.

*Duplexing.* Two arrays may be disposed side-by-side, e.g., as mirror images (Fig. 29). In such an arrangement, the critical size of the two arrays may be the same or different. Moreover, the arrays may be arranged so that the major flux flows to the boundary of the two arrays, to the edge of each array, or a combination thereof. Such a multiplexed array may also contain a central region disposed between the arrays, e.g., to collect particles above the critical size or to alter the sample, e.g., through buffer exchange, reaction, or labeling.

*Multiplexing on a device.* In addition to forming a duplex, two or more arrays that have separated inputs may be disposed on the same device (Fig. 30A). Such an arrangement could be employed for multiple samples, or the plurality of arrays may be connected to the same inlet for parallel processing of the same sample. In parallel processing of the same sample, the outlets may or may not be fluidically connected. For example, when the plurality of arrays has the same critical size, the outlets may be connected for high throughput samples processing. In another example, the arrays may not all have the same critical size or the particles in the arrays may not all be treated in the same manner, and the outlets may not be fluidically connected.

Multiplexing may also be achieved by placing a plurality of duplex arrays on a single device (Fig. 30B). A plurality of arrays, duplex or single, may be placed in any possible three-dimensional relationship to one another.

Devices of the invention also feature a small footprint. Reducing the footprint of an array may lower cost, and reduce the number of collisions with obstacles to eliminate any potential mechanical damage or other effects to particles. The length of a multiple stage array may be reduced if the boundaries between stages are not perpendicular to the direction of flow. The length reduction becomes significant as the number of stages increases. Fig. 31 shows a small-footprint three-stage array.

**Additional components**

In addition to an array of gaps, devices of the invention may include additional elements or modules, e.g., for isolation, enrichment, collection, manipulation, or detection, e.g., of CTCs. Such elements are known in the art. For example, devices may include one or more inlets for sample or buffer input, and one or more outlets for sample output. Arrays may also be employed on a device having components for other types of enrichment or other manipulation, including affinity, magnetic, electrophoretic, centrifugal, and dielectrophoretic enrichment. Devices of the invention may also be employed with a component for two-dimensional imaging of the output from the device, e.g., an array of wells or a planar surface. Preferably, arrays of gaps as described herein are employed in conjunction with an affinity enrichment.

In one example, a detection module is fluidically coupled to a separation or enrichment device of the invention. The detection module may operate using any method of detection disclosed herein, or other methods known in the art. For example, the detection module includes a microscope, a cell counter, a magnet, a biocavity laser (see, e.g., Gourley et al., J. Phys. D: Appl. Phys. 36: R228-R239 (2003)), a mass spectrometer, a PCR device, an RT-PCR device, a matrix, a microarray, or a hyperspectral imaging system (see, e.g., Vo-Dinh et al., IEEE Eng. Med. Biol. Mag. 23:40-49 (2004)). In one embodiment, a computer terminal may be connected to the detection module. For instance, the detection module may detect a label that selectively binds to cells of interest.

In another example, a capture module is fluidically coupled to a separation or enrichment device of the invention. For example, a capture module includes one or more binding moieties that selectively bind a particular cell type, e.g., a cancer cell or other rare cell. In capture module embodiments that include an array of obstacles, the obstacles may include such binding moieties.

Additionally, a cell counting module, e.g., a Coulter counter, may be fluidically coupled to a separation or enrichment device of the invention. Other modules, e.g., a programmable heating unit, may alternatively be fluidically coupled.

The methods of the invention may be employed in connection with any enrichment or analytical device, either on the same device or in different devices. Examples include affinity columns, particle sorters, e.g., fluorescent activated cell sorters, capillary electrophoresis, microscopes, spectrophotometers, sample storage devices, and sample preparation devices. Microfluidic devices are of particular interest in connection with the systems described herein.

Exemplary analytical devices include devices useful for size, shape, or deformability based enrichment of particles, including filters, sieves, and enrichment or separation devices, e.g., those described in International Publication Nos. WO 2004/029221 and WO 2004/113877, Huang et al. Science 304:987-990 (2004), U.S. Publication No. 2004/0144651, U.S. Patent Nos. 5,837,115 and 6,692,952, and U.S. Application Nos. 60/703,833, 60/704,067, and 11/227,904; devices useful for affinity capture, e.g., those described in International Publication No. WO 2004/029221 and U.S. Publication No. 2005/0266433; devices useful for preferential lysis of cells in a sample, e.g., those described in International Publication No. WO 2004/029221, U.S. Patent No. 5,641,628, and U.S. Application No. 60/668,415; devices useful for arraying cells, e.g., those described in International Publication No. WO 2004/029221, U.S. Patent No. 6,692,952, U.S. Publication No. 2004/0166555, and U.S. Application No. 11/146,581; and devices useful for fluid delivery, e.g., those described in U.S. Publication No. 2005/0282293 and U.S. Application No. 11/227,469. Two or more devices may be combined in series, e.g., as described in International Publication No. WO 2004/029221.

**Methods of fabrication**

Devices of the invention may be fabricated using techniques well known in the art. The choice of fabrication technique will depend on the material used for the device and the size of the array. Exemplary materials for fabricating the devices of the invention include glass, silicon, steel, nickel, polymers, e.g., poly(methylmethacrylate) (PMMA), polycarbonate, polystyrene, polyethylene, polyolefins, silicones (e.g., poly(dimethylsiloxane)), polypropylene, cis-polyisoprene (rubber), poly(vinyl chloride) (PVC), poly(vinyl acetate) (PVAc), polychloroprene (neoprene), polytetrafluoroethylene (Teflon), poly(vinylidene chloride) (SaranA), and cyclic olefin polymer (COP) and cyclic olefin copolymer (COC), and combinations thereof. Other materials are known in the art. For example, deep Reactive Ion Etch (DRIE) is used to fabricate silicon-based devices with small gaps, small obstacles and large aspect ratios (ratio of obstacle height to lateral dimension). Thermoforming (embossing, injection molding) of plastic devices may also be used, e.g., when the smallest lateral feature is >20 microns and the aspect ratio of these features is ≤10. Additional methods include photolithography (e.g., stereolithography or x-ray photolithography), molding, embossing, silicon micromachining, wet or dry chemical etching, milling, diamond cutting, Lithographie Galvanofonnung and Abformung (LIGA), and electroplating. For example, for glass, traditional silicon fabrication techniques of photolithography followed by wet (KOH) or dry etching (reactive ion etching with fluorine or other reactive gas) may be employed. Techniques such as laser micromachining may be adopted for plastic materials with high photon absorption efficiency. This technique is suitable for lower throughput fabrication because of the serial nature of the process. For mass-produced plastic devices, thermoplastic injection molding, and compression molding may be suitable. Conventional thermoplastic injection molding used for mass-fabrication of compact discs (which preserves fidelity of features in sub-microns) may also be employed to fabricate the devices of the invention. For example, the device features are replicated on a glass master by conventional photolithography. The glass master is electroformed to yield a tough, thermal shock resistant, thermally conductive, hard mold. This mold serves as the master template for injection molding or compression molding the features into a plastic device. Depending on the plastic material used to fabricate the devices and the requirements on optical quality and throughput of the finished product, compression molding or injection molding may be chosen as the method of manufacture. Compression molding (also called hot embossing or relief imprinting) has the advantages of being compatible with high molecular weight polymers, which are excellent for small structures and may replicate high aspect ratio structures but has longer cycle times. Injection molding works well for low aspect ratio structures and is most suitable for low molecular weight polymers.

A device may be fabricated in one or more pieces that are then assembled. Layers of a device may be bonded together by clamps, adhesives, heat, anodic bonding, or reactions between surface groups (e.g., wafer bonding). Alternatively, a device with channels in more than one plane may be fabricated as a single piece, e.g., using stereolithography or other three-dimensional fabrication techniques.

To reduce non-specific adsorption of cells or compounds released by lysed cells onto the channel walls, one or more channel walls may be chemically modified to be non-adherent or repulsive. The walls may be coated with a thin film coating (e.g., a monolayer) of commercial non-stick reagents, such as those used to form hydrogels. Additional examples chemical species that may be used to modify the channel walls include oligoethylene glycols, fluorinated polymers, organosilanes, thiols, poly-ethylene glycol, hyaluronic acid, bovine serum albumin, poly-vinyl alcohol, mucin, poly-HEMA, methacrylated PEG, and agarose. Charged polymers may also be employed to repel oppositely charged species. The type of chemical species used for repulsion and the method of attachment to the channel walls will depend on the nature of the species being repelled and the nature of the walls and the species being attached. Such surface modification techniques are well known in the art. The walls may be functionalized before or after the device is assembled. The channel walls may also be coated in order to capture materials in the sample, e.g., membrane fragments or proteins.

**Methods of operation**

Devices of the invention may be employed in any application where the production of a sample enriched in particles above or below a critical size is desired. A preferred use of the device is to produce samples enriched in CTCs or other rare cells. Once an enriched sample is produced, it may be collected for analysis or otherwise manipulated.

Devices of the invention may be employed in concentrated samples, e.g., where particles are touching, hydrodynamically interacting with each other, or exerting an effect on the flow distribution around another particle. For example, the method may enrich CTCs from other cells in whole blood from a human donor. Human blood typically contains ~45% of cells by volume. Cells are in physical contact and/or coupled to each other hydrodynamically when they flow through the array. Fig. 32 shows schematically that cells are densely packed inside an array and could physically interact with each other.

**Enrichment**

In one embodiment, devices of the invention are employed to produce a sample enriched in particles of a desired hydrodynamic size. Applications of such enrichment include concentrating CTCs or other cells of interest, and size fractionization, e.g., size filtering (selecting cells in a particular size range). Devices may also be used to enrich components of cells, e.g., nuclei. Desirably, the methods of the invention retain at least 54%, 75%, 80%, 81%, 82%, 83%, 84%, 85%, 86%, 87%, 88%, 89%, 90%, 91%, 92%, 93%, 94%, 95%, 96%, 97%, 98%, or even 99% of the desired particles compared to the initial mixture, while potentially enriching the desired particles by a factor of at least 100, 1,000, 10,000, 1.00,000, 1,000,000, 10,000,000, or even 100,000,000 relative to one or more non-desired particles. Desirably, if a device produces any output sample in addition to the enriched sample, this additional output sample contains less than 50%, 25%, 20%, 19%, 18%, 17%, 16%, 15%, 14%, 13%, 12%, 11%, 10%, 9%, 8%, 7%, 6%, 5%, 4%, 3%, 2%, or even 1% of the desired particles compared to the initial mixture. The enrichment may also result in a dilution of the enriched particles compared to the original sample, although the concentration of the enriched particles relative to other particles in the sample has increased. Preferably, the dilution is at most 90%, e.g., at most 75%, 50%, 33%, 25%, 10%, or 1%.

In a preferred embodiment, the device produces a sample enriched in a rare particles, e.g., cells. In general, a rare particle is a particle that is present as less than 10% of a sample. Rare particles include, depending on the sample, rare cells, e.g., CTCs, epithelial cells, fetal cells, stem cells (e.g., undifferentiated), bone marrow cells, progenitor cells, foam cells, mesenchymal cells, endothelial cells, endometrial cells, trophoblasts, cancer cells, immune system cells (host or graft), connective tissue cells, bacteria, fungi, and pathogens (e.g., bacterial or protozoa). Rare particles also include viruses, as well as cellular components such as organelles (e.g., mitochondria and nuclei). Rare particles may be isolated from samples including bodily fluids, e.g., blood, or environmental sources, e.g., pathogens in water samples. Fetal red blood cells may be enriched from maternal peripheral blood, e.g., for the purpose of determining sex and identifying aneuploidies or genetic characteristics, e.g., mutations, in the developing fetus. CTCs, which are of epithelial type and origin, may also be enriched from peripheral blood for the purpose of diagnosis and monitoring therapeutic progress. Circulating endothelial cells may be similarly enriched from peripheral blood. Bodily fluids or environmental samples may also be screened for pathogens, e.g., for colifonn bacteria, blood borne illnesses such as sepsis, or bacterial or viral meningitis. Rare cells also include cells from one organism present in another organism, e.g., cells from a transplanted organ.

In addition to enrichment of rare particles, devices of the invention may be employed for preparative applications. An exemplary preparative application includes generation of cell packs from blood. Devices of the invention may be configured to produce fractions enriched in platelets, red blood cells, and white blood cells. By using multiplexed devices or multistage devices, all three cellular fractions may be produced in parallel or in series from the same sample. In other embodiments, the device may be employed to separate nucleated from non-nucleated cells, e.g., from cord blood sources.

Using the devices of the invention is advantageous in situations where the particles being enriched are subj ect to damage or other degradation. As described herein, devices of the invention may be designed to enrich cells with a minimum number of collisions between the cells and obstacles. This minimization reduces mechanical damage to cells and also prevents intracellular activation of cells caused by the collisions. This gentle handling of the cells preserves the limited number of rare cells in a sample, prevents rupture of cells leading to contamination or degradation by intracellular components, and prevents maturation or activation of cells, e.g., stem cells or platelets. In preferred embodiments, cells are enriched such that fewer than 30%, 10 %, 5%, 1%, 0.1%, or even 0.01% are activated or mechanically lysed.

Fig. 33A shows a typical size distribution of cells in human peripheral blood. The white blood cells range from ~4 μm to ~12 μm, whereas the red blood cells are ~1.5-3 μm (short axis). Fig. 33B shows that CTCs are generally significantly larger than blood cells, with the majority of CTCs ranging from ~8 to ~22 μm. Thus, a size-based enrichment using a device of the invention, in which the size cutoff is chosen to be, e.g., 12 μm (Fig. 33C), would be effective in enriching CTCs from other blood cells. Any cell population with a similar distribution to CTCs may be similarly enriched from blood cells (Fig. 33B).

In an alternative embodiment, a cellular sample is added through a sample inlet of the device, and buffer medium is added through the fluid inlet (Fig. 42A). Cells below the critical size move through the device undeflected, emerging from the edge outlets in their original sample medium. Cells above the critical size, e.g., epithelial cells, in particular, CTCs, are deflected and emerge from the center outlet contained in the buffer medium added through the fluid inlet. Operation of the device thus produces samples enriched in cells above and below the critical size. Because epithelial cells are among the largest cells in the bloodstream, the size and geometry of the gaps of the device may be chosen so as to direct virtually all other cell types to the edge outlets, while producing a sample from the center outlet that is substantially enriched in epithelial cells after a single pass through the device.

A device of the invention need not be duplexed as shown in Fig. 42A in order to operate as described herein. The schematized representation shown in Fig. 42B may represent either a duplexed device or a single array.

Enrichment may be enhanced in numerous ways. For example, target cells may be labeled with immunoaffinity beads, thereby increasing their size (as depicted in Fig. 44). In the case of epithelial cells, e.g., CTCs, this may further increase their size and thus result in an even more efficient enrichment. Alternatively, the size of smaller cells may be increased to the extent that they become the largest objects in solution or occupy a unique size range in comparison to the other components of the cellular sample, or so that they copurify with other cells. The hydrodynamic size of a labeled target cell may be at least 10%, 100%, or even 1,000% greater than the hydrodynamic size of such a cell in the absence of label. Beads may be made of polystyrene, magnetic material, or any other material that may be adhered to cells. Desirably, such beads are neutrally buoyant so as not to disrupt the flow of labeled cells through the device of the invention.

Enrichment methods of the invention include devices that include obstacles that are capable of selectively capturing cells of interest, e.g., epithelial cells, e.g., CTCs,

The methods of the invention may also be used to deplete or remove an analyte from a cellular sample, for example, by producing a sample enriched in another analyte using the above-described methods. For example, a cellular sample may be depleted of cells having a hydrodynamic size less than or equal to 12 microns by enriching for cells having a hydrodynamic size greater than 12 microns. Any method of depletion or removal may be used in conjunction with the arrays and devices of the invention. In methods of the invention featuring depletion of removal of an analyte, sample processing may be continuous and may occur in vivo or ex vivo. Furthermore, in some embodiments, if the analyte to be depleted or removed is retained in a device of the invention, the analyte may be released from the device by applying a hypertonic solution to said device. The analyte may then be detected in the effluent from the device.

**Alteration**

In other embodiments, in addition to enrichment, CTCs or other cells of interest are contacted with an altering reagent that may chemically or physically alter the particle or the fluid in the suspension. Such applications include purification, buffer exchange, labeling (e.g., immunohistochemical, magnetic, and histochemical labeling, cell staining, and flow in-situ fluorescence hybridization (FISH)), cell fixation, cell stabilization, cell lysis, and cell activation.

Such methods allow for the transfer of particles, e.g., CTCs, from a sample into a different liquid. Fig. 34A shows this effect schematically for a single stage device, Fig. 34B shows this effect for a multistage device, Fig. 34C shows this effect for a duplex array, and Fig. 34D shows this effect for a multistage duplex array. By using such methods, blood cells may be separated from plasma. Such transfers of particles from one liquid to another may be also employed to effect a series of alterations, e.g., Wright staining blood on-chip. Such a series may include reacting a particle with a first reagent and then transferring the particle to a wash buffer, and then another reagent.

Figs. 35A-35C illustrate a further example of alteration in a two stage device having two bypass channels. In this example, large blood particles are moved from blood to buffer and collected in stage 1, medium blood particles are moved from blood to buffer in stage 2, and small blood particles that are not moved from the blood in stages 1 and 2 are also collected. Fig. 35B illustrates the size cut-off of the two stages, and Fig. 35C illustrates the size distribution of the three fractions collected.

Fig. 36 illustrates an example of alteration in a two stage device having bypass channels that are disposed between the lateral edge of the array and the channel wall. Fig. 37 illustrates a device similar to that in Fig. 36, except that the two stages are connected by fluidic channels. Fig. 38 illustrates alteration in a device having two stages with a small footprint. Figs. 39A-39B illustrate alteration in a device in which the output from the first and second stages is captured in a single channel. Fig. 40 illustrates another device for use in the methods of the invention.

Fig. 41 illustrates the use of a device to perform multiple, sequential alterations on a particle. In this device a blood particles is moved from blood into a regent that reacts with the particle, and the reacted particle is then moved into a buffer, thereby removing the unreacted reagent or reaction byproducts. Additional steps may be added.

Enrichment and alteration may also be combined, e.g., where desired cells are contacted with a lysing reagent and cellular components, e.g., nuclei, are enriched based on size. In another example, particles may be contacted with particulate labels, e.g., magnetic beads, which bind to the particles. Unbound particulate labels may be removed based on size.

**Separation of free labeling reagent from labeling reagent bound to cells**

Devices of the invention may be employed in order to separate free labeling reagent from labeling reagent bound to CTCs or other cells. As shown in Fig. 45, a labeling reagent may be pre-incubated with a cellular sample prior to introduction to the device. Desirably, the labeling reagent specifically or preferentially binds the cell population of interest, e.g., epithelial cells such as CTCs. Exemplary labeling reagents include antibodies, quantum dots, phage, aptamers, fluorophore-containing molecules, enzymes capable of carrying out a detectable chemical reaction, or functionalized beads. Generally, the labeling reagent is smaller than the cell of interest, or the cell of interest bound to the bead; thus, when the cellular sample combined with the labeling reagent is introduced to the device, free labeling reagent moves through the device undeflected and emerges from the edge outlets, while bound labeling reagent emerges from the center outlet along with epithelial cells. Advantageously, this method simultaneously achieves size separation and separation of free labeling reagent from bound labeling reagent. Additionally, this method of separation facilitates downstream sample analysis without the need for a release step or destructive methods of analysis, as described below.

Fig. 46 shows a more general case, in which the enriched labeled sample contains a population of non-target cells that co-separate with the target cells due to similar size. The non-target cells do not interfere with downstream sample analysis that relies on detection of the bound labeling reagent, because this reagent binds selectively to the cells of interest.

**Buffer exchange**

Devices of the invention may be employed for purposes of buffer exchange. To achieve this result, a protocol similar to that used for enrichment is followed: a cellular sample is added through a sample inlet of the device, and the desired final buffer medium is added through a fluid inlet. As described above, cells above the critical size are deflected and enter the buffer.

**Concentration**

Devices of the invention may be employed in order to concentrate a cellular sample of interest, e.g., a sample containing CTCs. As shown in Fig. 47, a cellular sample is introduced to the sample inlet of the device. By reducing the volume of buffer introduced into the fluid inlet so that this volume is significantly smaller than the volume of the cellular sample, concentration of target cells in a smaller volume results. This concentration step may improve the results of any downstream analysis performed.

**Cell lysis**

Devices of the invention may be employed for purposes of cell lysis. To achieve this, a protocol similar to that used for enrichment is followed: a cellular sample is added through a sample inlet of the device (Fig. 48), and lysis buffer is added through the fluid inlet. As described above, cells above the critical size are deflected and enter the lysis buffer, leading to lysis of these cells. As a result, the sample emerging from the center outlet includes lysed cell components including organelles, while undeflected whole cells emerge from the other outlet. Thus, the device provides a method for selectively lysing target cells.

**Multiple stages**

Devices of the invention may be joined together to provide multiple stages of enrichment and reaction. For example, Fig. 43A shows the "cascade" configuration, in which outlet 1 of one device is joined to a sample inlet of a second device. This allows for an initial enrichment step using the first device so that the sample introduced to the second device is already enriched for cells of interest. The two devices may have either identical or different critical sizes, depending on the intended application.

In Fig. 49, an unlabeled cellular sample is introduced to the first device in the cascade via a sample inlet, and a buffer containing labeling reagent is introduced to the first device via the fluid inlet. Epithelial cells, e.g., CTCs, are deflected and emerge from the center outlet in the buffer containing labeling reagent. This enriched labeled sample is then introduced to the second device in the cascade via a sample inlet, while buffer is added to the second device via the fluid inlet. Further enrichment af target cells and separation of free labeling reagent is achieved, and the enriched sample may be further analyzed. Alternatively, labeling reagent may be added directly to the sample emerging from the center outlet of the first device before introduction to the second device. The use of a cascade configuration may allow for the use of a smaller quantity or a higher concentration of labeling reagent at less expense than the single-device configuration of Fig. 55; in addition, any nonspecific binding that may occur is significantly reduced by the presence of an initial enrichment step using the first device.

An alternative configuration of two or more device stages is the "bandpass" configuration. Fig. 43B shows this configuration, in which outlet 2 of one device is joined to a sample inlet of a second device. This allows for an initial enrichment step using the first device so that the sample introduced to the second device contains cells that remained undeflected within the first device. This method may be useful when the cells of interest are not the largest cells in the sample; in this instance, the first stage may be used to reduce the number of large non-target cells by deflecting them to the center outlet. As in the cascade configuration, the two devices may have either identical or different critical sizes, depending on the intended application. For example, different critical sizes are appropriate for an application requiring the enrichment of epithelial cells, e.g., CTCs, in comparison with an application requiring the enrichment of smaller endothelial cells.

In Fig. 51, a cellular sample pre-incubated with labeling reagent is introduced to a sample inlet of the first device of the bandpass configuration, and a buffer is introduced to the first device via the fluid inlet. The first device is disposed in such a manner that large, non-target cells are deflected and emerge from the center outlet, while a mixture of target cells, small non-target cells, and labeling reagent emerge from outlet 2 of the first device. This mixture is then introduced to the second device via a sample inlet, while buffer is added to the second device via the fluid inlet. Enrichment of target cells and separation of free labeling reagent is achieved, and the enriched sample may be further analyzed. Non-specific binding of labeling reagent to the deflected cells in the first stage is acceptable in this method, as the deflected cells and any bound labeling reagent are removed from the system.

In any of the multiple device configurations described above, the devices and the connections joining them may be integrated into a single device. For example, a single cascade device including two or more stages is possible, as is a single bandpass device including two or more stages.

## Downstream analysis

A useful step for many diagnostic assays is the removal of free labeling reagent from the sample to be analyzed. As described above, devices of the invention are able to separate free labeling reagent from labeling reagent bound to cells, e.g., CTCs. It is then possible to perform a bulk measurement of the labeled sample without significant levels of background interference from free labeling reagent. For example, fluorescent antibodies selective for a particular epithelial cell marker such as EpCAM may be used. The fluorescent moiety may include Cy dyes, Alexa dyes, or other fluorophore-containing molecules. The resulting labeled sample is then analyzed by measuring the fluorescence of the resulting sample of labeled enriched cells using a fluorometer. Alternatively, a chromophore-containing label may be used in conjunction with a spectrometer, e.g., a UV or visible spectrometer. The measurements obtained may be used to quantify the number of target cells or all cells in the sample. Alternatively, the ratio of two cells types in the sample, e.g., the ratio of cancer cells to endothelial cells, may be determined. This ratio may be a ratio of the number of each type of cell, nr alternatively it may be a ratio of any measured characteristic of each type of cell.

Any method of identifying cells, e.g., cells that have a cell surface marker associated with cancer, e.g., Ber-Ep4, CD34+, EpCAM, E-Cadherin, Mucin-1, Cytokeratin 8, EGFR, and leukocyte associated receptor (LAR), may be used. For example, an enriched sample of CTCs may be contacted with a device that includes a surface with one or more binding moieties that selectively bind one or more cells of the enriched sample. The binding moieties may include a polypeptide, e.g., an antibody or fragment thereof, e.g., monoclonal. For example, such a monoclonal antibody could be specific for EpCAM, e.g., anti-human EpCAMITROP1 (catalog #AF960, R&D Systems).

Many other methods of measurement and labeling reagents are useful in the methods of the invention. Any imaging techniques, e.g., hyperspectral imaging, may be used. Labeling antibodies, e.g., antibodies selective for any cancer marker, e.g., those listed in Table 1, may possess covalently bound enzymes that cleave a substrate, altering its absorbance at a given wavelength; the extent of cleavage is then quantified with a spectrometer. Colorimetric or luminescent readouts are possible, depending on the substrate used. Advantageously, the use of an enzyme label allows for significant amplification of the measured signal, lowering the threshold of detectability.

Quantum dots, e.g., Qdots® from QuantumDot Corp., may also be utilized as a labeling reagent that is covalently bound to a targeting antibody. Qdots are resistant to photobleaching and may be used in conjunction with two-photon excitation measurements.

Other possible labeling reagents useful in the methods of the invention are phage. Phage display is a technology in which binding peptides are displayed by engineered phage strains having strong binding affinities for a target protein, e.g., those found on the surface of cells of interest. The peptide sequence corresponding to a given phage is encoded in that phage's nucleic acid, e.g., DNA or RNA. Thus, phage are useful labeling reagents in that they are small relative to epithelial cells such as CTCs and thus may be easily separated, and they additionally carry nucleic acid that may be analyzed and quantified using PCR or similar techniques, enabling a quantitative determination of the number of cells present in an enriched bound sample.

Fig. 50 depicts the use of phage as a labeling reagent in which two device stages are arrayed in a cascade configuration. The method depicted in Fig. 50 fits the general description of Fig. 49, with the exception of the labeling reagent employed.

Desirably, downstream analysis results in an accurate determination of the number of target cells in the sample being analyzed. In order to produce accurate quantitative results, the surface antigen being targeted on the cells of interest typically has known or predictable expression levels, and the binding of the labeling reagent should also proceed in a predictable manner, free from interfering substances. Thus, methods of the invention that result in highly enriched cellular samples prior to introduction of labeling reagent are particularly useful. In addition, labeling reagents that allow for amplification of the signal produced are preferred, because of the low incidence of target cells, such as epithelial cells, e.g., CTCs, in the bloodstream. Reagents that allow for signal amplification include enzymes and phage. Other labeling reagents that do not allow for convenient amplification but nevertheless produce a strong signal, such as quantum dots, are also desirable.

It is not necessary to include a labeling reagent in the methods of the invention. For example, one method includes the steps of introducing a cellular sample, e.g., a sample of peripheral blood, into a device of the invention. For example, the device enriches cells having a hydrodynamic size greater than 12 microns, 14 microns, 16 microns, 18 microns, or even 20 microns from smaller cells in the sample. Alternatively, the device may enrich cells having a hydrodynamic size greater than or equal to 6 microns and less than or equal to 12 microns, e.g.. cells having a hydrodynamic size greater than or equal to 8 microns and less than or equal to 10 microns, from other cells. The device may also enrich cells having a hydrodynamic size greater than or equal to 5 microns and less than or equal to 10 microns from cells having a hydrodynamic size greater than 10 microns; alternatively, it may enrich cells having a hydrodynamic size greater than or equal to 4 microns and less than or equal to 8 microns from cells having a hydrodynamic size greater than 8 microns. Each of these subsets of cells may then be collected and analyzed, e.g., by detecting the presence of a particular cell type, e.g., a rare cell, e.g., an epithelial cell or progenitor endothelial cell, in one of the samples thus collected. Because of the enrichment that this method generally achieves, the concentration of rare cells may be higher in a recovered sample than in the starting cellular sample, allowing for rare cell detection by a variety of means. In one embodiment, the cellular sample is applied to an inlet of the device; a second reagent, e.g., a buffer, e.g., a buffer containing BSA, a lysis reagent, a nucleic acid amplification reagent, an osmolarity regulating reagent, a labeling reagent, a preservative, or a fixing reagent, is optionally applied to a second inlet; and two output samples flow out of two outlets of the device. For example, application of a cellular sample containing cancer cells to an inlet of the device could result in one output sample that is enriched in such cells, while the other sample is depleted in these cells or even completely devoid of them. Any of the second reagents listed above may be employed in any of the devices and methods of the invention, e.g., those in which the device contains a second inlet.

In embodiments in which two cell types are directed in different directions, the first cell type being the cell type of interest, the second cell type may be any other cell type. For example, the second cell type may include white blood cells or red blood cells, e.g., enucleated red blood cells.

The methods of the invention need not employ either magnetic particles or interaction with an antibody or fragment thereof in order to enrich cells of interest, e.g., cancer cells, from a cellular sample. Any method based on cell size, shape, or deformability may be used in order to enrich cells of interest; subsequently, cell detection or any other downstream applications, e.g., those described herein, may be performed.

The methods of the invention allow for enrichment, quantification, and molecular biology analysis of the same set of cells. The gentle treatment of the cells in the devices of the invention, coupled with the described methods of bulk measurement, maintain the integrity of the cells so that further analysis may be performed if desired. For example, techniques that destroy the integrity of the cells may be performed subsequent to bulk measurement; such techniques include DNA or RNA analysis, proteome analysis, or metabolome analysis. For example, the total amount of DNA or RNA in a sample may be determined; alternatively, the presence of a particular sequence or mutation, e.g., a deletion, in DNA or RNA may be detected, e.g., a mutation in a gene encoding a polypeptide listed in Table 1. Furthermore, mitochondrial DNA, telomerase, or nuclear matrix proteins in the sample may be analyzed (for mitochondrial mutations in cancer, see, e.g., Parrella et al., Cancer Res. 61:7623-7626 (2001), Jones et al., Cancer Res. 61:1299-1304 (2001), and Fliss et al., Science 287:2017-2019 (2000); for telomerase, see, e.g., Soria et al., Clin. Cancer Res. 5:971-975 (1999)). For example, the sample may be analyzed to determine whether any mitochondrial abnormalities (see, e.g., Carew et al., Mol. Cancer 1:9 (2002), and Wallace, Science 283:1482-1488 (1999)) or perinuclear compartments are present. One useful method for analyzing DNA is PCR, in which the cells are lysed and levels of particular DNA sequences are amplified. Such techniques are particularly useful when the number of target cells isolated is very low. In-cell PCR may be employed; in addition, gene expression analysis (see, e.g., Giordano et al., Am. J.. Pathol. 159:1231-1238 (2001), and Buckhaults et al., Cancer Res. 63:4144-4149 . (2003)) or fluorescence in-situ hybridization may be used, e.g., to determine the tissue or tissues of origin of the cells being analyzed. A variety of cellular characteristics may be measured using any of the above techniques, such as protein phosphorylation, protein glycosylation, DNA methylation (see, e.g., Das et al., J. Clin. Oncol. 22:4632-4642 (2004)), microRNA levels (see, e.g., He et al., Nature 435:828-833 (2005), Lu et al., Nature 435:834-838 (2005), O'Donnell et al., Nature 435:839-843 (2005), and Calin et al., N. Engl. J. Med. 353:1793-1801 (2005)), cell morphology or other structural characteristics, e.g., pleomorphisms, adhesion, migration, binding, division, level of gene expression, and presence of a somatic mutation. This analysis may be performed on any number of cells, including a single cell of interest, e.g., a cancer cell. In addition, the size distribution of cells may be analyzed. Desirably, downstream analysis, e.g., detection, is performed on more than one sample, preferably from the same subject.

**Quantification of cells**

Cells found in blood are of various types and span a range of sizes. Using the methods of the invention, it is possible to distinguish, size, and count blood cell populations, e.g., CTCs. For example, a Coulter counter may be used. Fig. 33A shows a typical size distribution for a normal blood sample. Under some conditions, e.g., the presence of a tumor in the body that is exfoliating tumor cells, cells that are not native to blood may appear in the peripheral circulation. The ability to isolate and count large cells, or other desired cells, that may appear in the blood provides powerful opportunities for diagnosing disease states.

Desirably, a Coulter counter, or other cell detector, is fluidically coupled to an outlet of a device of the invention, and a cellular sample is introduced to the device of the invention. Cells flowing through the outlet fluidically coupled to the Coulter counter then pass through the Coulter aperture, which includes two electrodes separated by an opening through which the cells pass, and which measures the volume displaced as each cell passes through the opening. Preferably, the Coulter counter determines the number of cells of cell volume greater than 500 fL in the enriched sample. Alternatively, the Coulter counter preferably determines the number of cells of diameter greater than 14 $\mu$m in the enriched sample. The Coulter counter, or other cell detector, may also be an integral part of a device of the invention rather than constituting a separate device. The counter may utilize any cellular characteristic, e.g., impedance, light absorption, light scattering, or capacitance.

In general, any means of generating a cell count is useful in the methods of the invention. Such means include optical, such as scattering, absorption, or fluorescence means. Alternatively, non-aperture electrical means, such as determining capacitance, are useful.

**Combination with other enrichment techniques**

Enrichment and alteration methods employing devices of the invention may be combined with other particulate sample manipulation techniques. In particular, further enrichment or purification of CTCs or other particles may be desirable. Further enrichment may occur by any technique, including affinity enrichment. Suitable affinity enrichment techniques include contacting particles of interest with affinity agents bound to channel walls or an array of obstacles. Such affinity agents may be selective for any cell type, e.g., cancer cells. This includes using a device of the invention in which antibodies specific for target cells are immobilized within the device. This allows for binding and enrichment of target cells within the device; subsequently the target cells are eluted using a higher flow rate, competing ligands, or another method.

**Diagnosis**

As described herein, epithelial cells exfoliated from solid tumors have been found in the circulation of patients with cancers of the breast, colon, liver, ovary; prostate, and lung. In general, the presence of CTCs after therapy has been associated with tumor progression and spread, poor response to therapy, relapse of disease, and/or decreased survival over a period of several years. Therefore, enumeration of CTCs offers a means to stratify patients for baseline characteristics that predict initial risk and subsequent risk based upon response to therapy.

The devices and methods of the invention may be used, e.g., to evaluate cancer patients and those at risk for cancer. In any of the methods of diagnosis described herein, either the presence or the absence of an indicator of cancer, e.g., a cancer cell, or of any other disorder, may be used to generate a diagnosis. In one example, a blood sample is drawn from the patient and introduced to a device of the invention with a critical size chosen appropriately to enrich epithelial cells, e.g., CTCs, from other blood cells. Using a method of the invention, the number of epithelial cells in the blood sample is determined. For example, the cells may be labeled with an antibody that binds to EpCAM, and the antibody may have a covalently bound fluorescent label. A bulk measurement may then be made of the enriched sample produced by the device, and from this measurement, the number of epithelial cells present in the initial blood sample may be determined. Microscopic techniques may be used to visually quantify the cells in order to correlate the bulk measurement with the corresponding number of labeled cells in the blood sample.

Besides epithelial tumor cells, there are other cell types that are involved in metastatic tumor formation. Studies have provided evidence for the involvement of hematopoietic bone marrow progenitor cells and endothelial progenitor cells in metastasis (see, e.g., Kaplan et al., Nature 438:820-827 (2005), and Brugger et al., Blood 83:636-640 (1994)). The number of cells of a second cell type, e.g., hematopoietic bone marrow progenitor cells, e.g., progenitor endothelial cells, may be determined, and the ratio of epithelial tumor cells to the number of the second cell type may be calculated. Such ratios are of diagnostic value in selecting the appropriate therapy and in monitoring the efficacy of treatment.

Cells involved in metastatic tumor formation may be detected using any methods known in the art. For example, antibodies specific for particular cell surface markers may be used. Useful endothelial cell surface markers include CD105, CD106, CD 144, and CD146; useful tumor endothelial cell surface markers include TEM1, TEM5, and TEM8 (see, e.g., Carson-Walter et al., Cancer Res. 61:6649-6655 (2001)); and useful mesenchymal cell surface markers include CD133. Antibodies to these or other markers may be obtained from, e.g., Chemicon, Abcam, and R&D Systems.

By making a series of measurements, optionally made at regular intervals such as one day, two days, three days, one week, two weeks, one month, two months, three months, six months, or one year, one may track the level of epithelial cells present in a patient's bloodstream as a function of time. In the case of existing cancer patients, this provides a useful indication of the progression of the disease and assists medical practitioners in making appropriate therapeutic choices based on the increase, decrease, or lack of change in epithelial cells, e.g., CTCs, in the patient's bloodstream. For those at risk of cancer, a sudden increase in the number of cells detected may provide an early warning that the patient has developed a tumor. This early diagnosis, coupled with subsequent therapeutic intervention, is likely to result in an improved patient outcome in comparison to an absence of diagnostic information.

Diagnostic methods include making bulk measurements of labeled epithelial cells, e.g., CTCs, isolated from blood, as well as techniques that destroy the integrity of the cells. For example, PCR may be performed on a sample in which the number of target cells isolated is very low; by using primers specific for particular cancer markers, information may be gained about the type of tumor from which the analyzed cells originated. Additionally, RNA analysis, proteome analysis, or metabolome analysis may be performed as a means of diagnosing the type or types of cancer present in the patient.

One important diagnostic indicator for lung cancer and other cancers is the presence or absence of certain mutations in EGFR (see, e.g., International Publication WO 2005/094357). EGFR consists of an extracellular ligand-binding domain, a transmembrane portion, and an intracellular tyrosine kinase (TK) domain. The normal physiologic role of EGFR is to bind ErbB ligands, including epidermal growth factor (EGF), at the extracellular binding site to trigger a cascade of downstream intracellular signals leading to cell proliferation, survival, motility and other related activities. Many non-small cell lung tumors with EGFR mutations respond to small molecule EGFR inhibitors, such as gefitinib (Iressa; AstraZeneca), but often eventually acquire secondary mutations that make them drug resistant. Using the devices and method of the invention, one may monitor patients taking such drugs by taking frequent samples of blood and determining the number of epithelial cells, e.g., CTCs, in each sample as a function of time. This provides information as to the course of the disease. For example, a decreasing number of circulating epithelial cells over time suggests a decrease in the severity of the disease and the size of the tumor or tumors. Immediately following quantification of epithelial cells, these cells may be analyzed by PCR to determine what mutations may be present in the EFGR gene expressed in the epithelial cells. Certain mutations, such as those clustered around the ATP-binding pocket of the EGFR TK domain, are known to make the cancer cells susceptible to gefitinib inhibition. Thus, the presence of these mutations supports a diagnosis of cancer that is likely to respond to treatment using gefitinib. However, many patients who respond to gefitinib eventually develop a second mutation, often a methionine-to-threonine substitution at position 790 in exon 20 of the TK domain, which renders them resistant to gefitinib. By using the devices and method of the invention, one may test for this mutation as well, providing further diagnostic information about the course of the disease and the likelihood that it will respond to gefitinib or similar compounds. Since many EGFR mutations, including all EGFR mutations in NSC lung cancer reported to date that are known to confer sensitivity or resistance to gefitinib, lie within the coding regions of exons 18 to 21, this region of the EGFR gene may be emphasized in the development of assays for the presence of mutations (see Examples 4-6).

The invention includes a method of detecting the presence or absence of at least one nucleic acid variant in the kinase domain of the erbB1 gene of said patient. The presence of at least one variant indicates that the EGFR targeting treatment is likely to be effective. Preferably, the nucleic acid variant increases the kinase activity of the EGFR. The patient can then be treated with an EGFR targeting treatment. In one embodiment of the present invention, the EGFR targeting treatment includes administration of a tyrosine kinase inhibitor.

Accordingly, the present invention provides a method to determine the likelihood of effectiveness of an epidermal growth factor receptor (EGFR) targeting treatment in a human patient affected with cancer. The method includes detecting the presence or absence of at least one nucleic acid variant in the kinase domain of the erbB1 gene of said patient relative to the wild type erbB1 gene. The presence of at least one variant indicates that the EGFR targeting treatment is likely to be effective. Preferably, the nucleic acid variant increases the kinase activity of the EGFR. The patient can then be treated with an EGFR targeting treatment. In one embodiment of the present invention, the EGFR targeting treatment is a tyrosine kinase inhibitor.

Determining the presence or absence of a particular variant or plurality of variants in the kinase domain of the erbB1 gene in a patient with or at risk for developing cancer can be performed in a variety of ways. Such tests are commonly performed using DNA or RNA collected from biological samples, e.g., tissue biopsies, urine, stool, sputum, blood, cells, tissue scrapings, breast aspirates, or other cellular materials, and can be performed by a variety of methods including, but not limited to, PCR, hybridization with allele-specific probes, enzymatic mutation detection, chemical cleavage of mismatches, mass spectrometry, or DNA sequencing, including minisequencing. In particular embodiments, hybridization with allele specific probes can be conducted in two formats: (1) allele specific oligonucleotides bound to a solid phase (e.g., glass, silicon, or nylon membranes) and the labeled sample in solution, as in many DNA chip applications, or (2) bound sample (e.g., cloned DNA or PCR amplified DNA) and labeled oligonucleotides in solution (either allele specific or short so as to allow sequencing by hybridization). Diagnostic tests may involve a panel of variants, often on a solid support, which enables the simultaneous determination of more than one variant. The method involves determining whether the kinase domain of the EGFR of a patient contains at least one nucleic acid variant. The presence of such a variant is indicative of the effectiveness of an EGFR targeted treatment, e.g., administration of a tyrosine kinase inhibitor.

EGFR genetic testing can detect mutations in the kinase domain of EGFR. DNA is first obtained from CTCs enriched utilizing the microfluidic device of the invention, as outlined above. The DNA sequence of 7 exons (18, 19, 20, 21, 22, 23, and 24) of EGFR is then determined by direct bi-directional gene sequencing. The sequence obtained is then compared to known EGFR sequences to identify DNA sequence changes. If a DNA sequence change is detected in a CTC sample, the test may be repeated on the original tissue sample.

In another aspect, Determining the presence of at least one kinase activity increasing nucleic acid variant in the erbB1 gene may entail a haplotyping test. Methods of determining haplotypes are known to those of skill in the art, as for example, in International Publication No. WO 00/04194. Preferably, the determination of the presence or absence of a kinase activity increasing nucleic acid variant involves determining the sequence of the variant site or sites by methods such as polymerase chain reaction (PCR). Alternatively, the determination of the presence or absence of a kinase activity increasing nucleic acid variant may encompass chain terminating DNA sequencing or minisequencing, oligonucleotide hybridization or mass spectrometry.

The methods of the present invention may be used to predict the likelihood of effectiveness (or lack of effectiveness) of an EGFR targeting treatment in a patient affected with or at risk for developing cancer. Preferably, cancers include cancer of epithelial origin, including, but are not limited to, gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer and bladder cancer. In a preferred embodiment, the cancer is non-small cell lung cancer. The present invention generally concerns the identification of variants in the kinase domain of the erbB1 gene which are indicative of the effectiveness of an EGFR targeting treatment in a patient with or at risk for developing cancer. Additionally, the identification of specific variants in the kinase domain of EGFR, in effect, can be used as a diagnostic or prognostic test. For example, the presence of at least one variant in the kinase domain of erbB1 indicates that a patient will likely benefit from treatment with an EGFR targeting compound, such as, for example, a tyrosine kinase inhibitor.

In one embodiment, the invention provides a method of screening for variants in the kinase domain of the erbB1 gene in a test biological sample by PCR or, alternatively, in a ligation chain reaction (LCR) (see, e.g., Landegran et al., 1988, Science 241: 1077-1080; and Nakazawa et al., 1994, Proc. Natl. Acad. Sci. USA 91:360-364), the latter of which can be particularly useful for detecting point imitations in the EGFR-gene (see, e.g., Abravaya et al., 1995, Nucl. Acids Res. 23:675-682). The method includes the steps of designing degenerate primers for amplifying the target sequence, the primers corresponding to one or more conserved regions of the gene, amplifying reaction with the primers using, as a template, a DNA or cDNA obtained from a test biological sample and analyzing the PCR products. Comparison of the PCR products of the test biological sample to a control sample indicates variants in the test biological sample. The change can be either an absence or presence of a nucleic acid variant in the test biological sample.

Alternative amplification methods include: self sustained sequence replication (see, e.g., Guatelli et al., 1990, Proc. Natl. Acad. Sci. USA 87:1874-1 878), transcriptional amplification system (see, e.g., Kwoh, et al., 1989, Proc. Natl. Acad. Sci. USA 86: 1173-1177), Qb Replicase (see, e.g., Lizardi, et al, 1988, BioTechnology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

All sequence variants can be confirmed by multiple independent PCR amplifications. Primers useful according to the present invention are designed using amino acid sequences of the protein or nucleic acid sequences of the kinase domain of the erbB1 gene as a guide, e.g., SEQ ID NO: 493, SEQ ID NO: 494, SEQ ID NO: 509, and SEQ ID NO: 510. The primers are designed in the homologous regions of the gene wherein at least two regions of homology are separated by a divergent region of variable sequence, the sequence being variable either in length or nucleic acid sequence.

Primers for PCR reactions are obtained my methods available and known to one of ordinary skill in the art, utilizing the known EGFR nucleotide/protein sequence. For example, the cDNA sequences of receptor tyrosine kinases may be obtained from GenBank and compared to the human genome assembly using the BLAT alignment to identify exon/intron boundaries. External gene specific primer pairs are designed to amplify exon sequences and at least 250 bp of flanking intronic sequence or adjacent exonic sequence on each side using the PrimerS program. The resulting predicted amplicons are then used to design internal primers flanking the exon (generally greater than 50 bp from the exon/intron boundary) and containing appended M13 forward or reverse primer tails. These nested primer sets are tested for appropriate amplicon size and high-quality sequence from control DNA. Amplicons encompassing exons encoding the receptor tyrosine kinase activation loop of 47 tyrosine kinases are amplified and sequenced from test samples, e.g., cells enriched utilizing the microfluidic device of the invention. In addition, amplicons covering the full length EGFR are also amplified. Exemplary primers and other sequences for use in the methods of the invention are shown in Table 2.

**Table 2**

| SEQUENCE | SEQ ID NO |
|---|---|
| CAGGAATGGGTGAGTCTCTGTGTG | 567 |
| GTGGAATTCTGCCCAGGCCTTTC | 568 |
| GATTCTACAAACCAGCCAGCCAAAC | 569 |
| CCTACTGGTTCACATCTGACCCTG | 570 |
| GTTTGAATGTGGTTTCGTTGGAAG | 571 |
| CTTTGTCACCAGGCAGAGGGCAATATC | 572 |
| GACAGTAACTTGGGCTTTCTGAC | 573 |
| CATCCACCCAAAGACTCTCCAAG | 574 |
| CTGTTCATATAATACAGAGTCCCTG | 575 |
| GAGAGATGCAGGAGCTCTGTGC | 576 |
| GCAGTTTGTAGTCAATCAAAGGTGG | 577 |
| GTAATTTAAATGGGAATAGCCC | 578 |
| CAACTCCTTGACCATTACCTCAAG | 579 |
| GATGGCCGTCCTGCCCACACAGG | 580 |
| GAGTAGTTTAGCATATATTGC | 581 |
| GACAGTCAGAAATGCAGGAAAGC | 582 |
| CAAGTGCCGTGTCCTGGCACCCAAGC | 583 |
| CCAAACACTCA GTGAAACAAAGAG | 584 |
| GCACCCAAGCCCATGCCGTGGCTGC | 677 |
| GAAACAAAGAGTAAAGTAGATGATGG | 678 |
| CCTTAGGTGCGGCTCCACAGC | 585 |
| CATTTAGGATGTGGAGATGAGC | 586 |
| GAAACTCAAGATCGCATTCATGC | 587 |
| GCAAACTCTTGCTATCCCAGGAG | 588 |
| CAGCCATAAGTCCTCGACGTGG | 589 |
| CATCCTCCCCT GCATGTGTTAAAC | 590 |
| GTAGGTTTCTAAACATCAAGAAAC | 591 |
| GTGATGACATTTCTCCAGGGATGC | 592 |
| CATCACCAATGCCTTCTTTAAGC | 593 |
| GCTGGAGGGTTTAATAATGCGATC | 594 |
| GCAAACACACAGGCACCTGCTGGC | 595 |
| CATTTCCATGTGAGTTTCACTAGATGG | 596 |
| CACCTTCACAATATACCCTCCATG | 679 |
| GACAGCCGTGCAGGGAAAAACC | 680 |
| GAACCAGCATCTCAAGGAGATCTC | 681 |

(continued)

| SEQUENCE | SEQ ID NO |
|---|---|
| GAGCACCTGGCTTGGACACTGGAG | 682 |
| GACCGGACGACAGGCCACCTCGTC | 597 |
| GAAGAACGAAACGTCCCGTTCCTCC | 598 |
| GTTGAGCACT CGTGTGCATTAGG | 599 |
| CTCAGTGCACGTGTACTGGGTA | 600 |
| GTTCACTGGGCTAATTGCGGGACTCTTGTTCGCAC | 601 |
| GGTA AATACATGCTTTTCTAGTGGTCAG | 602 |
| GGAGGATGGA GCCTTTCCATCAC | 603 |
| GAAGAGGAAGATGTGTTCCTTTGG | 604 |
| GAATGAAGGATGATGTGGCAGTGG | 605 |
| GTATGTGTGAAGGAGTCACTGAAAC | 606 |
| GGTGAGTCACAGGTTCAGTTGG | 607 |
| CAAAACATCAGCCATTAACGG | 608 |
| GTAGCCAGCATGTC TGTGTCAC | 609 |
| CAGAATGCCTGTAAAGCTATAAC | 610 |
| CATTTGGCTTTCCCCACTCACAC | 611 |
| GACCAAAACACCTTAAGTAACTGACTC | 612 |
| GAAGCTACATAGTGTCTCACTTTCC | 613 |
| CACAACTGCTAATGGCCCGTTCTCG | 614 |
| GAGCAGCCCTGAACTCCGTCAGACTG | 683 |
| CTCAGTACAATAGATAGACAGCAATG | 684 |
| GCTCC TGCTCCCTGTCATAAGTC | 615 |
| GAAGTCCTGCTGGTAGTCAGGGTTG | 616 |
| CTGCAGTGGGCAACCCCGAGTATC | 617 |
| CAGTCTGTGGGTCTAAGAGCTAATG | 618 |
| GACAGGCCACCTCGTCGGCGTC | 619 |
| CAGCTGATCTCAAGGAAACAGG | 620 |
| CTCGTGTGCATTAGGGTTCAACTGG | 621 |
| CCTTCTCCGAGGTGGAATTGAGTGAC | 622 |
| GCTAATTGCGGGACTCTTGTTCGCAC | 623 |
| TACATGCTTT TCTAGTGGTCAG | 624 |
| CCTTTCCATCACCCCTCAAGAGG | 625 |
| GATGTGTTCCTTTGGAGGTGGCATG | 626 |
| GATGTGGCAGTGGCGGTTCCGGTG | 627 |
| GGAGTCACTGAAACAAACAACAGG | 628 |
| GGTTCAGTTGCTTGTATAAAG | 629 |
| CCATTAACGGTAAAATTTCAGAAG | 630 |
| CCAAGGTCATGGAGCACAGG | 631 |

(continued)

| SEQUENCE | SEQ ID NO |
|---|---|
| CTGTAAAGCTATAACAACAACCTGG | 632 |
| CCACTCACACACACTAAATATTTTAAG | 633 |
| GTAACTGACTCAAATACAAACCAC | 634 |
| GAAGCTACATAGTGTCTCACTTTCC | 635 |
| CACAACTGCTAATGGCCCGTTCTCG | 636 |
| GACGGGTCCTGGGGTGATCTGGCTC | 685 |
| CTCAGTACAATAGATAGACAGCAATG | 686 |
| CCTGTCATAAG TCTCCTTGTTGAG | 637 |
| GGTAGTCAGGGTTGTCCAGG | 638 |
| CGAGTATCTCAACACTGTCCAGC | 639 |
| CTAAGAGCTAATGCGGGC ATGGCTG | 640 |
| GCAATATCAGCCTTAGGTGCGGCTC | 505 |
| CATAGAA AGTGAACATTTAGGATGTG | 506 |
| CTAACGTTCG CCAGCCATAAGTCC | 507 |
| GCTGCGAGCTCACCCAG AATGTCTGG | 508 |
| CAGATTTGGCTCGACCTGGACATAG | 513 |
| CAGCTGATCTCAAGGAAACAGG | 514 |
| GTATTATCAGTCAC TAAAGCTCAC | 515 |
| CACACTTCAAGTGGAATTCTGC | 516 |
| CTCGTGTGCATTAGGGTTCAACTGG | 517 |
| CCTTCTCCGAGGTGGAATTGAGTGAC | 518 |
| GCTAATTGCGGGACTCTTGTTCGCAC | 519 |
| TACATGC TTTTCTAGTGGTCAG | 520 |
| GGTCTCAAGTGATTCTACAAACCAG | 521 |
| CCTTCACCTACTGGTTCACATCTG | 522 |
| CATGGT TTGACTTAGTTTGAATGTGG | 523 |
| GGATACTAAAGATACTTTGTCAC CAGG | 524 |
| GAACACTAGGCTGCAAAGACAGTAAC | 525 |
| CCAAGCAAGGCAAACACATCCACC | 526 |
| GGAGGATGGAGCCTTTCCATCAC | 527 |
| GAAGAGGAAGATGTGTTCCTTTGG | 528 |
| GAATGAAGGATGATGTGGCAGTGG | 529 |
| CAAAACATCAGCCATTAACGG | 530 |
| CCACTTACTGTTCATATAATACAGAG | 531 |
| CATGTGAGATAGCATTTGGGAATGC | 532 |
| CATGACCT ACCATCATTGGAAAGCAG | 533 |
| GTAATTTCACAGTTAGGAATC | 534 |
| GTCACCCAAGGTCATGGAGCACAGG | 535 |

(continued)

| SEQUENCE | SEQ ID NO |
|---|---|
| CAGAATGC CTGTAAAGCTATAAC | 536 |
| GTCCTGGAGTCCCAACTCCTTGAC | 537 |
| GGAAGTGGCTCTGA TGGCCGTCCTG | 538 |
| CCAC TCACACACACTAAATATTTTAAG | 539 |
| GACCAAAACACCTTAAGTAA CTGACTC | 540 |
| CCAA TCCAACATCCAGACACATAG | 541 |
| CCAGAGCCATAGAAACTTGATCAG | 542 |
| GTATGGACTATGGCACTTCAATTGCATGG | 543 |
| CCAGAGAACATGGCAACCAGCACAGGAC | 544 |
| CAAATGAGCTGGCAAGTGCCGTGTC | 545 |
| GAGTTTCCCAAACACTCAGTGAAAC | 546 |
| CAAGTGCCGTGTCCTGGCAGCCAAGC | 675 |
| CCAAACACTCAGTGAAACAAAGAG | 676 |
| GCAATATCAGCC TTAGG TGCGGCTC | 547 |
| CATAGAAAGTGAACATTTAGGATGTG | 548 |
| CCATGAGTACGTATTTTGAAACTC | 549 |
| CATATCC CCATGGC AAACTCTTGC | 550 |
| CTAACGTTCGCCAG CCATAAGTCC | 551 |
| GCTGCGAGCTCACCCAGAATGTCTGG | 552 |
| GACGGGTCCTGGGGTGATCTGGCTC | 553 |
| CTCAGTACAATAGATAGACAGCAATG | 684 |
| CAGGACTACAGAAATGTAGGTTTC | 555 |
| GTGCCTG CCTTAAGTAATGTGATGAC | 556 |
| GACTGGAAGTGTCGCATCACCAATG | 557 |
| GGTTTAATAATGCGATCTGGGACAC | 558 |
| GCAGCTATAATTTAGAGAACCAAGG | 559 |
| AAAATTGACTTCATTTCCATG | 560 |
| CCTAGTTGCTCTAAA ACTAACG | 561 |
| CTGTGAGGCGTGACAGCCGTGCAG | 562 |
| CAACCTACTAATCAG AACCAGCATC | 563 |
| CCTTCACTGTGTCTGC AAATCTGC | 564 |
| CCTGTCATAAGTCTCCTTGTTGAG | 565 |
| CAGTCTGTGGGTCTAAGAGCTAATG | 566 |
| CAGGAATGGGTGAGTCTCTGTGTG | 567 |
| GTGGAATTCTGCCCAGGCCTTTC | 568 |
| GATTCTACAAACCAGCCAGCCAAAC | 569 |
| CCTACTGGTTCACATCTGACCCTG | 570 |
| GTTTGAATGTGGTTTCGTTGGAAG | 571 |

(continued)

| SEQUENCE | SEQ ID NO |
|---|---|
| CTTTGTCACCAGG CAGAGG GCAATATC | 572 |
| GACAGTAACTTGGGCTTTCTGAC | 573 |
| CATCCACCCAAAGACTCTCCAAG | 574 |
| CTGTTCATATAATACAGAGTCCCTG | 575 |
| GAGAGATGCAGGAGCTCTGTGC | 576 |
| GCAGTTTGTAGTCAATCAAAGGTGG | 577 |
| GTAATTTAAATGGGAAT AGCCC | 578 |
| CAACTCCTTGACCATTACCTCAAG | 579 |
| GATGGCCGTCCTGCCCACACAGG | 580 |
| GAGTAGTTTAGCA TATATTGC | 581 |
| GACAGTCAGAAATGCAGGAAAGC | 582 |
| CAAGTGCCGTGTCCTGGCACCCAAGC | 583 |
| CCAAACACTCA GTGAAACAAAGAG | 584 |
| GCACCCAAGCCCATGCCGTGGCTGC | 677 |
| GAAACAAAGAGTAAAGTAGATGATGG | 678 |
| CCTTAGGTGCGGCTCCACAGC | 585 |
| CATTTAGGATGTGGAGATGAGC | 586 |
| GAAACTCAAG ATCGCATTCATGC | 587 |
| GCAAACTCTTGCTATCCCAGGAG | 588 |
| CAGCCATAAGTCCTCGACGTGG | 589 |
| CATCCTCCCCT GCATGTGTTAAAC | 590 |
| GTAGGTTTCTAAACATCAAGAAAC | 591 |
| GTGATGACATTTCTCCAGGGATGC | 592 |
| CATCACCA ATGCCTTCTTTAAGC | 593 |
| GCTGGAGGGTTTAATAATGCGATC | 594 |
| GCAAACACACAGGCACCTGCTGGC | 595 |
| CATTTCCATGTGAGTTTCACTAGATGG | 596 |
| CACCTTCACAATATACCCTCCATG | 679 |
| GACAGCCGTGCAGGGAAAAACC | 680 |
| GAACCAGCATCTCAAGGAGATCTC | 681 |
| GAGCACCTGGCTTGGACACTGGAG | 682 |
| GACCGGACGACAGGCCACCTCGTC | 597 |
| GAAGAACGAAACGTCCCGTTCCTCC | 598 |
| GTTGAGCACTCGTGTGCATTAGG | 599 |
| CTCAGTGCACGTGTACTGGGTA | 600 |
| GTTCACTGGGCTAATTGCGGGACTCTTGTTCGCAC | 601 |
| GGTAAATACATGCTTTTCTAGTGGTCAG | 602 |
| GGAGGATGGAGCCTTTCCATCAC | 603 |

(continued)

| SEQUENCE | SEQ ID NO |
|---|---|
| GAAGAGGAAGATGTGTTCCTTTGG | 604 |
| GAATGAAGGATGATGTGGCAGTGG | 605 |
| GTATGTGTGAAGGAG TCACTGAAAC | 606 |
| GGTGAGTCACAGGTTCAGTTGG | 607 |
| CAAAACATCAGCCATTAACGG | 608 |
| GTAGCCAGCATGTC TGTGTCAC | 609 |
| CAGAATGCCTGTAAAGCTATAAC | 610 |
| CATTTGGCTTTCCCCACTCACAC | 611 |
| GACCAAAACACCTTAA GTAACTGACTC | 612 |
| GAAGCTACATAGTGTCTCACTTTCC | 613 |
| CACAACTGCTAATGGCCCGTTCTCG | 614 |
| GAGCAGCCCTGAACTCCGTCAGACTG | 683 |
| CTCAGTACAATAGATAGACAGCAATG | 684 |
| GCTCC TGCTCCCTGTCATAAGTC | 615 |
| GAAGTCCTGCTGGTAGTCAGGGTTG | 616 |
| CTGCAGTGGGCAACCCCGAGTATC | 617 |
| CAGTCTGTGGGTCTAAGAGCTAATG | 618 |
| GACAGGCCACCTCGTCGGCGTC | 619 |
| CAGCTGATCTCAAGGAAACAGG | 620 |
| CTCGTG TGCATTA GGGTTCAACTGG | 621 |
| CCTTCTCCGAGGTGGAATTGAGTGAC | 622 |
| GCTAATTGCGGGACTCTTGTTCGCAC | 623 |
| TACATGCTTT TCTAGTGGTCAG | 624 |
| CCTTTCCATCACCCCTCAAGAGG | 625 |
| GATGTGTTCCTTTGGAGGTGGCATG | 626 |
| GATGTGG CAGTGGCGGTTCCGGTG | 627 |
| GGAGTCACTGAAACAAACAACAGG | 628 |
| GGTTCAGTTGCTTGTATAAAG | 629 |
| CCATTAACGGT AAAATTTCAGAAG | 630 |
| CCAAGGTCATGGAGCACAGG | 631 |
| CTGTAAAGCTATAACAACAACCTGG | 632 |
| CCACTCACA CACACTAAATATTTTAAG | 633 |
| GTAACTGACTCAAATACAAACCAC | 634 |
| GAAGCTACATAGTGTCTCACTTTCC | 635 |
| CACAACTGCTAATGGCCCGTTCTCG | 636 |
| GACGGGTCCTGGGGTGATCTGGCTC | 685 |
| CTCAGTACAATAGATAGACAGCAATG | 686 |
| CCTGTCATAAG TCTCCTTGTTGAG | 637 |

(continued)

| SEQUENCE | SEQ ID NO |
|---|---|
| GGTAGTCAGGGTTGTCCAGG | 638 |
| CGAGTATCTCAACACTGTCCAGC | 639 |
| CTAAGAGCTAATGCGGGCATGGCTG | 640 |
| KIPVAIKELREATSPKAN | 509 |
| FGLAKLLG | 510 |
| AAAATTCCCGTCGCTATCAAGGAATTAAGAGAAGCAACATCTCC GAAAGCCAAC | 493 |
| TTTGGGCTGGCCAAACTGCTGGGT | 494 |
| AAAATTCCCGTCGCTATCAA-AACATCTCCGAAAGCAAC | 495 |
| TTTGGGCTGGCCAAACTGCTGGGT | 496 |
| AAAATTCCCGTCGCTATCAAGGAAT-CATCTCCGAAAGCCAAC | 497 |
| TTTGGGCTGGCCAAACTGCTGGGT | 498 |
| AAAATTCCCGTCGCTATCAAGGAAT-CGAAAGCCAAC | 499 |
| TTTGGGCTGGCCAAACTGCTGGGT | 500 |
| AAAATTCCCGTCGCTATCAAGGAATTAAGAGAAGCAACATCTCC GAAAGCCAAC | 501 |
| TTTGGGCGGGCCAAACTGCTGGGT | 502 |
| AAAATTCCCGTCGCTATCAAGGAATTAAGAGAAGCAACATCTCC GAAAGCCAAC | 503 |
| TTTGGGCTGGCCAAACAGCTGGGT | 504 |
| TGTAAAACGACGGCCAGTCGCCCAGACCGGACGACA | 447 |
| CAGGAAACAGCTATGACCAGGGCAATGAGGACATAACCA | 448 |
| TGTAAAACGACGGCCAGTGGTGGTCCTTGGGAATTTGG | 449 |
| CAGGAAACAGCTATGACCCCATCGACATGTTGCTGAGAAA | 450 |
| TGTAAAACGACGGCCAGTGAAGGAGCTGCCCATGAGAA | 451 |
| CAGGAAACAGCTATGACCCGTGGCTTCGTCTCGGAATT | 452 |
| TGTAAAACGACGGCCAGTGAAACTGACCAAAATCATCTGT | 453 |
| CAGGAAACAGCTATGACCTACCTATTCCGTTACACACTTT | 454 |
| TGTAAAACGACGGCCAGTCCGTAATTATGTGGTGACAGAT | 455 |
| CAGGAAACAGCTATGACCGCGTATGATTTCTAGGTTCTCA | 456 |
| TGTAAAACGACGGCCAGTCTGAAAACCGTAAAGGAAATCAC | 457 |
| CAGGAAACAGCTATGACCCCTGCCTCGGCTGACATTC | 458 |
| TGTAAAACGACGGCCAGTTAAGCAACAGAGGTGAAAACAG | 459 |
| CAGGAAACAGCTATGACCGGTGTTGTTTTCTCCCATGACT | 460 |
| TGTAAAACGACGGCCAGTGGACCAGACAACTGTATCCA | 461 |
| CAGGAAACAGCTATGACCTTCCTTCAAGATCCTCAAGAGA | 462 |
| TGTAAAACGACGGCCAGTGATCGGCCTCTTCATGCGAA | 463 |

(continued)

| SEQUENCE | SEQ ID NO |
|---|---|
| CAGGAAACAGCTATGACCACGGTGGAGGTGAGGCAGAT | 464 |
| TGTAAAACGACGGCCAGTCGAAAGCCAACAAGGAAATCC | 465 |
| CAGGAAACAGCTATGACCATTCCAATGCCATCCACTTGAT | 466 |
| TGTAAAACGACGGCCAGTAACACCGCAGCATGTCAAGAT | 467 |
| CAGGAAACAGCTATGACCCTCGGGCCATTTTGGAGAATT | 468 |
| TGTAAAACGACGGCCAGTTCAGCCACCCATATGTACCAT | 469 |
| CAGGAAACAGCTATGACCGCTTTGCAGCCCATTTCTATC | 470 |
| TGTAAAACGACGGCCAGTACAGCAGGGCTTCTTCAGCA | 471 |
| CAGGAAACAGCTATGACCTGACACAGGTGGGCTGGACA | 472 |
| TGTAAAACGACGGCCAGTGAATCCTGTCTATCACAATCAG | 473 |
| CAGGAAACAGCTATGACCGGTATCGAAAGAGTCTGGATTT | 474 |
| TGTAAAACGACGGCCAGTGCTCCACAGCTGAAAATGCA | 475 |
| CAGGAAACAGCTATGACCACGTTGCAAAACCAGTCTGTG | 476 |
| 2155G>A | 425 |
| 2155G>A | 426 |
| 2235_2249delGGAATTAAGAGAAGC | 427 |
| 2235_2249delGGAATTAAGAGAAGC | 428 |
| 2235_2249delGGAATTAAGAGAAGC | 429 |
| 2235_2249delGGAATTAAGAGAAGC | 430 |
| 2235_2249delGGAATTAAGAGAAGC | 431 |
| 2235_2249delGGAATTAAGAGAAGC | 432 |
| 2235_2249delGGAATTAAGAGAAGC | 433 |
| 2236_2250delGAATTAAGAGAAGCA | 434 |
| 2236_2250delGAATTAAGAGAAGCA | 435 |
| 2236_2250delGAATTAAGAGAAGCA | 436 |
| 2254_2277delTCTCCGAAAGCCAACAAGGAAATC | 437 |
| 2573T>G | 438 |
| 2573T>G | 439 |
| 2573T>G | 440 |
| CATTTCCCCTAATCCTTTTCCA | 213 |
| GTGATCCCAGATTTAGGCCTTC | 214 |
| GCCTCTCGTGGTTTGTTTTGTC | 215 |
| CCCAGGGTAGGGTCCAATAATC | 216 |
| CTTCCTGGTGGAGGTGACTGAT | 217 |
| CAGGCATAGTGTGTGATGGTCA | 218 |
| TCACGATACACATTCTCAGATCC | 219 |
| GAAGATCTCCCAGAGGAGGATG | 220 |
| CGTAACGTGCTGTTGACCAAT | 221 |

(continued)

| SEQUENCE | SEQ ID NO |
|---|---|
| AAACGAGGGAAGAGCCAGAAAG | 222 |
| TGGGGAGCACAATAAAAGAAGA | 223 |
| ACTCTTGGCTCCTGGATTCTTG | 224 |
| GGAAGTCAGTGTGCAGGGAATA | 225 |
| TTTTAGCAGAAATAGGCAAGCA | 226 |
| TGGTAATCCTAAACACAATGCAGA | 227 |
| CTGGGCAACACAGTGAGATCCT | 228 |
| TCACAAATTTCTTTGCTGTGTCC | 229 |
| CATGGAACTCCAGATTAGCCTGT | 230 |
| GATTGTTGCAGATCGTGGACAT | 231 |
| CGCTTAAATCTTCCCATTCCAG | 232 |
| CTCCATGGCACCATCATTAACA | 233 |
| CTCAGGACACAAGTGCTCTGCT | 234 |
| GCAGTTCATGGTTCATCTTCTTTT | 235 |
| CAAAATAGCCCACCCTGGATTA | 236 |
| CTTTCTGCATTGCCCAAGATG | 237 |
| CAAGGTCTCAGTGAGTGGTGGA | 238 |
| GAGAAGGGTCTTTCTGACTCTGC | 239 |
| CAGGTGTTTCTCCTGTGAGGTG | 240 |
| CACATTGCGGCCTAGAATGTTA | 241 |
| ACCCCGTCACAACCTTCAGT | 242 |
| GCCGTAGCCCCAAAGTGTACTA | 243 |
| TCAGCTCAAACCTGTGATTTCC | 244 |
| CTCACTCTCCATAAATGCTACGAA | 245 |
| GACTTAACGTGTCCCCTTTTGC | 246 |
| GCCTCTTCGGGGTAATCAGATA | 247 |
| GAAGTCTGTGGTTTAGCGGACA | 248 |
| ATCTTTTGCCTGGAGGAACTTT | 249 |
| CAGGGTAAATTCATCCCATTGA | 250 |
| CAGCAGCCAGCACAACTACTTT | 251 |
| TTGGCTAGATGAACCATTGATGA | 252 |
| TGAATGAAGCTCCTGTGTTTACTC | 253 |
| ATGTTCATCGCAGGCTAATGTG | 254 |
| AAAACAGGGAGAACTTCTAAGCAA | 255 |
| CATGGCAGAGTCATTCCCACT | 256 |
| CAATGCTAGAACAACGCCTGTC | 257 |
| TCCCTCCACTGAGGACAAAGTT | 258 |
| GGGAGAGCTTGAGAAAGTTGGA | 259 |

(continued)

| SEQUENCE | SEQ ID NO |
|---|---|
| ATTTCCTCGGATGGATGTACCA | 260 |
| TCAGAGCCTGTGTTTCTACCAA | 261 |
| TGGTCTCACAGGACCACTGATT | 262 |
| AAATAATCAGTGTGATTCGTGGAG | 263 |
| GAGGCCAGTGCTGTCTCTAAGG | 264 |
| ACTTCACAGCCCTGCGTAAAC | 265 |
| ATGGGACAGGCACTGATTTGT | 266 |
| GCAGCGGGTTACATCTTCTTTC | 267 |
| CAGCTCTGGCTCACACTACCAG | 268 |
| CCTGAACTCCGTCAGACTGAAA | 269 |
| GCAGCTGGACTCGATTTCCT | 270 |
| CCTTACAGCAATCCTGTGAAACA | 271 |
| TGCCCAATGAGTCAAGAAGTGT | 272 |
| ATGTACAGTGCTGGCATGGTCT | 273 |
| CACTCACGGATGCTGCTTAGTT | 274 |
| TAAGGCACCCACATCATGTCA | 275 |
| TGGACCTAAAAGGCTTACAATCAA | 276 |
| GCCTTTTAGGTCCACTATGGAATG | 277 |
| CCAGGCGATGCTACTACTGGTC | 278 |
| TCATAGCACACCTCCCTCACTG | 279 |
| ACACAACAAAGAGCTTGTGCAG | 280 |
| CCATTACTTTGAGAAGGACAGGAA | 281 |
| TATTCTTGCTGGATGCGTTTCT | 282 |
| AGGAGGGCAGAGGACTAGCTG | 283 |
| GGCAATGTGAATGTGCACTG | 284 |
| CTTGAACCTGGGAGGTGGAG | 285 |
| ATCAGGGTGGGAGGAGTAAAGA | 286 |
| CCCACTTACCTCTCACCTGTGC | 287 |
| GTGAACTTCCGGTAGGAAATGG | 288 |
| AGGGGACCTCAAGGGAGAAG | 289 |
| AGATCATGCCAGTGAACTCCAG | 290 |
| GGACCAGGAAAGTCCTTGCTTT | 291 |
| GGTGGGGAACATTAAACTGAGG | 292 |
| GCTTCAGGTTGTTTTGTTGCAG | 293 |
| ACCCTTGCTTGAGGGAAATATG | 294 |
| CCCAGCTCCTAGGGTACAGTCT | 295 |
| CAGTCAGCTTCAAAATCCCTCTT | 296 |
| TCACTTCCCTGTGAGTAAAGAAAA | 297 |

(continued)

| SEQUENCE | SEQ ID NO |
|---|---|
| GGCCATTTAATTCTTGTCCTTGA | 298 |
| TGGACTTGTGCAAACTCAAACTG | 299 |
| TCCCAATATAGGGCAGTCATGTT | 300 |
| TCTCAATCAGTTGAGTTGCCTTG | 301 |
| AGCTGTGCAAGTGTGGAAACAT | 302 |
| GCTGTGAGGGTAAATGAGACCA | 303 |
| GTCTCCTGGTGAGTGACTGTGG | 304 |
| CCTTCCTTCGTCTCCACAGC | 305 |
| GTCCTTGTGCCAACAGTCGAG | 306 |
| GCTTGGCAAGGAGAAGAGAACA | 307 |
| GCTTGCTTTCTTGCTTGAACAAC | 308 |
| GCTGGTCACCTTGAGCTTCTCT | 309 |
| CCATGCTGGGCTCTTTGATTA | 310 |
| CACCACTCTGAAGTTGGCCTCT | 311 |
| ATGGCTCTGCACATTTGTTCC | 312 |
| CAGAGTGGGAAAAGGCACTTCA | 313 |
| CCAGAGTCCTGTGCAGACATTC | 314 |
| ATGGGGATTAACTGGGATGTTG | 315 |
| CGTAGCTCCAGACATCACTAGCA | 316 |
| GCAACCTGGTCTGCAAAGTCTC | 317 |
| ACCCAGCAGTCCAGCATGAG | 318 |
| TCAGAGCCTGTGTTTCTACCAA | 653 |
| TGGTCTCACAGGACCACTGATT | 646 |
| AAATAATCAGTGTGATTCGTGGAG | 654 |
| GAGGCCAGTGCTGTCTCTAAGG | 647 |
| ACTTCACAGCCCTGCGTAAAC | 655 |
| ATGGGACAGGCACTGATTTGT | 648 |
| GCAGCGGGTTACATCTTCTTTC | 656 |
| CAGCTCTGGCTCACACTACCAG | 649 |
| CCTGAACTCCGTCAGACTGAAA | 657 |
| GCAGCTGGACTCGATTTCCT | 650 |
| CCTTACAGCAATCCTGTGAAACA | 658 |
| TGCCCAATGAGTCAAGAAGTGT | 651 |
| ATGTACAGTGCTGGCATGGTCT | 659 |
| CACTCACGGATGCTGCTTAGTT | 652 |
| TCCAAATGAGCTGGCAAGTG | 660 |
| TCCCAAACACTCAGTGAAACAAA | 667 |
| GTGCATCGCTGGTAACATCC | 661 |

(continued)

| SEQUENCE | SEQ ID NO |
|---|---|
| TGTGGAGATGAGCAGGGTCT | 668 |
| ATCGCATTCATGCGTCTTCA | 662 |
| ATCCCCATGGCAAACTCTTG | 669 |
| GCTCAGAGCCTGGCATGAA | 663 |
| CATCCTCCCCTGCATGTGT | 670 |
| TGGCTCGTCTGTGTGTGTCA | 664 |
| CGAAAGAAAATACTTGCATGTCAGA | 671 |
| TGAAGCAAATTGCCCAAGAC | 665 |
| TGACATTTCTCCAGGGATGC | 672 |
| AAGTGTCGCATCACCAATGC | 666 |
| ATGCGATCTGGGACACAGG | 673 |
| TGTAAAACGACGGCCAGT | 645 |
| AACAGCTATGACCATG | 674 |

As noted above, preferably, the variant in the kinase domain of EGFR is an in frame deletion or a substitution in exon 18, 19, 24 or 21. However, all the analysis discussed herein, i.e., generally genetic tests that detect mutations in the kinase domain of EGFR, can be applied to additional or all exons. In one embodiment, DNA is first obtained from cells that are isolated from blood, e.g., CTCs. The DNA sequence of 7 exons (18, 19, 20, 21, 22, 23, 24) of EGFR is then determined by direct bi-directional gene sequencing. The sequence obtained is then compared to known EGFR sequence (e.g., SEQ ID NO: 511, Fig. 71) to identify DNA sequence changes. If a DNA sequence change is detected, the test may be repeated on the original sample. If the change has not previously been reported in a gefitinib- or erlotinib-responder, the test will also be conducted with a sample from reference or control cells, from the same or different source (e.g., patient or animal), so as to determine whether the mutation is constitutive (and therefore likely a normally occurring polymorphism) or occurred somatically in the tumor tissue. If PCR amplification for an individual sample fails, a new round of PCR should be attempted with a two-fold increase in input DNA template. For example, the DNA can undergo pre-amplification. Appropriate pre-amplification methods include multiple displacement amplification, and linear amplification methods such as in vitro transcription, or primer extension whole genome pre-amplification. If PCR amplification fails again, a new DNA sample for that patient should be acquired if available.

In one embodiment, the in-frame deletion is in exon 19 of EGFR (erbB1). The in-frame deletion in exon 19 preferably includes deletion of at least amino acids leucine, arginine, glutamic acid and alanine, at codons 747, 748, 749, and 750. In one embodiment, the in-frame deletion includes nucleotides 2235 to 2249 and deletes amino acids 746 to 750 (the sequence glutamic acid, leucine, arginine, glutamic acid, and alanine). In another embodiment, the in-frame deletion includes nucleotides 2236 to 2250 and deletes amino acids 746 to 750. Alternatively, the in-frame deletion includes nucleotides 2240 to 2251, or nucleotides 2240 to 2257. Alternatively, the in-frame deletion includes nucleotides 2239 to 2247 together with a substitution of cytosine for guanine at nucleotide 2248, or a deletion of nucleotides 2238 to 2255 together with a substitution of thymine for adenine at nucleotide 2237, or a deletion of nucleotides 2254 to 2277. Alternatively, the in-frame deletion includes nucleotides 2239-2250delTTAAGAGAAGCA; 2251A>C, or 2240-2254delTAAGAGAAGCA, or 2257-2271delCCGAAAGCCAACAAG. Additional mutations may be an EGFR mutation that substitutes a glycine (G) for a valine (V) at position 857 ("G857V") or one that substitutes a leucine (L) with a serine (S) at position 883 ("L883S") in a metastatic sarcoma. Figs. 72A-E and Table 3 show exemplary EGFR mutations.

**Table 3: Epidermal Growth Factor Receptor Somatic Gene Mutations Identified**

| Histology | Exon | Nucleotide Change | Amino Acid Change |
|---|---|---|---|
| Adeno | 18<br>18 | 2126A>T<br>2155G>A | E709V<br>G719S |
| A+BAC | 18<br>20 | 2156G>C<br>2327G>A | G719A<br>R776H |

(continued)

| Histology | Exon | Nucleotide Change | Amino Acid Change |
|---|---|---|---|
| A+BAC | 19 | 2235_2249 del | K745_A750 del ins K |
| A+BAC | 19 | 2235_2249 del | K745_A750 del ins K |
| Adeno | 19 | 2235_2249 del | K745_A750 del ins K |
| Adeno | 19 | 2235_2249 del | K745_A750 del ins K |
| Adeno | 19 | 2236 2250 del | E746_A750 del |
| Adeno | 19 | 2236_2250 del | E746_A750 del |
| Adeno | 19 | 2236_2250 del | E746_A750 del |
| A+BAC | 19 | 2237_2255 del ins T | E746_S752 del ins V |
| Adeno | 19 | 2239_2248 del ins C | L747_A750 del ins P |
| A+BAC | 19 | 2239_2251 del ins C | L747_T751 del ins P |
| Adeno | 19 | 2253_2276 del | T751_I759 del ins T |
| Adeno | 19 | 2254_2277 del | S752_1759 del |
| Adeno | 20 | 2303_2311 dup | D770_N771 ins SVD |
| Adeno | 20 | 2313_2318 dup CCCCCA | P772_H773 dup |
| Adeno | 21 | 2543C>T | P848L* |
| BAC | 21 | 2573T>G | L858R |
| A+BAC | 21 | 2573T>G | L858R |
| A+BAC | 21 | 2573T>G | L858R |
| Adeno | 21 | 2573T>G | L858R |
| Adeno | 21 | 2573T>G | L858R |
| Adeno | 21 | 2582T>A | L861Q |
| Adeno - Adenocarcinoma, Adeno+BAC = Adenocarcinoma with Bronchioloalveolar Carcinoma Features, BAC = Pure Bronchioloalveolar Carcinoma<br>* This mutation was identified as a germline variant. | | | |

In another embodiment, the substitution is in exon 21 of EGFR. The substitution in exon 21 includes at least one amino acid. In one embodiment, the substitution in exon 21 includes a substitution of a guanine for a thymine at nucleotide 2573. This substitution results in an amino acid substitution, where the wildtype Leucine is replaced with an Arginine at amino acid 858. Alternatively, the substitution in exon 21 includes a substitution of an adenine for a thymine at nucleotide 2582. This substitution results in an amino acid substitution, where the wild type Leucine is replaced with a Glutamine at amino acid 861.

The substitution may also be in exon 18 of EGFR. In one embodiment, the substitution is in exon 18 is a thymine for a guanine at nucleotide 2155. This substitution results in an amino acid substitution, where the wildtype Glycine is substituted with a Cysteine at codon 719. In another embodiment, the substitution in exon 18 is an adenine for a guanine at nucleotide 2155 resulting in an amino acid substitution, where the wildtype Glycine is substituted for a Serine at codon 719.

In another embodiment, the substitution is an insertion of guanine, guanine and thymine (GOT) after nucleotide 2316 and before nucleotide 2317 of SEQ ID NO: 511 (2316_2317 ins GGT). This can also be described as an insertion of valine (V) at amino acid 772 (P772_H733 insV). Other mutations include, for example, an insertion of CAACCCGG after nucleotide 2309 and before nucleotide 2310 of SEQ ID NO: 511 and an insertion of GCGTGGACA after nucleotide 2311 and before nucleotide 2312 of SEQ ID NO: 511. The substitution may also be in exon 20 and in one embodiment is a substitution of AA for GG at nucleotides 2334 and 2335.

Therefore, in preferred embodiments, the nucleic acid variant of the erbB1 gene is a substitution of a thymine for a guanine or an adenine for a guanine at nucleotide 2155 of SEQ ID NO: 511, a deletion of nucleotides 2235 to 2249, 2240 to 2251, 2240 to 2257, 2236 to 2250, 2254 to 2277, or 2236 to 2244 of SEQ ID NO: 511, an insertion of nucleotides guanine, guanine, and thymine (GGT) after nucleotide 2316 and before nucleotide 2317 of SEQ ID NO: 511, and a substitution of a guanine for a thymine at nucleotide 2573 or an adenine for a thymine at nucleotide 25 82 of SEQ ID NO: 511.

The detection of the presence or absence of at least one nucleic acid variant can be detennined by amplifying a segment of nucleic acid encoding the receptor. The segment to be amplified is 1000 nucleotides in length, preferably, 500 nucleotides in length, and most preferably 100 nucleotides in length or less. The segment to be amplified can include a plurality of variants. In another embodiment, the detection of the presence or absence or at least one variant provides for contacting EGFR nucleic acid containing a variant site with at least one nucleic acid probe. The probe preferentially hybridizes with a nucleic acid sequence including a variant site and containing complementary nucleotide bases at the variant site under selective hybridization conditions. Hybridization can be detected with a detectable label.

In yet another embodiment, the detection of the presence or absence of at least one variant includes sequencing at least one nucleic acid sequence and comparing the obtained sequence with the known erbB1 nucleic acid sequence. Alternatively, the presence or absence of at least one variant includes mass spectrometric determination of at least one nucleic acid sequence.

In a preferred embodiment, the detection of the presence or absence of at least one nucleic acid variant includes performing a polymerase chain reaction (PCR). The erbB1 nucleic acid sequence containing the hypothetical variant is amplified and the nucleotide sequence of the amplified nucleic acid is determined. Determining the nucleotide sequence of the amplified nucleic acid includes sequencing at least one nucleic acid segment. Alternatively, amplification products can analyzed by using any method capable of separating the amplification products according to their size, including automated and manual gel electrophoresis and the like. Alternatively, the detection of the presence or absence of at least one variant includes determining the haplotype of a plurality of variants in a gene.

In another embodiment, the presence or absence of an EGFR variant can be detected by analyzing the erbB1 gene product (protein). In this embodiment, a probe that specifically binds to a variant EGFR is utilized. In a preferred embodiment, the probe is an antibody that preferentially binds to a variant EGFR. The presence of a variant EGFR predicts the likelihood of effectiveness of an EGFR targeting treatment. Alternatively, the probe may be an antibody fragment, chimeric antibody, humanized antibody, or aptamer.

Furthermore, a probe may specifically binds under selective binding conditions to a nucleic acid sequence comprising at least one nucleic acid variant in the EGFR gene (erbB1). In one embodiment, the variant is a mutation in the kinase domain of erbB1 that confers a structural change in the ATP-binding pocket. The probe of the present invention may comprise a nucleic acid sequence of about 500 nucleotide bases, preferably about 100 nucleotides bases, and most preferably about 50 or about 25 nucleotide bases or fewer in length. The probe may be composed of DNA, RNA, or peptide nucleic acid (PNA). Furthermore, the probe may contain a detectable label, such as, for example, a fluorescent or enzymatic label.

Analysis of amplification products can be performed using any method capable of separating the amplification products according to their size, including automated and manual gel electrophoresis, mass spectrometry, and the like. Alternatively, the amplification products can be separated using sequence differences, using SSCP, DGGE, TGGE, chemical cleavage or restriction fragment polymorphisms as well as hybridization to, for example, a nucleic acid arrays.

Sequence analysis and validation: Forward (F) and reverse (R) chromatograms are analyzed in batch by Mutation Surveyor 2.03 (SoftGenetics, State College, PA), followed by manual review. High quality sequence variations found in one or both directions are scored as candidate mutations. Exons harboring candidate mutations can be reamplified from the original DNA sample and re-sequenced as above.

Preferably, exons 19 and 21 of human EGFR are amplified by the polymerase chain reaction (PCR) using the following primers: Exon19 sense primer, 5'- GCAATATCAGCCTTAGGTGCGGCTC-3' (SEQ ID NO: 505); Exon 19 antisense primer, 5'-CATAGAAAGTGAACATTTAGGATGTG-3' (SEQ ID NO: 506); Exon 21 sense primer, 5'-CTAACGTTCG CCAGCCATAAGTCC-3' (SEQ ID NO: 507); and Exon21 antisense primer, 5'-GCTGCGAGCTCACCCAGAATGTCTGG-3' (SEQ ID NO: 508).

In an alternative embodiment, mutations in a EGFR gene from a sample cell can be identified by alterations in restriction enzyme cleavage patterns. For example, sample and control DNA is isolated, optionally amplified, digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, e.g., U.S. Patent No. 5,493,531) can be used to score for the presence of specific mutations by development or loss of a ribozyme cleavage site.

Other methods for detecting mutations in the EGFR gene include methods in which protection from cleavage agents is used to detect mismatched bases in KNA/RNA or RNA/DNA heteroduplexes. See, e.g., Myers et al., 1985, Science 230: 1242. In general, the technique of "mismatch cleavage" starts by providing heteroduplexes formed by hybridizing (labeled) RNA or DNA containing the wildtype EGFR sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, e.g., Cotton et al., 1988, Proc. Natl. Acad. Sci. USA 85:4397, and Saleeba et al., 1992, Methods Enzymol. 2 17: 286-295. In an embodiment, the control DNA or RNA can be labeled for detection.

In still another embodiment, the mismatch cleavage reaction employs one or more proteins that recognize mismatched base pairs in doublestranded DNA (so called "DNA mismatch repair" enzymes) in defined systems for detecting and mapping point mutations in EGFR cDNAs obtained from samples of cells. For example, the mutY enzyme of E. coli cleaves A at Gr/A mismatches and the thymidine DNA glycosylase from HeLa cells cleaves T at G/T mismatches. See, e.g., Hsu et al., 1994, Carcinogenesis 15:1657-1662. According to an exemplary embodiment, a probe based on a mutant EGFR sequence, e.g., a DEL-1 through DEL-5, G719S, G857V, L883S or L858R EGFR sequence, is hybridized to a cDNA or repair enzyme, and the cleavage products, if any, can be detected from electrophoresis protocols or the like. See, e.g., U.S. Patent No. 5,459,039.

In other embodiments, alterations in electrophoretic mobility is used to identify mutations in EGFR genes. For example, single strand conformation polymorphism (SSCP) may be used to detect differences in electrophoretic mobility between mutant and wild type nucleic acids. See, e.g., Orita et al., 1989, Proc. Natl. Acad. Sci. USA: 86: 2766; Cotton, 1993, Mutat. Res. 285: 125-144; Hayashi, 1992, Genet. Anal. Tech. Appl. 9:73-79. Single-stranded DNA fragments of sample and control EGFR nucleic acids will be denatured and allowed to renature. The secondary structure of single-stranded nucleic acids varies according to sequence; the resulting alteration in electrophoretic mobility enables the detection of even a single base change. The DNA fragments may be labeled or detected with labeled probes. The sensitivity of the assay may be enhanced by using RNA (rather than DNA) in which the secondary structure is more sensitive to a change in sequence. In one embodiment, the subject method utilizes heteroduplex analysis to separate double-stranded heteroduplex molecules on the basis of changes in electrophoretic mobility. See, e.g., Keen et al., 1991, Trends Genet. 7:5.

In yet another embodiment, the movement of mutant or wild-type fragments in polyacrylamide gels containing a gradient of denaturant is assayed using denaturing gradient gel electrophoresis (DGGE). See, e.g., Myers et al., 1985, Nature 313:495. When DGGE is used as the method of analysis, DNA will be modified to ensure that it does not completely denature, for example, by adding a GC clamp of approximately 40 bp of high-melting GC-rich DNA by PCR. In a further embodiment, a temperature gradient is used in place of a denaturing gradient to identify differences in the mobility of control and sample DNA. See, e.g., Rosenbaum and Reissner, 1987, Biophys. Chem. 265:12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found See, e.g., Saiki et al., 1986, Nature 324:163, and Saiki et al., 1989, Proc. Natl. Acad. Sci. USA 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology depending on selective PCR amplification may be used in conjunction with the instant invention. Oligonucleotides used as primers for specific amplification may carry the mutation of interest in the center of the molecule (so that amplification depends on differential hybridization; see, e.g., Gibbs, et al., 1989, Nucl. Acids Res. 17: 2437-24-48) or at the extreme 3-terminus of one primer where, under appropriate conditions, mismatch can prevent, or reduce polymerase extension (see, e.g., Prossner, 1993, Tibteeli. 11: 238). In addition, it may be desirable to introduce a novel restriction site in the region of the mutation to create cleavage-based detection. See, e.g., Gasparini, et al., 1992, Mol. Cell Probes 6:1. It is anticipated that in certain embodiments, amplification may also be performed using Taq ligase for amplification. See, e.g., Barany 1991, Proc. Natl. Acad. Sci. USA 88:189. In such cases, ligation will occur only if there is a perfect match at the 3-terminus of the 5' sequence, making it possible to detect the presence of a known mutation at a specific site by looking for the presence or absence of amplification.

PCR and sequencing methods for genomic DNA: Tyrosine kinase exons and flanking intronic sequences may be amplified using specific primers in a 384-well format nested PCR setup. Each PCR reaction contains 5 ng of DNA, IX HotStar Buffer, 0.8 mM dNTPs, 1 mM $MgCl_2$, 0.2U HotStar Enzyme (Qiagen, Valencia, CA), and 0.2 uM forward and reverse primers in a 10 uL reaction volume. PCR cycling parameters are: one cycle of 95°C for 15 minutes, 35 cycles of 95°C for 20 seconds, 60°C for 30 seconds, and 72°C for 1 minute, followed by one cycle of 72°C for 3 minutes. The resulting PCR products are purified by solid phase reversible immobilization chemistry followed by bi-directional dye-terminator fluorescent sequencing with universal M13 primers. Sequencing fragments are detected via capillary electrophoresis using ABI Prism 3700 DNA Analyzer (Applied Biosystems, Foster City, CA). PCR and sequencing are performed by Agencourt BioscienceCorporation (Beverly, MA).

**EGFR Mutations**

CTC samples from patients with striking responses to Gefitinib might harbor mutations in EGFR, indicating an essential role played by this growth factor signaling pathway in tumors that are present in such patients. To search for such mutations, rearrangements within the extracellular domain of EGFR can be tested that are characteristic of gliomas. If no such rearrangements are detected, the entire coding region may be sequenced using PCR-amplification of individual exons. Heterozygous mutations may include in-frame deletions removing amino acids 746-750 (delE75&-A750), 747 to 750 (delL747-T751), and 747 to 752 (delL747-P753). The latter two deletions are associated with the insertion of a serine residue resulting from the generation of a novel codon at the deletion breakpoint. Additional mutations may include amino acid substitutions within exon 21: leucine to arginine at codon 858 (L858R), and leucine to glutamine at codon 861 (L861Q). The L861Q mutation is of particular interest, since the same amino acid change in the mouse egfr gene is responsible for the Dark Skin (dsk5) trait, associated with altered EGFR signaling. A missense mutation in the kinase domain can result in a glycine to cysteine substitution at codon 719 within exon 18 (G719C).

Additional mutations include, delL747-P753 and delE746-A750. The (2253_2276 del) deletion overlaps previously described exon 19 deletions. The deletions may be categorized into one of two groups: those spanning codons 747-749 at a minimum (amino acid sequence LKE), and those spanning codons 752-759. Analysis of all exon 19 deletions reported to date suggests that a wide variety of amino acids can be deleted from the TK region spanning codons 747-759. There does not appear to be a required common codon deleted.

In other embodiments, the kinase domain of EGFR (exons 18-24 and flanking intronic regions) is amplified in a set of individual nested polymerase chain reaction (PCR) reactions. The primers used in the nested PCR amplifications are described with the addition of universal sequences to the 5' ends of the primers (5' tgtaaaacgacggccagt). The PCR products can be directly sequenced bi-directionally by dye-terminator sequencing. PCR is performed in a 3 84-well plate in a volume of 15 ul containing 5 ng genomic DNA, 2 mM $MgCl_2$, 0.75 ul DMSO, 1 M Betaine, 0.2 mM dNTPs, 20 pmol primers, 0.2 ul AmpliTaq Gold® (Applied Biosystems), and IX buffer (supplied with AmpliTaq Gold). Thermal cycling conditions are as follows: 95°C for 10 minutes; 95°C for 30 seconds, 60°C for 30 seconds, 72°C for 1 minute for 30 cycles; and 72°C for 10 minutes. PCR products are purified with Ampure® Magnetic Beads (Agencourt). Sequencing products are purified using Cleanseq™ Magnetic Beads (Agencourt) and separated by capillary electrophoresis on an ABI3730 DNA Analyzer (Applied Biosystems). Sequence analysis is performed by Mutation Surveyor (SoftGenetics, State College, PA) and manually by two reviewers. Nonsynonymous DNA sequence variants are confirmed by analysis of 3-5 independent PCR reactions of the original genomic DNA sample. Control or reference samples are analyzed to determine whether the sequence changes are unique to tumor tissue, via the enriched CTCs, or are consitutive or background variants.

Additional exons 2 through 25 of EGFR can also be detected in CTCs. For example, exon sequencing of genomic DNA will reveal missense and deletion mutations af EGFR, which usually occur within exons 18 through 21 of the kinase domain. Sequence alterations may be heterozygous in the tumor DNA; in each case, paired normal lung tissue from the same patient will have wild-type sequence, confirming that the mutations are somatic in origin. Some examples of a substitution mutation are G719S and L858R. The "G719S" mutation changes the glycine (G) at position 719 to serine. These mutations are located in the GXGXXG motif of the nucleotide triphosphate binding domain or P-loop and adjacent to the highly conserved DFG motif in the activation loop, respectively. The mutated residues are nearly invariant in all protein kinases and the analogous residues (G463 and L596) in the B-Raf protein serine-threonine kinase are somatically mutated in colorectal, ovarian and lung carcinomas.

Examples of multiple deletion mutations include those clustered in the region spanning codons 746 to 759 within the kinase domain af EGFR. For example, one of two overlapping 15-nucleotide deletions eliminating EGFR codons 746 to 750, starting at either nucleotide 2235 or 2236 (Del-1). EGFR DNA from another tumor displayed a heterozygous 24-nucleotide gap leading to the deletion of codons 752 to 759 (Del-2).

We next examined exons 2 through 25 of EGFR in the complete collection of 119 NSCLC tumors. Exon sequencing of genomic DNA revealed missense and deletion mutations ot EGFR in a total of 16 tumors, all within exons 18 through 21 of the kinase domain. All sequence alterations in this group were heterozygous in the tumor DNA; in each case, paired normal lung tissue from the same patient showed wild-type sequence, confirming that the mutations are somatic in origin. Substitution mutations G719S and L858R were detected in two and three tumors, respectively. The "G719S" mutation changes the glycine (G) at position 719 to serine (S). These mutations are located in the GXGXXG motif of the nucleotide triphosphate binding domain or P-loop and adjacent to the highly conserved DFG motif in the activation loop, respectively.

Additional deletion mutations clustered in the region spanning codons 746 to 759 within the kinase domain of EGFR. Ten tumors carried one of two overlapping 15-nucleotide deletions eliminating EGFR cordons 746 to 750, starting at either nucleotide 2235 or 2236. Two other mutations include EGFR mutation G857V in Acute Myelogenous Leukemia (AML) and the EGFR mutation L883S in a metastatic sarcoma. The "G857V" mutation has the glycine (G) at position 857 substituted with a valine (V), while the "L883S" mutation has the leucine (L) at position 883 substituted with a serine (S). Mutations in EGFR can occur in several tumor types, thus enriching/selecting CTCs as outlined in the methods herein will allow a determination for selection of EGFR inhibitors that would efficacious in the treatment of patients harboring such mutations. This expands the use of kinase inhibitors such as, e.g., the tyrosine kinase inhibitors gefitinib (marketed as Iressa™), erlotinib (marketed as Tarceva™), and the like in treating tumor types other than NSCLC. Moreover, the EGFR mutations may show a striking correlation with the differential patient characteristics in the response to the tyrosine kinase inhibitor gefitinib (Iressa™), with higher responses seen in one cohort versus another. Examples of differential patient characteristics include smokers, non-smokers, sex, age, race, and acute versus chronic versus no exposure to air pollution.

The methods of the invention described above are not limited to epithelial cells and cancer, but rather may be used to diagnose any condition. Exemplary conditions that may be diagnosed using the methods of the invention are hematological conditions, inflammatory conditions, ischemic conditions, neoplastic conditions, infections, traumas, endometriosis, and kidney failure (see, e.g., Takahashi et al., Nature Med. 5:434-438 (1999), Healy et al., Hum. Reprod. Update 4:736-740 (1998), and Gill et al., Circ. Res. 88:167-174 (2001)). Neoplastic conditions include acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, and Wilm's tumor. In one embodiment, neoplastic cells associated with thyroid cancer are not detected. A cellular sample taken from a patient, e.g., a sample of less than 50 mL, 40 mL, 30 mL, 20 mL, or even 10 mL, may be processed through a device of the invention in order to produce a sample enriched in any cell of interest, e.g., a rare cell. Detection of this cell in the enriched sample may then enable one skilled in the art to diagnose the presence or absence of a particular condition in the patient. Furthermore, determination of ratios of numbers of cells, e.g., cancer cells to endothelial cells, in the sample may be used to generate a diagnosis. Alternatively, detection of cancer biomarkers, e.g., any of those listed in Table 1, or a nucleic acid associated with cancer, e.g., a nucleic acid enoding any marker listed in Table 1, may result in the diagnosis of a cancer or another condition. For example, analysis of the expression level or pattern of such a polypeptide or nucleic acid, e.g., cell surface markers, genomic DNA, mRNA, or microRNA, may result in a diagnosis.

Cell detection may be combined with other information, e.g., imaging studies af the patient, in order to diagnose a patient. For example, computed axial tomography, positron emission tomography, or magnetic resonance imaging may be used.

A diagnosis may also be made using a cell pattern associated with a particular condition. For example, by comparing the size distribution of cells in an enriched sample, e.g., a sample containing cells having a hydrodynamic size greater than 12 microns, with a size distribution associated with a condition, e.g., cancer, a diagnosis may be made based on this comparison. A cell pattern for comparison may be generated by any method. For example, an association study may be performed in which cellular samples from a plurality of control subjects (e.g., 50) and a plurality of case subjects (e.g., 50) having a condition of interest are processed, e.g., by enriching cells having a hydrodynamic size greater than 12 microns, the results samples are analyzed, and the results of the analysis are compared. To perform such a study, it may be useful to analyze RNA levels, e.g., mRNA or microRNA levels, in the enriched cells. Alternatively, it is useful to count the number of cells enriched in each case, or to determine a cellular size distribution, e.g., by using a microscope, a cell counter, or a microarray device. The presence of particular cell types, e.g., rare cells, may also be identified.

Once a drug treatment is administered to a patient, it is possible to determine the efficacy of the drug treatment using the methods of the invention. For example, a cellular sample taken from the patient before the drug treatment, as well as one or more cellular samples taken from the patient concurrently with or subsequent to the drug treatment, may be processed using the methods of the invention. By comparing the results of the analysis of each processed sample, one may determine the efficacy of the drug treatment. For example, an enrichment device may be used to enrich cells having a hydrodynamic size greater than 12 microns, or cells having a hydrodynamic size greater than or equal to 6 microns and less than or equal to 12 microns, from other cells. Any other detection or analysis described above may be performed, e.g., identification of the presence or quantity of specific cell types.

### Methods of using sample mobilization devices

A sample mobilization device of the invention may be used to enrich CTCs or other cells from a sample. In one embodiment, a cellular sample is placed in a sample mobilization device, e.g., a device that includes a receptacle, a lid with a functionalized surface, and a sample mobilizer. The receptacle containing the sample is then covered with the lid, the sample mobilizer is employed to mobilize the sample, and the lid is removed. Such a device may be used to enrich a CTC or other cell of interest.

Any type of sample mobilization, e.g., centrifugation, may be applied. Any centrifugal field that is known in the art may be applied, e.g., a centrifugal field between 100g and 100,000g. For example, the centrifugal field may be between 1,000g and 10,000g. The application of this field results in a centrifugal force on the sample. Additional forces may also be applied, e.g., a force opposite to the centrifugal force; furthermore, forces may be applied repeatedly and in alternation, with an optional time interval between applications of each force.

General considerations

Samples may be employed in the methods described herein with or without purification, e.g., stabilization and removal of certain components. Some sample may be diluted or concentrated prior to introduction into the device.

In one embodiment, reagents are added to the sample, to selectively or nonselectively increase the hydrodynamic size of the particles within the sample. This modified sample is then pumped through an obstacle array. Because the particles are swollen and have an increased hydrodynamic size, it will be possible to use obstacle arrays with larger and more easily manufactured gap sizes. In a preferred embodiment, the steps of swelling and size-based enrichment are performed in an integrated fashion on a device. Suitable reagents include any hypotonic solution, e.g., deionized water, 2% sugar solution, or neat non-aqueous solvents. Other reagents include beads, e.g., magnetic or polymer, that bind selectively (e.g., through antibodies or avidin-biotin) or non-selectively.

In another embodiment, reagents are added to the sample to selectively or nonselectively decrease the hydrodynamic size of the particles within the sample. Nonuniform decrease in particles in a sample will increase the difference in hydrodynamic size between particles. For example, nucleated cells are separated from enucleated cells by hypertonically shrinking the cells. The enucleated cells may shrink to a very small particle, while the nucleated cells cannot shrink below the size of the nucleus. Exemplary shrinking reagents include hypertonic solutions.

In an alternative embodiment, affinity functionalized beads and other appropriate beads are used to increase the volume of particles of interest relative to the other particles present in a sample, thereby allowing for the operation of a obstacle array with a larger and more easily manufactured gap size.

Fluids may be driven through a device either actively or passively. Fluids may be pumped using electric field, a centrifugal field, pressure-driven fluid flow, an electro-osmotic flow, and capillary action. In preferred embodiments, the average direction of the field will be parallel to the walls of the channel that contains the array.

### Sample preparation

Samples may be employed in the methods described herein with or without manipulation, e.g., stabilization and removal of certain components. In one embodiment, the sample is enriched in CTCs or other cells of interest prior to introduction to a device of the invention. Methods for enriching cell populations are known in the art, e.g., affinity mechanisms, agglutination, and size, shape, and deformability based enrichments. Exemplary methods for enriching a sample in a cell of interest are found in U.S. Patent Nos. 5,837,115 and 5,641,628, International Publication Nos. WO 2004/029221 and WO 2004/113877, and U.S. Publication No. 2004/0144651.

## EXAMPLES

### Example 1

Microfluidic devices of the invention were designed by computer-aided design (CAD) and microfabricated by photolithography. A two-step process was developed in which a blood sample is first debulked to remove the large population of small cells, and then the rare target epithelial cells target cells are recovered by immunoaffinity capture. The devices were defined by photolithography and etched into a silicon substrate based on the CAD-generated design. The cell enrichment module, which is approximately the size of a standard microscope slide, contains 14 parallel sample processing sections and associated sample handling channels that connect to common sample and buffer inlets and product and waste outlets. Each section contains an array of microfabricated obstacles that is optimized to enrich the target cell type by hydrodynamic size via displacement of the larger cells into the product stream. In this example, the microchip was designed to separate red blood cells (RBCs) and platelets from the larger leukocytes and CTCs. Enriched populations of target cells were recovered from whole blood passed through the device. Performance of the cell enrichment microchip was evaluated by separating RBCs and platelets from white blood cells (WBCs) in normal whole blood (Fig. 52). In cancer patients, CTCs are found in the larger WBC fraction. Blood was minimally diluted (30%), and a 6 ml sample was processed at a flow rate of up to 6 m1/hr. The product and waste stream were evaluated in a Coulter Model "A$^{c}$-T diff" clinical blood analyzer, which automatically distinguishes, sizes, and counts different blood cell populations. The enrichment chip achieved separation of RBCs from WBCs, in which the WBC fraction had >99% retention of nucleated cells, >99% depletion of RBCs, and >97% depletion of platelets. Representative histograms of these cell fractions are shown in Fig. 53. Routine cytology confirmed the high degree of enrichment of the WBC and RBC fractions (Fig. 54).

Next, epithelial cells were recovered by affinity capture in a microfluidic module that is functionalized with immobilized antibody. A capture module with a single chamber containing a regular array of antibody-coated microfabricated obstacles was designed. These obstacles are disposed to maximize cell capture by increasing the capture area approximately four-fold, and by slowing the flow of cells under laminar flow adjacent to the obstacles to increase the contact time between the cells and the immobilized antibody. The capture modules may be operated under conditions of relatively high flow rate but low shear to protect cells against damage. The surface of the capture module was functionalized by sequential treatment with 10% silane, 0.5% gluteraldehyde, and avidin, followed by biotinylated anti-EpCAM. Active sites were blocked with 3% bovine serum albumin in PBS, quenched with dilute Tris HCl, and stabilized with dilute L-histidine. Modules were washed in PBS after each stage and finally dried and stored at room temperature. Capture performance was measured with the human advanced lung cancer cell line NCI-H1650 (ATCC Number CRL-5883). This cell line has a heterozygous 15 bp in-frame deletion in exon 19 of EGFR that renders it susceptible to gefitinib. Cells from confluent cultures were harvested with trypsin, stained with the vital dye Cell Tracker Orange (CMRA reagent, Molecular Probes, Eugene, OR), resuspended in fresh whole blood, and fractionated in the microfluidic chip at various flow rates. In these initial feasibility experiments, cell suspensions were processed directly in the capture modules without prior fractionation in the cell enrichment module to debulk the red blood cells; hence, the sample stream contained normal blood red cells and leukocytes as well as tumor cells. After the cells were processed in the capture module, the device was washed with buffer at a higher flow rate (3ml/hr) to remove the nonspecifically bound cells. The adhesive top was removed and the adherent cells were fixed on the chip with parafonnaldehyde and observed by fluorescence microscopy. Cell recovery was calculated from hemacytometer counts; representative capture results are shown in Table 4. Initial yields in reconstitution studies with unfractionated blood were greater than 60% with less than 5% of non-specific binding.

**Table 4**

| Run number | Avg. flow rate | Length of run | No. cells processed | No. cells captured | Yield |
|---|---|---|---|---|---|
| 1 | 3.0 | 1 hr | 150.000 | 36,012 | 25% |
| 2 | 1.5 | 2 hr | 150.000 | 30.000/ml | 60% |
| 3 | 1.08 | 2 hr | 108.000 | 65.661 | 64% |
| 4 | 1.21 | 2 hr | 121.000 | 75.491 | 62% |

Next, NCI-H1650 cells that were spiked into whole blood and recovered by size fractionation and affinity capture as described above were successfully analyzed in situ. In a trial run to distinguish epithelial cells from leukocytes, 0.5 ml of a stock solution of fluorescein-labeled CD45 pan-leukocyte monoclonal antibody were passed into the capture module and incubated at room temperature for 30 minutes. The module was washed with buffer to remove unbound antibody, and the cells were fixed on the chip with 1% paraformaldehyde and observed by fluorescence microscopy. As shown in Fig. 55, the epithelial cells were bound to the obstacles and floor of the capture module. Background staining of the flow passages with CD45 pan-leukocyte antibody is visible, as are several stained leukocytes, apparently because of a low level of non-specific capture.

**Example 2: Device embodiments**

A design for preferred device embodiments of the invention is shown in Fig. 57A, and parameters corresponding to three preferred device embodiments associated with this design are shown in Fig. 57B. These embodiments are particularly useful for enrich epithelial cells from blood.

**Example 3: Determining counts for non-epithelial cell types**

Using the methods of the invention, one may make a diagnosis based on counting cell types other than CTCs or other epithelial cells. A diagnosis of the absence, presence, or progression of cancer may be based on the number of cells in a cellular sample that are larger than a particular cutoff size. For example, cells with a hydrodynamic size of 14 microns or larger may be selected. This cutoff size would eliminate most leukocytes. The nature of these cells may then be determined by downstream molecular or cytological analysis.

Cell types other than epithelial cells that would be useful to analyze include endothelial cells, endothelial progenitor cells, endometrial cells, or trophoblasts indicative of a disease state. Furthermore, determining separate counts for epithelial cells, e.g., cancer cells, and other cell types, e.g., endothelial cells, followed by a determination of the ratios between the number of epithelial cells and the number of other cell types, may provide useful diagnostic information.

A device of the invention may be configured to isolate targeted subpopulations of cells such as those described above, as shown in Figs. 33A-D. A size cutoff may be selected such that most native blood cells, including red blood cells, white blood cells, and platelets, flow to waste, while non-native cells, which could include endothelial cells, endothelial progenitor cells, endometrial cells, or trophoblasts, are collected in an enriched sample. This enriched sample may be further analyzed.

Using a device of the invention, therefore, it is possible to isolate a subpopulation of cells from blood or other bodily fluids based on size, which conveniently allows for the elimination of a large proportion of native blood cells when large cell types are targeted. As shown schematically in Fig. 56, a device of me invention may include counting means to determine the number of cells in the enriched sample, or the number of cells of a particular type, e.g., cancer cells, within the enriched sample, and further analysis of the cells in the enriched sample may provide additional information that is useful for diagnostic or other purposes.

**Example 4: Method for detection of EGFR mutations**

A blood sample from a cancer patient is processed and analyzed using the devices and methods of the invention, e.g., those of Example 1, resulting in an enriched sample of epithelial cells containing CTCs. This sample is then analyzed to identify potential EGFR mutations. The method permits both identification of known, clinically relevant EGFR mutations as well as discovery of novel mutations. An overview of this process is shown in Fig. 58.

Below is an outline of the strategy for detection and confirmation of EGFR mutations:

1) Sequence CTC EGFR mRNA

- a) Purify CTCs from blood sample;
- b) Purify total RNA from CTCs;
- c) Convert RNA to cDNA using reverse transcriptase;
- d) Use resultant cDNA to perform first and second PCR reactions for generating sequencing templates; and
- e) Purify the nested PCR amplicon and use as a sequencing template to sequence EGFR exons 18-21.

2) Confirm RNA sequence using CTC genomic DNA

- a) Purify CTCs from blood sample;
- b) Purify genomic DNA (gDNA) from CTCs;
- c) Amplify exons 18, 19, 20, and/or 21 via PCR reactions; and
- d) Use the resulting PCR amplicon(s) in real-time quantitative allele-specific PCR reactions in order to confirm the sequence of mutations discovered via RNA sequencing.

Further details for each step outlined above are as follows.

1) Sequence CTC EGFR mRNA

*a) Purify CTCs from blood sample.* CTCs are isolated using any of the size-based enrichment and/or affinity purification devices of the invention.

*b) Purify total RNA from CTCs.* Total RNA is then purified from isolated CTC populations using, e.g., the Qiagen Micro RNeasy kit, or a similar total RNA purification protocol from another manufacturer; alternatively, standard RNA purification protocols such as guanidium isothiocyanate homogenization followed by phenol/chloroform extraction and ethanol precipitation may be used. One such method is described in "Molecular Cloning - A Laboratory Manual, Second Edition" (1989) by J. Sambrook, E.F. Fritch and T. Maniatis, p. 7.24.

*c) Convert RNA to cDNA using reverse transcriptase.* cDNA reactions are carried out based on the protocols of the supplier of reverse transcriptase. Typically, the amount of input RNA into the cDNA reactions is in the range of 10 picograms (pg) to 2 micrograms (μg) total RNA. First-strand DNA synthesis is carried out by hybridizing random 7mer DNA primers, or oligo-dT primers, or gene-specific primers, to RNA templates at 65°C followed by snap-chilling on ice. cDNA synthesis is initiated by the addition of iScript Reverse Transcriptase (BioRad) or SuperScript Reverse Transcriptase (Invitrogen) or a reverse transcriptase from another commercial vendor along with the appropriate enzyme reaction buffer. For iScript, reverse transcriptase reactions are carried out at 42°C for 30-45 minutes, followed by enzyme inactivation for 5 minutes at 85°C. cDNA is stored at -20°C until use or used immediately in PCR reactions. Typically, cDNA reactions are carried out in a final volume of 20 μl, and 10% (2 μl) of the resultant cDNA is used in subsequent PCR reactions.

*d) Use resultant cDNA to perform first and second PCR reactions for generating sequencing templates.* cDNA from the reverse transcriptase reactions is mixed with DNA primers specific for the region of interest (Fig. 59). See Table 5 for sets of primers that may be used for amplification of exons 18-21. In Table 5, primer set M13(+)/M12(-) is internal to primer set M11(+)/M14(-). Thus primers M13(+) and M12(-) may be used in the nested round of amplification, if primers M11(+) and M14(-) were used in the first round of expansion. Similarly, primer set M11(+)/M14(-) is internal to primer set M15(+)/M16(-), and primer set M23(+)/M24(-) is internal to primer set M21(+)/M22(-). Hot Start PCR reactions are performed using Qiagen Hot-Star Taq Polymerase kit, or Applied Biosystems HotStart TaqMan polymerase, or other Hot Start thermostable polymerase, or without a hot start using Pramega GoTaq Green Taq Polymerase master mix, TaqMan DNA polymerase, or other thermostable DNA polymerase. Typically, reaction volumes are 50 μl, nucleotide triphosphates are present at a final concentration of 200 μM for each nucleotide, $MgCl_2$ is present at a final concentration of 1-4 mM, and oligo primers are at a final concentration of 0.5 μM. Hot start protocols begin with a 10-15 minute incubation at 95°C, followed by 40 cycles of 94°C for one minute (denaturation), 52°C for one minute (annealing), and 72°C for one minute (extension). A 10 minute terminal extension at 72°C is performed before samples are stored at 4°C until they are either used as template in the second (nested) round ot PCRs, or purified using QiaQuick Spin Columns (Qiagen) prior to sequencing. If a hot-start protocol is not used, the initial incubation at 95°C is omitted. If a PCR product is to be used in a second round of PCRs, 2 μl (4%) of the initial PCR product is used as template in the second round reactions, and the identical reagent concentrations and cycling parameters are used.

**Table 5. Primer Sets for expanding EGFR mRNA around Exons 18-21**

| Name | SEQ ID NO | Sequence (5' to 3') | cDNA Coordinates | Amplicon Size |
|---|---|---|---|---|
| NXK-M11(+) | 1 | TTGCTGCTGGTGGTGGC | (+) 1966-1982 | 813 |
| NXK-M14(-) | 2 | CAGGGATTCCGTCATATGGC | (-) 2778-2759 | |
| NXK-M13(+) | 3 | GATCGGCCTCTTCATGCG | (+) 1989-2006 | 747 |
| NXK M12(-) | 4 | GATCCAAAGGTCATCAACTCCC | (-) 2735-2714 | |
| NXK-M15(+) | 5 | GCTGTCCAACGAATGGGC | (+) 1904-1921 | 894 |
| NXK-M16(-) | 6 | GGCGTTCTCCTTTCTCCAGG | (-) 2797-2778 | |
| NXK-M21(+) | 7 | ATGCACTGGGCCAGGTCTT | (+) 1881-1899 | 944 |
| NXK-M22(-) | 8 | CGATGGTACATATGGGTGGCT | (-) 2824-2804 | |
| NXK-M23(+) | 9 | AGGCTGTCCAACGAATGGG | (+) 1902-1920 | 904 |
| NXK-M24(-) | 10 | CTGAGGGAGGCGTTCTCCT | (-) 2805-2787 | |

*e) Purify the nested PCR amplicon and use as a sequencing template to sequence EGFR exons 18-21.* Sequencing is performed by ABI automated fluorescent sequencing machines and fluorescence-labeled DNA sequencing ladders generated via Sanger-style sequencing reactions using fluorescent dideoxynucleotide mixtures. PCR products are purified using Qiagen QuickSpin columns, the Agencourt AMPure PCR Purification System, or PCR product purification kits obtained from other vendors. After PCR products are purified, the nucleotide concentration and purity is determined with a Nanodrop 7000 spectrophotometer, and the PCR product concentration is brought to a concentration of 25 ng/μl. As a quality control measure, only PCR products that have a UV-light absorbance ratio ($A_{260}/A_{280}$) greater than 1.8 are used for sequencing. Sequencing primers are brought to a concentration of 3.2 pmol/μl.

2) Confirm RNA sequence using CTC genomic DNA

*a) Purify CTCs from blood sample.* As above, CTCs are isolated using any of the size-based enrichment and/or affinity purification devices of the invention.

*b) Purify genomic DNA (gDNA) from CTCs.* Genomic DNA is purified using the Qiagen DNeasy Mini kit, the Invitrogen ChargeSwitch gDNA kit, or another commercial kit, or via the following protocol:

1. Cell pellets are either lysed fresh or stored at -80°C and are thawed immediately before lysis.
2. Add 500 μl 50mM Tris pH 7.9/100mM EDTA/0.5%SDS (TES buffer).
3. Add 12.5 μl Proteinase K (IBI5406, 20mg/ml), generating a final [ProtK] = 0.5 mg/ml.
4. Incubate at 55°C overnight in rotating incubator.
5. Add 20 μl of RNase cocktail (500 U/ml RNase A + 20,000 U/ml RNase T1, Ambion #2288) and incubate four hours at 37°C.
6. Extract with Phenol (Kodak, Tris pH 8 equilibrated), shake to mix, spin 5 min. in tabletop centrifuge.
7. Transfer aqueous phase to fresh tube.
8. Extract with Phenol/Chloroform/Isoamyl alcohol (EMD, 25:24:1 ratio, Tris pH 8 equilibrated), shake to mix, spin five minutes in tabletop centrifuge.
9. Add 50 μl 3M NaOAc pH = 6.
10. Add 500 μl EtOH.
11. Shake to mix. Strings of precipitated DNA may be visible. If anticipated DNA concentration is very low; add carrier nucleotide (usually yeast tRNA).
12. Spin one minute at max speed in tabletop centrifuge.
13. Remove supernatant.
14. Add 500 μl 70% EtOH, Room Temperature (RT)
15. Shake to mix.
16. Spin one minute at max speed in tabletop centrifuge.
17. Air dry 10-20 minutes before adding TE.
18. Resuspend in 400 μl TE. Incubate at 65°C for 10 minutes, then leave at RT overnight before quantitation on Nanodrop.

*c) Amplify exons 18, 19, 20, and/or 21 via PCR reactions.* Hot start nested PCR amplification is carried out as described above in step 1d, except that there is no nested round of amplification. The initial PCR step may be stopped during the log phase in order to minimize possible loss of allele-specific information during amplification. The primer sets used for expansion of EGFR exons 18-21 are listed in Table 6 (see also Paez et al., Science 304:1497-1500 (Supplementary Material) (2004)).

**Table 6. Primer sets for expanding EGFR genomic DNA**

| Name | SEQ ID NO | Sequence (5' to 3') | Exon | Amplicon Size |
|---|---|---|---|---|
| NXK-ex18.1(+) | 11 | TCAGAGCCTGTGTTTCTACCAA | 18 | 534 |
| NXK-ex18.2(-) | 12 | TGGTCTCACAGGACCACTGATT | 18 | |
| NXK-ex18.3(+) | 13 | TCCAAATGAGCTGGCAAGTG | 18 | 397 |
| NXK-ex18.4(-) | 14 | TCCCAAACACTCAGTGAAACAAA | 18 | |
| NXK-ex19.1(+) | 15 | AAATAATCAGTGTGATTCGTGGAG | 19 | 495 |
| NXK-ex19.2(-) | 16 | GAGGCCAGTGCTGTCTCTAAGG | 19 | |
| NXK-ex19.3(+) | 17 | GTGCATCGCTGGTAACATCC | 19 | 298 |
| NXK-ex19.4(-) | 18 | TGTGGAGATGAGCAGGGTCT | 19 | |
| NXK-ex20.1(+) | 19 | ACTTCACAGCCCTGCGTAAAC | 20 | 555 |
| NXK-ex20.2(-) | 20 | ATGGGACAGGCACTGATTTGT | 20 | |
| NXK-ex20.3(+) | 21 | ATCGCATTCATGCGTCTTCA | 20 | 379 |
| NXK-ex20.4(-) | 22 | ATCCCCATGGCAAACTCTTG | 20 | |

(continued)

| Name | SEQ ID NO | Sequence (5' to 3') | Exon | Amplicon Size |
|---|---|---|---|---|
| NXK-ex21.1(+) | 23 | GCAGCGGGTTACATCTTCTTTC | 21 | 526 |
| NXK-ex21.2(-) | 24 | CAGCTCTGGCTCACACTACCAG | 21 | |
| NXK-ex21.3(+) | 25 | GCAGCGGGTTACATCTTCTTTC | 21 | 349 |
| NXK-ex21.4(-) | 26 | CATCCTCCCCTGCATGTGT | 21 | |

*d) Use the resulting PCR amplicon(s) in real-time quantitative allele-specific PCR reactions in order to confirm the sequence of mutations discovered via RNA sequencing.* An aliquot of the PCR amplicons is used as template in a multiplexed allele-specific quantitative PCR reaction using TaqMan PCR 5' Nuclease assays with an Applied Biosystems model 7500 Real Time PCR machine (Fig. 60). This round of PCR amplifies subregions of the initial PCR product specific to each mutation of interest. Given the very high sensitivity of Real Time PCR, it is possible to obtain complete information on the mutation status of the EGFR gene even if as few as 10 CTCs are isolated. Real Time PCR provides quantification of allelic sequences over 8 logs of input DNA concentrations; thus, even heterozygous mutations in impure populations are easily detected using this method.

Probe and primer sets are designed for all known mutations that affect gefitinib responsiveness in NSCLC patients, including over 40 such somatic mutations, including point mutations, deletions, and insertions, that have been reported in the medical literature. For illustrative purposes, examples of primer and probe sets for five of the point mutations are listed in Table 7. In general, oligonucleotides may be designed using the primer optimization software program Primer Express (Applied Biosystems), with hybridization conditions optimized to distinguish the wild type EGFR DNA sequence from mutant alleles. EGFR genomic DNA amplified from lung cancer cell lines that are known to carry EGFR mutations, such as H358 (wild type), H1650 (15-bp deletion, $\Delta$2235-2249), and H1975 (two point mutations, 2369 C$\rightarrow$T,2573 T$\rightarrow$G), is used to optimize the allele-specific Real Time PCR reactions. Using the TaqMan 5' nuclease assay, allele-specific labeled probes specific for wild type sequence or for known EGFR mutations are developed. The oligonucleotides are designed to have melting temperatures that easily distinguish a match from a mismatch, and the Real Time PCR conditions are optimized to distinguish wild type and mutant alleles. All Real Time PCR reactions are carried out in triplicate.

Initially, labeled probes containing wild type sequence are multiplexed in the same reaction with a single mutant probe. Expressing the results as a ratio of one mutant allele sequence versus wild type sequence may identify samples containing or lacking a given mutation. After conditions are optimized for a given probe set, it is then possible to multiplex probes for all of the mutant alleles within a given exon within the same Real Time PCR assay, increasing the ease of use of this analytical tool in clinical settings.

A unique probe is designed for each wild type allele and mutant allele sequence. Wild-type sequences are marked with the fluorescent dye VIC at the 5' end, and mutant sequences with the fluorophore FAM. A fluorescence quencher and Minor Groove Binding moiety are attached to the 3' ends of the probes. ROX is used as a passive reference dye for normalization purposes. A standard curve is generated for wild type sequences and is used for relative quantitation. Precise quantitation of mutant signal is not required, as the input cell population is of unknown, and varying, purity. The assay is set up as described by ABI product literature, and the presence of a mutation is confirmed when the signal from a mutant allele probe rises above the background level of fluorescence (Fig. 61), and this threshold cycle gives the relative frequency of the mutant allele in the input sample.

**Table 7. Probes and Primers for Allele-Specific qPCR**

| Name | SEQ ID NO | Sequence (5' to 3', mutated position in bold) | EMBL Chromosome 7 Genomic Coordinates | Description | Mutation |
|---|---|---|---|---|---|
| NXK-M01 | 27 | CCGCAGCATGTCAAGATCAC | (+)55,033,694-55,033,713 | (+) primer | L858R |
| NXK-M02 | 28 | TCCTTCTGCATGGTATTCTTTCTCT | (-)55,033,769-55,033,745 | (-) primer | |
| Pwt-L858R | 29 | VIC-TTTGGGCTGGCCAA-MGB | (+)55,033,699-55,033,712 | WT allele probe | |
| Pmut-L858R | 30 | FAM-TTTTGGGCGGGCCA-MGB | (+)55,033,698-55,033,711 | Mutant allele probe | |
| NXK-M03 | 31 | ATGGCCAGCGTGGACAA | (+)55,023,207-55,023,224 | (+) primer | T790M |
| NXK-M04 | 32 | AGCAGGTACTGGGAGCCAATATT | (-)55,023,355-55,023,333 | (-) primer | |
| Pwt-T790M | 33 | VIC-ATGAGCTGCGTGATGA-MGB | (-)55,023,290-55,023,275 | WT allele probe | |
| Pmut-T790M | 34 | FAM-ATGAGCTGCATGATGA-MGB | (-)55,023,290-55,023,275 | Mutant allele probe | |
| NXK-M05 | 35 | GCCTCTTACACCCAGTGGAGAA | (+)55,015,831-55,015,852 | (+) primer | G719S,C |
| NXK-ex18.5 | 36 | GCCTGTGCCAGGGACCTT | (-)55,015,965-55,015,948 | (-) primer | |
| Pwt-G719SC | 37 | VIC-ACCGGAGCCCAGCA-MGB | (-)55,015,924-55,015,911 | WT allele probe | |
| Pmut-G719S | 38 | FAM-ACCGGAGCTCAGCA-MGB | (-)55,015,924-55,015,911 | Mutant allele probe | |
| Pmut-G719C | 39 | FAM-ACCGGAGCACAGCA-MGB | (-)55,015,924-55,015,911 | Mutant allele probe | |
| NXK-ex21.5 | 40 | ACAGCAGGGTCTTCTCTGTTTCAG | (+)55,033,597-55,033,620 | (+) primer | H835L |
| NXK-M10 | 41 | ATCTTGACATGCTGCGGTGTT | (-)55,033,710 55,033,690 | (-) primer | |
| Pwt-H835L | 42 | VIC-TTGGTGCACCGCGA-MGB | (+)55,033,803-55,033,816 | WT allele probe | |
| Pmut-H835L | 43 | FAM-TGGTGCTCCGCGAC-MGB | (+)55,033,803-55,033,816 | Mutant allele probe | |
| NXK-M07 | 101 | TGGATCCCAGAAGGTGAGAAA | (+)55,016,630-55,016,650 | (+) primer | delE746-A750 |
| NXK-ex19.5 | 1.02 | AGCAGAAACTCACATCGAGGATTT | (-)55,016,735-55,016,712 | (-) primer | |
| Pwt-delE746-A750 | 103 | AAGGAATTAAGAGAAGCAA | (+)55,016,681-55,016,699 | WT allele probe | |
| Pmut-delE746-A750var1 | 104 | CTATCAAAACATCTCC | (+)55,016,676-55,016,691 | Mutant allele probe, variant 1 | |
| Pmut-delE746-A750var1 | 105 | CTATCAAGACATCTCC | (+)55,016,676-55,016,691 | Mutant allele probe, variant 2 | |

**Example 5: Absence of EGFR expression in leukocytes**

The protocol of Example 4 would be most useful if EGFR were expressed in target cancer cells but not in background leukocytes. To test whether EGFR mRNA is present in leukocytes, several PCR experiments were performed. Four sets of primers, shown in Table 8, were designed to amplify four corresponding genes:

1) BCKDK (branched-chain a-ketoacid dehydrogenase complex kinase) - a "housekeeping" gene expressed in all types of cells, a positive control for both leukocytes and tumor cells;
2) CD45 - specifically expressed in leukocytes, a positive control for leukocytes and a negative control for tumor cells;
3) EpCaM - specifically expressed in epithelial cells, a negative control for leukocytes and a positive control for tumor cells; and
4) EGFR - the target mRNA to be examined.

**Table 8**

| Name | SEQ ID NO | Sequence (5' to 3') | Description | Amplicon Size |
|---|---|---|---|---|
| BCKD_1 | 44 | AGTCAGGACCCATGCACGG | BCKDK (+) primer | 273 |
| BCKD_2 | 45 | ACCCAAGATGCAGCAGTGTG | BCKDK (-) primer | |
| CD_1 | 46 | GATGTCCTCCTTGTTCTACTC | CD45 (+) primer | 263 |
| CD_2 | 47 | TACAGGGAATAATCGAGCATGC | CD45 (-) primer | |
| EpCAM_1 | 48 | GAAGGGAAATAGCAAATGGACA | EpCAM (+) primer | 222 |
| EpCAM_2 | 49 | CGATGGAGTCCAAGTTCTGG | EpCAM (-) primer | |
| EGFR_1 | 50 | AGCACTTACAGCTCTGGCCA | EGFR (+) primer | 371 |
| EGFR_2 | 51 | GACTGAACATAACTGTAGGCTG | EGFR (-) primer | |

Total RNAs of approximately $9\times10^6$ leukocytes isolated using a cell enrichment device of the invention (cutoff size 4 $\mu$m) and $5\times10^6$ H1650 cells were isolated by using RNeasy mini kit (Qiagen). Two micrograms of total RNAs from leukocytes and H1650 cells were reverse transcribed to obtain first strand cDNAs using 100 pmol random hexamer (Roche) and 200 U Superscript II (Invitrogen) in a 20 $\mu$l reaction. The subsequent PCR was carried out using 0.5 $\mu$l of the first strand cDNA reaction and 10 pmol of forward and reverse primers in total 25 $\mu$l of mixture. The PCR was run for 40 cycles of 95°C for 20 seconds, 56°C for 20 seconds, and 70°C for 30 seconds. The amplified products were separated on a 1% agarose gel. As shown in Fig. 62A, BCKDK was found to be expressed in both leukocytes and H1650 cells; CD45 was expressed only in leukocytes; and both EpCAM and EGFR were expressed only in H1650 cells. These results, which are fully consistent with the profile of EGFR expression shown in Fig. 62B, confirmed that EGFR is a particularly useful target for assaying mixtures of cells that include both leukocytes and cancer cells, because only the cancer cells will be expected to produce a signal.

**Example 6: EGFR assay with low quantities of target RNA or high quantities of background RNA**

In order to determine the sensitivity of the assay described in Example 4, various quantities of input NSCLC cell line total RNA were tested, ranging from 100 pg to 50 ng. The results of the first and second EGFR PCR reactions (step 1d, Example 4) are shown in Fig. 63. The first PCR reaction was shown to be sufficiently sensitive to detect 1 ng of input RNA, while the second round increased the sensitivity to 100 pg or less of input RNA. This corresponds to 7-10 cells, demonstrating that even extremely dilute samples may generate detectable signals using this assay.

Next, samples containing 1 ng of NCI-H1975 RNA were mixed with varying quantities of peripheral blood mononuclear cell (PBMC) RNA ranging from 1 ng to 1 $\mu$g and used in PCR reactions as before. As shown in Fig. 64A, the first set of PCR reactions demonstrated that, while amplification occurred in all cases, spurious bands appeared at the highest contamination level. However, as shown in Fig. 64B, after the second, nested set of PCR reactions, the desired specific amplicon was produced without spurious bands even at the highest contamination level. Therefore, this example demonstrates that the EGFR PCR assays described herein are effective even when the target RNA occupies a tiny fraction of the total RNA in the sample being tested.

Table 9 lists the RNA yield in a variety of cells and shows that the yield per cell is widely variable, depending on the cell type. This information is useful in order to estimate the amount of target and background RNA in a sample based on cell counts. For example, 1 ng of NCL-H1975 RNA corresponds to approximately 100 cells, while 1 μg of PBMC RNA corresponds to approximately $10^6$ cells. Thus, the highest contamination level in the above-described experiment, 1,000:1 of PBMC RNA to NCL-H1975 RNA, actually corresponds to a 10,000:1 ratio of PBMCs to NCL-H1975 cells. Thus, these data indicate that EGFR may be sequenced from as few as 100 CTCs contaminated by as many as $10^6$ leukocytes.

**Table 9. RNA Yield versus Cell Type**

| Cells | Count | RNA Yield | [RNA]/Cell |
|---|---|---|---|
| NCI-H1975 | $2\times10^6$ | 26.9 μg | 13.5 μg |
| NCI-H1650 | $2\times10^6$ | 26.1 μg | 13.0 pg |
| H358 | $2\times10^6$ | 26.0 μg | 13.0 μg |
| HT29 | $2\times10^6$ | 21.4 μg | 10.7 μg |
| MCF7 | $2\times10^6$ | 25.4 μg | 12.7 pg |
| PBMC #1 | $19\times10^6$ | 10.2 μg | 0.5 pg |
| PBMC #2 | $16.5\times10^6$ | 18.4 μg | 1.1 pg |

Next, whole blood spiked with 1,000 cells/ml of Cell Tracker (Invitrogen)-labeled H1650 cells was run through the capture module chip of Fig. 57C. To avoid inefficiency in RNA extraction from fixed samples, the captured H1650 cells were immediately counted after running and subsequently lysed for RNA extraction without formaldehyde fixation. Approximately 800 captured H1650 cells and >10,000 contaminated leukocytes were lysed on the chip with 0.5 ml of 4M guanidine thiocyanate solution. The lysate was extracted with 0.5 ml of phenol/chloroform and precipitated with 1ml of ethanol in the presence of 10 μg of yeast tRNA as carrier. The precipitated RNAs were DNase I-treated for 30 minutes and then extracted with phenol/chloroform and precipitated with ethanol prior to first strand cDNA synthesis and subsequent PCR amplification. These steps were repeated with a second blood sample and a second chip. The cDNA synthesized from chip1 and chip2 RNAs along with H1650 and leukocyte cDNAs were PCR amplified using two sets of primers, CD45_1 and CD45_2 (Table 8) as well as EGFR_5 (forward primer, 5'-GTTCGGCACGGTGTATAAGG-3') (SEQ ID NO: 52) and EGFR_6 (reverse primer, 5'-CTGGCCATCACGTAGGCTTC-3') (SEQ ID NO: 53). EGFR_5 and EGFR_6 produce a 138 bp wild type amplified fragment and a 123 bp mutant amplified fragment in H1650 cells. The PCR products were separated on a 2.5% agarose gel. As shown in Fig. 65, EGFR wild type and mutant amplified fragments were readily detected, despite the high leukocyte background, demonstrating that the EGFR assay is robust and does not require a highly purified sample.

**Example 7: Protocol for processing a blood sample through an enrichment module coupled to a capture module**

Using a sample of healthy blood spiked with tumor cells, a device of the invention containing an enrichment module coupled to a capture module was tested for the ability to enrich and capture tumor cells from blood.

To prepare the blood sample, a human non-small-cell lung cancer line, NCI-H1650 from ATCC) was stained with cell tracker orange (CMRA from Molecular Probes) and then spiked into fresh blood from a healthy patient (Research Blood Component). The spike level was 1,000 cells/ml. The spiked blood was diluted to a ratio of 2:1 (blood to buffer, 1 % BSA in PBS). Both leukocytes and tumor cells were labeled with nuclear staining dye, Hoechst 33342; labeling the tumor cells with an additional stain, cell tracker orange, helped to distinguish tumor cells from leukocytes.

Next, the enrichment module manifold, chip, and tubing were set up, and the enrichment module chip was primed with degassed buffer. The spiked blood sample was run through the enrichment module at a pressure of 2.4 psi, and the flow rate of product was 6.91 ml/hr.

Prior to running the product through the capture module, the product was characterized. Taking into account the dilution factor in the product, the number of leukocytes per ml of equivalent whole blood was $7.02\times10^5$. The removal efficiency of leukocytes was 90%. The yield of tumor cells was 89.5%, and the purity of the tumor cells was 0.14%.

The product from the enrichment module was then run through the capture module, which contained anti-Ep-CAM-coated obstacles. The tumor cells expressing epithelial cell adhesion molecule were captured on the obstacles. The flow rate was 2.12 ml/hr, and the running time was one hour. The device was then washed with buffer at a higher flow rate, 3 ml/hr, to remove the nonspecifically-bound cells. The yield was 74%. The purity was not determined.

The results of these experiments are summarized in Table 10.

**Table 10**

| | Yield of enrichment module (%) | Number of leukocytes/ml of whole blood | Tumor cell purity (%) | Yield of capture module (%) | Number of leukocytes/ml of whole blood | Tumor cell purity (%) | Combined yield (%) |
|---|---|---|---|---|---|---|---|
| Enrichment module (V1)-capture module | 89 | $7.02\times10^5$ | 0.14 | 74 (2.12 ml/hr) | Not measured | N/A | 66 |

**Example 8: Cell capture using staggered arrays**

In one embodiment of the invention, CTCs or other cells larger than a chosen cutoff size may be captured using a device that includes obstacles arranged in an array of subarrays. The subarrays are arrayed over the field with a slight stagger, or uneven spacing, initially designed in order to introduce variation in the flow lines and encourage the interaction of cells with the obstacles. One effect of this arrangement is that each subarray gives rise to a region in which the flow path is narrowed, as shown in Fig. 66A. In the array shown in the figure, the regular gap between obstacles is 46 $\mu$m, while the narrowed gap is 17 $\mu$m. The array and subarrays may be varied in order to result in any desirable gap sizes, as well as any desired density of narrowed gaps in relation to regular gaps.

Such a staggered array is particularly useful for preferential capture of CTCs in a blood sample, since CTCs tend to be larger than most other blood cells. CTCs or other large cells may be captured within the array without the need for a functionalized surface containing antibodies or other binding moieties, since cell capture is based on array geometry. Fabrication of such a device is therefore simplified.

A staggered array of the invention is shown in Fig. 66B. Narrowed flow paths are dispersed regularly throughout the device, and these paths may be sized to capture cells of a given hydrodynamic size or larger, while allowing cells smaller than this cutoff size to flow through the array without being retained. If a large cell is lodged in a narrow flow path, thereby blocking it, smaller cells are still able to flow around via the unblocked larger flow paths, as shown in Fig. 66C. This design avoids the problem of clogging that may occur in a uniform array.

Desirably, the device is configured such that CTCs or other cells of interest are statistically likely to encounter and be trapped in the areas of narrowed gaps. Devices may be optimized for particular applications by varying the density of the restricted flow paths to alter the probability of capture of target cells.

In one configuration, a larger percentage of flow paths near the device outlet may be designed to be narrow (Fig. 66D), thereby allowing for capture of any large cells that were not captured elsewhere in the array. Unless all available narrow gaps are occupied by target cells, clogging is still avoided in this configuration.

Some devices of the invention have a relatively large depth dimension in order to accommodate high throughput of sample, whereas in other embodiments, the depth dimension is much smaller, with the result that captured cells are largely found in one focal plane and are easier to view under a microscope. In the device shown in Fig. 66E, the depth dimension is structured to create narrowed flow paths, resulting in capture of cells in a single focal plane (Fig. 66F). The captured cells are directly below the transparent window for simplified viewing. Fabrication of such devices may be achieved readily by a variety of means, e.g., injection molding or hot embossing of polymer substrates.

Once captured, cells may be released, e.g., by treatment with a hypotonic solution that causes the cells to shrink and be released from the device. Upon release and collection, cells may be returned to their original osmolarity and subjected to further analysis, e.g, molecular analysis. Alternatively, analysis may be conducted within the device without releasing the cells.

**Example 9: Cell capture of H1650 cells using staggered arrays**

A capture module chip (Fig. 57C) was used to process a sample of H1650 lung cancer cells. Parameters of the capture module are as follows: the chip dimensions are 66.0x24.9 mm; the obstacle field dimensions are $51.3\times18.9$ mm; the obstacle diameter is 104 $\mu$m; the port dimensions are 2.83 x2.83 mm on the front side and 1.66 x 1.66 mm on the back side; the substrate is silicon; and the etch depth is 100 $\mu$m. The H1650 lung cancer cells were spiked at 10,000 cells/ml into buffy coat and run at 1.6 ml/hour (Fig. 66G). An estimated 12,700 H1650 cells passed through the device. The device contained approximately 7,230 capture locations in the active area. The yield of H1650 cells following the experiment was 16%, indicating that a substantial portion of available capture locations was occupied by H1650 cells.

**Example 10: Size distribution of cancer cells**

In order to determine the size distribution of cancer cells, several cancer cell lines were passed through a Beckman Coulter Model Z2 counting device (Fig. 67A). Cell, lines that were tested in this experiment included H3 5 8, H1650, H1975, HT29, and MCF7 cells, which include colon, lung, and breast cancer cells. As Fig. 67A shows, each of these cell lines consists of cells that are larger than most white blood cells. The size distributions of each cancer cell line are similar to each other and are well-separated from the distribution of white blood cells shown. A closeup of the size distribution of the cancer cells (Fig. 67B) reveals a generally Gaussian distribution of cells in each case, with only a small minority of cells below 8, 10, or even 12 μm in size (Fig. 67C). These data offer strong support for the principle of enrichment of CTC from other blood cells based on size.

**Example 11: Capture device using a microscope slide**

The invention encompasses a variety of cell capture devices and methods. In one embodiment, a capture device of the invention utilizes a functionalized surface, e.g., a glass microscope slide, as shown in Fig. 68A. The slide may be functionalized with an antibody or other capture moiety specific for the cell type of interest, e.g., CTCs, using standard chemistries. The device includes a sample fluid chamber, which may have, for example, a capacity of 10 ml or greater, with the functionalized slide on the bottom of the chamber. Any fluid, e.g., blood or a blood fraction, may be placed within the chamber for processing.

Cells within the fluid sample sediment to the bottom of the chamber via gravity, or optionally centrifugation (see Example 12), or application of other forces, and are bound by the functionalized surface. In order to keep the remaining cells tumbling, the chamber may be rocked (Fig. 68B) or rotated (Fig. 68C). Subsequently, the chamber may be washed and removed, and the slide is then available for staining, visualization, and/or other subsequent analysis.

Several advantages of such a device and method are evident. For example, the flat capture surface allows for easy visualization of captured cells. Furthermore, the uniform cell capture on the flat surface simplifies cell quantification. In addition, the residence time for cells contacting the surface is long in comparison to other methods, improving capture efficiency and allowing for the total duration of the experiment to be shortened. This duration may also be shortened in view of the fact that there is no limiting flow rate. Because the cells are not flowing through a device, they are also not subjected to flow-induced shear.

Other advantages include the fact that, in the configuration described here, surface area is generally not a limiting factor in the capture of rare cells. Furthermore, it is particularly straightforward to analyze captured cells using a light microscope or other visualization techniques, allowing for the analysis of morphology, organelle characteristics, or other cellular characteristics.

The capture device may be coupled to other devices for processing cellular samples or other fluid samples, and it is compatible with microcapture technologies.

In one variation, shown in Fig. 68D, two additional fluid chambers are present in the device. The fluid chambers, which may be filled with air, are alternately filled and emptied in order to cause fluid motion inside the main chamber of the device. The air chambers have a flexible wall separating them, and may be filled and emptied using any mechanism. The device mobilizes the cellular sample or other fluid sample, keeping sedimented cells tumbling and preventing the blockage of capture sites on the functionalized surface.

The capture surface of any of the above devices may be microstructured, e.g., with low relief, including micro-posts, micro-fins, and/or micro-corrugation. The functionalized surface may be, e.g., a microfabricated silicon chip surface or a plastic surface. This approach provides, for example, multiple, spatially patterned capture functionalities on the surface for differential capture, quantification, and/or targeting of multiple cell populations (Fig. 68E).

**Example 12: Centrifugal capture device using a microscope slide**

Prior to using a capture device of the invention, it is advantageous to perform microfluidics-based cell enrichment with a cell enrichment device of the invention. For example, by applying a first enrichment step to a blood sample, most erythrocytes, leukocytes, and platelets are removed. In one set of experiments, when blood samples were processed using cell enrichment devices of the invention having a cutoff of 8 μm, 10 μm, and 12 μm, erythrocytes and platelets were removed completely in each case, and the leukocyte concentration was reduced to $1.25\times10^5$ cells/ml, 2,900 cells/ml, and 111 cells/ml, respectively. Thus, a large portion of the contaminating cells in a blood sample or other cellular sample may be removed prior to a capture step, helping to avoid nonspecific sedimentation on a functionalized surface. However, the resulting enriched sample may be highly diluted, thereby increasing the processing time necessary to capture cells of interest, e.g., CTCs.

To decrease the time required to process a sample, the device described in Example 11 may be used in combination with a centrifuge (Fig. 69A). In this method, cells of interest, e.g., CTCs, are flattened against the functionalized slide (Fig. 69B) when the sample is exposed to a high centrifugal field of Nxg, where, for example, N is a large number, e.g., 1,000 or greater. This centrifugal method substantially increases the contact location and area between CTCs and binding moieties, e.g., antibodies.

Cell sedimentation velocity may be estimated by the equation:

$$u = \frac{a d_{cell}^2 (\rho_{cell} - \rho_{plasma})}{18 \mu_{plasma}}$$

where u represents velocity, d represents cell diameter, p represents density, μ represents viscosity, and a represents acceleration, i.e., gravitational or centrifugal field. The parameter a may be expressed as Nxg, where N equals 1 in the case of gravity, and N generally equals a large number, e.g., 1,000 or greater, in the case of centrifugation. When N equals 1, i.e., in the presence of gravity alone, it takes approximately one hour for a 14 μm diameter cell to settle in a 2 cm high liquid level chamber; however, with a centrifugal field of Nxg, sedimentation time is reduced by a factor of N, thereby significantly reducing the time required to perform the experiment.

Following capture of CTCs, leukocytes or other contaminating cells that are bound nonspecifically to the functionalized surface may be removed by inverting the chamber and subjecting it once again to a high centrifugal force (Fig. 69C). This step greatly reduces the number of contaminating cells that remain attached to the functionalized surface. In one embodiment, antibodies specific for contaminating cells such as leukocytes may be coupled to a functionalized surface opposite the surface that is used to capture the cells of interest (Fig. 69D), thereby capturing the contaminating cells and further minimizing contamination of the captured cells of interest. In another variation, the functionalized surface used to capture cells of interest may be inclined at an angle, resulting in a centrifugal force component that drives cell rolling along the planar surface, in addition to the perpendicular component of the centrifugal force (Fig. 69E). The component of the centrifugal force that drives cell rolling helps to spread clusters of cells and increases the efficiency of cell capture.

The applied centrifugal field may be optimized in a number of ways (Fig. 69F). For example, each period of centrifugation may be modified, including the "spin up" phase (period between starting centrifugation and attaining the desired rotational speed), "spin time" (period of centrifugation at desired rotational speed), "spin down" (period between beginning to slow centrifugation and coming to a stop), and "rest time" (period between spins). In each case, the duration, rotational speed, and/or rotational acceleration may be optimized to suit the application. This includes spinning the chamber in both the forward and reverse directions, as described above.

To improve capture efficiency, the functionalized surface may be microstructured (Fig. 69G), as in Example 11.

### Example 13: Capture device

In the enrichment devices of the invention that include obstacles (Fig. 70A, and described above), large cells generally have numerous interactions with the obstacles, while small cells are able to flow through the device with minimal contact with the obstacles. A capture device that includes antibodies or other binding moieties attached to the surfaces of arrayed obstacles may be designed using similar principles, and combines both size and affinity selectivity.

In a regular array of obstacles, the critical diameter depends on a number of parameters, including the gap size and the distance between obstacles (obstacle offset), as shown in Fig. 70B. As described above, cells that are larger than the critical diameter are deflected, while cells that are smaller than this parameter move in the average flow direction. Thus, based on the size of the cell type of interest, e.g., a particular type of CTC, the critical diameter may be optimized. This may be achieved, for example, by selecting an appropriate gap size and offset. The optimized device may provide efficient capture with very low contamination.

In one instance, the obstacle density may be varied throughout the device. For example, obstacles may be arrayed at a lower density near the sample inlet of the device, or order to prevent clogging, while the density may be increased near the device outlet, in order to maximize capture.

It is possible to vary the arrangement of obstacles while keeping the critical size constant. Thus, devices of the invention may include variable obstacle arrays in which the direction of deflection, the gap size, and/or the distance between obstacles is varied throughout the device, in order to increase flow rate, decrease clogging, or achieve other design goals (Fig. 70C).

In some devices, both target cells, e.g., CTCs, and contaminating cells, e.g., leukocytes, bind to the floor of the device. For example, this may occur in devices that include a functionalized silicon substrate containing obstacles, as all exposed surfaces of the silicon substrate are typically functionalized with antibody or other binding moiety. Thus, capture devices may be operated in an inverted orientation, such that any cells that sediment come into contact with a non-functionalized surface and do not bind. This may result in reduced clogging and may generally improve device performance.

The capture device described in this example, or other capture devices of the invention, may also include nonfunctionalized areas that may be used for enrichment or other purposes.

## Other Embodiments

All publications, patents, and patent applications mentioned in the above specification are hereby incorporated by reference. Various modifications and variations of the described method and system of the invention will be apparent to those skilled in the art without departing from the scope and spirit of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention.

Other embodiments are in the claims.

Furthermore, the present invention relates to the following items:

1. A method of detecting cancer cells in a cellular sample, said method comprising the steps of:

   a) introducing said cellular sample into a device comprising a channel comprising a structure that directs said cancer cells in a first direction to produce a first output sample enriched in said cancer cells and one or more second cells in a second direction to produce a second output sample enriched in said second cells; and
   b) detecting the presence or absence of said cancer cells in said first output sample.

2. The method of item 1, wherein said structure comprises an array of obstacles that form a network of gaps.

3. The method of item 2, wherein said obstacles are capable of selectively capturing said cancer cells.

4. The method of item 1., wherein said cellular sample is a blood sample.

5. The method of item 1, wherein step b) comprises reacting said first output sample with an antibody to a marker for said cancer cells.

6. The method of item 5, wherein said marker is selected from Table 1.

7. The method of item 1, wherein step b) comprises determining the number of cells in said first output sample.

8. The method of item 7, wherein said determining comprises determining the total amount of DNA in said first output sample.

9. The method of item 1, wherein step b) comprises determining the number of said cancer cells in said first output sample.

10. The method of item 9, said method further comprising determining the number of endothelial cells in said cellular sample.

11. The method of item 10, further comprising determining the ratio of said cancer cells to said endothelial cells.

12. The method of item 1, wherein step b) comprises detecting a mutation in DNA or RNA in said first output sample.

13. The method of item 12, wherein said mutation is in a gene encoding a polypeptide listed in Table 1.

14. The method of item 1, wherein step b) comprises analyzing protein phosphorylation, protein glycosylation, DNA methylation, microRNA levels, or cell morphology in said first output sample.

15. The method of, item 1, wherein step b) comprises detecting mitochondrial DNA, telomerase, or a nuclear matrix protein in said first output sample.

16. The method of item 1, wherein step b) comprises detecting one or more mitochondrial abnormalities in said first output sample.

17. The method of item 1, wherein step b) comprises detecting the presence or absence of perinuclear compartments in a cell of said first output sample.

18. The method of item 1, wherein step b) comprises performing gene expression analysis, in-cell PCR, or fluorescence in-situ hybridization of said first output sample.

19. The method of item 18, wherein said gene expression analysis is used to determine the tissue or tissues of origin of said cancer cells.

20. The method of item 18, wherein said gene expression analysis is performed on a single cancer cell.

21. The method of item 1, wherein said cellular sample comprises one or more progenitor endothelial cells, and wherein at least one of said progenitor endothelial cells is in said first output sample.

22. The method of item 1, said device comprising a continuous flow device comprising a first inlet, a first outlet, and a second outlet, wherein said cellular sample is applied to said first inlet, said first output sample flows out of said first outlet, and said second output sample flows out of said second outlet.

23. The method of item 22, wherein said second cells comprise noncancer cells.

24. The method of item 22, wherein said device comprises a second inlet, and wherein a second fluid is applied to said second inlet.

25. The method of item 24, wherein said second fluids comprises a buffer, a lysis reagent, a nucleic acid amplification reagent, an osmolarity regulating reagent, a labeling reagent, a preservative, or a fixing reagent.

26. A method of detecting cancer cells in a cellular sample, said method comprising the steps of:

a) enriching one or more of said cancer cells from said cellular sample without using magnetic particles, wherein said enriching is based on cell size, shape, or deformability; and
b) determining the number of said enriched cancer cells.

27. A method of detecting cancer cells in a cellular sample, said method comprising the steps of:

a) enriching one or more of said cancer cells from said cellular sample without using an antibody or fragment thereof, wherein said enriching is based on cell size, shape, or deformability; and
b) determining the number of said enriching cancer cells.

28. The method of item 26 or 27, said method further comprising the steps of:

i) enriching one or more endothelial cells from said cellular sample; and
ii) determining the number of said enriched endothelial cells.

29. The method of item 28, said method further comprising determining the ratio of said cancer cells to said endothelial cells.

30. The method of item 26 or 27, wherein step b) comprises counting said enriched cancer cells.

31. The method of item 26 or 27, wherein step b) comprises determining the total amount of DNA in said enriched cancer cells.

32. The method of item 26 or 27, further comprising repeating steps a) and b) with a second cellular sample.

33. The method of item 32, wherein said cellular sample and said second cellular sample are taken from a single subject.

34. The method of item 1, wherein said detecting comprises hyperspectral imaging of said first output sample.

35. The method of item 1, wherein, prior to or concurrently with step a), said cellular sample is contacted with a labeling reagent that preferentially labels said cancer cells.

36. The method of item 35, said labeling reagent comprising beads, wherein the hydrodynamic size of a labeled cancer cell is at least 10% greater than the hydrodynamic size of said cancer cell in the absence of said label.

37. A method for diagnosing a condition in a subject, said method comprising the steps of:

a) introducing a cellular sample from said subject into a device comprising a channel comprising a structure that directs one or more cancer cells in a first direction to produce a first output sample enriched in said cancer cells and one or more second cells in a second direction to produce a second output sample enriched in said second cells;
b) detecting the presence or absence of said cancer cells in said first output sample; and
c) diagnosing the presence or absence of said condition based on the results of step b).

38. The method of item 37, further comprising imaging a portion of said subject prior to said diagnosis, wherein said diagnosis is further based on the results of said imaging.

39. The method of item 38, wherein said imaging comprises computed axial tomography, positron emission tomography, or magnetic resonance imaging.

40. The method of item 37, said method further comprising:

i) determining the number of endothelial cells in said cellular sample; and
ii) determining the ratio of said cancer cells to said endothelial cells, wherein said diagnosis is further based on said ratio.

41. The method of item 40, wherein said endothelial cells comprise progenitor endothelial cells.

42. The method of item 40, wherein said condition is a hematological condition, an inflammatory condition, an ischemic condition, a neoplastic condition, infection, trauma, endometriosis, or kidney failure.

43. The method of item 1, wherein said channel comprises an array of obstacles forming a network of gaps, and wherein fluid flows through said gaps such that said fluid is divided unequally into a major flux and a minor flux.

44. A method for diagnosing a condition in a subject, said method comprising the steps of:

a) introducing a cellular sample from said subject into a device comprising a channel comprising a structure that directs one or more first cells in a first direction to produce a first output sample enriched in said first cells and one or more second cells in a second direction to produce a second output sample enriched in said second cells;
b) analyzing said first output sample; and
c) diagnosing the presence or absence of said condition based on the results of step b).

45. The method of item 44, wherein said device is configured to direct cells having a hydrodynamic size greater than 12 microns in said first direction, and cells having a hydrodynamic size less than or equal to 12 microns in said second direction.

46. The method of item 44, wherein said device is configured to direct cells having a hydrodynamic size greater than 14 microns in said first direction, and cells having a hydrodynamic size less than or equal to 14 microns in said second direction.

47. The method of item 44, wherein said device is configured to direct cells having a hydrodynamic size greater than or equal to 5 microns and less than or equal to 10 microns in said first direction, and cells having a hydrodynamic size greater than 10 microns in said second direction.

48. The method of item 44, wherein said device is configured to direct cells having a hydrodynamic size greater than or equal to 4 microns and less than or equal to 8 microns in said first direction, and cells having a hydrodynamic size greater than 8 microns in said second direction.

49. The method of item 44, wherein said first output sample comprises at least 90% of said first cells in said cellular sample.

50. The method of item 44, further comprising repeating steps a) through c) one or more times with additional cellular samples from said subject.

51. The method of item 50, wherein said cellular samples are obtained at regular intervals.

52. The method of item 51, wherein said regular interval is one day, two days, three days, one week, two weeks, one month, two months, three months, six months, or one year.

53. The method of item 44, wherein said first output sample is enriched in said first cells relative to said cellular sample by a factor of at least 1,000.

54. The method of item 44, wherein said condition is a hematological condition, an inflammatory condition, an ischemic condition, a neoplastic condition, infection, trauma, endometriosis, or kidney failure.

55. The method of item 54, wherein said neoplastic condition is selected from the group consisting of acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, and Wilm's tumor.

56. The method of item 44, wherein said cellular sample is less than 50 mL in volume.

57. The method of item 44, wherein step b) comprises detecting the presence or absence of a cancer biomarker in said first output sample.

58. The method of item 57, wherein said cancer biomarker is a polypeptide selected from Table 1, or a nucleic acid encoding said polypeptide.

59. The method of item 44, wherein step b) comprises identifying the presence or absence of a nucleic acid associated with cancer.

60. The method of item 59, wherein said nucleic acid comprises genomic DNA, mRNA, or microRNA.

61. The method of item 44, wherein step b) comprises analyzing the expression pattern of a nucleic acid associated with cancer.

62. The method of item 61, wherein said nucleic acid comprises genomic DNA, mRNA, or microRNA.

63. The method of item 59, wherein said nucleic acid contains a mutation and encodes a polypeptide selected from Table 1.

64. The method of item 44, wherein step b) comprises identifying the presence or absence of cells that have a cell surface marker associated with cancer.

65. The method of item 64, wherein said cell surface marker is selected from the group consisting af EpGAM, E-Cadherin, Mucin-1, Cytokeratin 8, EGFR, and leukocyte associated receptor (LAR).

66. The method of item 44, wherein step. b) further comprises contacting said first output sample with a device comprising a surface with one or more binding moieties that selectively bind one or more cells from said first output sample.

67. The method of item 66, wherein said cells from said first output sample comprise epithelial or neoplastic cells.

68. The method of item 67, wherein said neoplastic cell type is associated with a cancer selected from the group consisting of acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, and Wilm's tumor.

69. The method of item 68, wherein said cancer is not thyroid cancer.

70. The method of item 66, wherein said binding moieties comprise a polypeptide.

71. The method of item 70, wherein said polypeptide comprises an antibody or fragment thereof.

72. The method of item 71, wherein said antibody or fragment thereof is monoclonal.

73. The method of item 72, wherein said monoclonal antibody or fragment thereof binds to EpCAM.

74. The method of item 44, wherein said structure comprises an array of obstacles that form a network of gaps.

75. The method of item 74, wherein said obstacles are capable of selectively capturing said first cells.

76. The method of item 74, wherein said gaps between said obstacles are more than 15 microns, more than 20 microns, or less than 60 microns.

77. The method of item 44, wherein said cellular sample comprises blood, sweat, tears, ear flow, sputum, lymph, bone marrow suspension, urine, saliva, semen, vaginal flow, cerebrospinal fluid, brain fluid, ascites, milk, secretions of the respiratory, intestinal or genitourinary tract, amniotic fluid, or a water sample.

78. The method of item 44, wherein step b) comprises analyzing the size distribution of said first output sample.

79. The method of item 44, wherein step b) comprises determining the number of said first cells.

80. A method for identifying a cell pattern associated with a condition of interest, said method comprising the steps of:

a) obtaining a cellular sample from each of a plurality of control subjects and a plurality of case subjects having said condition of interest;
b) enriching by size, from each said cellular sample, cells having a hydrodynamic size greater than 12 microns;
c) analyzing cells enriched in step b); and
d) performing an association study using the results obtained in step c).

81. A method for diagnosing a condition in a subject, said method comprising the steps of:

a) providing a cell pattern associated with said condition;
b) obtaining a cellular sample from said subject;
c) enriching by size, from said cellular sample, cells having a hydrodynamic size greater than 12 microns;
d) analyzing cells enriched in step c); and
e) diagnosing the presence or absence of said condition in said subj ect based on said cell pattern of step a) together with said analysis of step d).

82. The method of item 80, wherein step c) comprises detecting RNA levels in said cells enriched in step b).

83. The method of item 82, wherein said RNA comprises mRNA or microRNA.

84. The method of item 80, said plurality of case subjects comprising at least 50 case subjects and said plurality of control subjects comprising at least 50 control subjects.

85. The method of item 80, wherein step c) comprises determining the number of said cells enriched in step b).

86. The method of item 85, wherein step c) utilizes a cellular characteristic selected from the group consisting of impedance, light absorption, light scattering, and capacitance.

87. The method of item 80, wherein step c) comprises analyzing the size distribution of said cells enriched in step b).

88. The method of item 87, wherein step c) comprises using a microscope, a cell counter, a magnet, a biocavity laser, a mass spectrometer, a PCR device, an RT-PCR device, a matrix, a microarray, or a hyperspectral imaging system in order to determine said size distribution.

89. The method of item 80, wherein said cellular sample comprises blood, sweat, tears, ear flow, sputum, lymph, bone marrow suspension, urine, saliva, semen, vaginal flow, cerebrospinal fluid, brain fluid, ascites, milk, secretions of the respiratory, intestinal or genitourinary tract, amniotic fluid, or a water sample.

90. The method of item 84, wherein step c) comprises determining the tissue or tissues of origin of said cells enriched in step b).

91. The method of item 80, wherein step c) comprises identifying, from said cells enriched in step b), one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

92. The method of item 80, wherein step e) comprises contacting said cells enriched in step b) with one or more binding moieties that selectively bind said first cells.

93. The method of item 92, wherein said binding moieties comprise a polypeptide.

94. The method of item 93, wherein said polypeptide comprises an antibody or fragment thereof.

95. The method of item 94, wherein said antibody or fragment thereof is monoclonal.

96. The method of item 95, wherein said monoclonal antibody or fragment thereof binds to EpCAM.

97. The method of item 94, wherein said wherein said antibody or fragment thereof is selected from the group consisting of anti-Ber-Ep4, anti-EpCAM, anti-E-Cadherin, anti-Mucin-1, anti-Cytokeratin 8, and anti-CD34+.

98. The method of item 80, wherein said device is configured to direct said cells having a hydrodynamic size greater than 12 microns in a first direction, and cells having a hydrodynamic size less than or equal to 12 microns in a second direction.

99. The method of item 80, wherein said device selectively captures said cells enriched in step b).

100. A method for determining the efficacy of a drug treatment administered to a subject, said method comprising the steps of:

a) obtaining a first cellular sample from said subject before said treatment;
b) introducing said first cellular sample into a device comprising a channel comprising a structure that directs one or more first cells in a first direction to produce a first output sample enriched in said first cells and one or more second cells in a second direction to produce a second output sample enriched in said second cells;
c) analyzing said first output sample;
d) obtaining a second cellular sample from said subject concurrently with or subsequent to said drug treatment;
e) repeating steps b) and c) for said second cellular sample; and
f) comparing the results of step c) for said first cellular sample and said second cellular sample,

wherein said comparing determines said efficacy of said drug treatment.

101. The method of item 100, wherein said device is configured to direct cells having a hydrodynamic size greater than 12 microns in said first direction, and cells having a hydrodynamic size less than or equal to 12 microns in said second direction:

102. The method of item 100, wherein said device is configured to direct cells having a hydrodynamic size greater than or equal to 6 microns and less than or equal to 12 microns in said first direction, and cells having a hydrodynamic size less than 6 microns or cells having a hydrodynamic size greater than 12 microns in said second direction.

103. The method of item 100, wherein step c) comprises detecting RNA levels in said first cells from said first cellular sample or said second cellular sample.

104. The method of item 103, wherein said RNA comprises mRNA or microRNA.

105. The method of item 100, wherein step c) comprises determining the number of said first cells from said first cellular sample or said second cellular sample.

106. The method of item 100, wherein step d) comprises identifying, from said first cells from said first cellular sample or said second cellular sample, one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

107. The method of item 44, wherein said channel comprises an array of obstacles forming a network of gaps, and wherein fluid flows through said gaps such that said fluid is divided unequally into a major flux and a minor flux.

108. A device for processing a cellular sample, said device comprising:

a) a channel comprising a structure that directs one or more first cells in a first direction to produce a first output sample enriched in said first cells and one or more second cells in a second direction to produce a second output sample enriched in said second cells, wherein said device is configured either:

i) to direct cells having a hydrodynamic size greater than 12 microns in said first direction, and cells having a hydrodynamic size less than or equal to 12 microns in said second direction; or
ii) to direct cells having a hydrodynamic size greater than or equal to 6 microns and less than or equal to 12 microns in said first direction, and cells having a hydrodynamic size less than 6 microns or cells having a hydrodynamic size greater than 12 microns in said second direction; and

b) a detection module for analyzing said first output sample or said second output sample, wherein said detection module is fluidically coupled to said channel.

109. The device of item 108, wherein said device is configured to direct cells having a hydrodynamic size greater than or equal to 6 microns and less than or equal to 12 microns in said first direction, and cells having a hydrodynamic size less than 6 microns or cells having a hydrodynamic size greater than 12 microns in said second direction.

110. The device of item 108, wherein said device is configured to direct cells having a hydrodynamic size greater than or equal to 8 microns and direct cells having a hydrodynamic size greater than or equal to 8 microns and less than or equal to 10 microns in said first direction, and cells having a hydrodynamic size less than 8 microns or cells having a hydrodynamic size greater than 10 microns in said second direction.

111. The device of item 108, wherein said detection module is adapted to identify a marker associated with cancer in said first cells.

112. The device of item 108, wherein said detection module comprises an antibody that specifically binds said first cells.

113. The device of item 112, wherein said antibody specifically binds one or more markers selected from Table 1.

114. The device of item 108, wherein said detection module is configured to detect one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

115. The device of item 108, wherein said detection module comprises a microscope, a cell counter, a magnet, a biocavity laser, a mass spectrometer, a PCR device, an RT-PCR device, a matrix, a microarray, or a hyperspectral imaging system.

116. A device for processing a cellular sample, said device comprising:

a) a channel comprising a structure that directs one or more cancer cells in a first direction to produce a first output sample enriched in said cancer cells and one or more second cells in a second direction to produce a second output sample enriched in said second cells; and
b) a capture module for capturing said cancer cells or said second cells, wherein said capture module is fluidically coupled to said channel, and wherein said capture module comprises one or more binding moieties that selectively bind said cancer cells or said second cells.

117. The device of item 116, wherein said structure comprises an array of obstacles that form a network of gaps.

118. The device of item 116, wherein said one or more binding moieties specifically bind one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

119. The device of item 117, wherein said obstacles comprise said binding moieties.

120. The device of item 116, wherein said device is configured to direct cells having a hydrodynamic size greater than 12 microns in said first direction.

121. The device of item 116, wherein said device is configured to direct cells having a hydrodynamic size greater than 14 microns in said first direction.

122. The device of item 116, wherein said device is configured to direct cells having a hydrodynamic size greater than 16 microns in said first direction.

123. The device of item 116, further comprising a cell counting module fluidically coupled to said capture module.

124. The device of item 116, wherein said one or more binding moieties comprise a polypeptide.

125. The device of item 124, wherein said polypeptide comprises an antibody or fragment thereof.

126. The device of item 125, wherein said antibody or fragment thereof is monoclonal.

127. The device of item 126, wherein said monoclonal antibody or fragment thereof binds to EpCAM.

128. A device for processing a cellular sample, said device comprising a channel comprising a structure that directs one or more first cells in a first direction to produce a first output sample enriched in said first cells and one or more second cells in a second direction to produce a second output sample enriched in said second cells, wherein said structure comprises an array of obstacles that form a network of gaps, and wherein at least some of said obstacles comprise monoclonal anti-EpCAM antibodies or fragments thereof that selectively bind said first cells or said second cells.

129. A device for processing a cellular sample, said device comprising:

a) an enrichment module that is capable of enriching cells in said cellular sample based on size; and
b) a cell counting module for determining the number of cells enriched by said enrichment module, wherein said cell counting module is fluidically coupled to said enrichment module.

130. The device of item 129, wherein said enrichment module comprises a channel comprising a structure that directs one or more first cells in a first direction to produce a first output sample enriched in said first cells and one or more second cells in a second direction to produce a second output sample enriched in said second cells.

131. The device of item 130, wherein said device is configured to direct cells having a hydrodynamic size greater than 12 microns in said first direction, and cells having a hydrodynamic size less than or equal to 12 microns in said second direction.

132. The device of item 130, wherein said device is configured to direct cells having a hydrodynamic size greater than or equal to 6 microns and less than or equal to 12 microns in said first direction, and cells having a hydrodynamic size less than 6 microns or cells having a hydrodynamic size greater than 12 microns in said second direction.

133. The device of item 130, wherein said first cells comprise cancer cells.

134. The device of item 130, wherein said structure comprises an array of obstacles that form a network of gaps.

135. The device of item 129, wherein said cell counting module utilizes impedance, optics, or capacitance to determine said number of cells in said first output sample or said second output sample.

136. The device of item 116, 128, or 129, wherein said device further comprises a detector adapted to visualize said first output sample or said second output sample, said detector fluidically coupled to said capture module.

137. The device of item 108, wherein said channel comprises an array of obstacles forming a network of gaps, and wherein fluid flows through said gaps such that said fluid is divided unequally into a major flux and a minor flux.

138. A device for processing a cellular sample, said device comprising a channel comprising a structure that directs one or more first cells in a first direction to produce a first output sample enriched in said first cells and one or more second cells in a second direction to produce a second output sample enriched in said second cells, wherein said device is capable of processing at least 20 mL of fluid per hour.

139. The device of item 138, wherein said structure comprises an array of obstacles that form a network of gaps.

140. The device of item 139, wherein said gaps are between 20 and 114 microns in size.

141. The device of item 139, wherein said array of obstacles comprises a staggered two-dimensional array of obstacles.

142. The device of item 139, wherein said array of obstacles comprises a plurality of rows, each successive row being offset by less than half of the period of the previous row.

143. The device of item 139, further comprising one or more additional arrays of obstacles in series or in parallel with said first array of obstacles.

144. The device of item 138, wherein said first cells have a larger average hydrodynamic size than said second cells.

145. The device of item 138, wherein said device is capable of processing at least 50 mL of fluid per hour.

146. The device of item 138, wherein said cellular sample comprises blood or a fraction thereof.

147. The device of item 138, wherein said device is configured to direct cells having a hydrodynamic size greater than 12 microns in said first direction.

148. The device of item 138, wherein said device is configured to direct cells having a hydrodynamic size greater than 14 microns in said first direction.

149. The device of item 138, wherein said device is configured to direct cells having a hydrodynamic size greater than 16 microns in said first direction.

150. The device of item 138, said device comprising a continuous flow device comprising a first inlet, a first outlet, and a second outlet, wherein said cellular sample is applied to said first inlet, said first output sample flows out of said first outlet, and said second output sample flows out of said second outlet.

151. The device of item 13 8, wherein said device is capable of producing a first output sample enriched in said first cells, wherein the volume of said first output sample is smaller than the volume of said cellular sample.

152. The device of item 138, wherein said first output sample comprises at least 80% of said first cells in said cellular sample.

153. The device of item 138, wherein said second output sample comprises less than 20% of said first cells in said cellular sample.

154. The device of item 150, wherein said device comprises a second inlet, and wherein a second fluid is applied to said second inlet.

155. The device of item 138, wherein said first cells comprise epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

156. The device of item 138, said device further comprising a detector module fluidically coupled to said channel.

157. The device of item 156, wherein said detector module comprises a microscope, a cell counter, a magnet, a biocavity laser, a mass spectrometer, a PCR device, an RT-PCR device, a matrix, a microarray, or a hyperspectral imaging system.

158. The device of item 157, wherein said detector module detects a label that selectively binds said first cells.

159. The device of item 138, wherein said device is adapted for implantation in a subject.

160. The device of item 159, wherein said device is adapted for placement in or near the circulatory system of a subject.

161. A system that is capable of being fluidically coupled to the circulatory system of a subject, said system comprising a device for processing a cellular sample, said device comprising a channel comprising a structure that directs one or more first cells in a first direction to produce a first output sample enriched in said first cells and one or more second cells in a second direction to produce a second output sample enriched in said second cells.

162. The system of item 161, wherein said system is fluidically coupled to said circulatory system through tubing or an arteriovenous shunt.

163. The system of item 161, wherein said system is capable of removing one or more analytes from said circulatory system.

164. The system of item 161, wherein said system is adapted for continuous blood flow through said device.

165. The system of item 161, wherein said device is disposable.

166. A method for depleting an analyte from a cellular sample, said method comprising introducing said cellular sample into a device for processing a cellular sample, said device comprising a channel comprising a structure that directs one or more first cells in a first direction to produce a first output sample enriched in said first cells and one or more second cells in a second direction to produce a second output sample enriched in said second cells, wherein said first output sample or said second output sample is depleted in said analyte relative to said cellular sample.

167. The method of item 166, wherein said cellular sample comprises blood, sweat, tears, ear flow, sputum, lymph, bone marrow suspension, urine, saliva, semen, vaginal flow, cerebrospinal fluid, brain fluid, ascites, milk, secretions of the respiratory, intestinal or genitourinary tract, amniotic fluid, or a water sample.

168. The method of item 166, wherein said cellular sample is taken from a subject afflicted with a hematological condition, an inflammatory condition, an ischemic condition, a neoplastic condition, infection, trauma, endometriosis, or kidney failure.

169. The method of item 166, wherein said neoplastic condition is selected from the group consisting of acute lymphoblastic leukemia, acute or chronic lymphocyctic or granulocytic tumor, acute myeloid leukemia, acute promyelocytic leukemia, adenocarcinoma, adenoma, adrenal cancer, basal cell carcinoma, bone cancer, brain cancer, breast cancer, bronchi cancer, cervical dysplasia, chronic myelogenous leukemia, colon cancer, epidermoid carcinoma, Ewing's sarcoma, gallbladder cancer, gallstone tumor, giant cell tumor, glioblastoma multiforma, hairy-cell tumor, head cancer, hyperplasia, hyperplastic comeal nerve tumor, in situ carcinoma, intestinal ganglioneuroma, islet cell tumor, Kaposi's sarcoma, kidney cancer, larynx cancer, leiomyomater tumor, liver cancer, lung cancer, lymphomas, malignant carcinoid, malignant hypercalcemia, malignant melanomas, marfanoid habitus tumor, medullary carcinoma, metastatic skin carcinoma, mucosal neuromas, mycosis fungoide, myelodysplastic syndrome, myeloma, neck cancer, neural tissue cancer, neuroblastoma, osteogenic sarcoma, osteosarcoma, ovarian tumor, pancreas cancer, parathyroid cancer, pheochromocytoma, polycythemia vera, primary brain tumor, prostate cancer, rectum cancer, renal cell tumor, retinoblastoma, rhabdomyosarcoma, seminoma, skin cancer, small-cell lung tumor, soft tissue sarcoma, squamous cell carcinoma, stomach cancer, thyroid cancer, topical skin lesion, veticulum cell sarcoma, and Wilm's tumor.

170. A method for diagnosing a condition in a subject, said method comprising the steps of:

a) introducing a cellular sample from said subject into a device for processing a cellular sample, said device comprising a channel comprising a structure that directs one or more first cells in a first direction to produce a first output sample enriched in said first cells and one or more second cells in a second direction to produce a second output sample enriched in said second cells, wherein said device is capable of processing at least 20 mL of fluid per hour;
b) analyzing said first output sample; and
c) diagnosing the presence or absence of said condition based on the results of step b).

171. The method of item 170, wherein step b) comprises analyzing the cells of said first output sample for one or more characteristics selected from the group consisting of adhesion, migration, binding, morphology, division, level of gene expression, and presence of a somatic mutation.

172. The method of item 170, wherein step b) comprises detecting the presence or absence of one or more markers selected from Table 1, detecting the presence or absence of a mutation in a nucleic acid that encodes one or more markers selected from Table 1, detecting the presence or absence of a deletion in a nucleic acid that encodes one or more markers selected from Table 1, detecting the level of expression of one or more markers selected from Table 1, or detecting the level of microRNA in said first output sample.

173. The method of item 170, wherein said condition is a hematological condition, an inflammatory condition, an ischemic condition, a neoplastic condition, infection, trauma, endometriosis, or kidney failure.

174. The method of item 170, wherein step b) comprises determining the number of said first cells in said first output sample.

175. The device of item 138, wherein said channel comprises an array of obstacles forming a network of gaps, and wherein fluid flows through said gaps such that said fluid is divided unequally into a major flux and a minor flux.

176. A device for processing a cellular sample, said device comprising a channel comprising an array of obstacles forming a network of gaps, wherein fluid flows through said gaps such that said fluid is divided unequally into a maj or flux and a minor flux, and wherein said array is configured to direct epithelial cells in a direction not parallel to the average direction of flow in said array.

177. The device of item 176, wherein said device further comprises two inlets.

178. The device of item 176, wherein said device further comprises two outlets.

179. The device of item 176, further comprising a second device of item 176, wherein said devices are fluidically coupled.

180. The device of item 178, wherein said gaps are sized to direct epithelial cells to one of said outlets.

181. The device of item 177, wherein said gaps are sized to direct epithelial cells from the fluid flowing into one said inlet to the fluid flowing into another said inlet.

182. The device of item 176, wherein said obstacles comprise a polymer, silicon, glass, or fused silica.

183. A device for processing a cellular sample, said device comprising:

a. a channel comprising an array of obstacles forming a network of gaps, wherein fluid flows through said gaps such that said fluid is divided unequally into a major flux and a minor flux so that the average direction of movement of cells larger than a critical size is not parallel to the average direction of fluidic flow;
b. an outlet disposed to collect cells larger than normal blood cells; and
c. a cell detector that counts cells.

184. A method of producing an enriched sample of epithelial cells, said method comprising introducing a cellular sample into the device of item 176, wherein, as said cellular sample flows through said device, epithelial cells present in said cellular sample are directed in a direction not parallel to the average flow direction of said cellular sample and another component of said cellular sample is directed along the average flow direction, thereby producing a sample enriched in epithelial cells relative to said other component.

185. The method of item 184, wherein said other component comprises red blood cells, platelets, leukocytes, or endothelial cells.

186. The method of item 184, wherein said epithelial cells range from 8 μm to 30 μm in diameter.

187. The method of item 184, wherein said cells are capable of undergoing DNA analysis, RNA analysis, protein analysis, or metabolome analysis.

188. The method of item 187, wherein said cells are analyzed using in-cell PCR.

189. The method of item 187, wherein said cells are analyzed by determining the level of cytokeratin mRNA present.

190. The method of item 184, wherein said epithelial cells are cancer cells.

191. The method of item 190, wherein said cancer cells are cancer stem cells.

192. The method of item 184, wherein the volume of said enriched sample is substantially smaller than the volume of said cellular sample, resulting in concentration of said epithelial cells.

193. The method of item 184, wherein a lysis buffer is co-introduced into said device, and wherein said enriched sample comprises components of epithelial cells.

194. The method of item 184, wherein an exchange buffer is co-introduced into said device, and wherein said enriched sample comprises exchange buffer.

195. The method of item 184, further comprising contacting said enriched sample with a labeling reagent that preferentially labels epithelial cells.

196. The method of item 184, further comprising quantifying the number of cells in said enriched sample.

197. The method of item 196, wherein said device determines the number of cells of cell volume greater than 500 fL in said enriched sample.

198. The method of item 196, wherein said device determines the number of cells of diameter greater than 14 $\mu$m in said enriched sample.

199. The method of item 184, wherein the shear stress on each of said cells inside said device is below 10 dynes per square centimeter at all times.

200. The method of item 184, wherein said cellular sample is contacted with a labeling reagent that preferentially labels epithelial cells, wherein said labeling reagent is first combined with said cellular sample either prior to introduction of said cellular sample to said device or following introduction of said cellular sample to said device.

201. The method of item 200, wherein said labeling reagent is a particulate labeling reagent.

202. The method of item 200, said method further comprising quantifying said labeled cells.

203. The method of item 202, wherein said labeling reagent comprises quantum dots, and said labeled cells are analyzed using 2-photon excitation.

204. The method of item 203, said method further comprising making a bulk measurement of said enriched sample.

205. The method of item 200, wherein labeling reagent bound to said cells is separated from labeling reagent that remains unbound to said cells.

206. The method of item 200, wherein said labeling reagent is co-introduced into said device, resulting in labeling of said cells within said device.

207. The method of item 200, wherein said labeling reagent comprises a quantum dot, an antibody, a phage, an aptamer, a fluorophore, an enzyme, or a bead.

208. The method of item 207, wherein said bead comprises an affinity reagent.

209. The method of item 207, wherein said bead comprises polystyrene.

210. The method of item 207, wherein said bead is neutrally buoyant.

211. The method of item 207, wherein said bead comprises an antibody.

212. The method of item 200, wherein said labeling reagent increases the size of said cells.

213. The method of item 212, wherein said size increases by at least 10%.

214. The method of item 212, wherein said size increases by at least 100%.

215. The method of item 212, wherein said size increases by at least 1000%.

216. A method of quantifying epithelial cells, said method comprising the steps of:

a. providing a cellular sample;
b. introducing said cellular sample into the device of item 176 to produce a sample enriched in epithelial cells; and
c. quantifying said epithelial cells.

217. A diagnostic method comprising the steps of:

a. introducing a cellular sample from a patient into the device of item 176 to produce a sample enriched in cells larger than normal blood cells; and
b. determining the number of cells larger than normal blood cells present in said sample to determine a disease state.

218. A diagnostic method comprising the steps of:

a. introducing a cellular sample from a patient into the device of item 176 to produce a sample enriched in cells larger than normal blood cells; and
b. performing DNA analysis, RNA analysis, proteome analysis, or metabolome analysis on said enriched sample in order to determine a disease state.

219. The method of item 218, wherein said DNA analysis comprises determining the presence and identity, or the absence, of one or more mutations in the epidermal growth factor receptor gene.

220. The method of item 219, wherein said DNA analysis comprises the steps of:

i. isolating genomic DNA from said enriched sample; and
ii. amplifying one or more of exons 18, 19, 20, and 21 of said epidermal growth factor receptor gene.

221. The method of item 220, further comprising the steps of:

iii. amplifying subregions of said amplification products corresponding to known epidermal growth factor receptor mutations, resulting in further amplification products;
iv. sequencing said further amplification products; and
v. comparing the resulting sequences to the wild-type sequence of said epidermal growth factor receptor gene and a list of known mutations of said epidermal growth factor receptor gene.

222. The method of item 217 or 218, wherein said cells larger than normal blood cells comprise epithelial cells.

223. The method of item 217 or 218, wherein said disease state comprises cancer.

224. The device of item 183, wherein said cells larger than normal blood cells comprise epithelial cells.

225. A device for processing a cellular sample, said device comprising a channel comprising a ceiling positioned over an array of obstacles that form a network of gaps, wherein said device is configured to allow cells having a diameter of less than or equal to a critical size to flow through said network of gaps, and to capture cells having a diameter of greater than said critical size between said ceiling and said obstacles.

226. The device of item 225, wherein said critical size is between 5 and 20 microns.

227. The device of item 225, wherein said critical size is 12 microns.

228. The device of item 225, wherein said critical size is 14 microns.

229. The device of item 225, wherein said cells having a diameter greater than said critical size comprise one or more rare cells.

230. The device of item 229, wherein said rare cells comprise one or more epithelial cells, cancer cells, bone marrow cells, fetal cells, progenitor cells, stem cells, foam cells, mesenchymal cells, immune system cells, endothelial cells, endometrial cells, connective tissue cells, trophoblasts, bacteria, fungi, or pathogens.

231. The device of item 225, wherein said ceiling is transparent.

232. A method of processing cells having a diameter of greater than a critical size in a cellular sample, said method comprising introducing said cellular sample into a device comprising a channel comprising a ceiling positioned over an array of obstacles that form a network of gaps, wherein said device is configured to allow cells having a diameter of less than or equal to said critical size to flow through said network of gaps, and to capture cells having a diameter of greater than said critical size between said ceiling and said obstacles.

233. The method of item 232, further comprising detecting the presence or absence of said cells having a diameter of greater than said critical size between said ceiling and said obstacles.

234. The method of item 233, wherein said ceiling is transparent, and wherein said detecting comprises using a microscope.

235. The method of item 232, further comprising introducing a buffer, a lysis reagent, a nucleic acid amplification reagent, an osmolarity regulating reagent, a labeling reagent, a preservative, or a fixing reagent into said device subsequent to introducing said cellular sample.

236. The method of item 235, wherein said osmolarity regulating reagent comprises a hypertonic solution.

237. The method of item 236, wherein said hypertonic solution causes said cells having a diameter of greater than said critical size between said ceiling and said obstacles to be released.

238. A method for determining the likelihood of effectiveness of an epidermal growth factor receptor (EGFR) targeting treatment in a human patient affected with or at risk for developing cancer comprising:

applying a cellular sample from said patient to a device comprising a channel comprising a structure which directs first cells of one hydrodynamic size in one direction to produce a first output enriched in first cells and one or more second cells in a second direction to produce a second output; and
in cells of said first or second output, detecting the presence or absence of at least one predetermined nucleic acid variant in the EGFR gene, wherein the presence of said at least one nucleic acid variant indicates that the EGFR targeting treatment is likely to be effective.

239. The method of item 238, wherein said variant is located in the kinase domain of the erbBl gene of cells of said first output, said variant being the wildtype erbB1 gene.

240. The method of item 239, wherein the nucleic acid variant increases kinase activity.

241. The method of item 238, wherein said, first cells are cancer cells.

242. The method of item 240, wherein said cancer is selected from the group consisting of gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer and bladder cancer.

243. The method of item 241, wherein said cancer is non-small cell lung cancer.

244. The method of item 239, wherein the variant in the kinase domain of the erbBl gene affects the conformational structure of the ATP-binding pocket.

245. The method of item 239, wherein the variant in the kinase domain of erbB 1 is in an exon of the erbB1 gene selected from the group consisting of exon 18, 19, 20, and 21.

246. The method of item 245, wherein the variant is in exon 18, 19 or 21.

247. The method of item 239, wherein the variant in the kinase domain of the erbBl gene is an in frame deletion, substitution, or insertion.

248. The method of item 238, wherein the detection of the presence or absence of said at least one variant comprises amplifying a segment of nucleic acid.

249. The method of item 239, wherein the detection of the presence or absence of said at least one variant comprises contacting the erbBl nucleic acid with at least one nucleic acid probe, wherein said at least one probe preferentially hybridizes with a nucleic acid sequence comprising said variant under selective hybridization conditions.

250. The method of item 239, wherein the detection of the presence or absence of at least one variant comprises performing a polymerase chain reaction (PCR) to amplify nucleic acid comprising the erbB1 coding sequence, and determining the nucleotide sequence of the amplified nucleic acid.

251. The method of item 238, wherein said structure comprises a two dimensional array of obstacles forming a network of gaps which separate said cells.

252. The method of item 251, further comprising contacting said first output with a device comprising specific binding moieties which selectively bind epithelial or neoplastic cells.

253. The method of item 252, wherein said specific binding moieties are antibodies or fragments thereof.

254. The method of item 253, wherein said antibodies specifically bind Ber-Ep4, EpCam, E-Cadherin, mucin-1, cytokeratin or CD-34.

255. A method for determining the likelihood of effectiveness of an EGFR targeting treatment in a patient comprising:

applying a cellular sample from said patient to a device comprising a channel comprising a structure which directs first cells of one hydrodynamic size in one direction to produce a first output enriched in first cells and one or more second cells in a second direction to produce a second output;
stimulating said cells with an EGFR ligand; and
determining the kinase activity of the erbB1 gene-encoded kinase in said first cells, wherein an increase in kinase activity, compared to a control, indicates that the EGFR targeting treatment is likely to be effective.

256. The method of item 255, wherein said first cells are cancer cells.

257. The method of item 256, wherein said cancer is selected from the group consisting of gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer, and bladder cancer.

258. The method of item 25 6, wherein said cancer is non-small cell lung cancer.

259. The method of item 255, wherein said structure comprises a two dimensional array of obstacles forming a network of gaps which separate said cells.

260. The method of item 259, further comprising contacting said first output with a device comprising specific binding moieties which selectively bind epithelial or neoplastic cells.

261. The method of item 260, wherein said specific binding moieties are antibodies or fragments thereof.

262. The method of item 261, wherein said antibodies specifically bind Ber-Ep4, EpCam, E-Cadherin, mucin-1, cytokeratin or CD-34.

263. The method of item 255, wherein the EGFR targeting treatment is a tyrosine kinase inhibitor.

264. The method of item 263, wherein the tyrosine kinase inhibitor is an artilinoquinazoline.

265. The method of item 264, wherein the anilinoquinazoline is a synthetic anilinoquinazoline.

266. The method of item 265, wherein the synthetic anilinoquinazoline is selected from the group consisting of gefitinib and erlotinib.

267. A method for determining the likelihood of effectiveness of an epidermal growth factor receptor (EGFR) targeting treatment in a human patient affected, with or at risk for developing cancer comprising:

applying a cellular sample from said patient to a device comprising a channel comprising a structure which directs first cells of one hydrodynamic size in one direction to produce a first output enriched in first cells and one or more second cells in a second direction to produce a second output;
in said first cells, detecting the presence or absence of at least one nucleic acid variant in exon 18, 19, 20, or 21 by performing a polymerase chain reaction (PCR) to amplify a portion of exon 18, 19, 20, or 21; and
determining the nucleotide sequence of the amplified nucleic acid by sequencing at least one portion of the amplified exon 18, 19, 20, or 21, wherein the presence of at least one nucleotide variant in exon 18, 19, 20, or 21 compared to a wildtype erbB1 control indicates that the EGFR targeting treatment is likely to be effective.

268. The method of item 267, wherein said structure comprises a two dimensional array of obstacles forming a network of gaps which separate said cells.

269. The method of item 268, further comprising contacting said first output with a device comprising specific binding moieties which selectively bind epithelial or neoplastic cells.

270. The method of item 269, wherein said specific binding moieties are antibodies or fragments thereof.

271. The method of item 270, wherein said antibodies specifically bind Ber-Ep4, EpCam, E-Cadherin, mucin-1, cytokeratin or CD-34.

272. A kit comprising:

a device comprising a channel comprising a structure which directs first cells of one hydrodynamic size in one direction to produce a first output enriched in first cells and one or more second cells in a second direction to produce a second output; and
reagents for detecting the presence or absence of at least one nucleic acid mutation in the EGFR gene.

273. The kit of item 272, wherein said reagents comprise at least one degenerate primer pair designed to anneal to nucleic acid and products and reagents required to carry out PCR amplification.

274. The kit of item 272, wherein said structure comprises a two dimensional array of obstacles forming a network of gaps which separate said cells.

275. The kit of item 274, further comprising a device adapted to receive said first output, which device comprises specific binding moieties which selectively bind epithelial or neoplastic cells.

276. The kit of item 275, wherein said specific binding moieties are antibodies or fragments thereof.

277. The kit of item 276, wherein said antibodies specifically bind Ber-Ep4, EpCam, E-Cadherin, mucin-1, cytokeratin or CD-34.

278. The kit of item 272, wherein said reagents comprise at least one probe capable of binding to the ATP-binding pocket of the EGFR kinase domain protein.

279. The kit of item 272 or 278, wherein said reagents comprise an antibody, antibody fragment of chimeric antibody.

280. The kit of item 279, wherein said reagents further comprise a detectable label.

281. A method for predicting the acquisition of secondary mutations in the EGFR gene of a cancer cell from a patient comprising:

contacting a cellular sample from said patient with a device comprising a channel comprising a structure which directs first cells of one hydrodynamic size in one direction to produce a first output enriched in first cells and one or more second cells in a second direction to produce a second output;
contacting said first cells with a sublethal dose of a tyrosine kinase inhibitor;
selecting cells that are resistant to the effect of the tyrosine inhibitor; and
analyzing the nucleic acid from said resistant cells for the presence of secondary mutations.

282. The method of item 281, wherein said first cells have a variant of the erbB1 gene.

283. The method of item 281, wherein said structure comprises a two dimensional array of obstacles forming a network of gaps which separate said cells.

284. The method of item 273, further comprising contacting said first output with a device comprising specific binding moieties which selectively bind epithelial or neoplastic cells.

285. The method of item 284, wherein said specific binding moieties are antibodies or fragments thereof.

286. The method of item 285, wherein said antibodies specifically bind Ber-Ep4, EpCam, E-Cadherin, mucin-1, cytokeratin or CD-34.

287. The method of item 281, wherein the cell is obtained from a tumor biopsy.

288. The method of item 281, further comprising first contacting said first cells with an effective amount of a mutagenizing agent.

289. The method of item 288, wherein the mutagenizing agent is selected from the group consisiting of ethyl methanesulfonate (EMS), N-ethyl-N-nitrosourea (ENU), N-methyl-N-nitrosourea (MNU), phocarbaxine hydrocfciloride (Prc), methyl methanesulfonate (MeMS), chlorambucil (Chi), melphalan, porcarbazine hydrochloride, cyclophosphamide (Cp), diethyl sulfate (Et2SO4), acrylamide monomer (AA), triethylene melamin (TEM), nitrogen mustard, vincristine, dimethylnitrosamine, N-methyl-N'-nitro-Nitrosoguanidine (MNNG), 7,12 dimethylbenz(a)anthracene (DMBA), ethylene oxide, hexamethylphosphoramide, bisulfan, and ethyl methanesulforate (EtMs).

290. A method for determining the likelihood of effectiveness of an epidermal growth factor receptor (EGFR) targeting treatment in a patient affected with or at risk for developing cancer comprising:

contacting a biological sample from a patient with a device comprising a channel comprising a structure which directs first cells of one hydrodynamic size in one direction to produce a first output enriched in first cells and one or more second cells in a second direction to produce a second output, wherein said first cells are epithelial or neoplastic cells; and

determining whether Akt, STAT5, or STAT3 are activated in said first cells from said first output, wherein activated Akt, STATS, or STATS indicates that said EGFR targeting treatment is likely to be effective.

291. The method of item 290, wherein the biological sample is a biopsy or an aspirate.

292. The method of item 290, wherein activated Akt, STAT3, or STATS is phosphorylated.

293. The method of item 290, wherein the activated Akt, STATS, or STATS is determined immunologically.

294. The method of item 293, wherein the immunological detection methods are selected from the group consisting of immimohistochemistry, immunocytochemistry, FACTS scanning, immunoblotting, radioimmunoassays, western blotting, immunoprecipitation, or enzyme-linked immunoadsorbant assays (ELISA).

295. The method of item 294, wherein the immunological detection method is immunohistochemistry or immunocytochemistry using anti-phospho Akt, antiphospho STATS or anti-phospho STATS antibodies.

296. The method of item 290, wherein said structure comprises a two dimensional array of obstacles forming a network of gaps which separate said cells.

297. The method of item 296, further comprising contacting said first output with a device comprising specific binding moieties which selectively bind epithelial or neoplastic cells.

298. The method of item 297, wherein said specific binding moieties are antibodies or fragments thereof.

299. The method of item 298, wherein said antibodies specifically bind Ber-Ep4, EpCam, E-Cadherin, mucin-1, cytokeratin, or CD-34.

300. The method of item 238, wherein said EGFR gene comprises the wild type sequence of SEQ ID NO: 511.

301. The method of item 238, wherein said variant differs from SEQ ID NO: 511 at one or more nucleotide positions.

302. The method of item 238, wherein the polypeptide encoded by said EGFR gene comprises the wild type sequence of SEQ ID NO: 512.

303. The method of item 23 8, wherein the polypeptide encoded by said variant differs from SEQ ID NO: 512 at one or more amino acid positions.

304. The method of item 238, wherein said variant is selected from Table 3.

## Claims

1. A device for detecting circulating cancer cells in a cellular sample, comprising:

   a channel comprising a first fluid inlet and a first fluid outlet;
   a first array of obstacles defining a first network of gaps and arranged within the channel to define flow paths between the inlet and a downstream end of the first array to the outlet; and
   a second array of obstacles defining a second network of gaps and arranged within the channel downstream of the first array of obstacles to define flow paths from the downstream end of the first array to the outlet;
   wherein the gaps of the second network of gaps are smaller than the gaps of the first network of gaps;
   wherein one or more of the obstacles in the first array, second array, or both the first array and the second array comprise or are bound to one or more binding moieties; and
   wherein either the first array or the second array of obstacles is configured to selectively capture at least one cancer cell in a cellular sample based on both (i) affinity of the cancer cell to the one or more binding moieties and (ii) one or more of cell size, shape, or deformability of the cancer cell.

2. The device of claim 1, wherein the obstacle of the first, second, or first and second arrays are staggered.

3. The device of claim 1 or 2, wherein the obstacles are arranged in rows and wherein obstacles in each successive row in the first array, second array, or first and second array are offset from obstacles in each previous row by less than half of the period of the previous row.

4. The device of any one of claims 1 to 3, wherein the obstacle of the first, second, or first and second arrays are uniformly spaced.

5. The device of any one of claims 1 to 4, wherein the gaps between the obstacles of the first network, the second network, or both the first and the second network, are between 15 and 60 micrometers.

6. The device of any one of claims 1 to 4, wherein the gaps between the obstacles of the first network, the second network, or both the first and the second network, are greater than 20 micrometers.

7. The device of any one of claims 1 to 6, further comprising a module for performing gene expression analysis on said cancer cell.

8. The device of any one of claims 1 to 7, wherein the one or more binding moieties comprise a polypeptide.

9. The device of claim 8, wherein the polypeptide comprises an antibody or fragment thereof.

10. The device of claim 9, wherein the antibody or fragment thereof is a monoclonal antibody or fragment thereof.

11. The device of claim 9 or 10, wherein the antibody or fragment thereof is selected from the group consisting of anti-EpCAM, anti-CK (cytokeratin), anti-EGFR, c-ERBB-2, and anti-HER-2.

12. The device of any one of claims 1 to 11, further comprising a cell counting module coupled to the device, wherein the cell counting module is capable of determining the number of cancer cells captured in the device.

13. A method of detecting cancer cells in a cellular sample, the method comprising:

(a) selectively capturing one or more cancer cells from the cellular sample by flowing the cellular sample through a device of any one of claims 1 to 12; and
(b) detecting the presence or absence of the one or more captured cancer cells.

**14.** The method of claim 13, further comprising performing genetic analysis on the one or more captured cancer cells.

**15.** The method of claim 13, further comprising counting the one or more captured cancer cells.

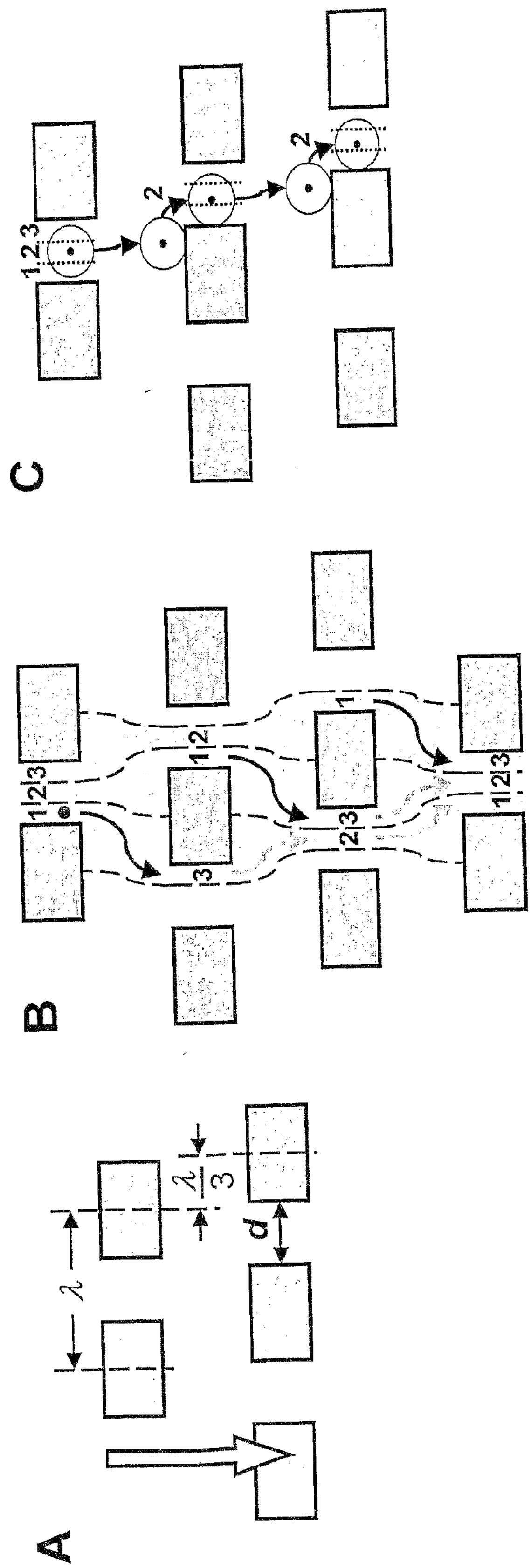


Figs. 1A-1C

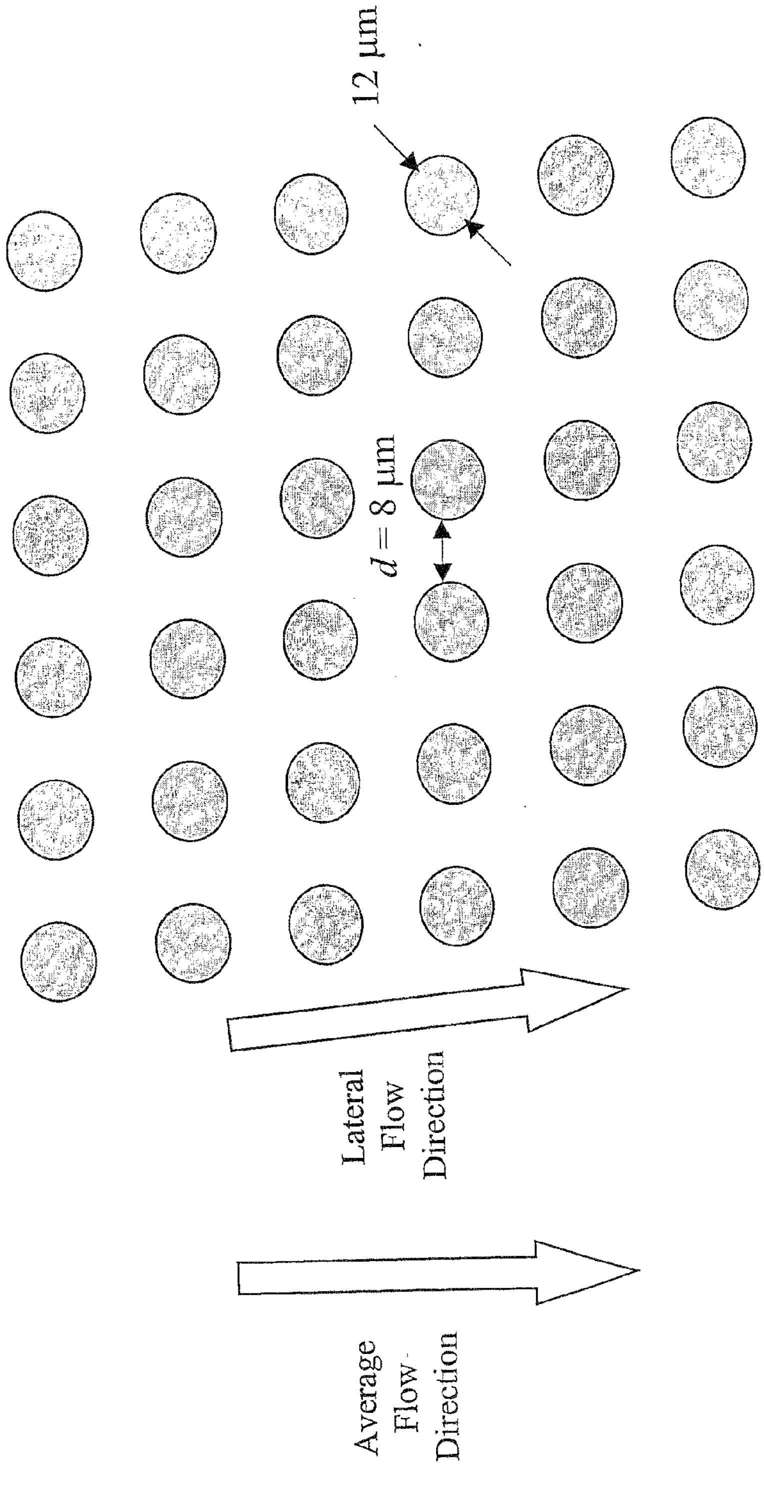


Fig. 1D

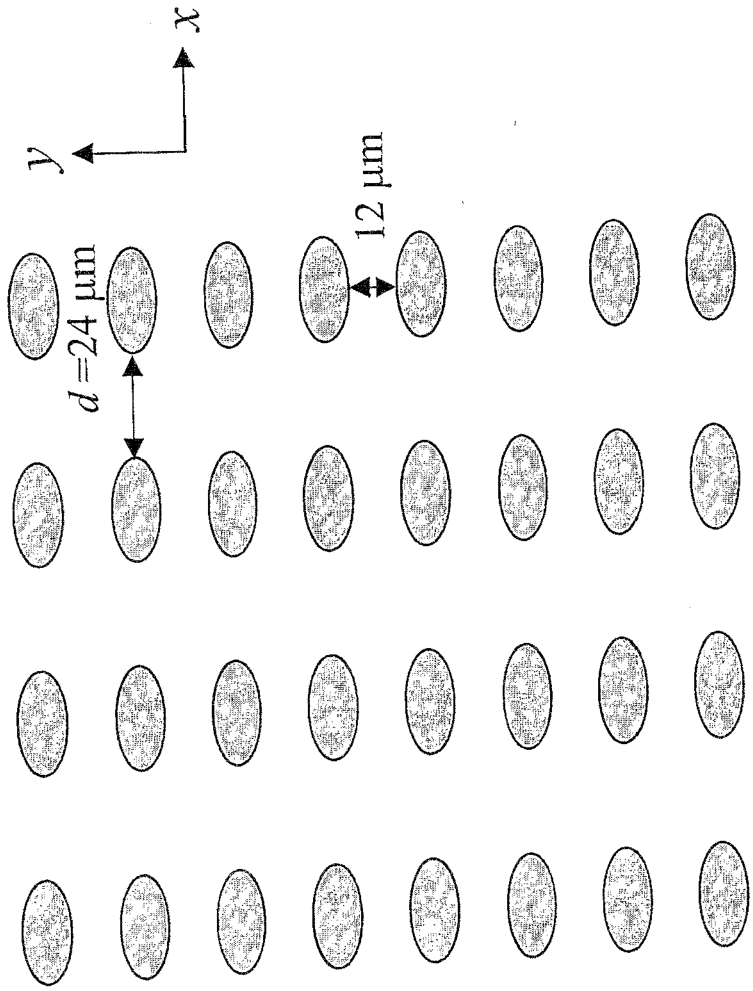


Fig. 1E

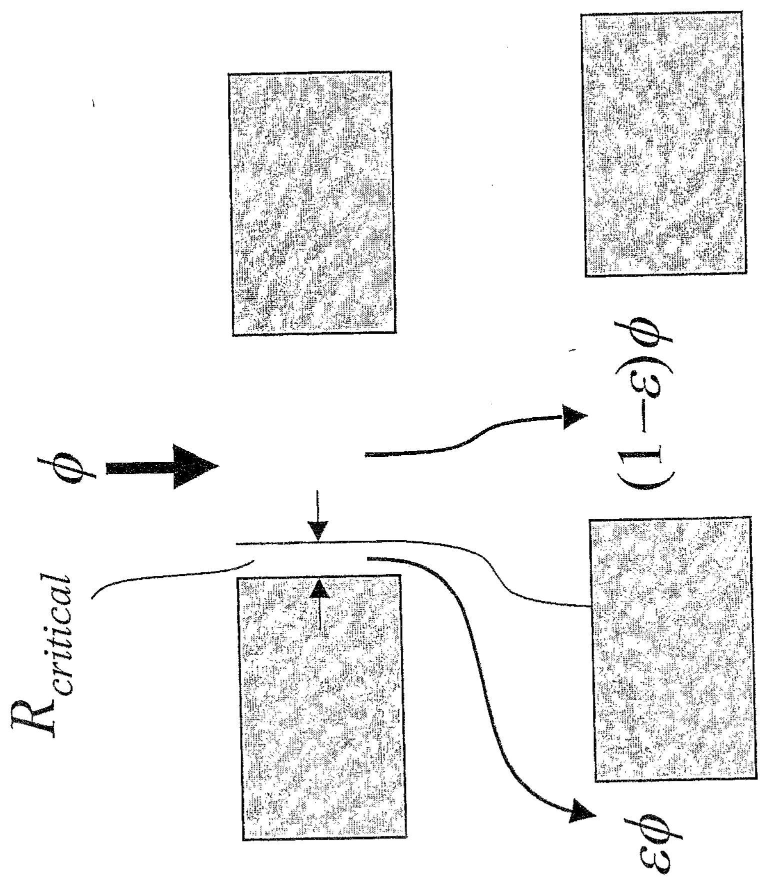


Fig. 2

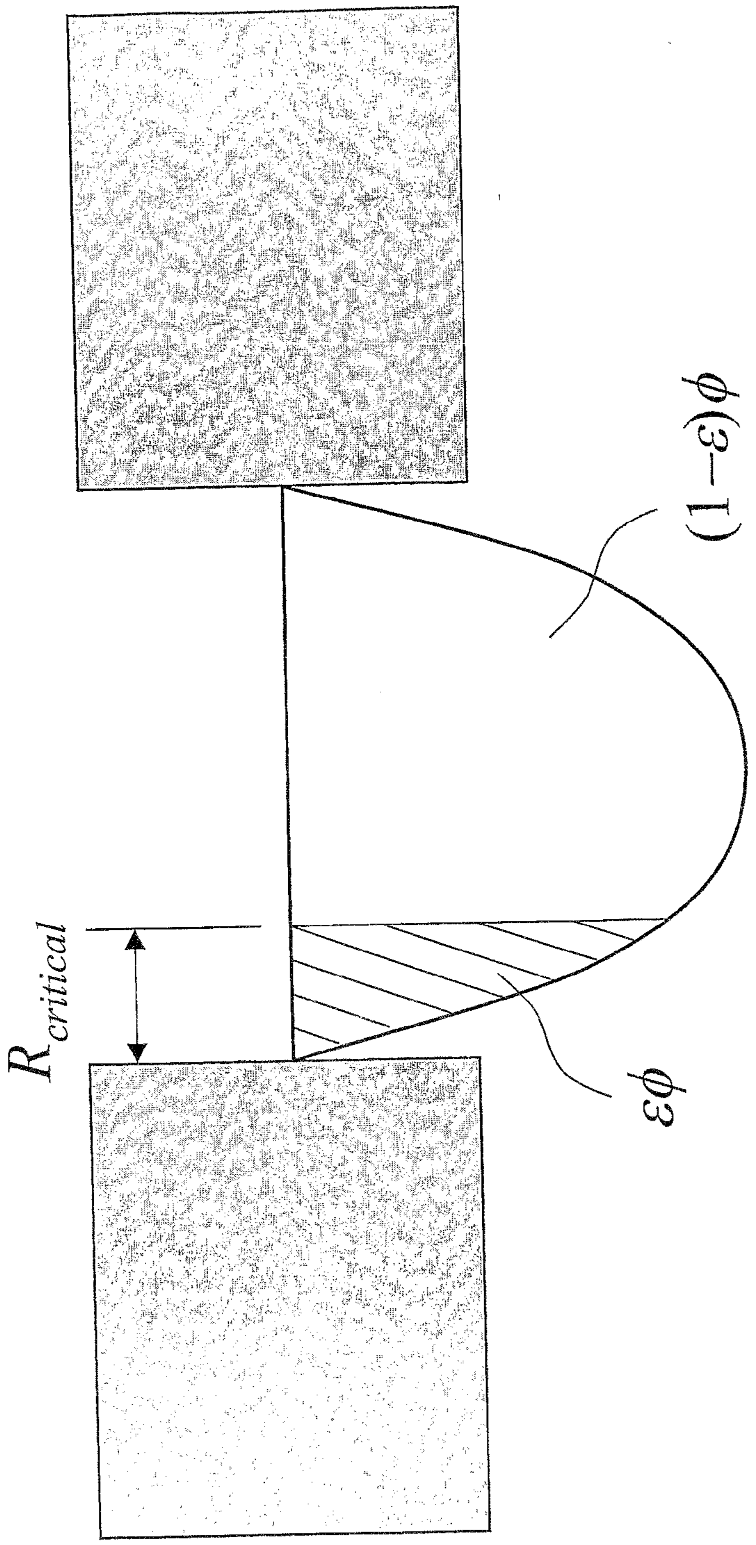


Fig. 3

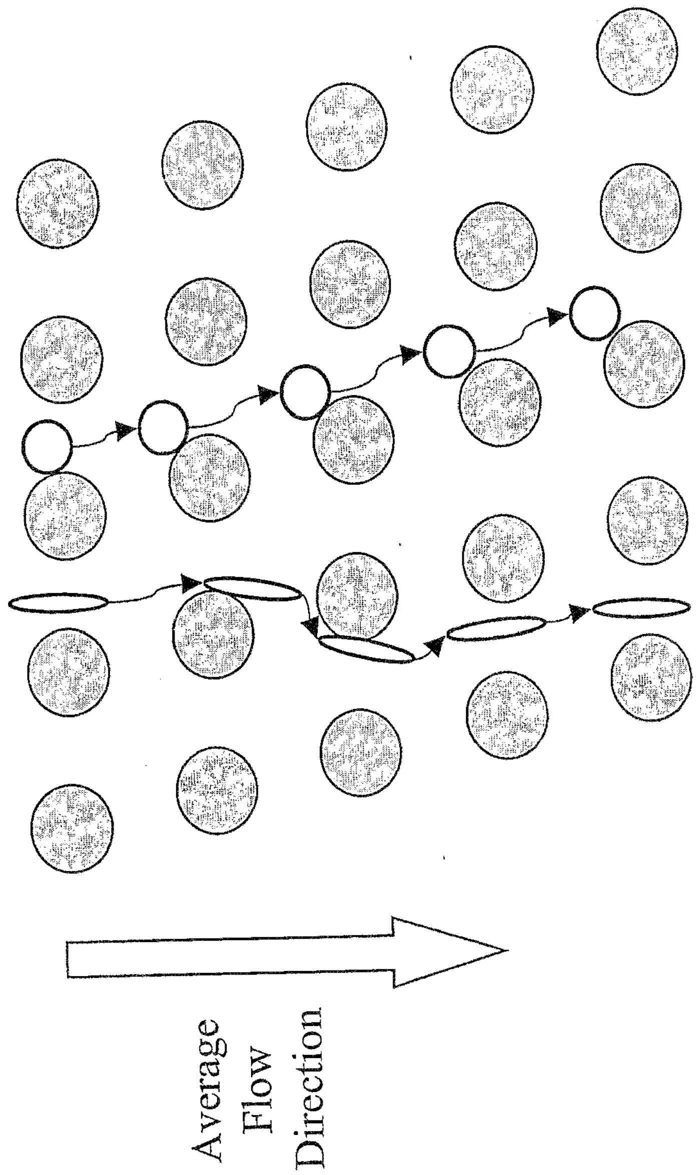


Fig. 4

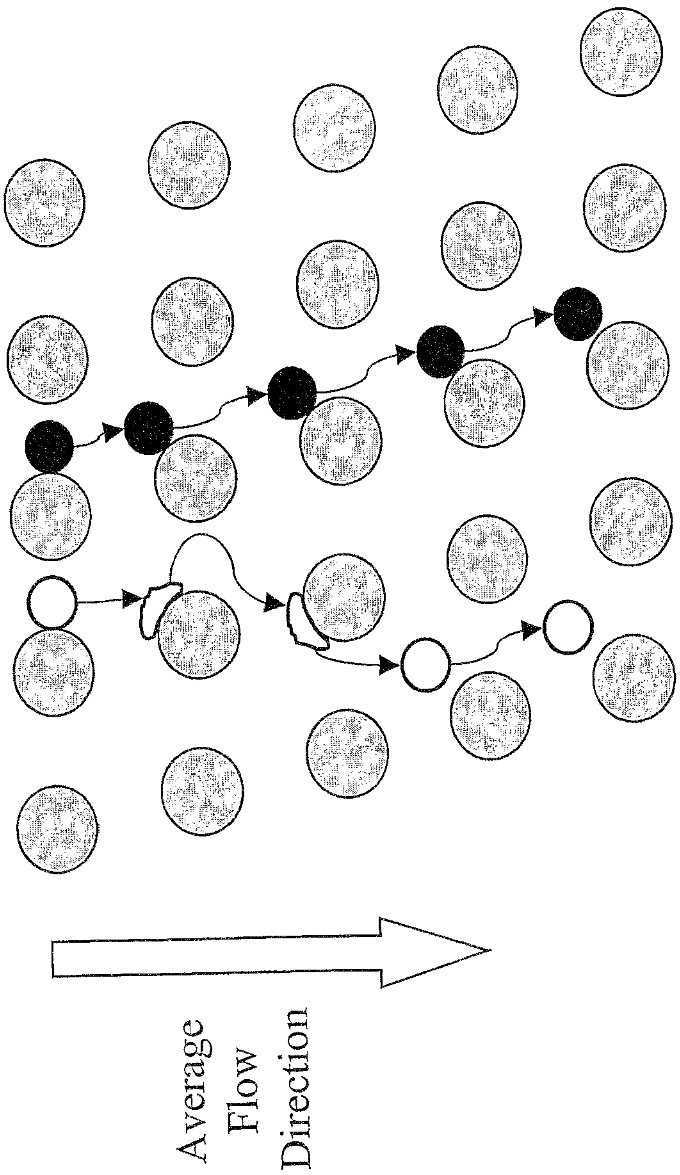


Fig. 5

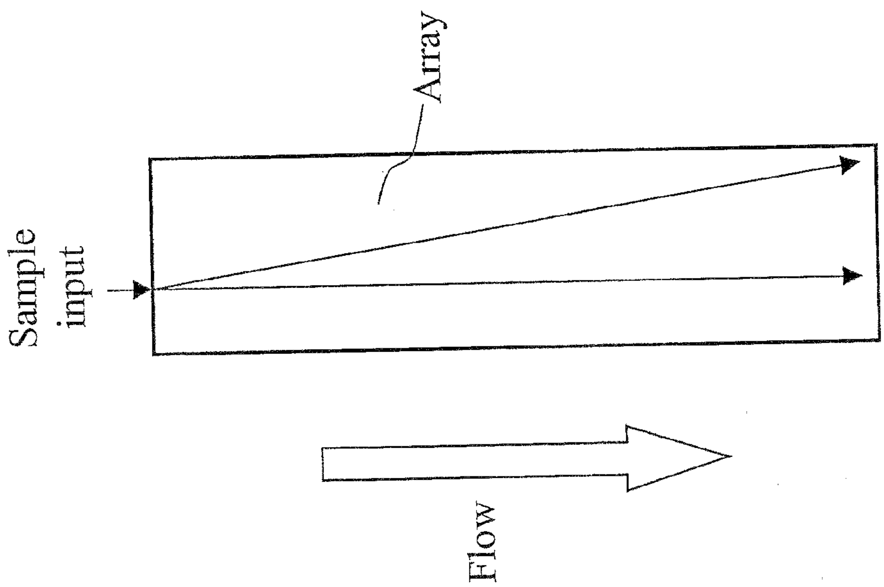


Fig. 6

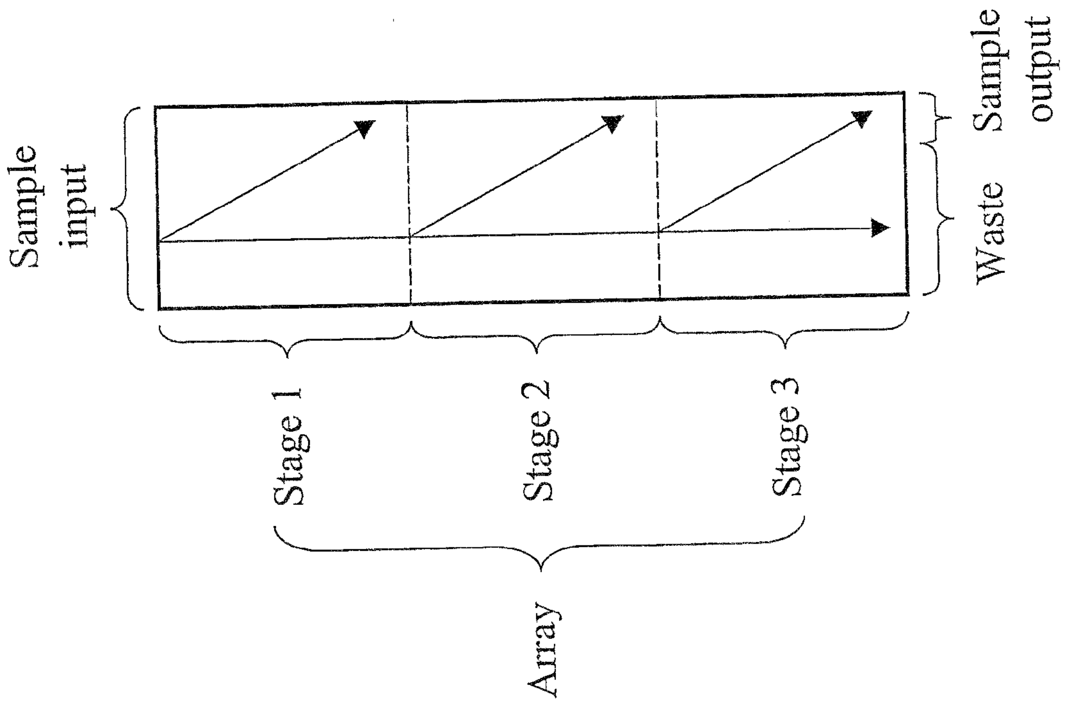
Sample input
Sample output
Waste
Stage 1
Stage 2
Stage 3
Array

Fig. 7

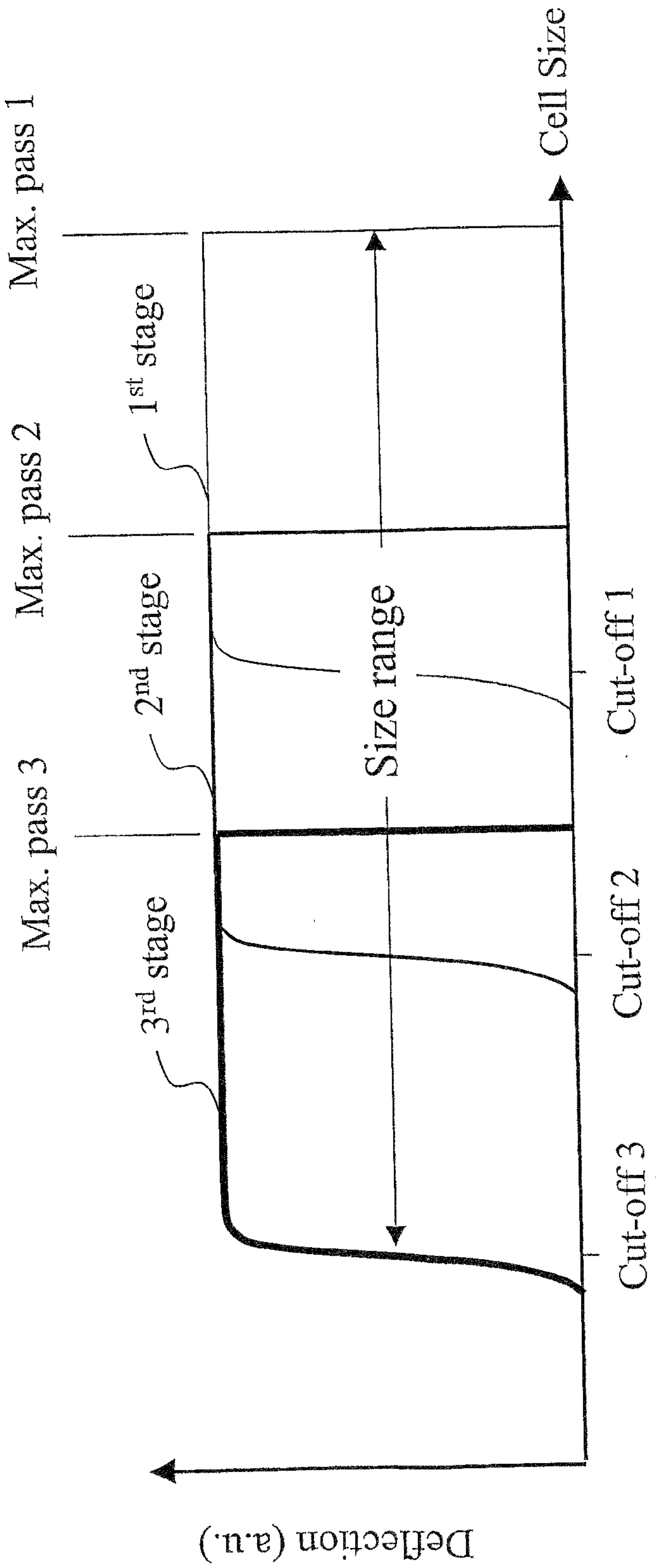
Max. pass 1
Max. pass 2
Max. pass 3
1st stage
2nd stage
3rd stage
Size range
Cut-off 1
Cut-off 2
Cut-off 3
Cell Size
Deflection (a.u.)

Fig. 8

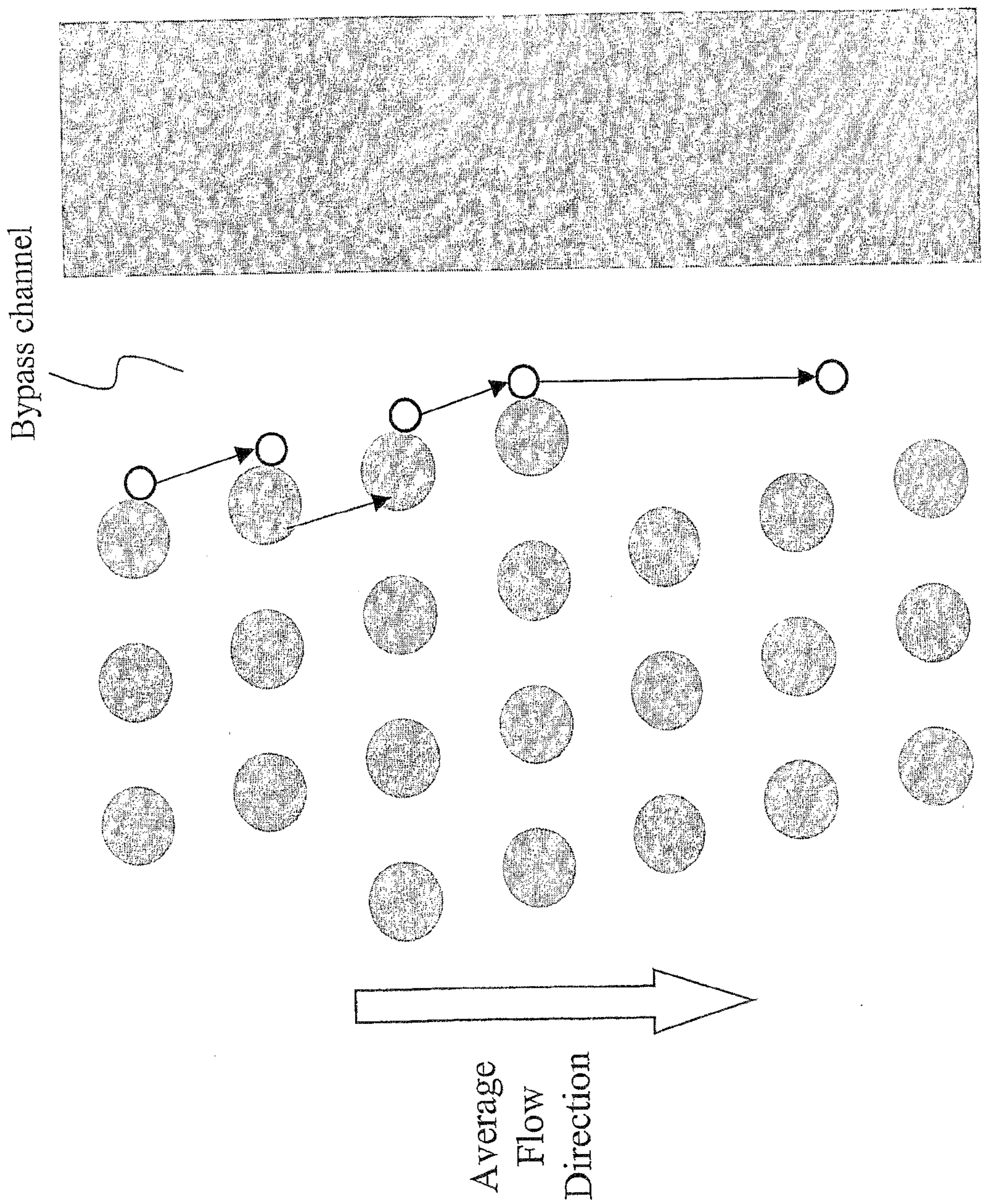


Fig. 9

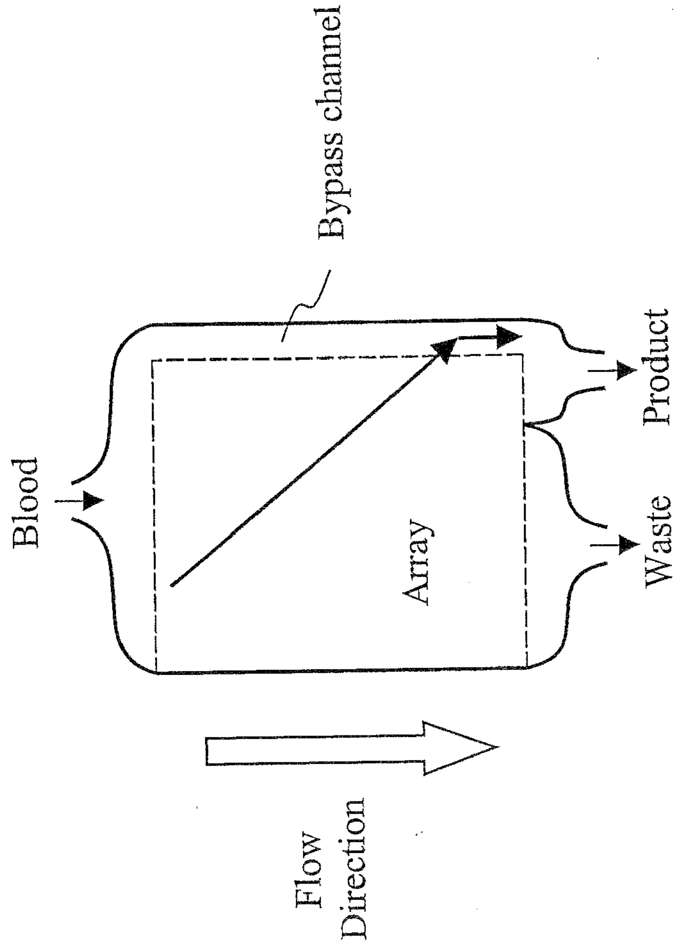


Fig. 10

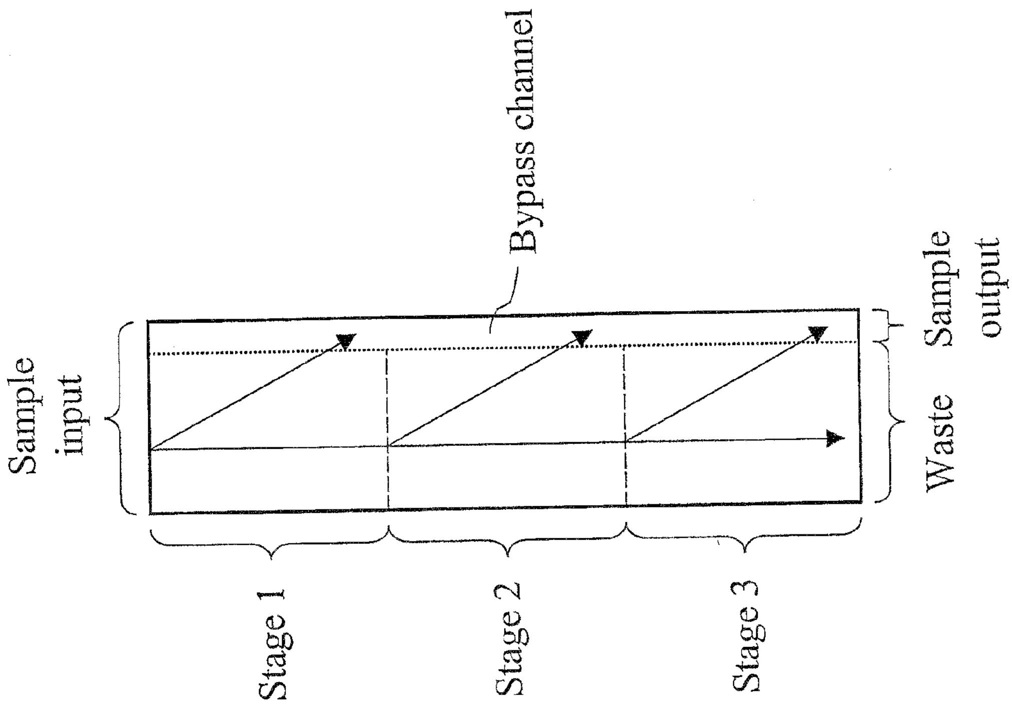
Bypass channel
Sample input
Sample output
Waste
Stage 1
Stage 2
Stage 3

Fig. 11

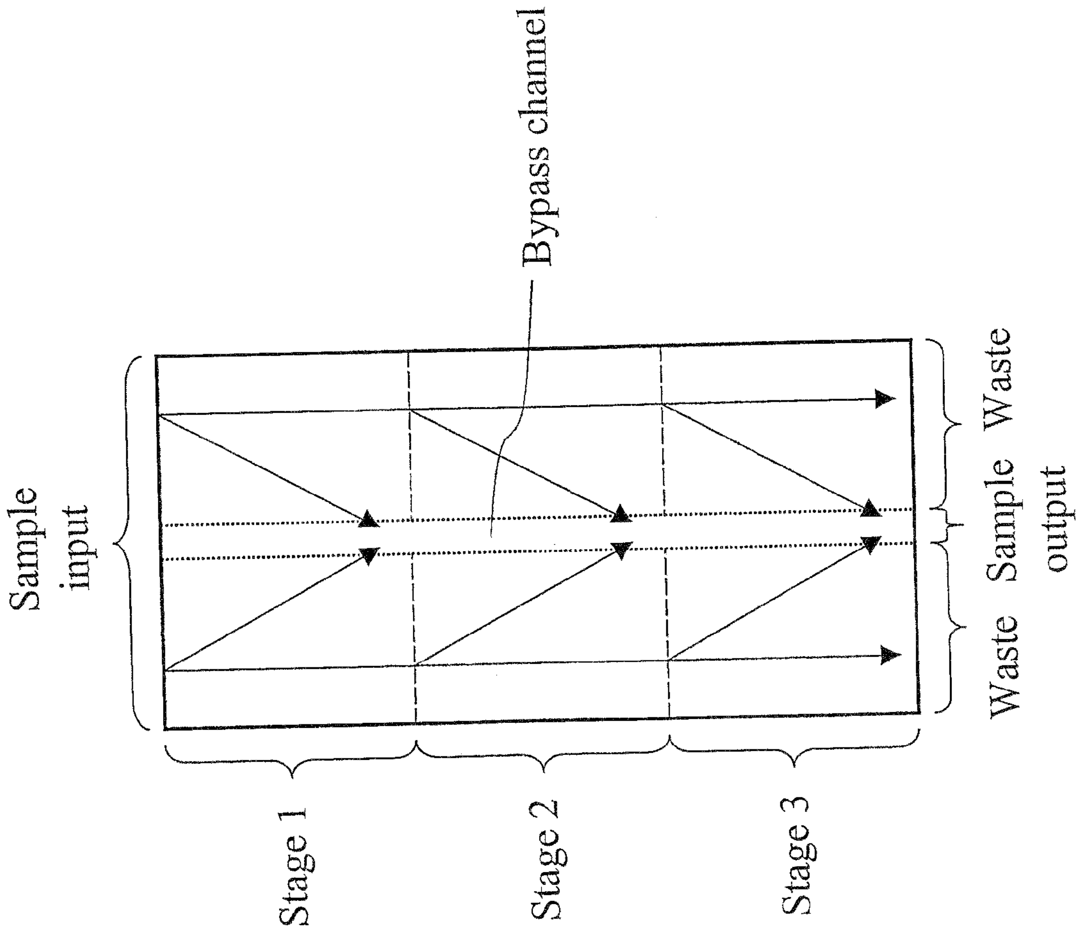
Sample input
Bypass channel
Waste
Sample output
Waste
Stage 1
Stage 2
Stage 3

Fig. 12

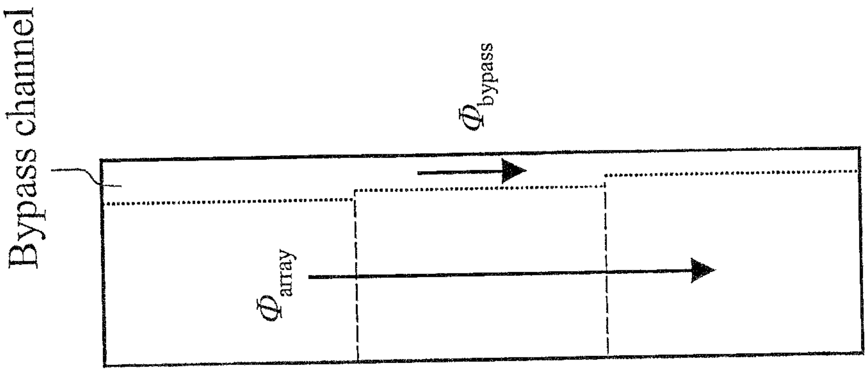


Fig. 13

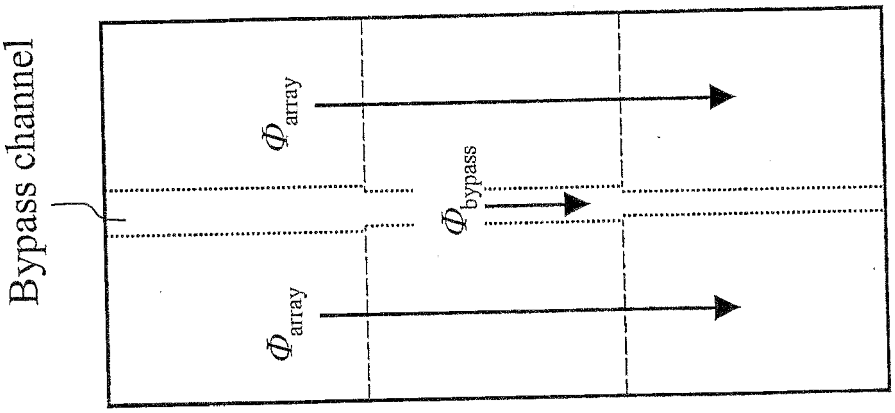


Fig. 14

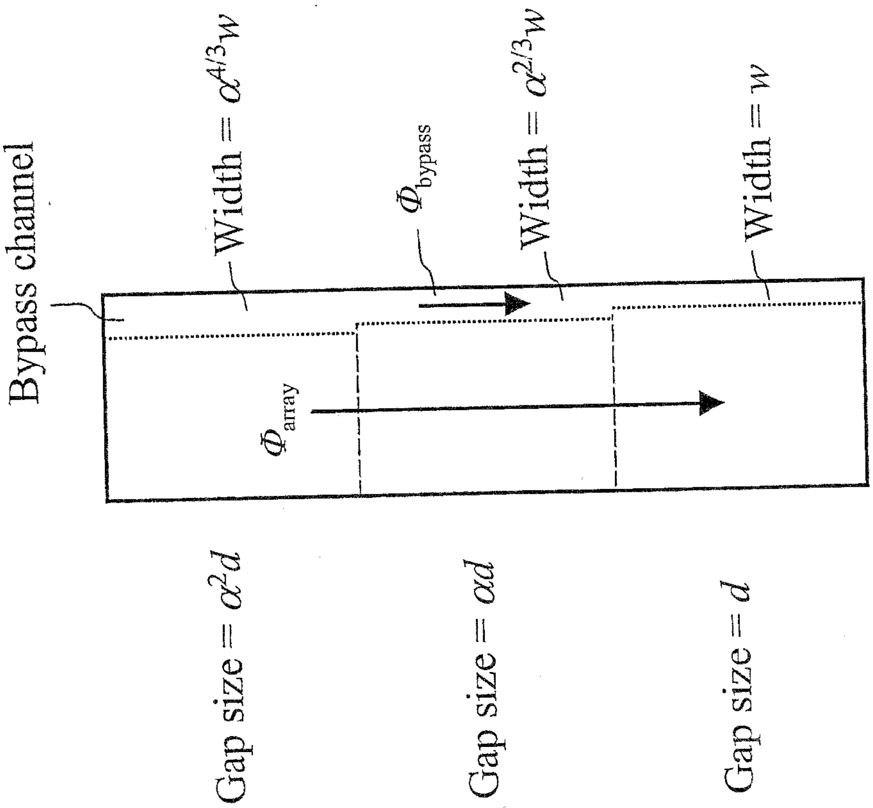
Bypass channel
Width = $\alpha^{4/3}w$
$\Phi_{bypass}$
Width = $\alpha^{2/3}w$
Width = $w$
$\Phi_{array}$
Gap size = $\alpha^2 d$
Gap size = $\alpha d$
Gap size = $d$

Fig. 15

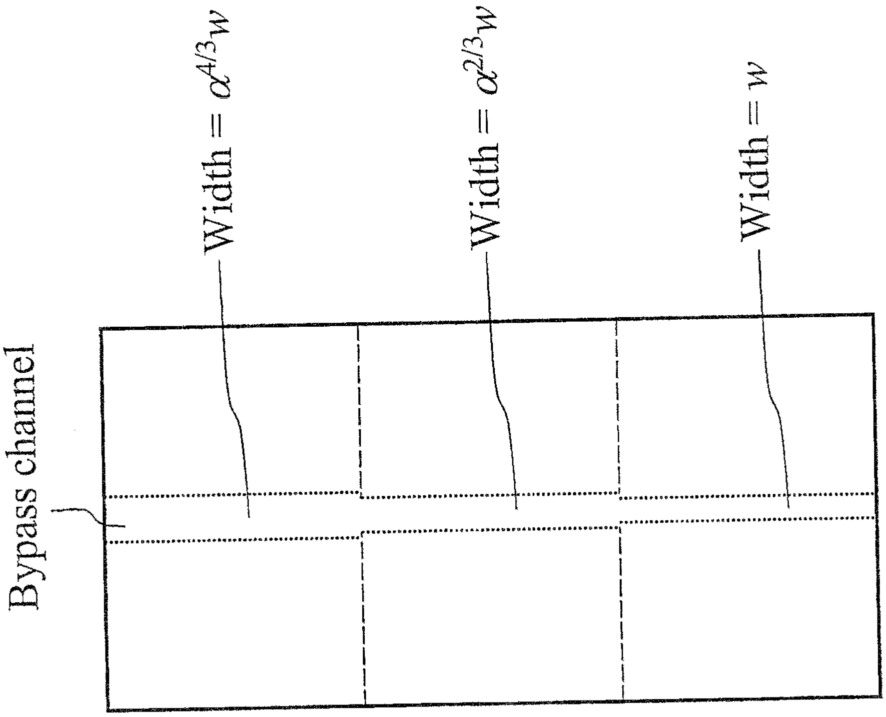


Fig. 16

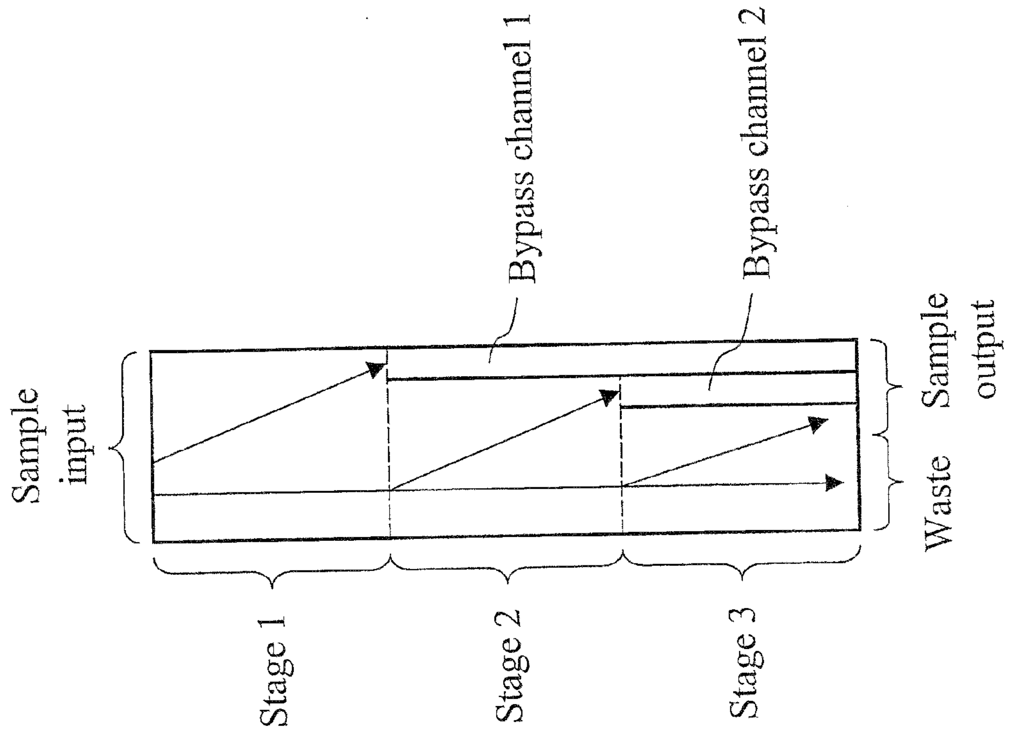
Sample input
Bypass channel 1
Bypass channel 2
Sample output
Waste
Stage 1
Stage 2
Stage 3

Fig. 17

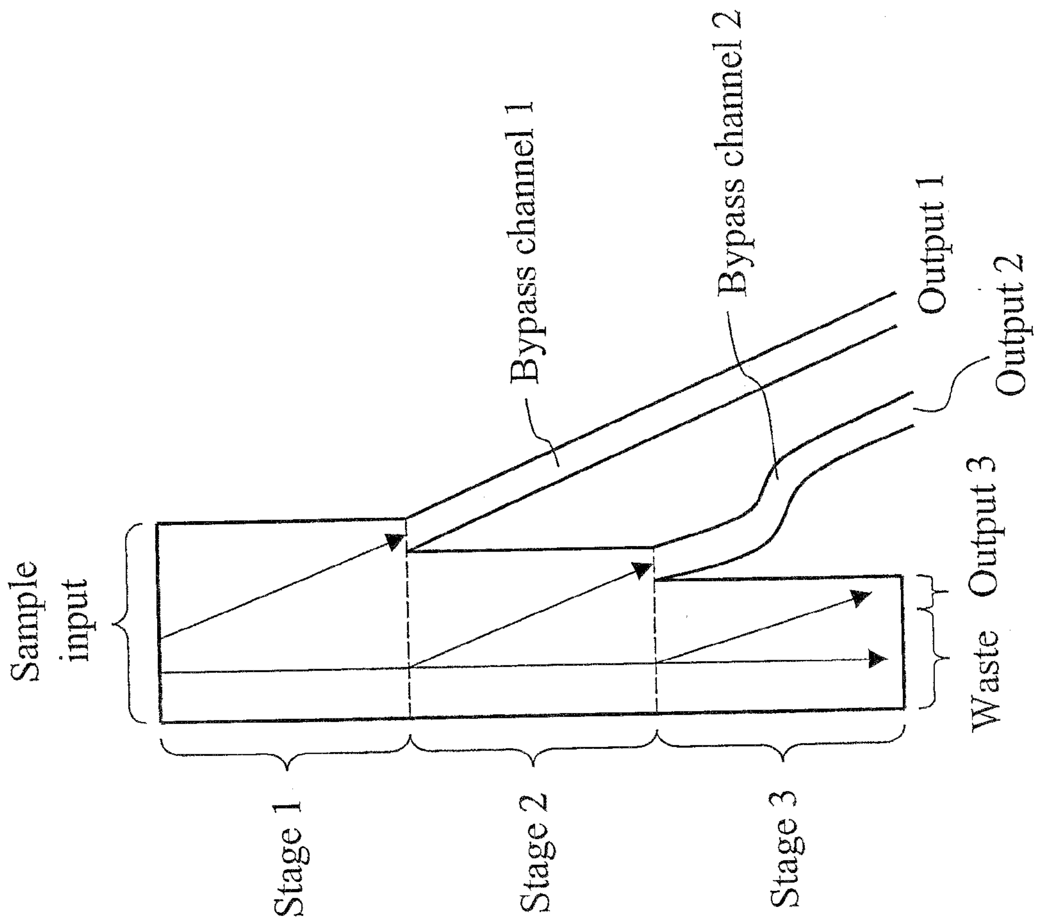
Sample input
Bypass channel 1
Bypass channel 2
Output 1
Output 2
Output 3
Waste
Stage 1
Stage 2
Stage 3

Fig. 18

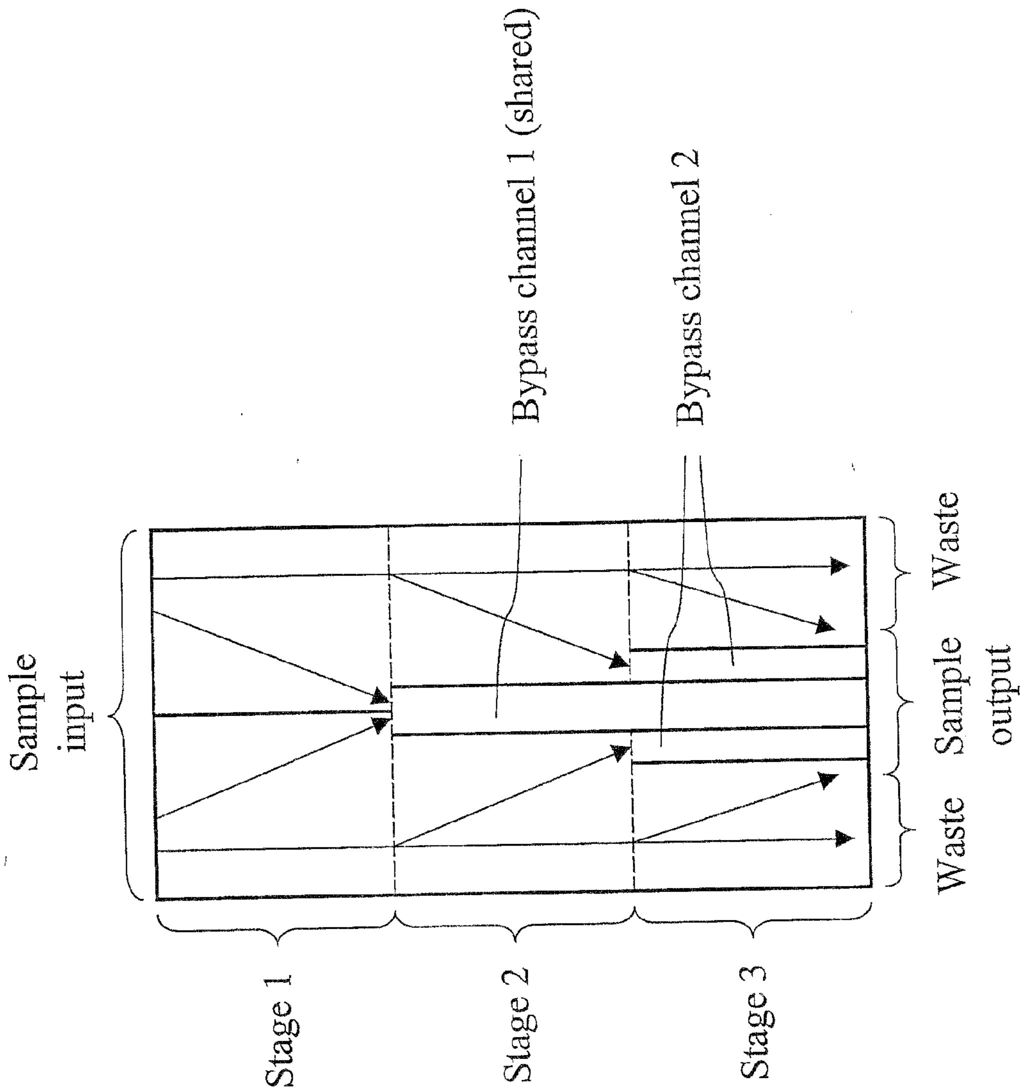
Sample input
Bypass channel 1 (shared)
Bypass channel 2
Waste
Sample output
Waste
Stage 1
Stage 2
Stage 3

Fig. 19

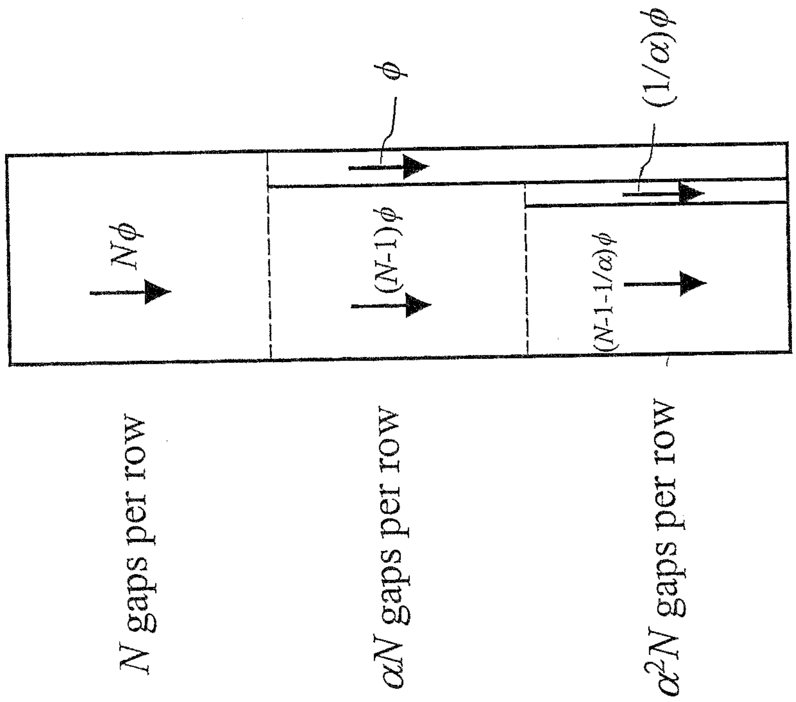
$N\phi$
$\phi$
$(N\text{-}1)\phi$
$(1/\alpha)\phi$
$(N\text{-}1\text{-}1/\alpha)\phi$
$N$ gaps per row
$\alpha N$ gaps per row
$\alpha^2 N$ gaps per row

Fig. 20

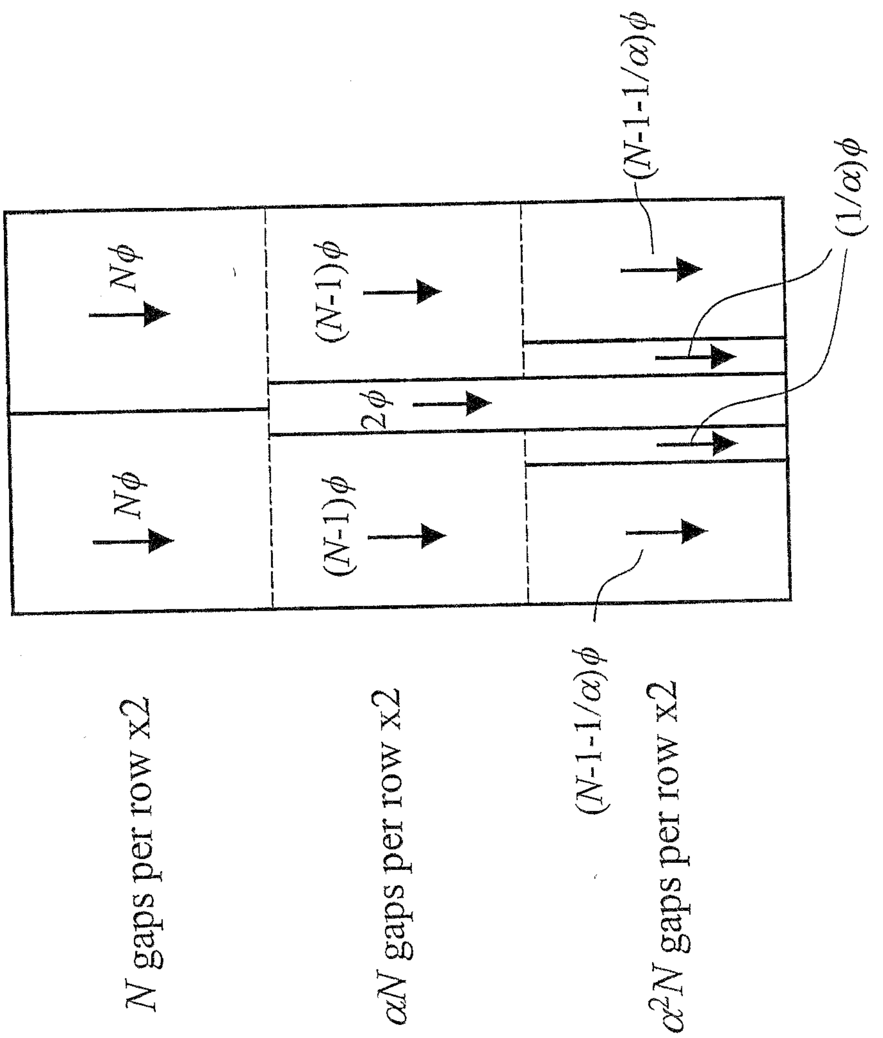

$N\phi$
$N\phi$
$(N\text{-}1)\phi$
$(N\text{-}1)\phi$
$2\phi$
$(N\text{-}1\text{-}1/\alpha)\phi$
$(1/\alpha)\phi$
$(N\text{-}1\text{-}1/\alpha)\phi$
$N$ gaps per row x2
$\alpha N$ gaps per row x2
$\alpha^2 N$ gaps per row x2


Fig. 21

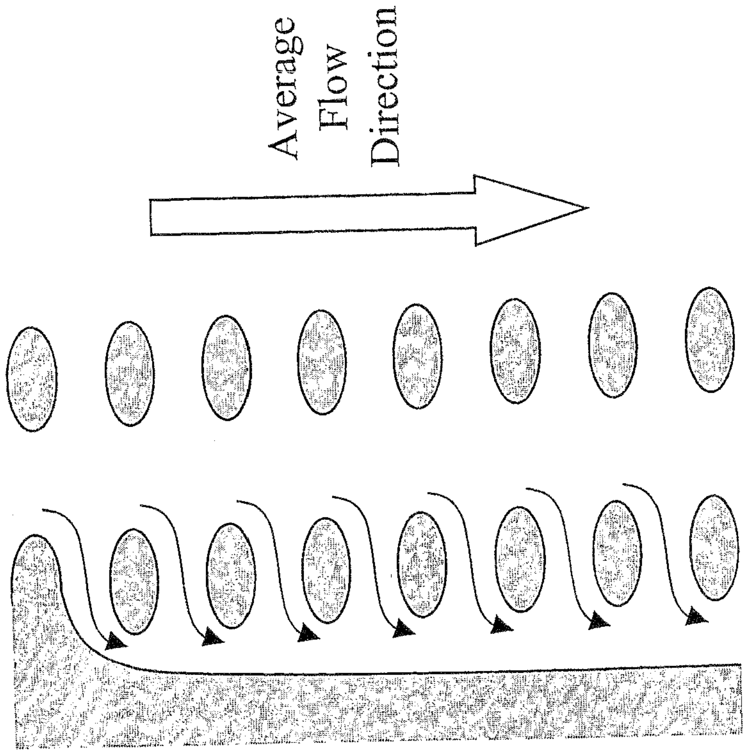


Fig. 22

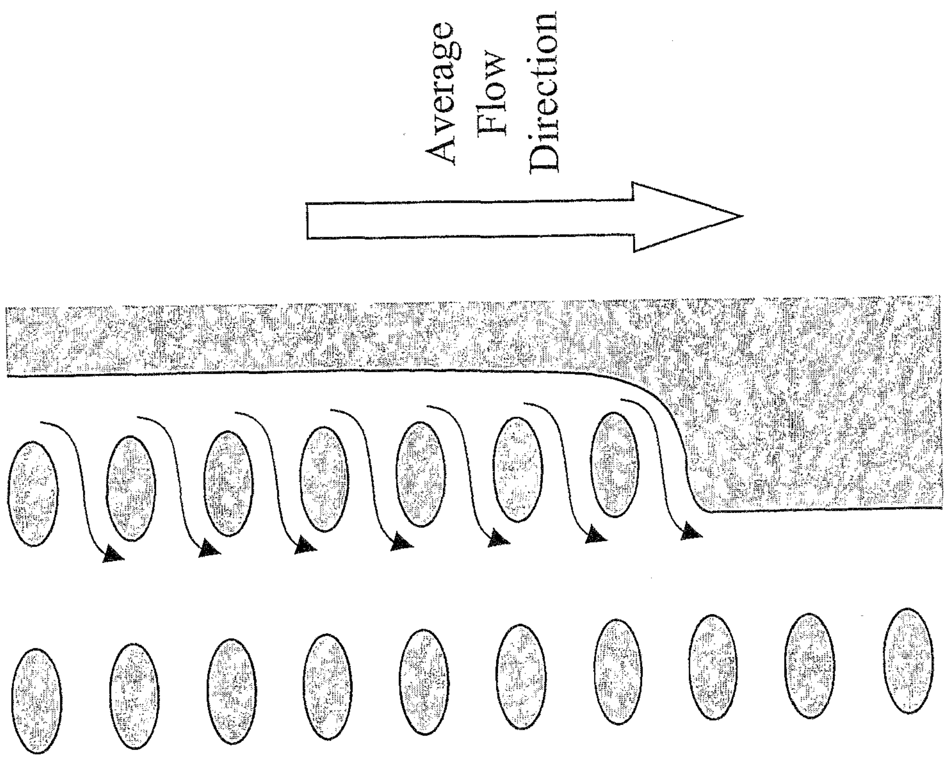


Fig. 23

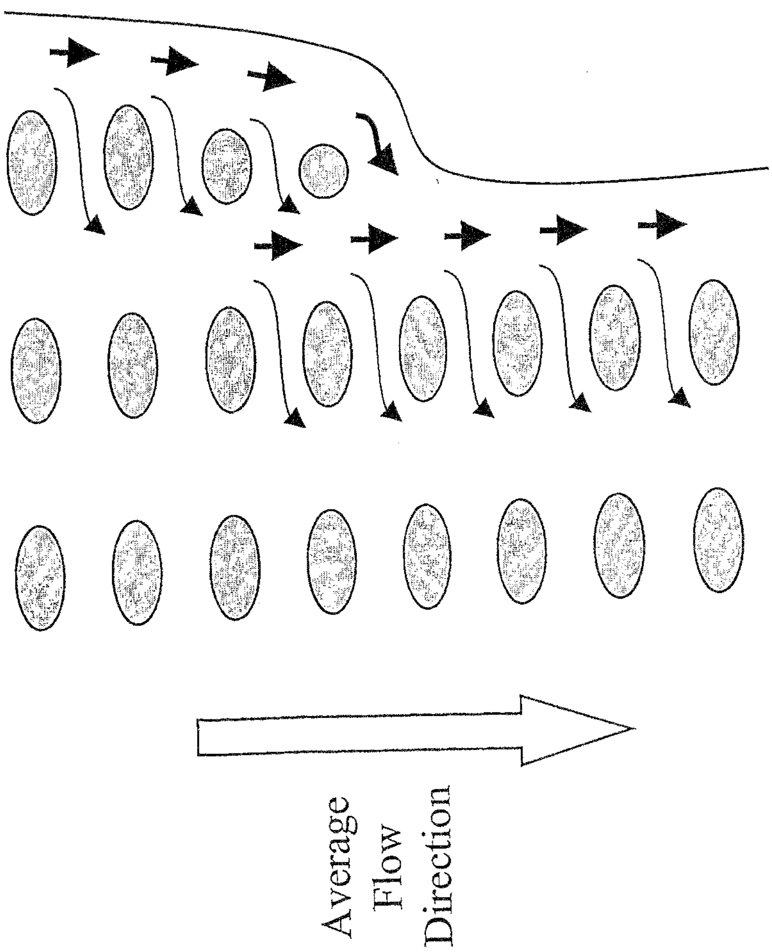


Fig. 24

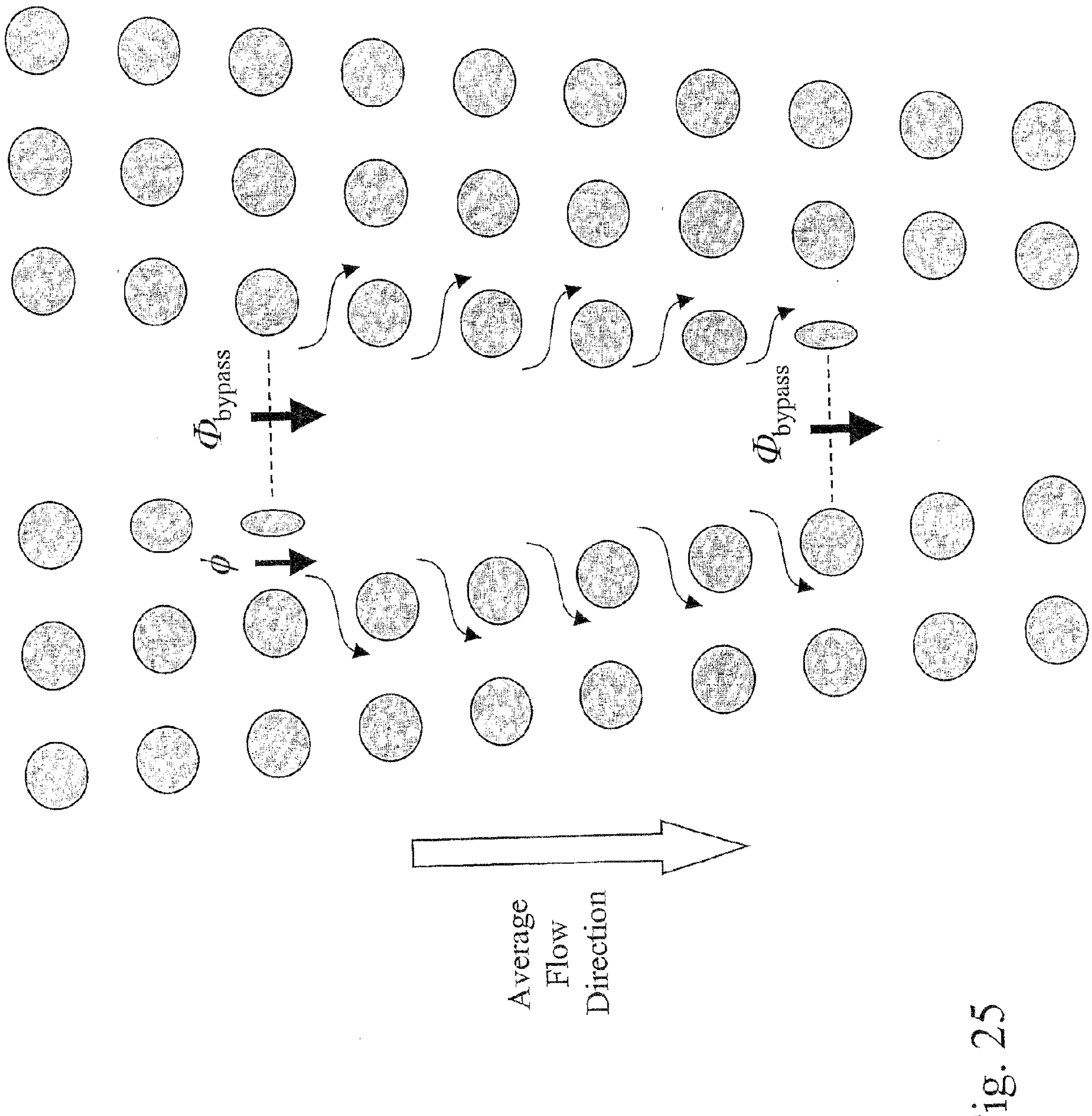


Fig. 25

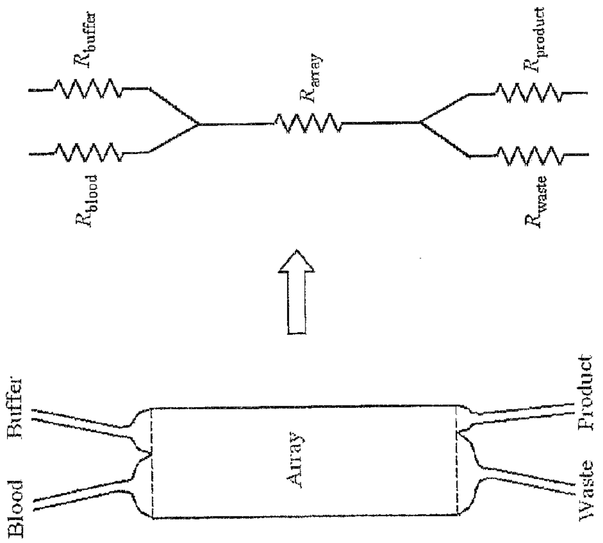

$R_{buffer}$
$R_{blood}$
$R_{array}$
$R_{product}$
$R_{waste}$
Blood
Buffer
Array
Waste
Product


Fig. 26

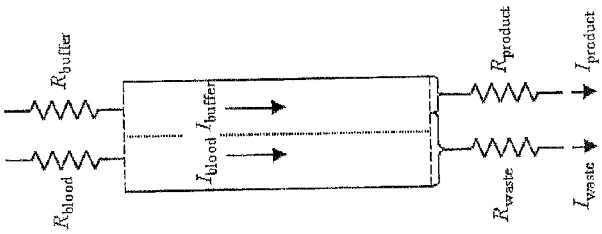
$R_{buffer}$
$R_{blood}$
$I_{blood}$
$I_{buffer}$
$R_{product}$
$I_{product}$
$R_{waste}$
$I_{waste}$

Fig. 27

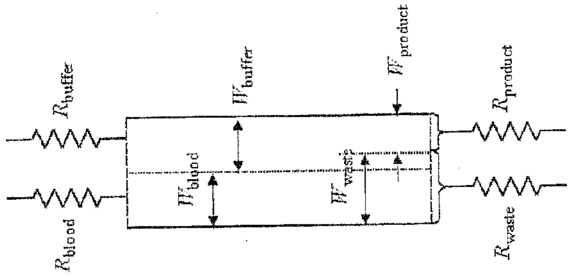
$R_{buffer}$
$R_{blood}$
$W_{buffer}$
$W_{blood}$
$W_{waste}$
$W_{product}$
$R_{product}$
$R_{waste}$

Fig. 28

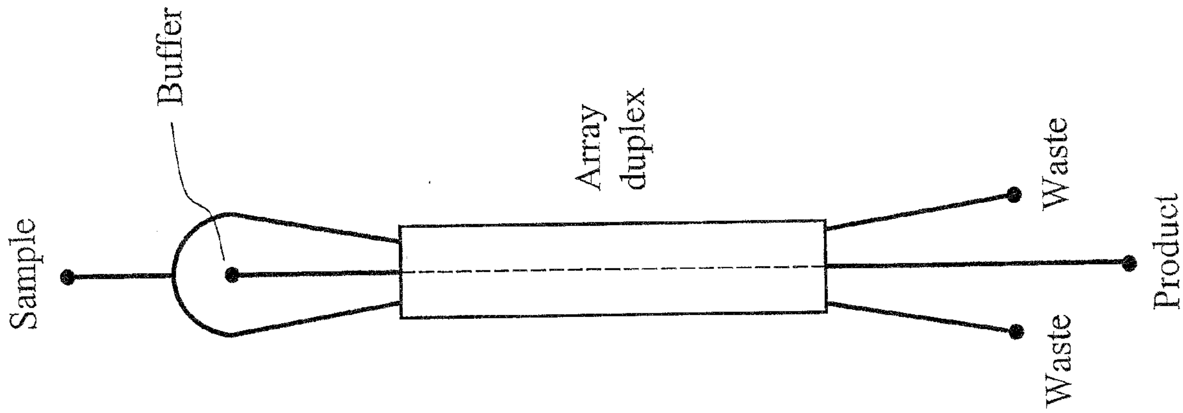


Fig. 29

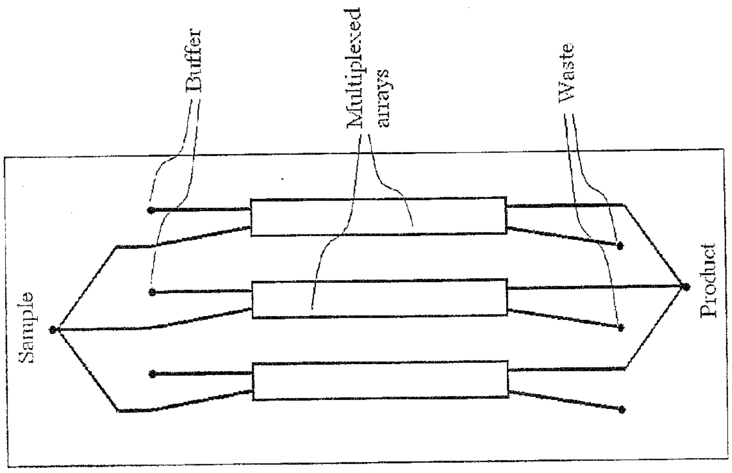


Fig. 30A

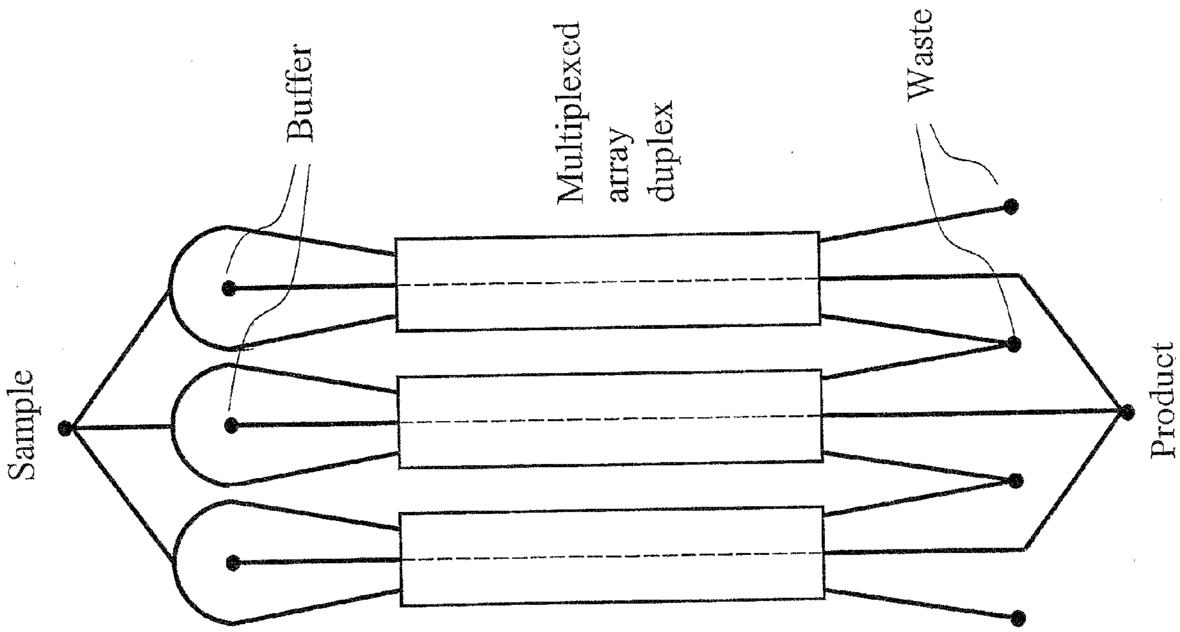


Fig. 30B

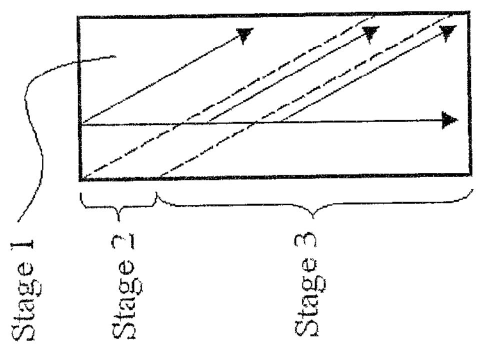


Fig. 31

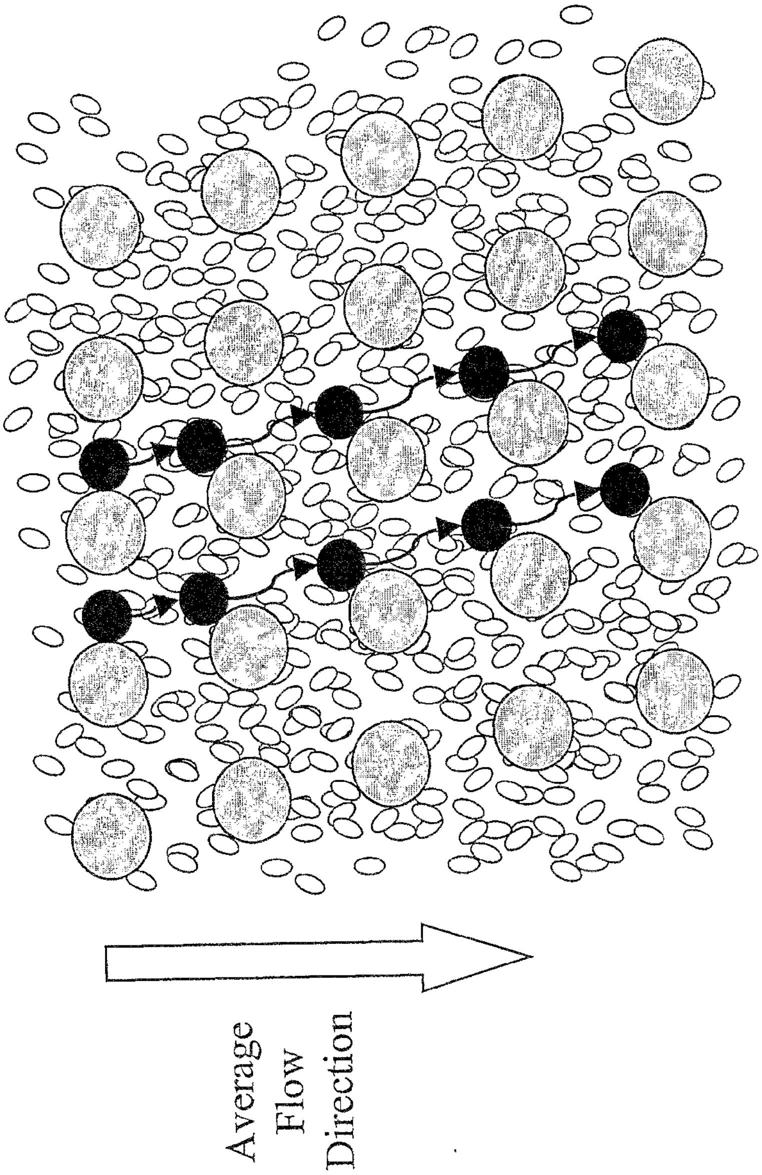


Fig. 32

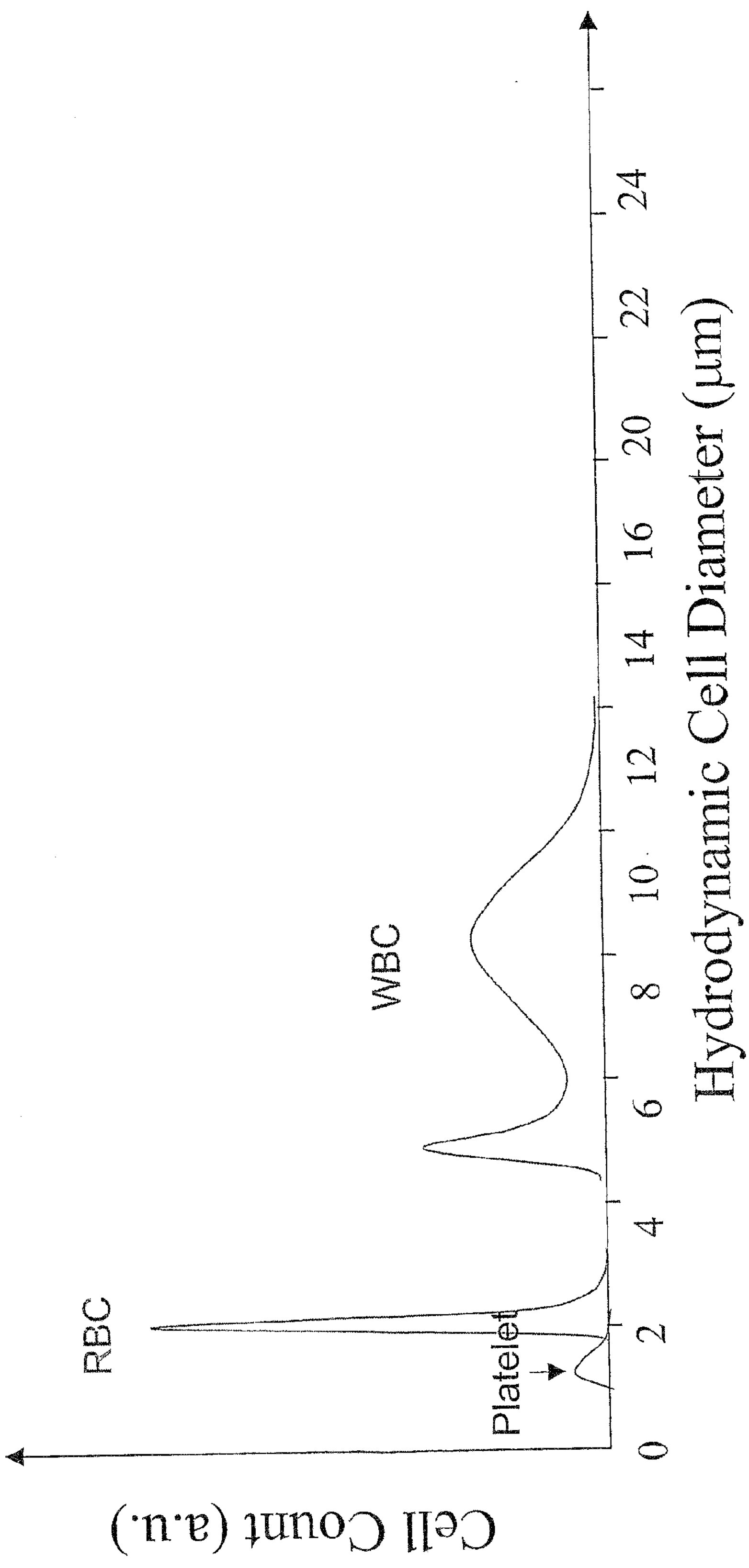
Cell Count (a.u.)
Hydrodynamic Cell Diameter (μm)
RBC
WBC
Platelet
0
2
4
6
8
10
12
14
16
20
22
24

Fig. 33A

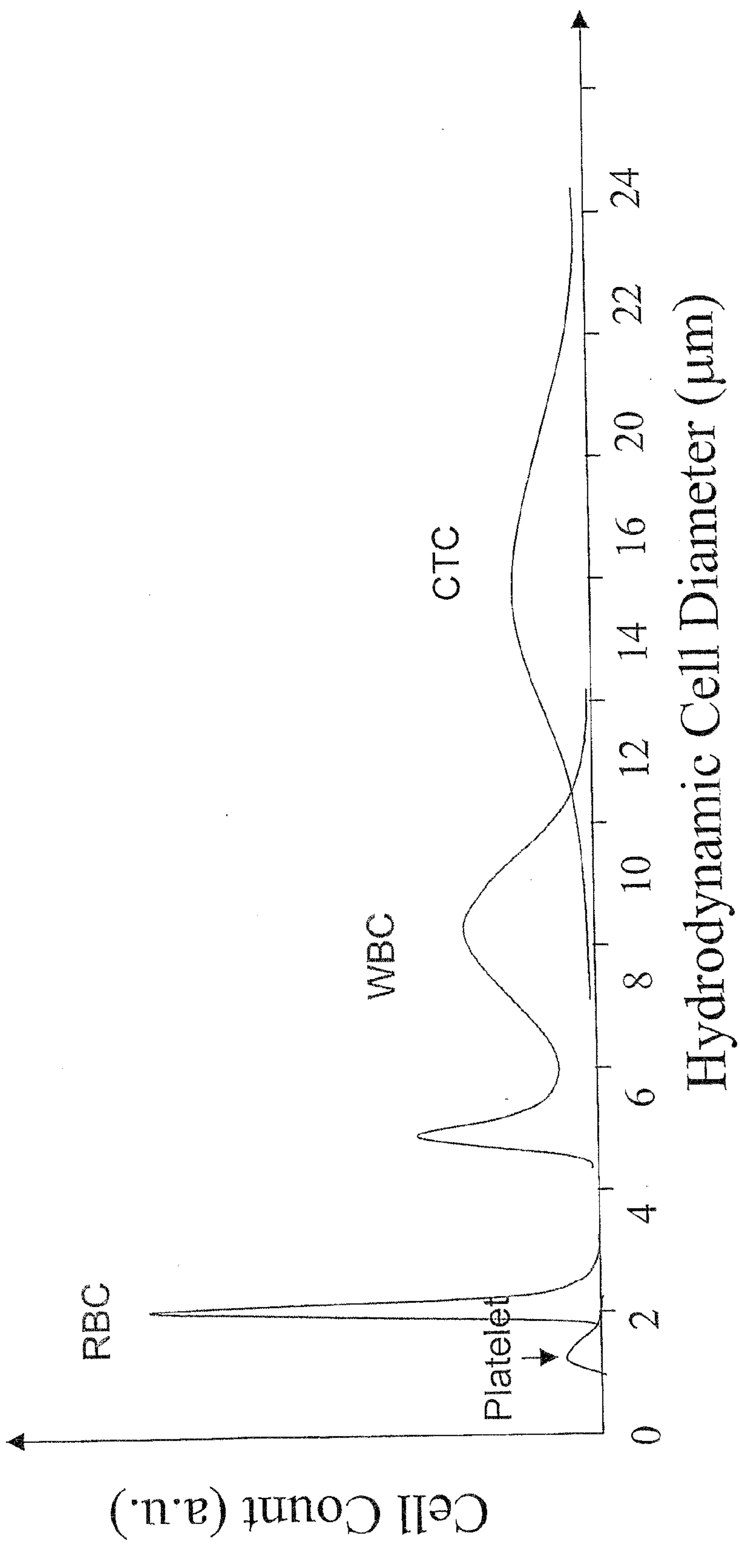

Cell Count (a.u.)
RBC
Platelet
WBC
CTC
0
2
4
6
8
10
12
14
16
20
22
24
Hydrodynamic Cell Diameter (μm)


Fig. 33B

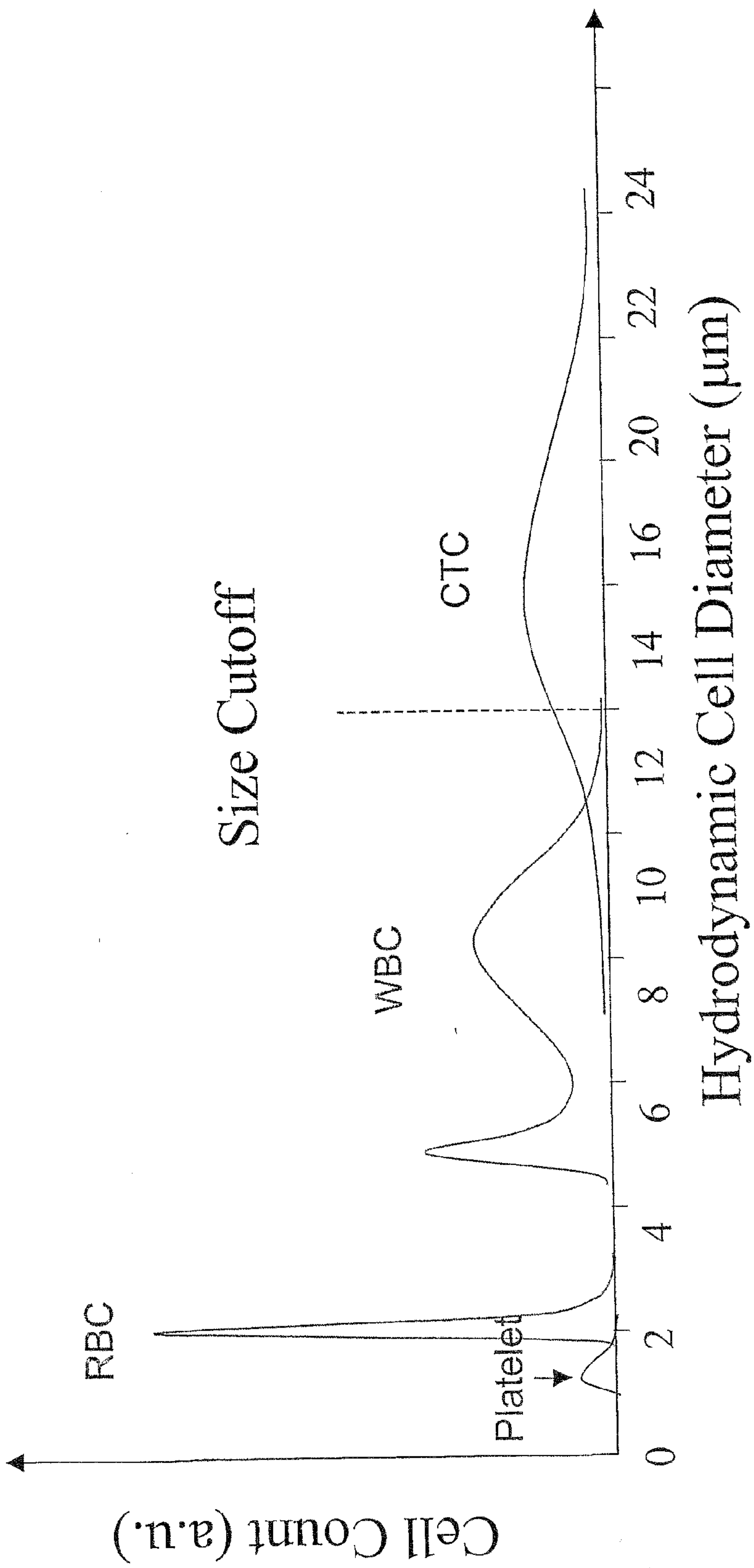
Size Cutoff
RBC
Platelet
WBC
CTC
Cell Count (a.u.)
Hydrodynamic Cell Diameter (μm)
0
2
4
6
8
10
12
14
16
20
22
24

Fig. 33C

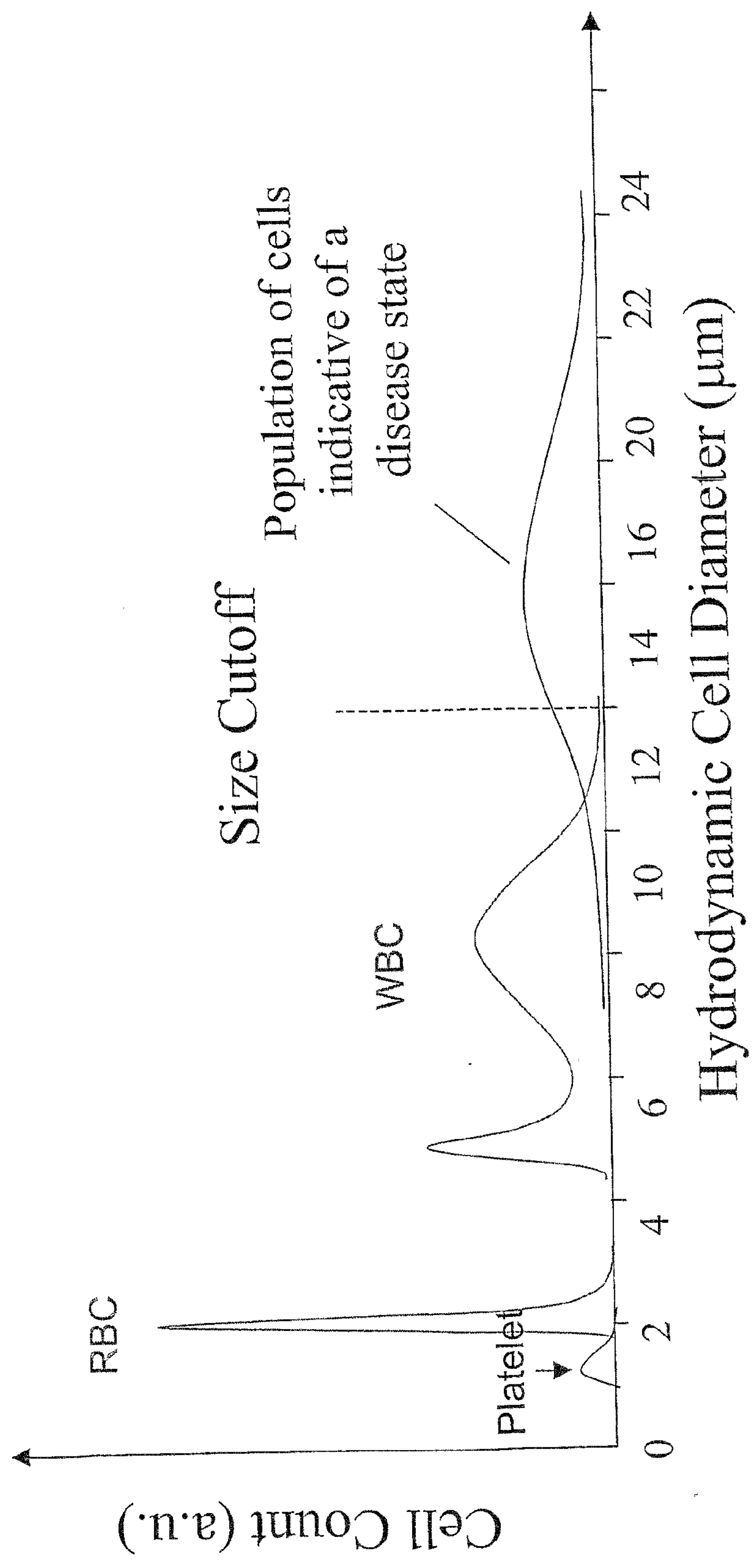
Size Cutoff
Population of cells indicative of a disease state
RBC
Platelet
WBC
0
2
4
6
8
10
12
14
16
20
22
24
Hydrodynamic Cell Diameter (µm)
Cell Count (a.u.)

Fig. 33D

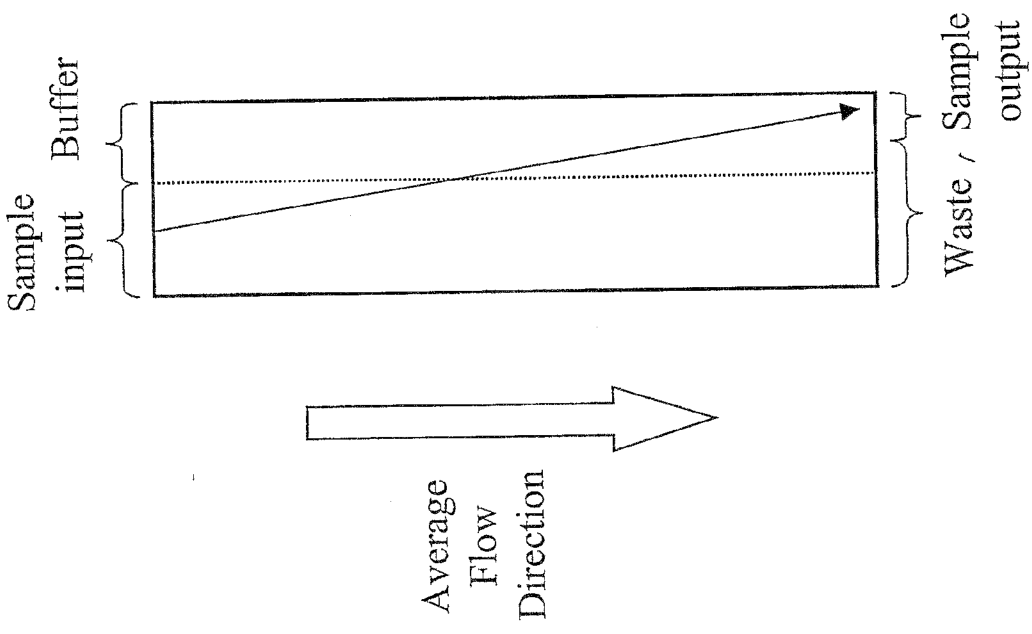
Sample input
Buffer
Waste
Sample output
Average Flow Direction

Fig. 34A

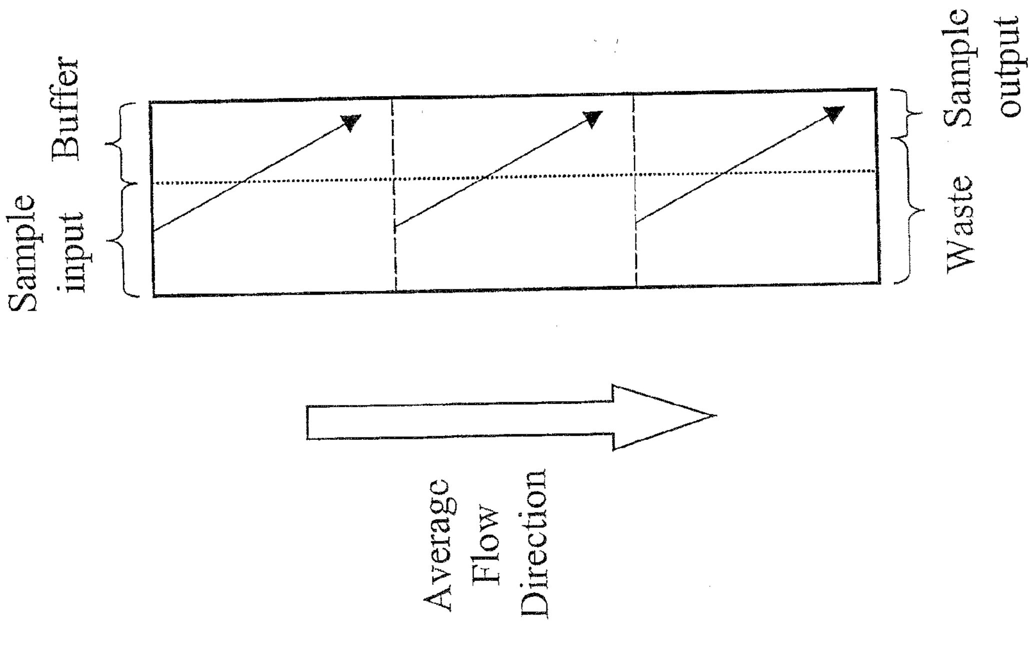
Sample input
Buffer
Sample output
Waste
Average Flow Direction

Fig. 34B

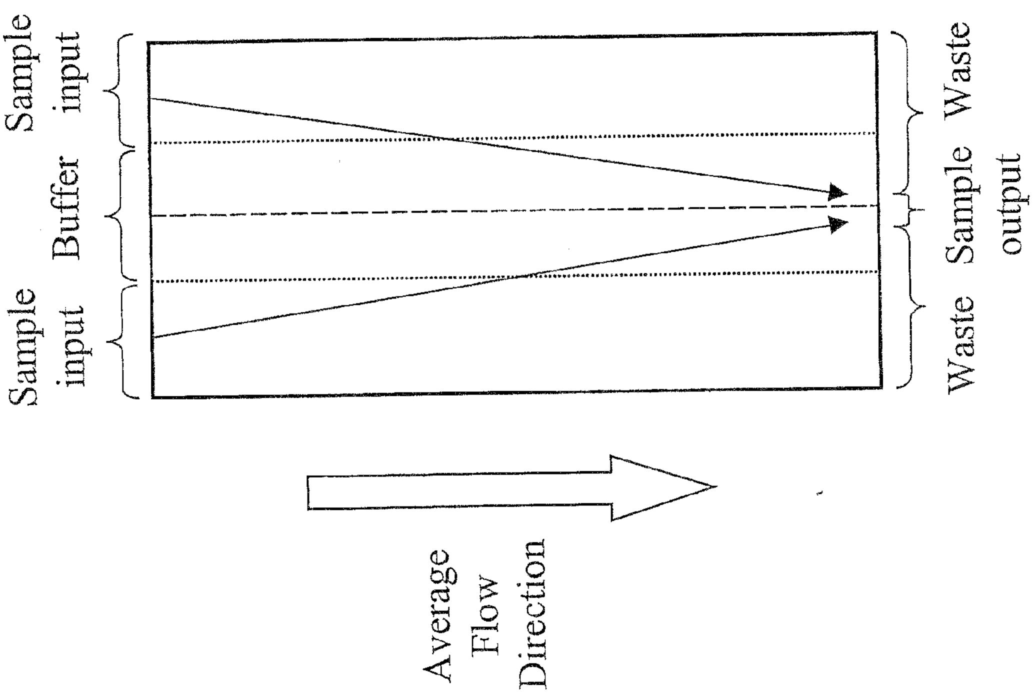
Sample input
Buffer
Sample input
Waste
Sample output
Waste
Average Flow Direction

Fig. 34C

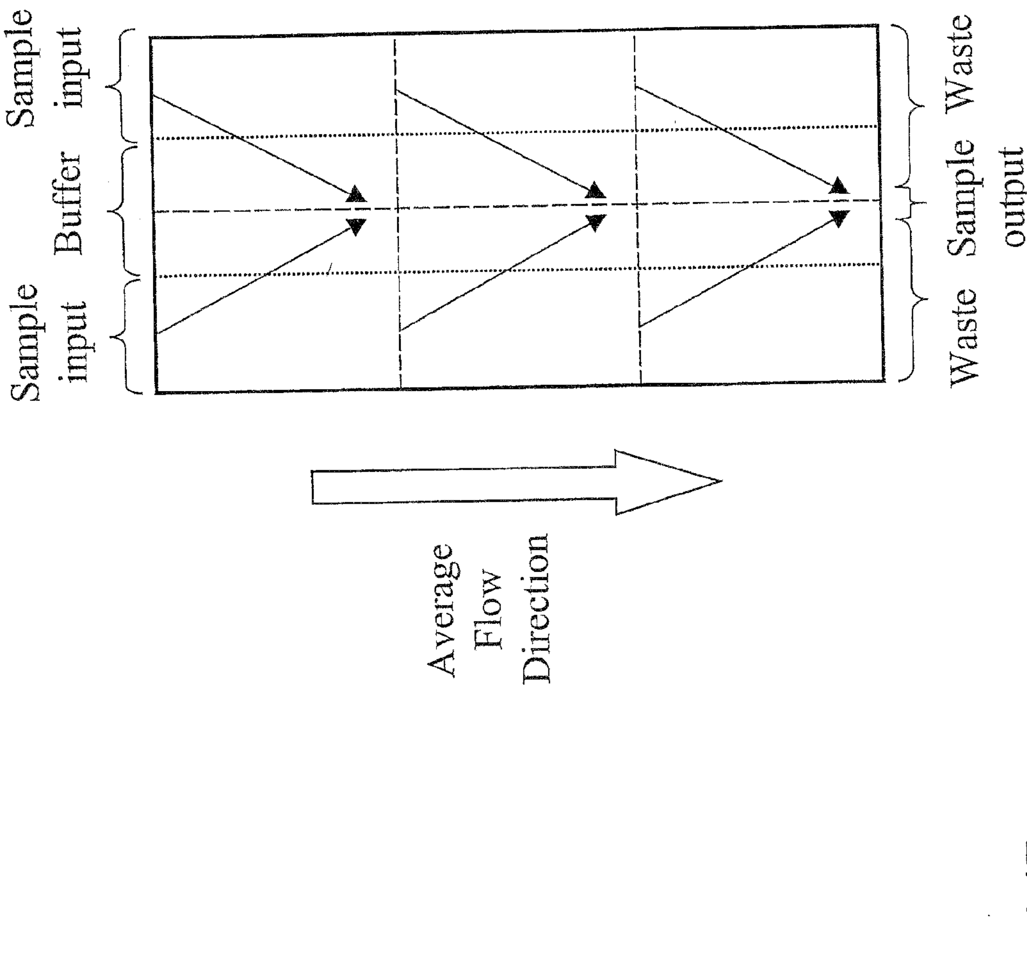
Sample input
Buffer
Sample input
Waste
Sample output
Waste
Average Flow Direction

Fig. 34D

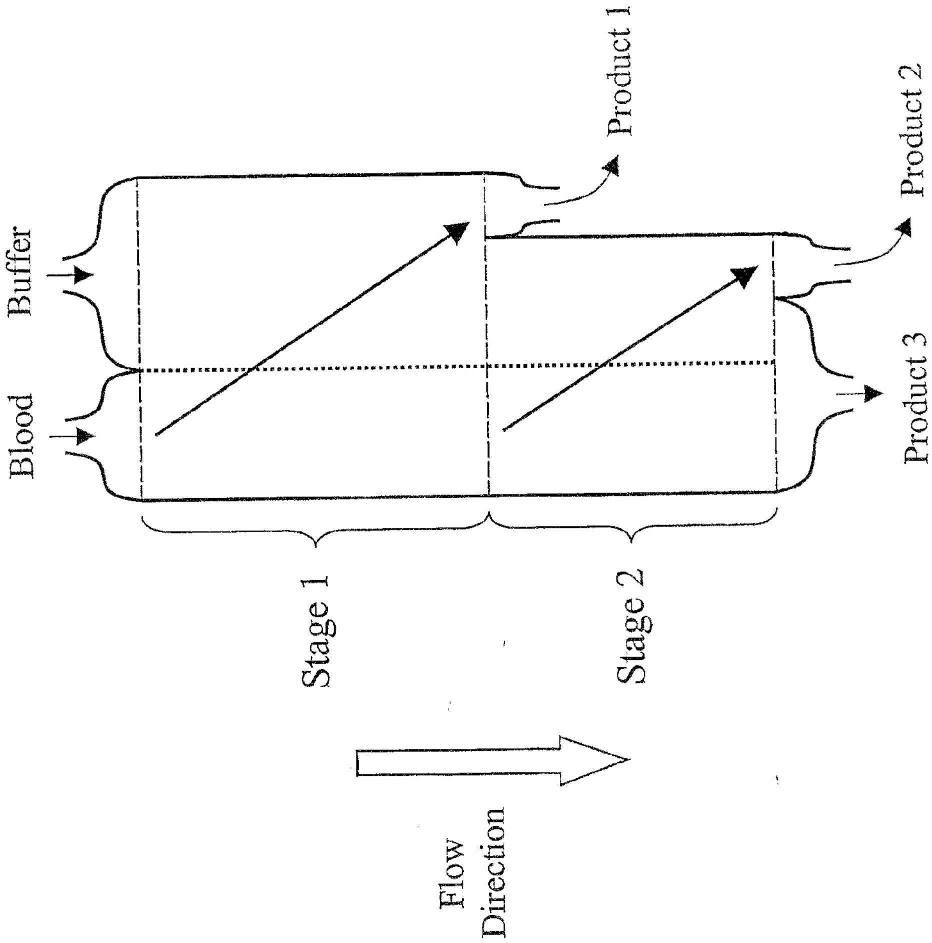
Blood
Buffer
Product 1
Product 2
Product 3
Stage 1
Stage 2
Flow Direction

Fig. 35A

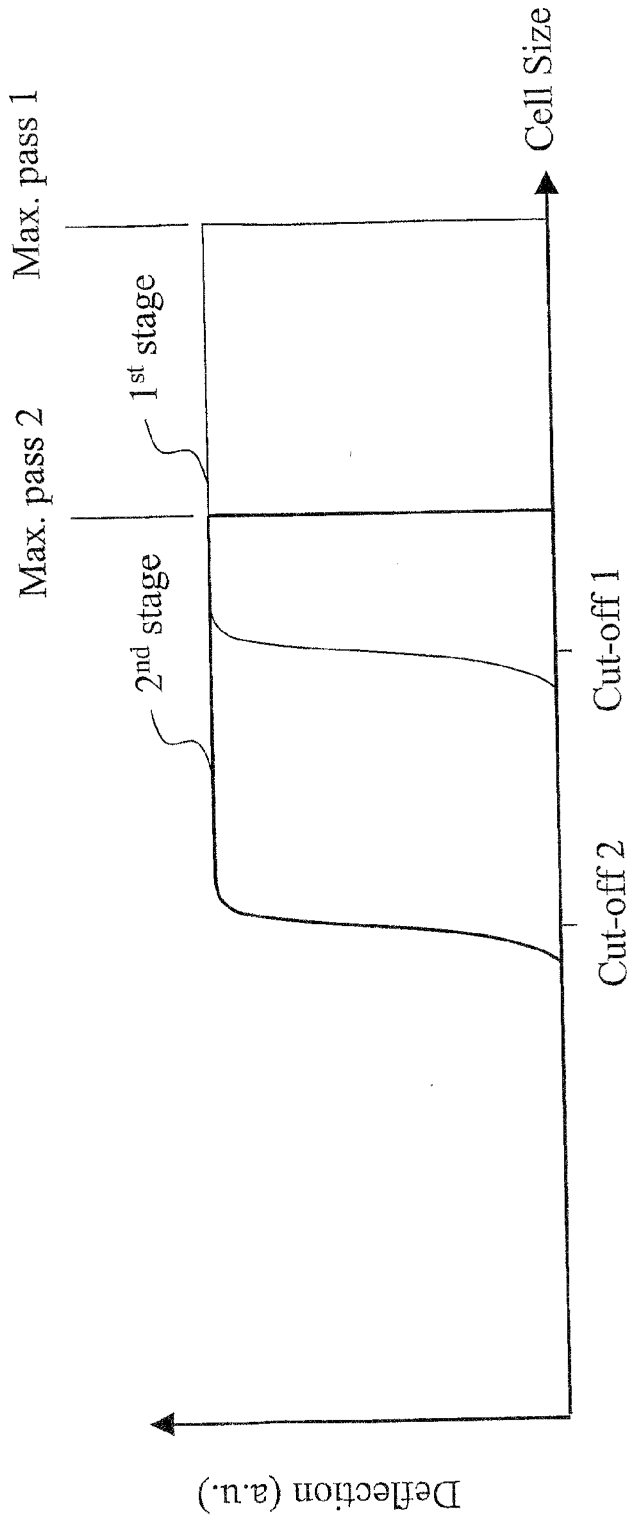
Cell Size
Max. pass 1
Max. pass 2
1st stage
2nd stage
Cut-off 1
Cut-off 2
Deflection (a.u.)

Fig. 35B

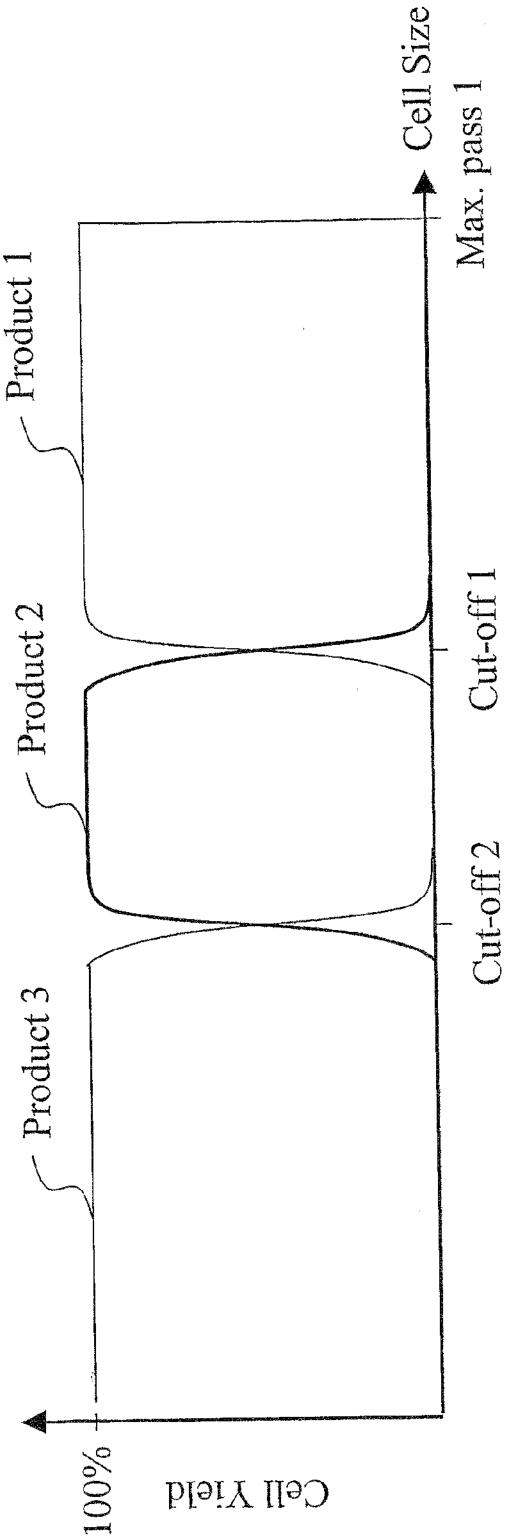
Cell Size
Max. pass 1
Product 1
Product 2
Product 3
Cut-off 1
Cut-off 2
100%
Cell Yield

Fig. 35C

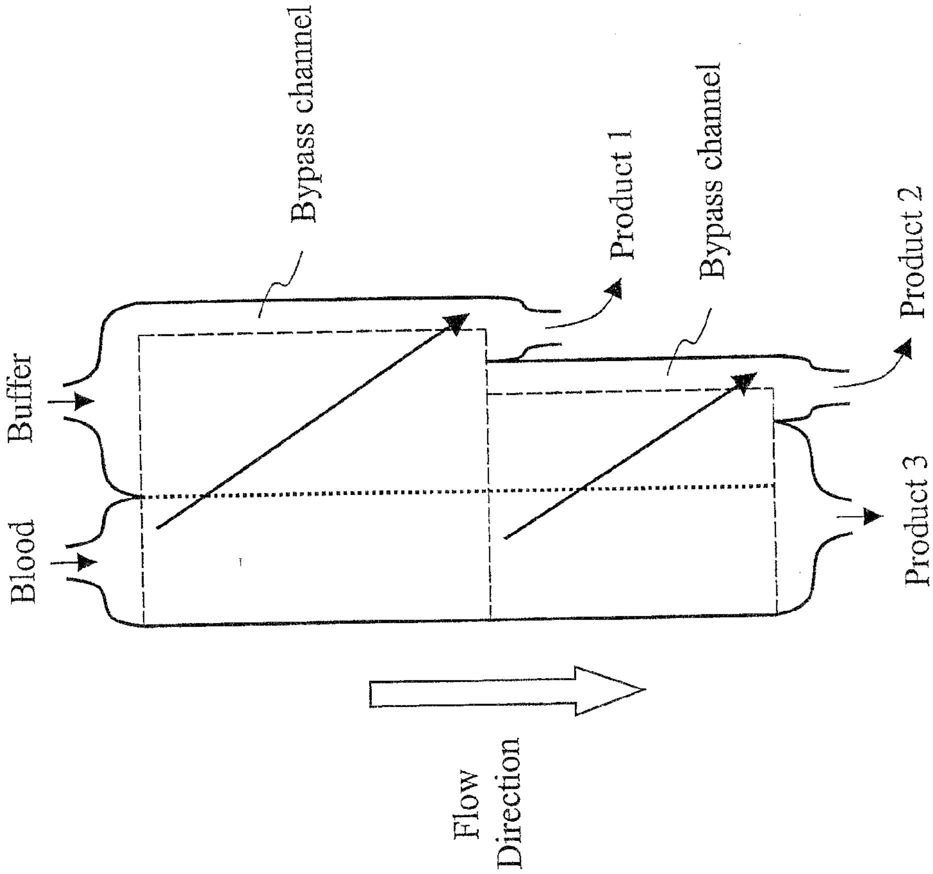
Blood
Buffer
Bypass channel
Product 1
Bypass channel
Product 2
Product 3
Flow
Direction

Fig. 36

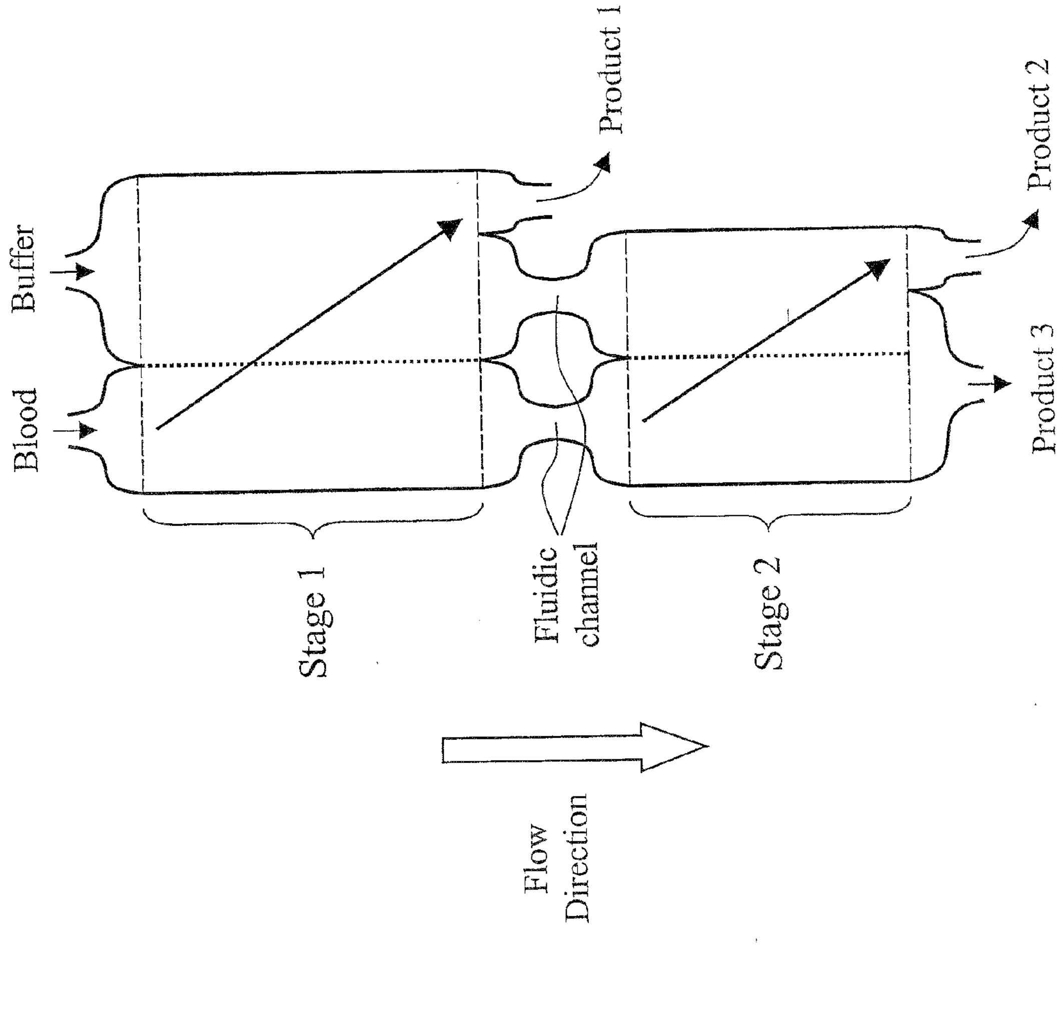
Blood
Buffer
Product 1
Product 2
Product 3
Stage 1
Fluidic channel
Stage 2
Flow Direction

Fig. 37

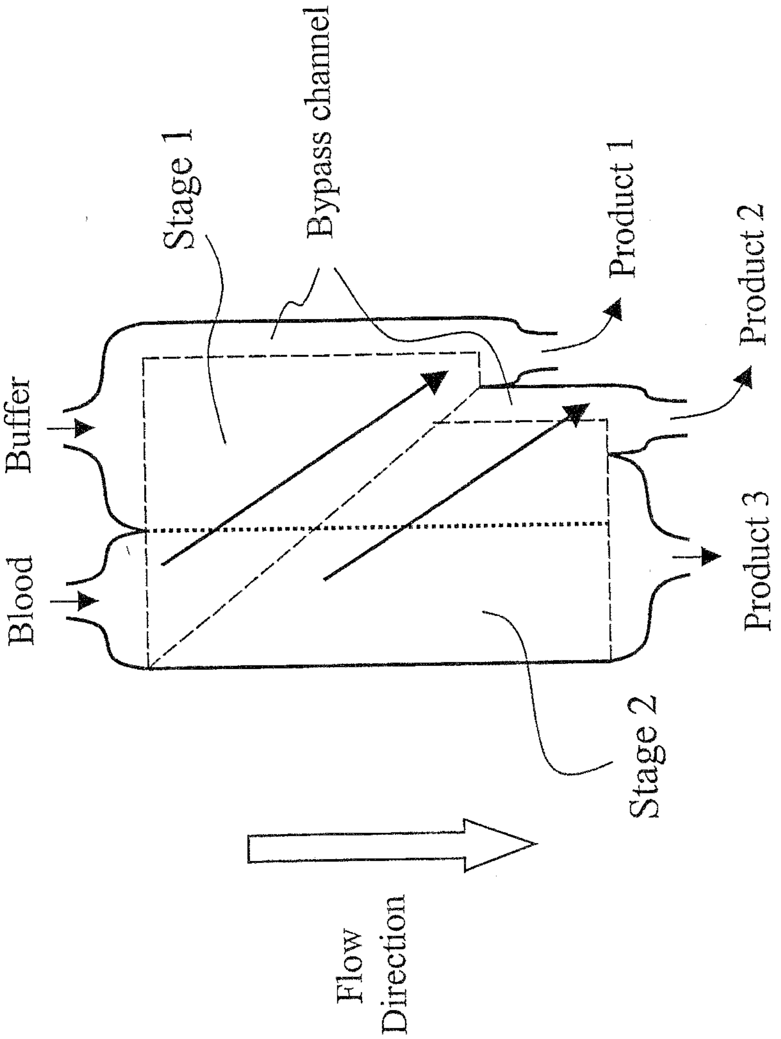
Stage 1
Bypass channel
Product 1
Product 2
Buffer
Blood
Product 3
Stage 2
Flow Direction

Fig. 38

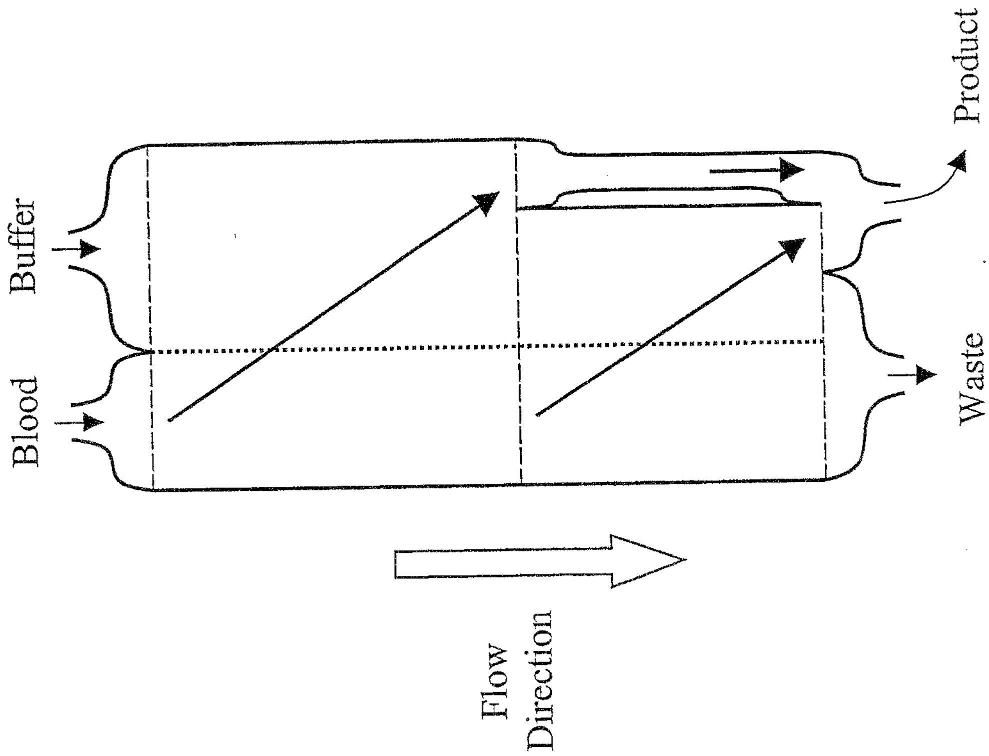


Fig. 39A

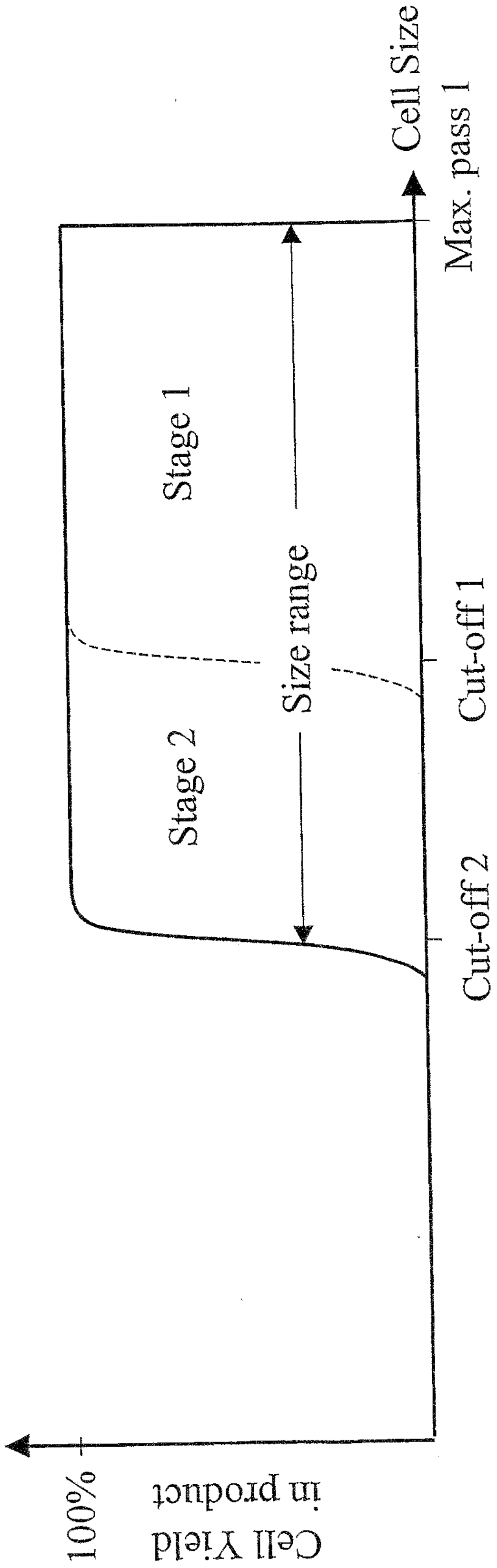
Cell Size
Max. pass 1
Stage 1
Size range
Cut-off 1
Stage 2
Cut-off 2
100%
Cell Yield
in product

Fig. 39B

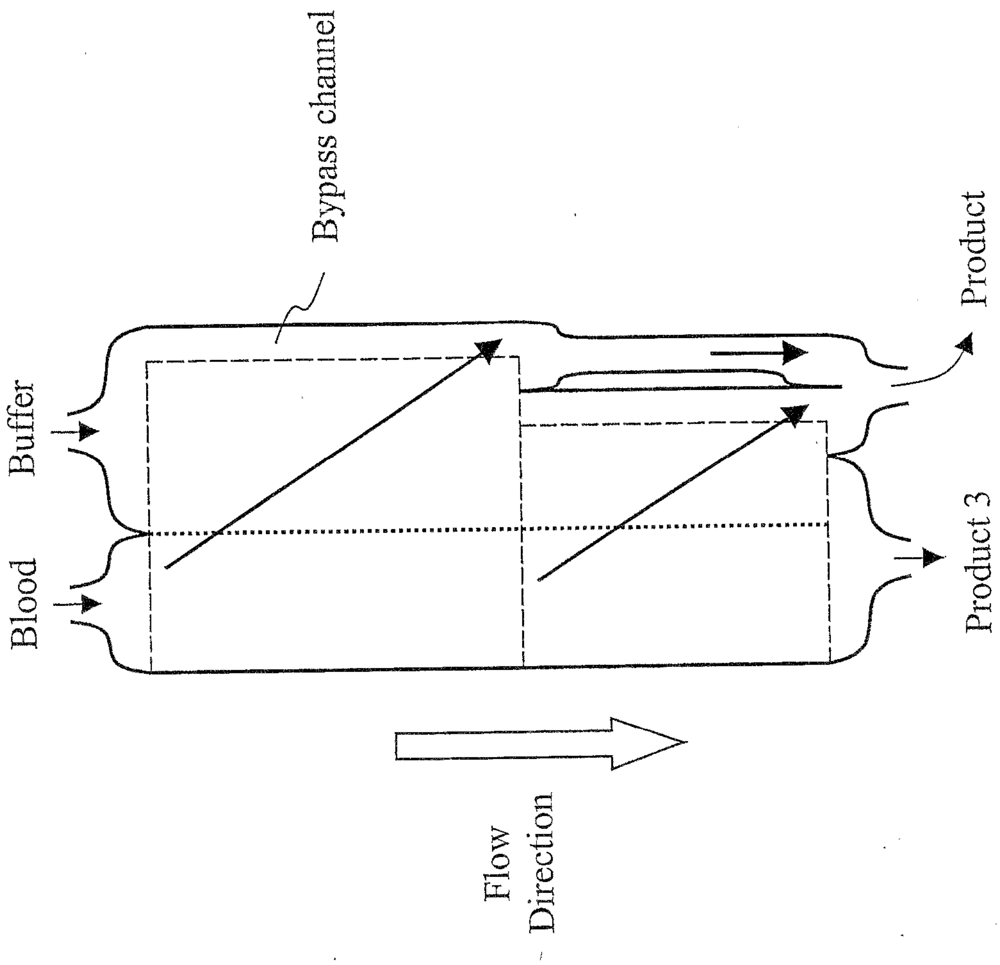
Bypass channel
Buffer
Blood
Product
Product 3
Flow Direction

Fig. 40

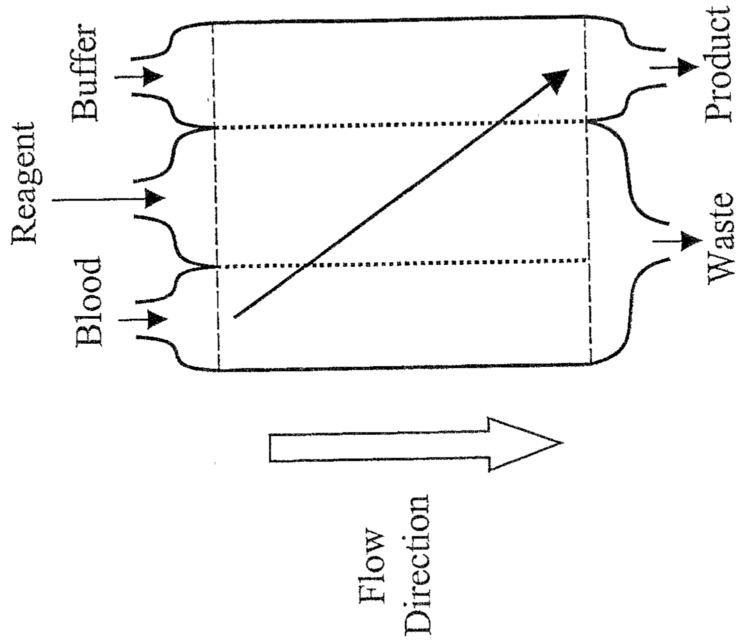


Fig. 41

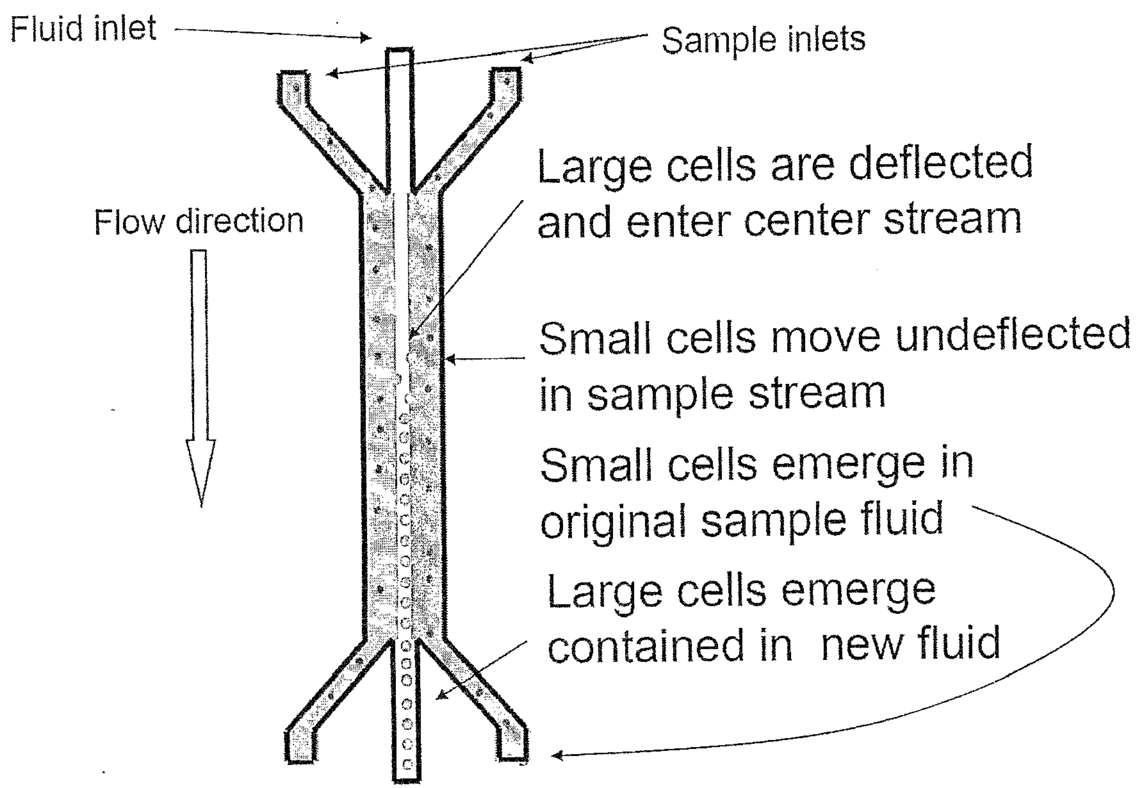
Fluid inlet
Sample inlets
Flow direction
Large cells are deflected and enter center stream
Small cells move undeflected in sample stream
Small cells emerge in original sample fluid
Large cells emerge contained in new fluid

Fig. 42A

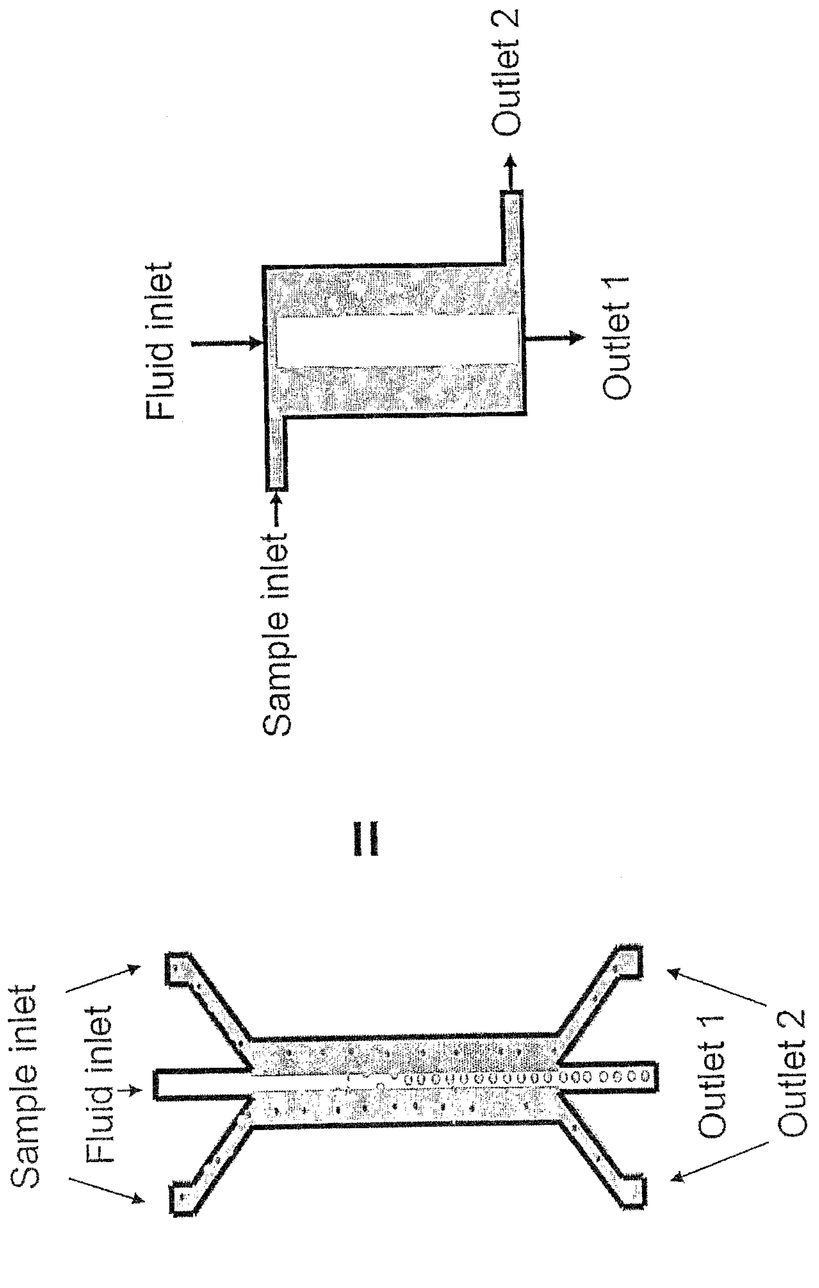
Fluid inlet
Sample inlet
Outlet 2
Outlet 1
II
Sample inlet
Fluid inlet
Outlet 1
Outlet 2

Fig. 42B

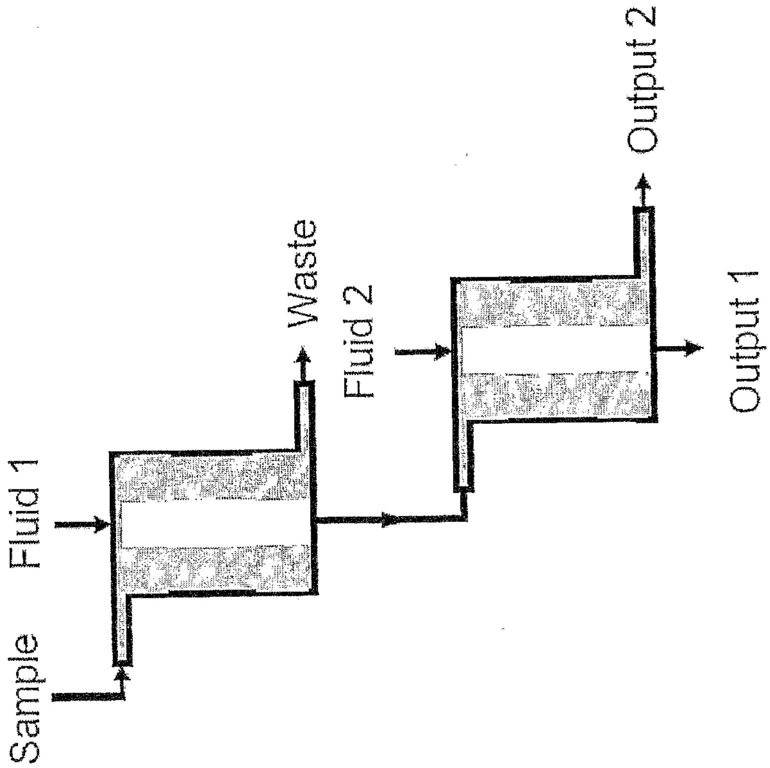


Fig. 43A

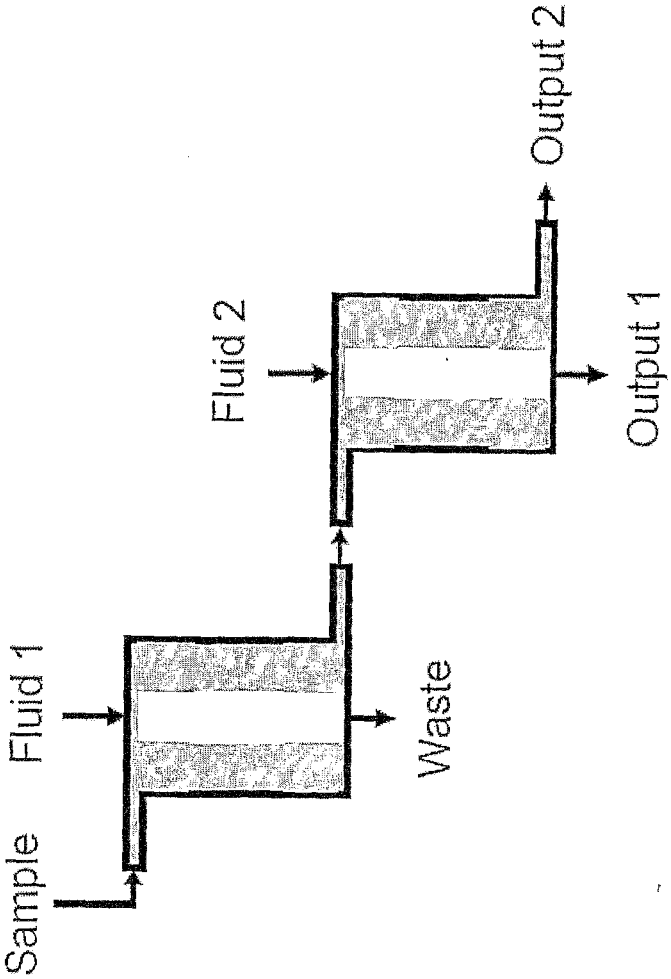


Fig. 43B

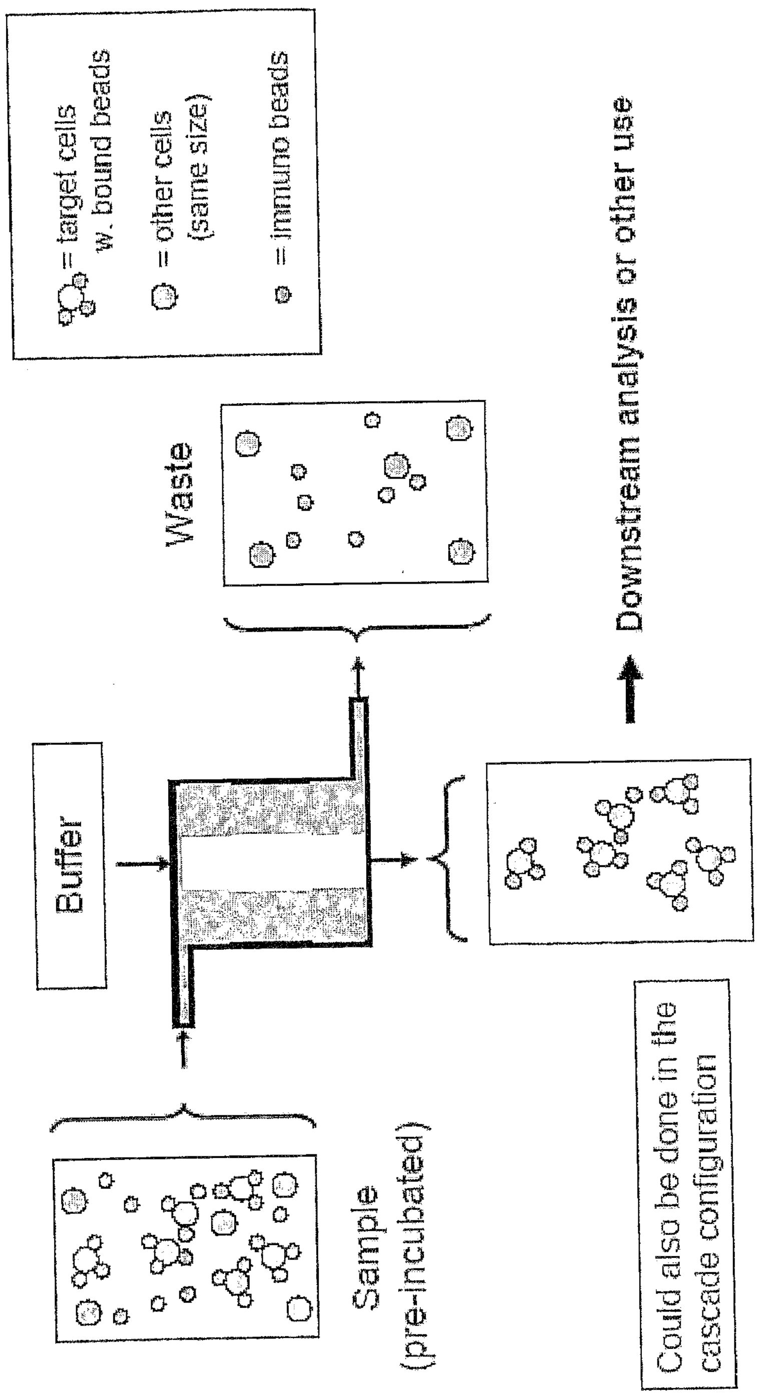
= target cells
w. bound beads
= other cells
(same size)
= immuno beads
Waste
Buffer
Downstream analysis or other use
Sample
(pre-incubated)
Could also be done in the
cascade configuration

Fig. 44

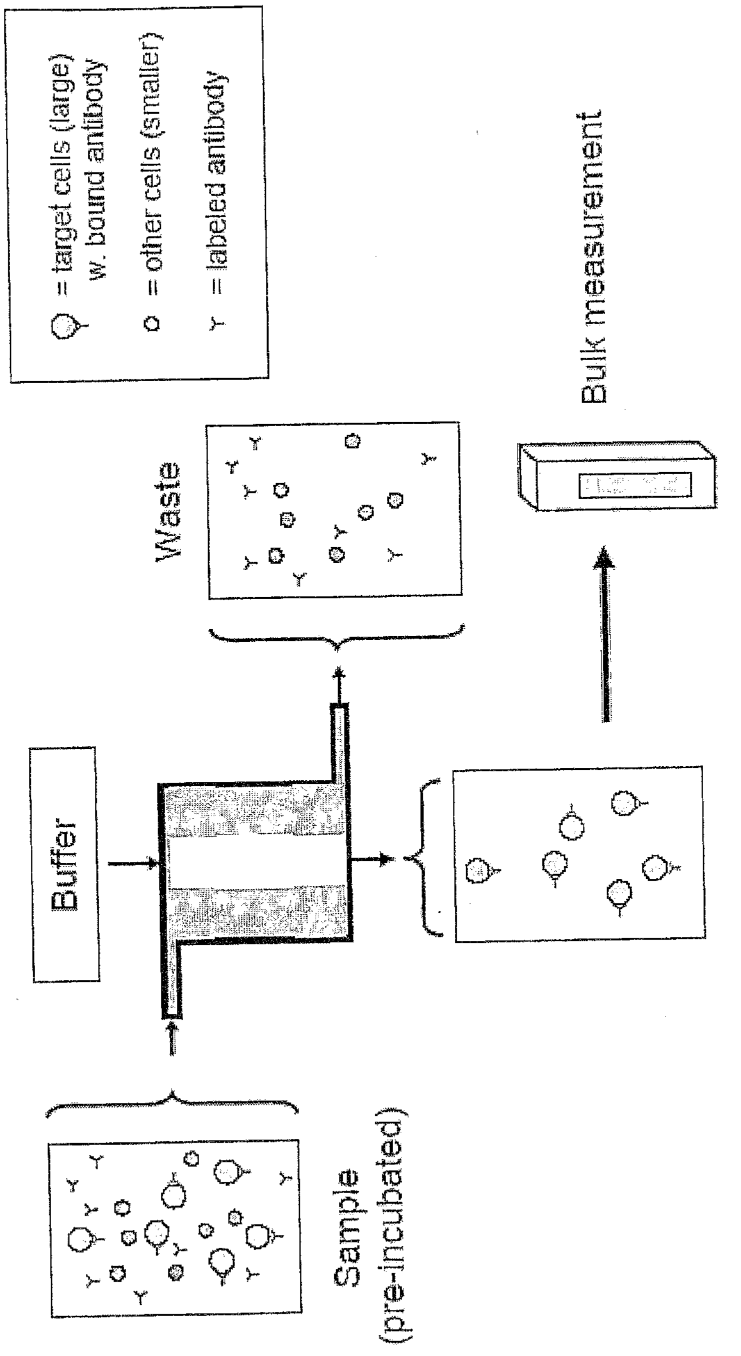
= target cells (large) w. bound antibody
= other cells (smaller)
= labeled antibody
Buffer
Waste
Bulk measurement
Sample (pre-incubated)

Fig. 45

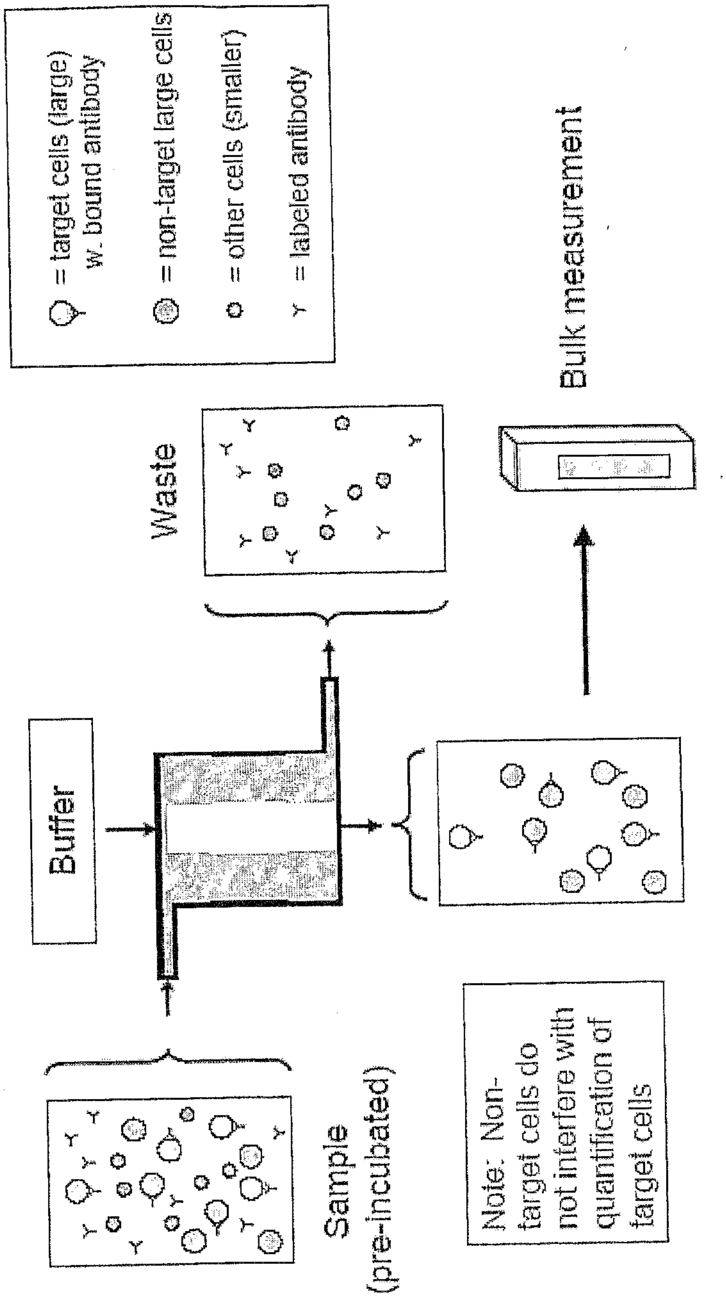

= target cells (large) w. bound antibody
= non-target large cells
= other cells (smaller)
= labeled antibody
Buffer
Waste
Bulk measurement
Sample (pre-incubated)
Note: Non-target cells do not interfere with quantification of target cells


Fig. 46

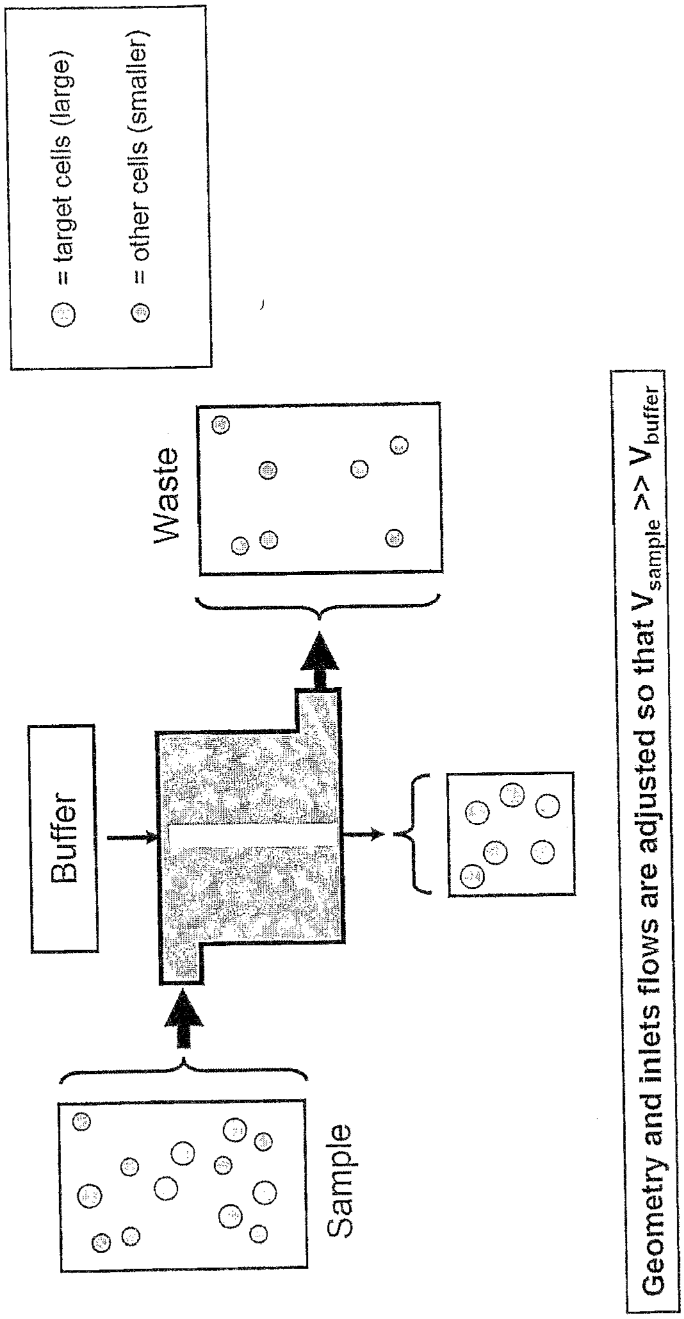
= target cells (large)
= other cells (smaller)
Buffer
Waste
Sample
Geometry and inlets flows are adjusted so that $V_{sample} >> V_{buffer}$

Fig. 47

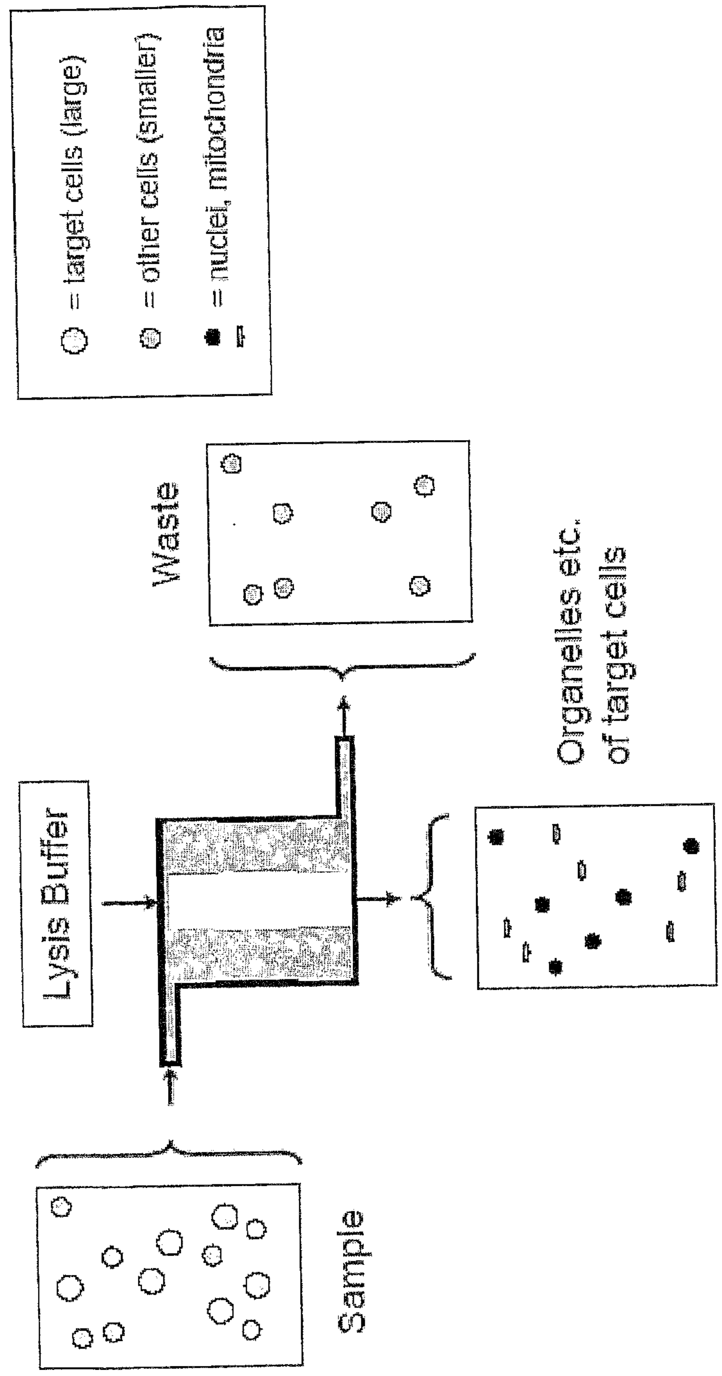
= target cells (large)
= other cells (smaller)
= nuclei, mitochondria
Waste
Organelles etc.
of target cells
Lysis Buffer
Sample

Fig. 48

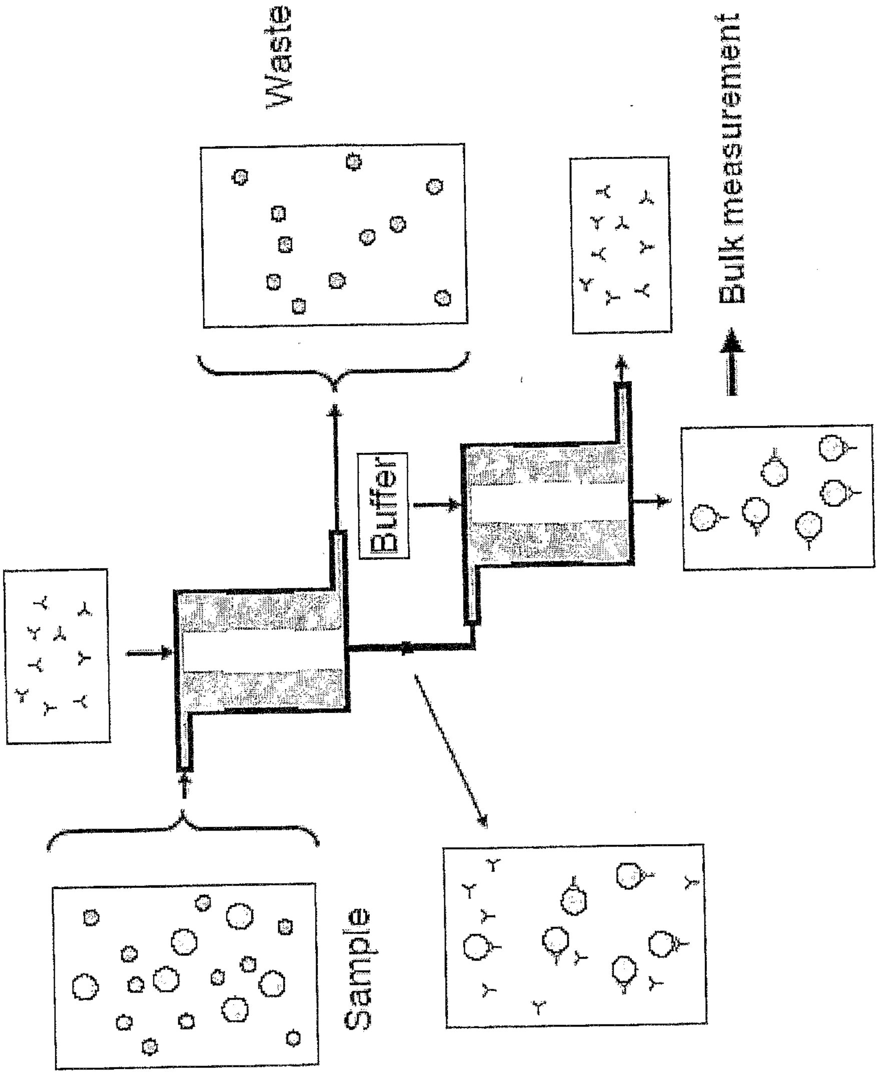


Fig. 49

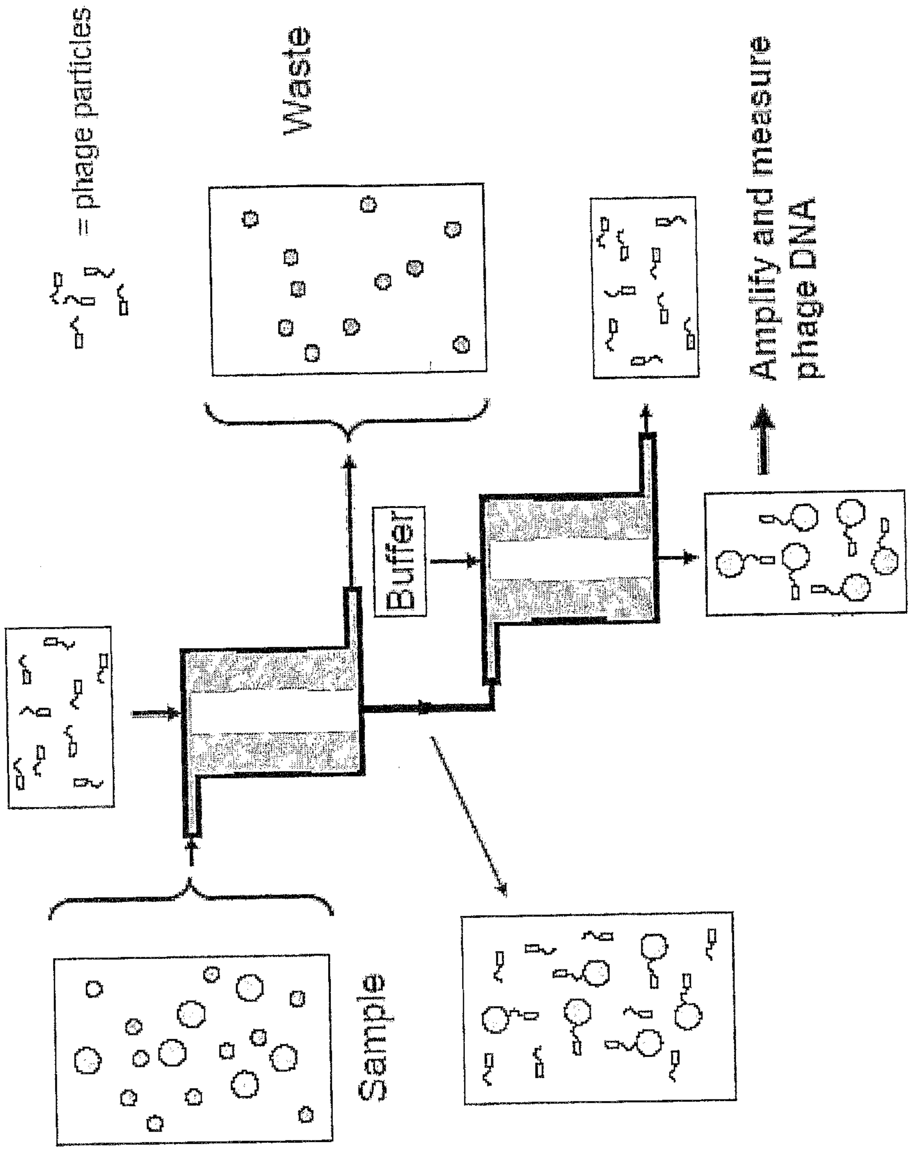
= phage particles
Waste
Buffer
Amplify and measure phage DNA
Sample

Fig. 50

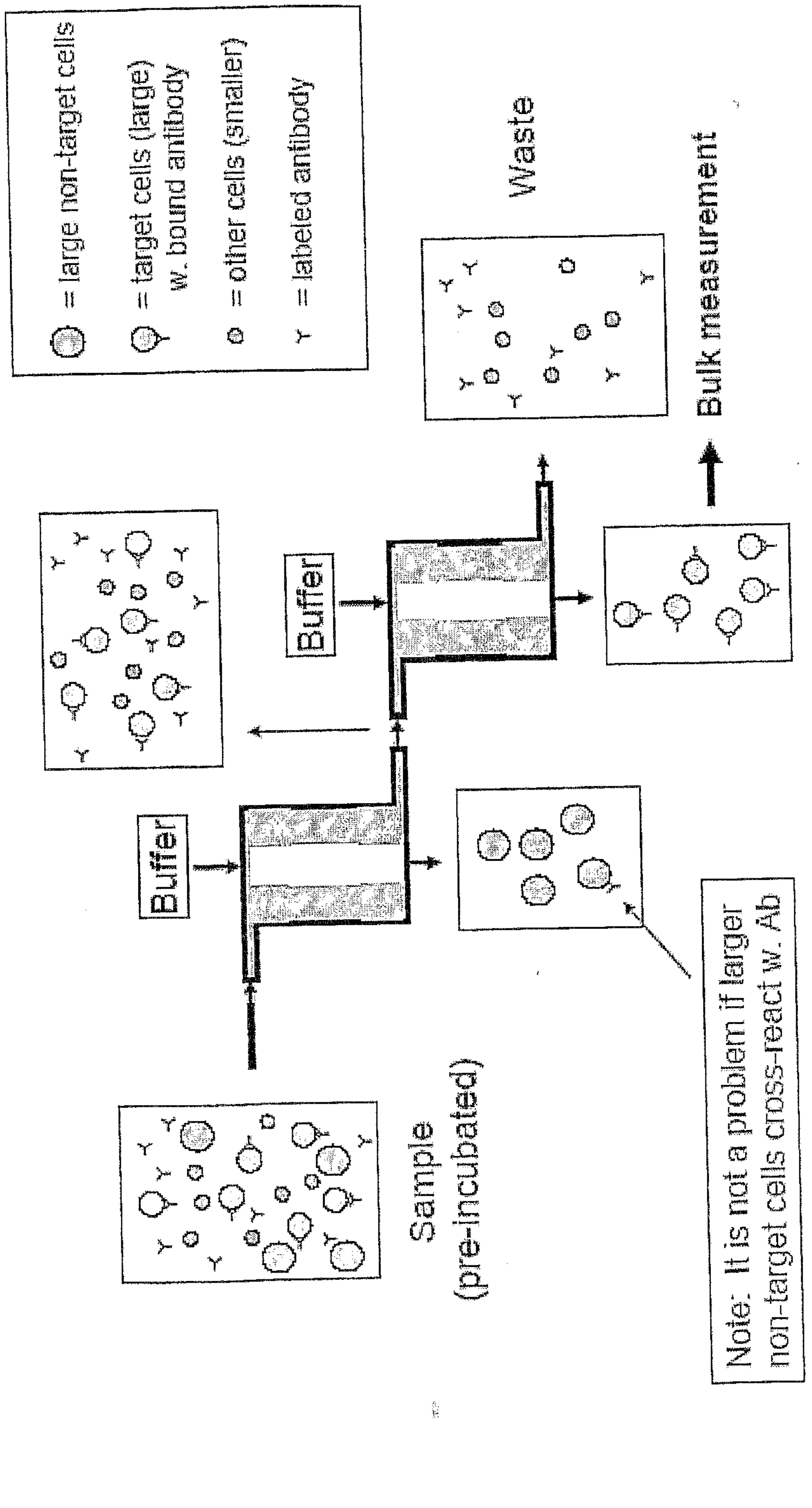
= large non-target cells
= target cells (large) w. bound antibody
= other cells (smaller)
= labeled antibody
Sample (pre-incubated)
Buffer
Buffer
Waste
Bulk measurement
Note: It is not a problem if larger non-target cells cross-react w. Ab

Fig. 51

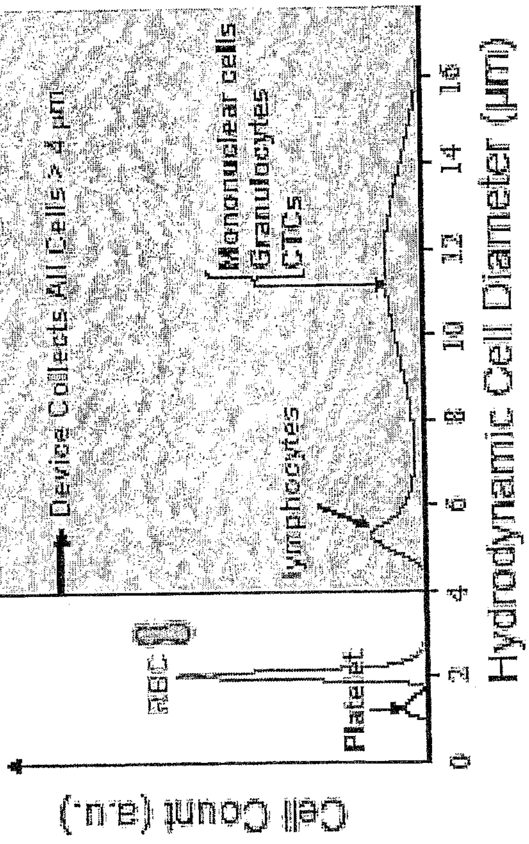
Device Collects All Cells > 4 μm
Mononuclear cells
Granulocytes
CTCs
lymphocytes
RBC
Platelet
Cell Count (a.u.)
Hydrodynamic Cell Diameter (μm)
0
2
4
6
8
10
12
14
16

Fig. 52

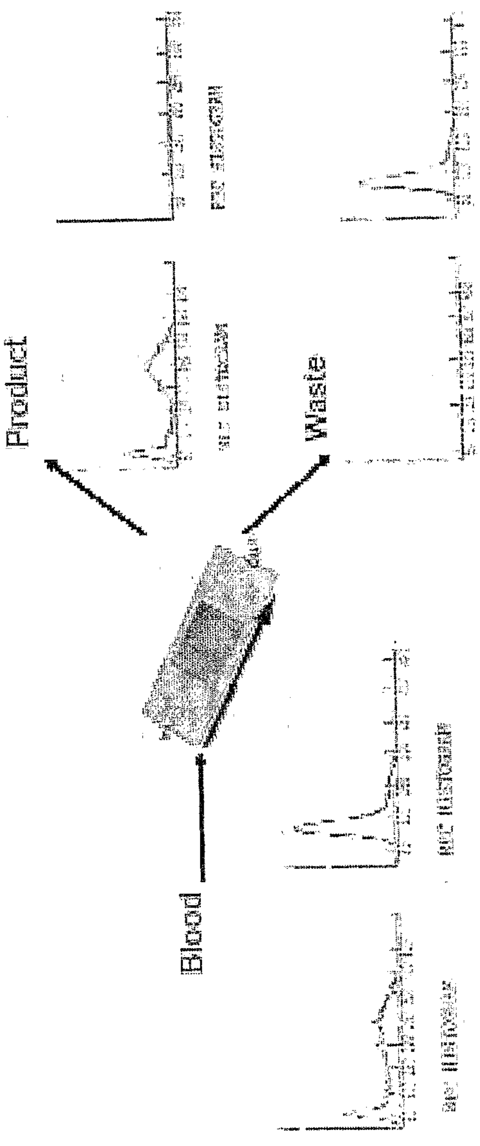


Fig. 53

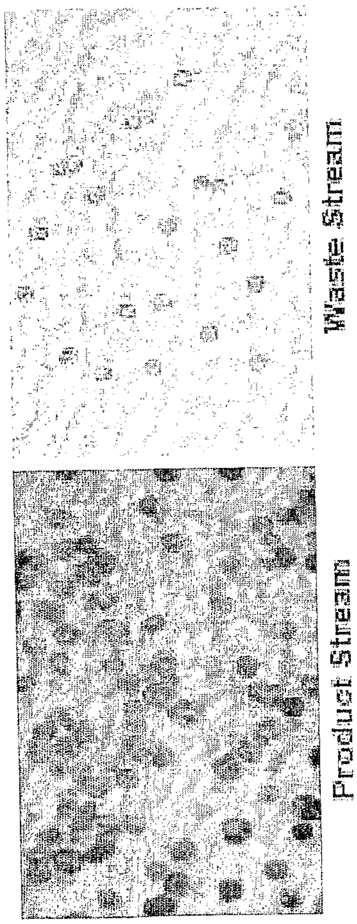


Fig. 54

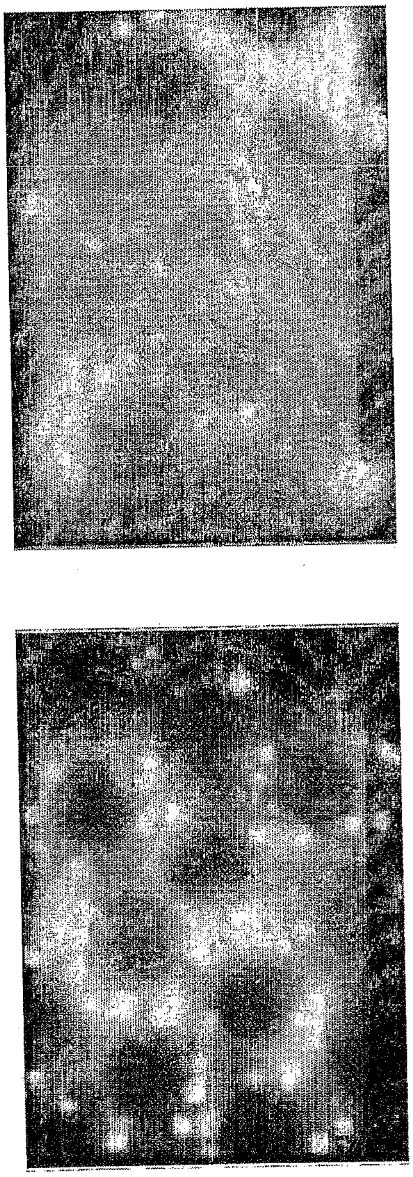

Fig. 55

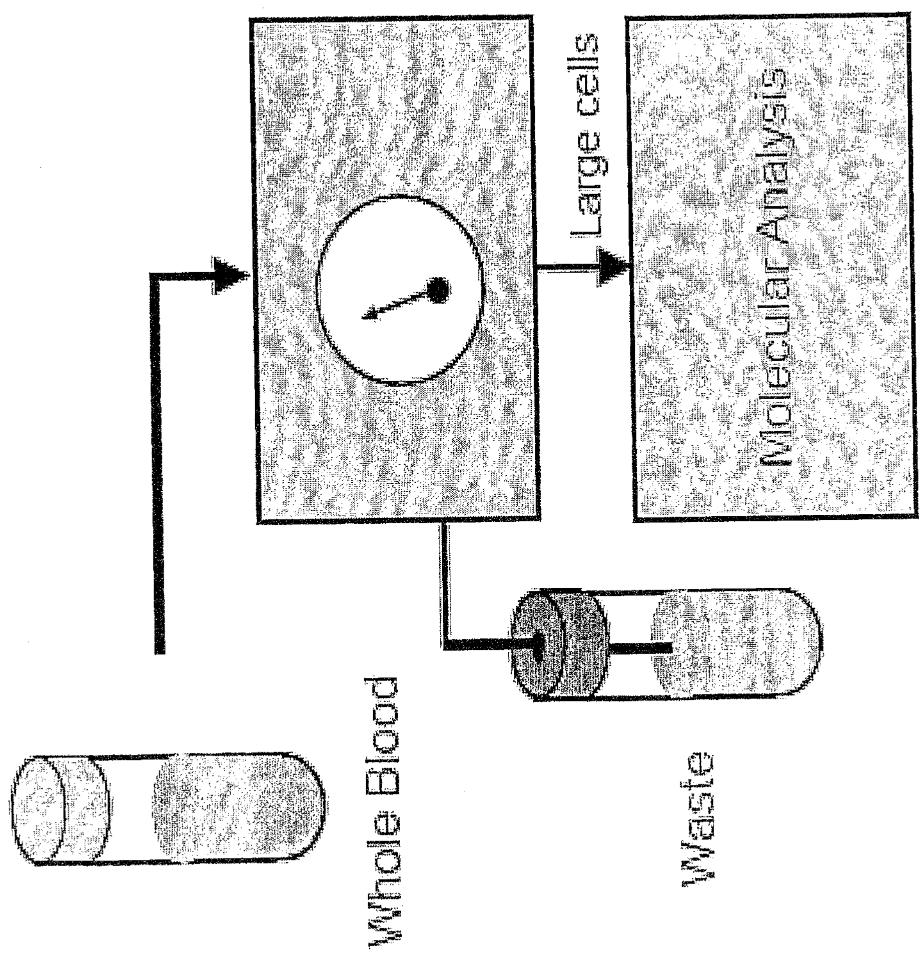


Fig. 56

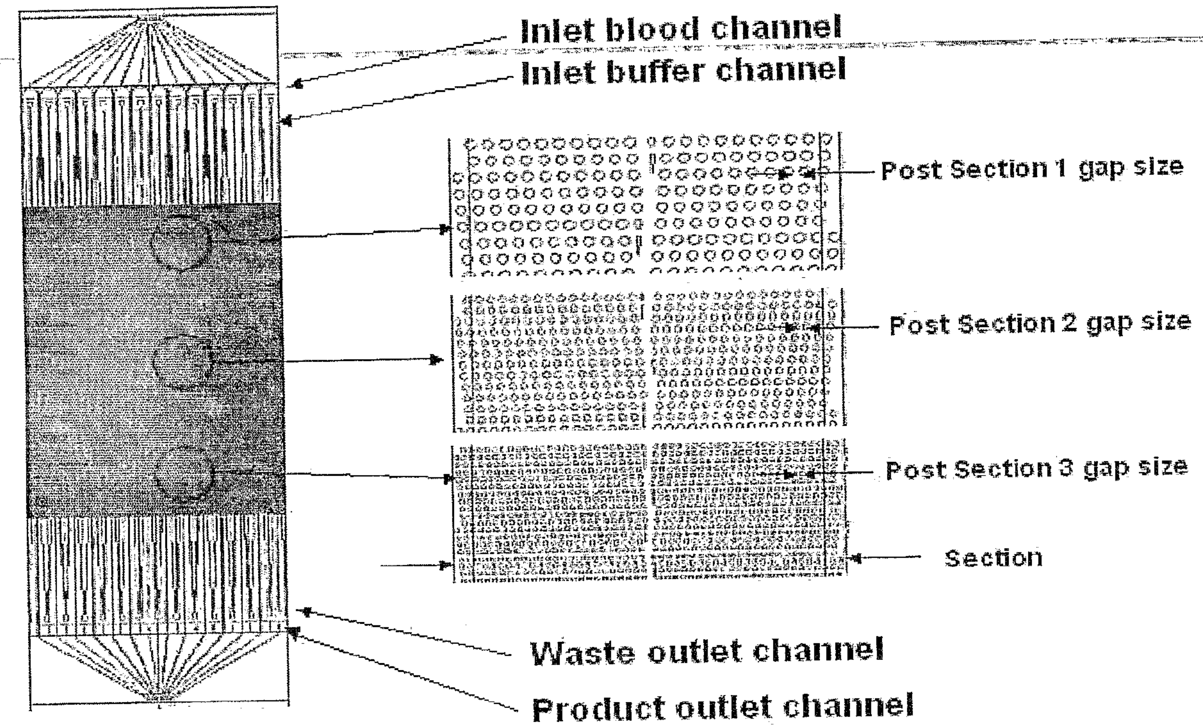
Inlet blood channel
Inlet buffer channel
Post Section 1 gap size
Post Section 2 gap size
Post Section 3 gap size
Section
Waste outlet channel
Product outlet channel

Fig. 57A

| | | Version 1 | Version 2 | Version 3 |
|---|---|---|---|---|
| Inlet channel width (blood) | 50 | 50 | 100 | 100 |
| Inlet channel width (buffer) | 55 | 55 | 110 | 110 |
| Outlet channel width (product) | 49 | 49 | 98 | 98 |
| Outlet channel width (waste) | 50 | 50 | 100 | 100 |
| | | | | |
| Gap size / Deflect cell size | | | | |
| Post section 1 | 18/9 | 36/18 | 44/22 | 50/25 |
| Post section 2 | 12/6 | 24/12 | 30/15 | 36/18 |
| Post section 3 | 8/4 | 16/8 | 20/10 | 24/12 |
| Number of parallel sections | 14 | 14 | 14 | 14 |
| Etch depth | 150 | 150 | 150 | 150 |
| Product cell size (cut off) | 4 | 8 | 10 | 12 |
| Estimated Flow rate, ml/hr | 5 | 10 | 20 | 30 |

Fig. 57B

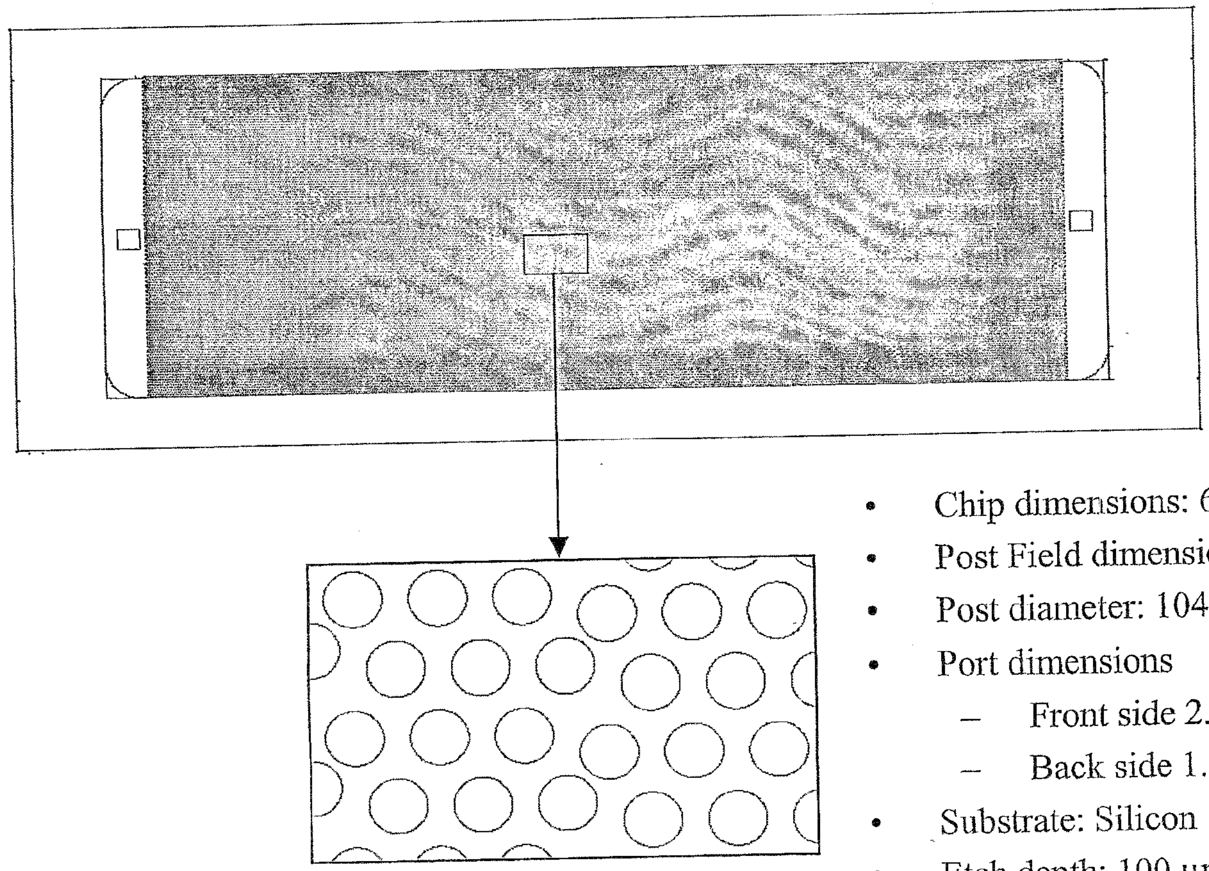

- Chip dimensions: 66.0 x 24.9 mm
- Post Field dimensions: 51.3 x 18.9 mm
- Post diameter: 104 µm
- Port dimensions
  - Front side 2.83 x2.83 mm
  - Back side 1.66 x 1.66 mm
- Substrate: Silicon
- Etch depth: 100 µm

Fig. 57C

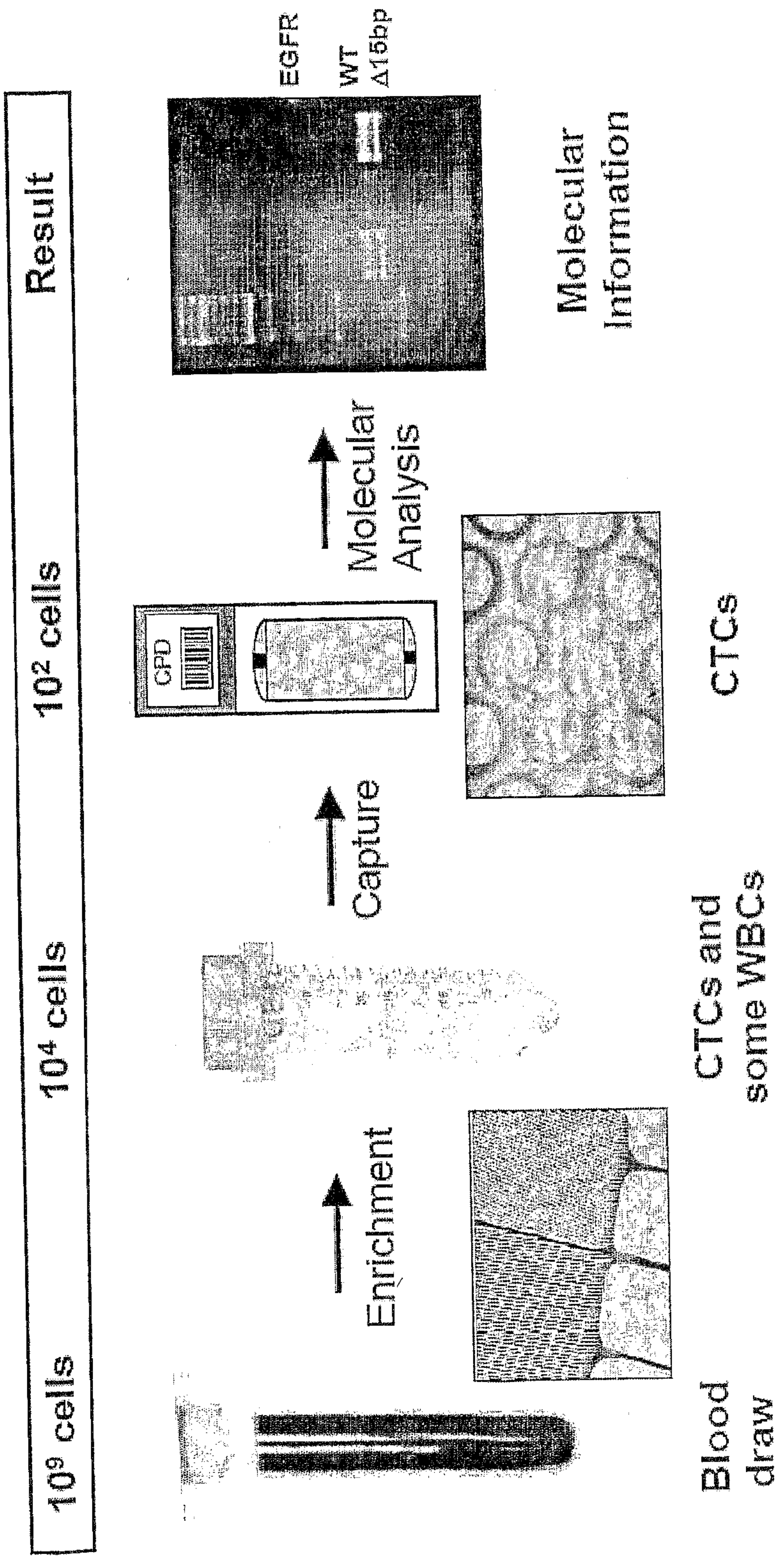
10⁹ cells
10⁴ cells
10² cells
Result
Enrichment
Capture
Molecular Analysis
CPD
EGFR
WT
Δ15bp
Blood draw
CTCs and some WBCs
CTCs
Molecular Information

Fig. 58

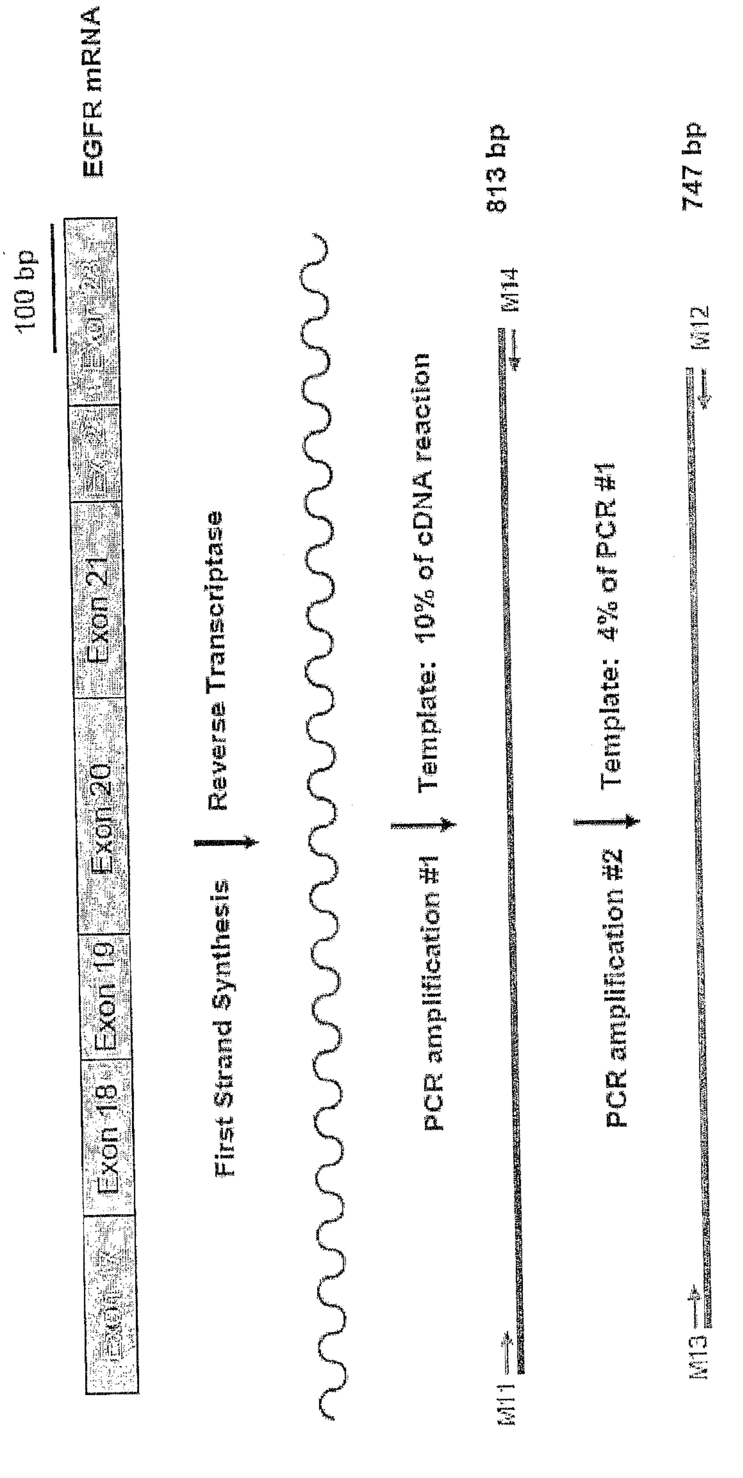
EGFR mRNA
100 bp
Exon 17
Exon 18
Exon 19
Exon 20
Exon 21
Exon 22
Exon 23
First Strand Synthesis
Reverse Transcriptase
PCR amplification #1
Template: 10% of cDNA reaction
M11
M14
813 bp
PCR amplification #2
Template: 4% of PCR #1
M13
M12
747 bp

Fig. 59

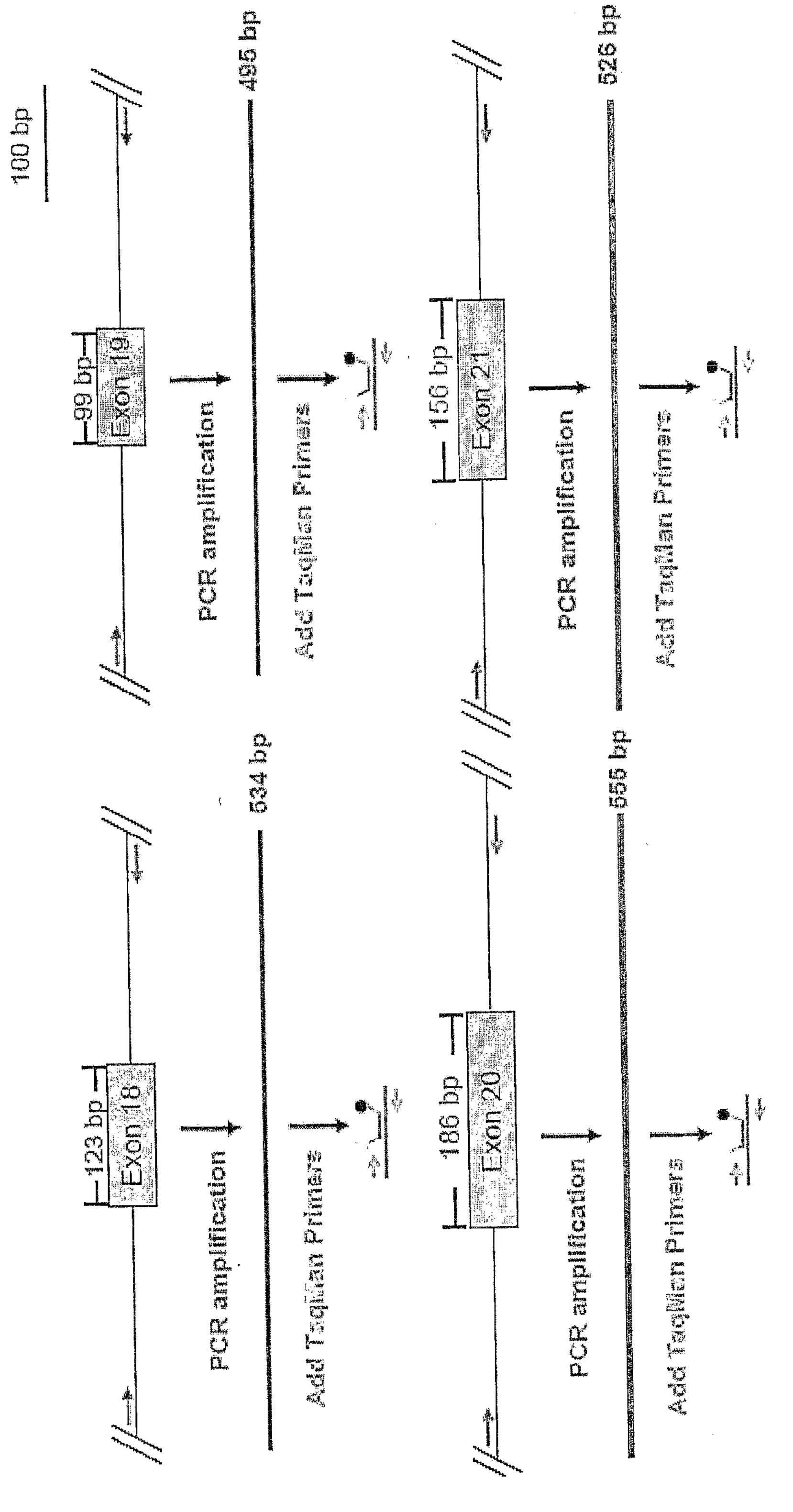
100 bp
99 bp
Exon 19
PCR amplification
495 bp
Add TaqMan Primers
156 bp
Exon 21
PCR amplification
526 bp
Add TaqMan Primers
123 bp
Exon 18
PCR amplification
534 bp
Add TaqMan Primers
186 bp
Exon 20
PCR amplification
555 bp
Add TaqMan Primers

Fig. 60

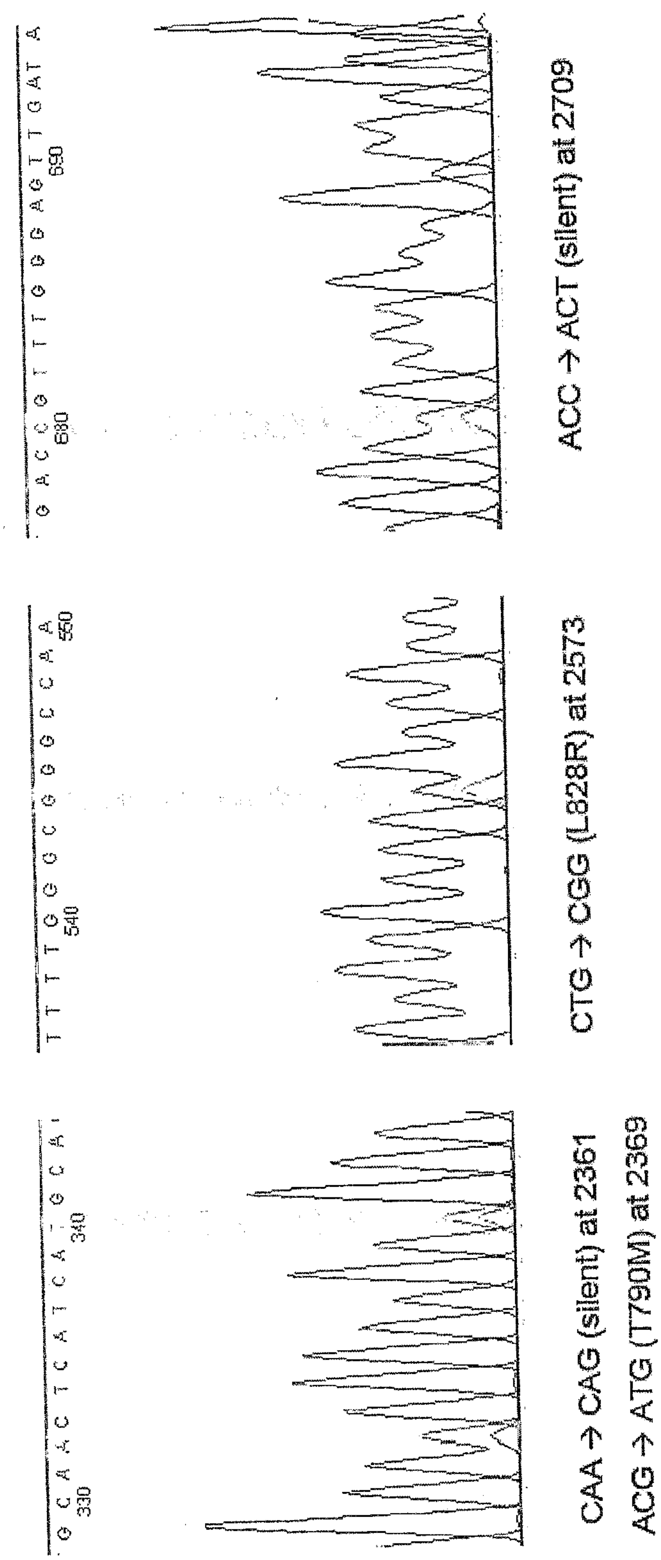
CAA → CAG (silent) at 2361
ACG → ATG (T790M) at 2369
CTG → CGG (L828R) at 2573
ACC → ACT (silent) at 2709

Fig. 61

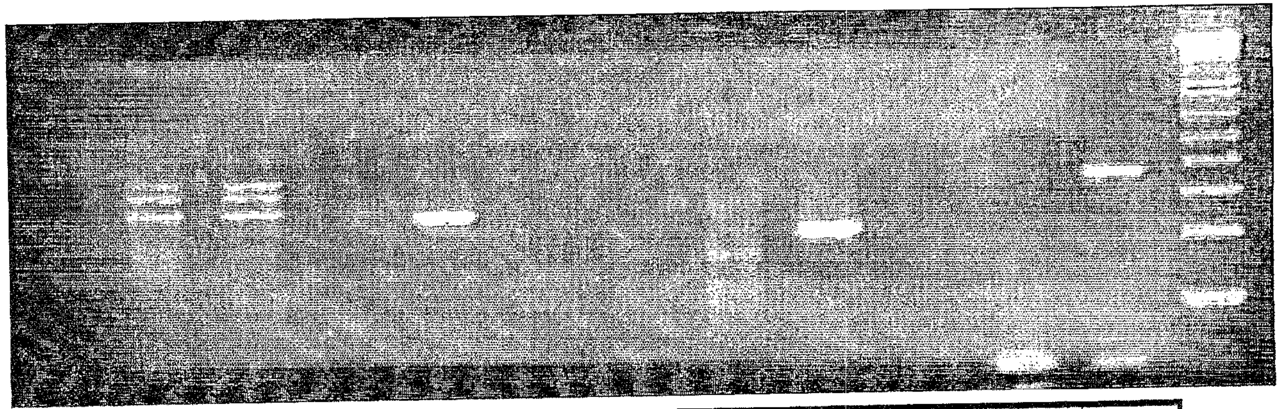


| | | | | | | | | | | | | |
|---|---|---|---|---|---|---|---|---|---|---|---|---|
| WBC cDNA | - | + | - | - | + | - | - | + | - | - | + | - |
| H1650 cDNA | - | - | + | - | - | + | - | - | + | - | - | + |
| Target mRNA | BCKD | | | CD45 | | | EpCaM | | | EGFR | | |

Fig. 62A

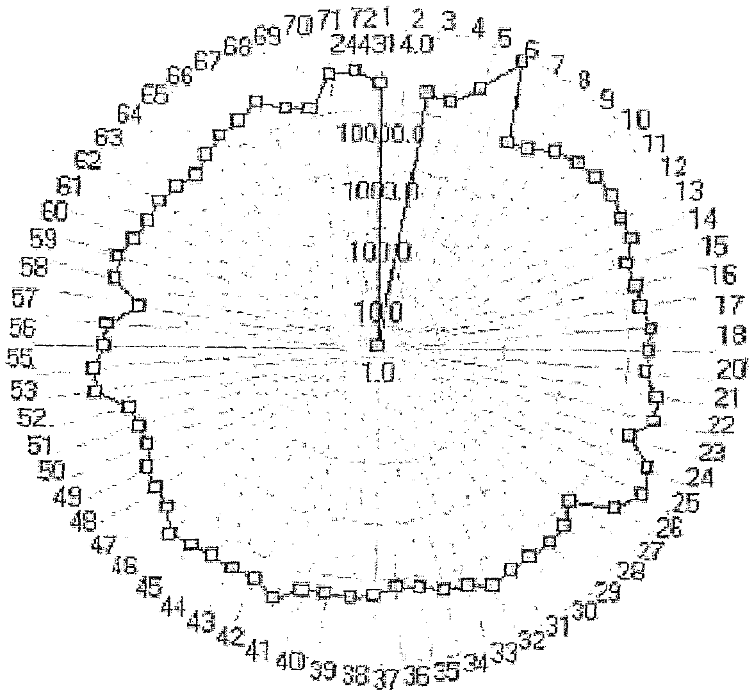


| | Tissue | Ct | [mRNA] |
|---|---|---|---|
| 1 | Renal Blood Vessel | 24.02 | 25,916 |
| 2 | Blood Mononuclear Cells (PBMC) | >40 | 0 |
| 3 | Colon | 24.28 | 21,968 |
| 4 | Primary Lung Bronchus | 24.49 | 19,224 |

Fig. 62B

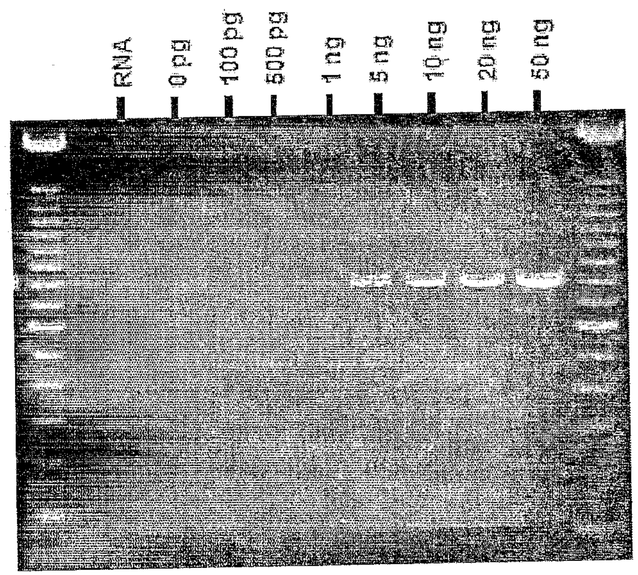


PCR #1

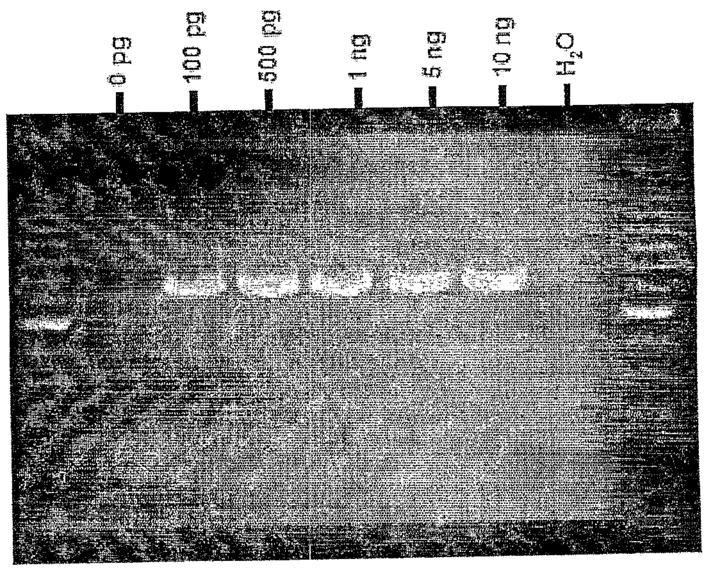


PCR #2 (Nested)

NIH-3T3 -- 100 pg total RNA = ~20 cells

NCI-H1975 -- 100 pg total RNA = ~10 cells

COS-7 -- 100 pg total RNA = ~6 cells

Fig. 63

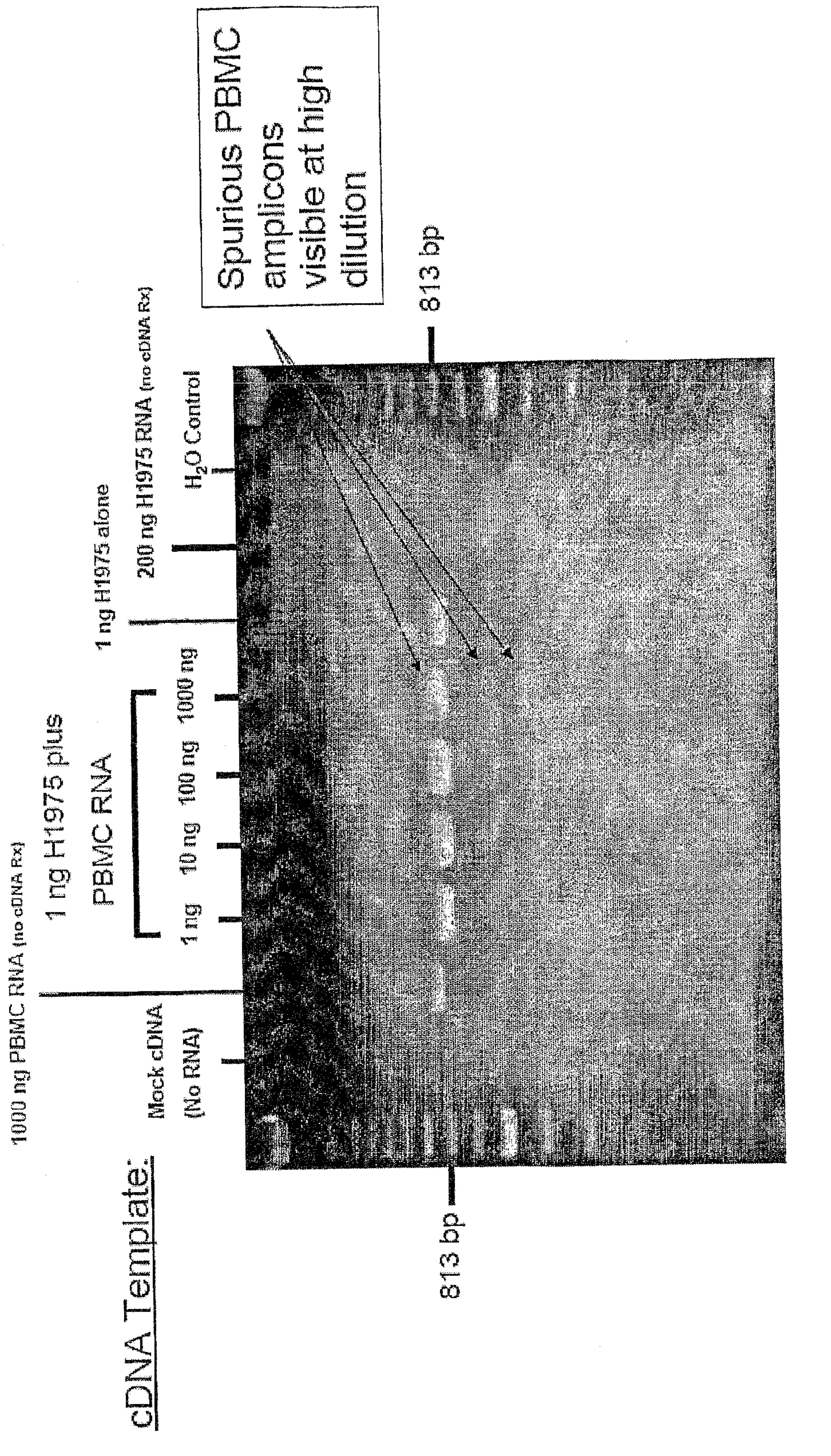
cDNA Template:
1000 ng PBMC RNA (no cDNA Rx)
Mock cDNA
(No RNA)
1 ng H1975 plus
PBMC RNA
1 ng
10 ng
100 ng
1000 ng
1 ng H1975 alone
200 ng H1975 RNA (no cDNA Rx)
$H_2O$ Control
Spurious PBMC amplicons visible at high dilution
813 bp
813 bp

Fig. 64A

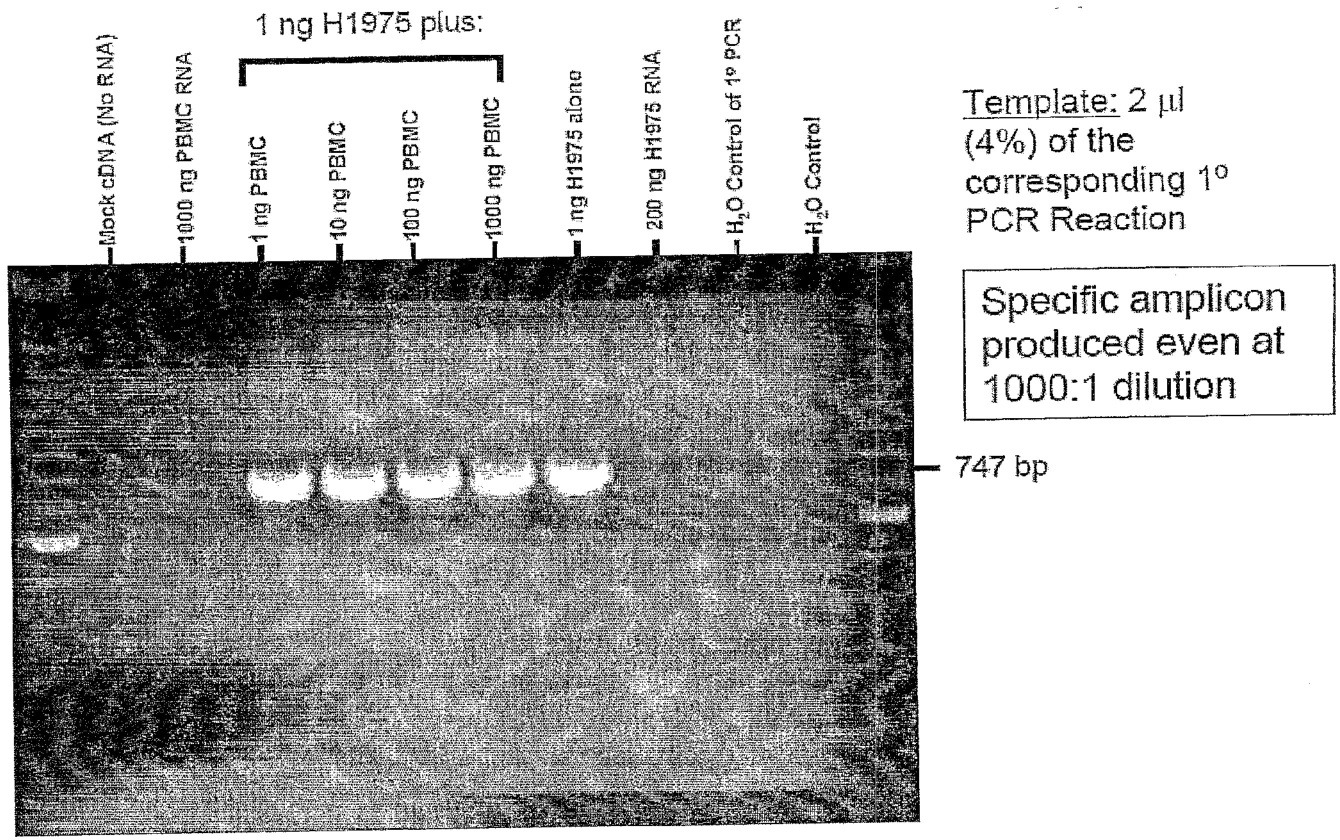
1 ng H1975 plus:
Mock cDNA (No RNA)
1000 ng PBMC RNA
1 ng PBMC
10 ng PBMC
100 ng PBMC
1000 ng PBMC
1 ng H1975 alone
200 ng H1975 RNA
H2O Control of 1º PCR
H2O Control
Template: 2 µl (4%) of the corresponding 1º PCR Reaction
Specific amplicon produced even at 1000:1 dilution
747 bp

Fig. 64B

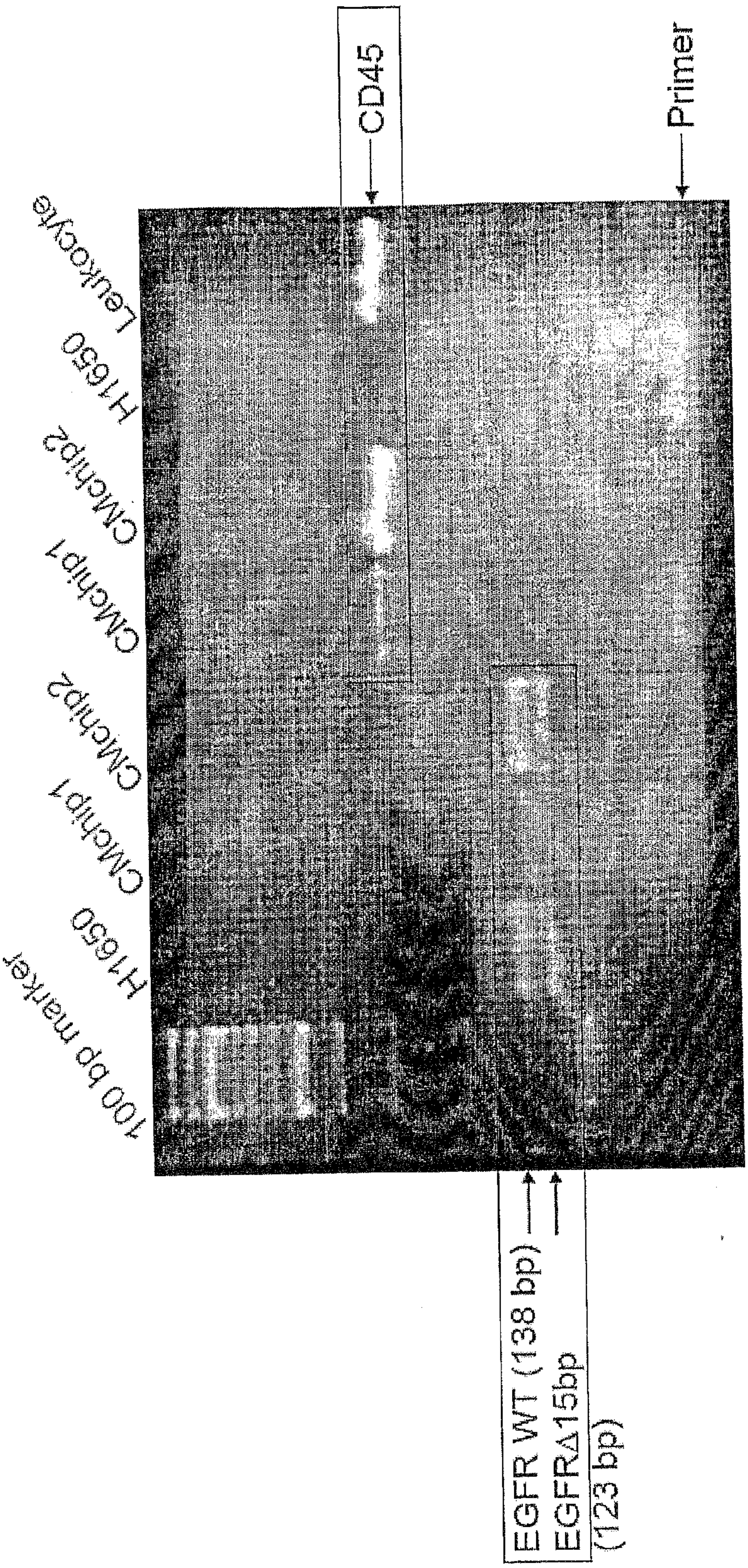
100 bp marker
H1650
CMchip1
CMchip2
CMchip1
CMchip2
H1650
Leukocyte
CD45
Primer
EGFR WT (138 bp)
EGFRΔ15bp (123 bp)

Fig. 65

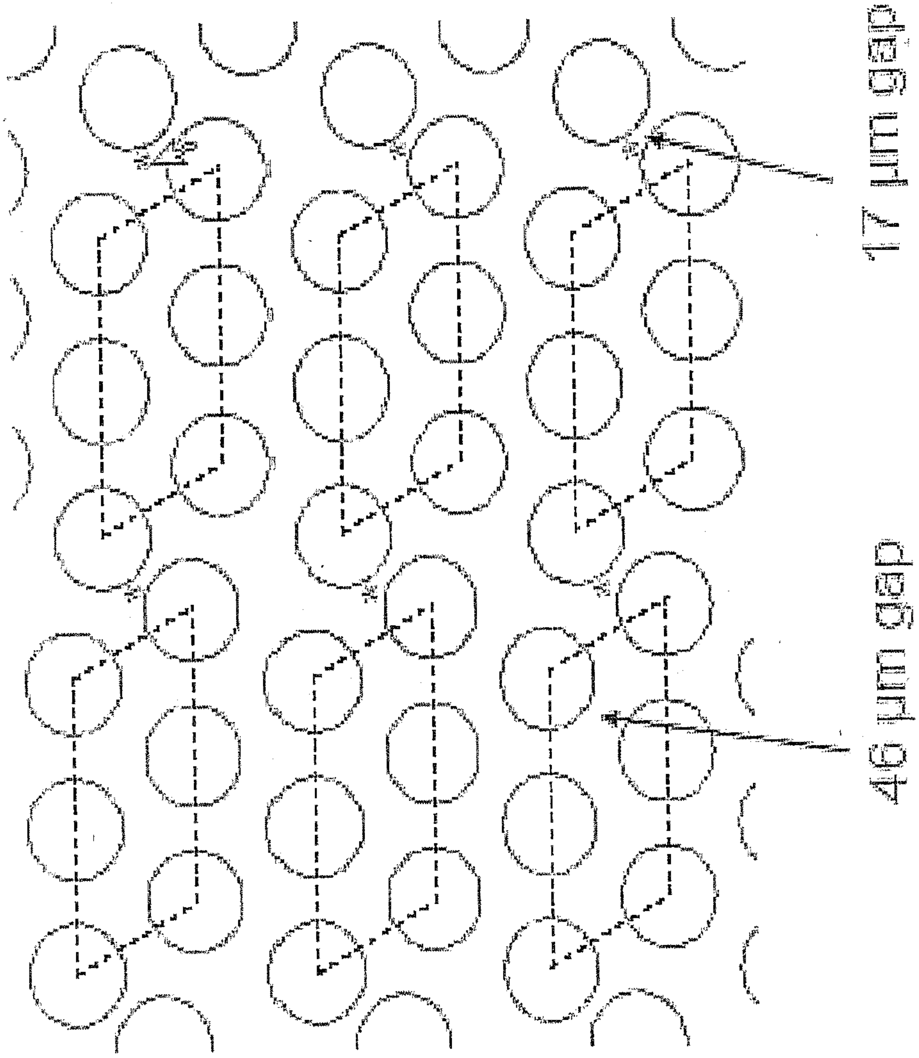


Fig. 66A

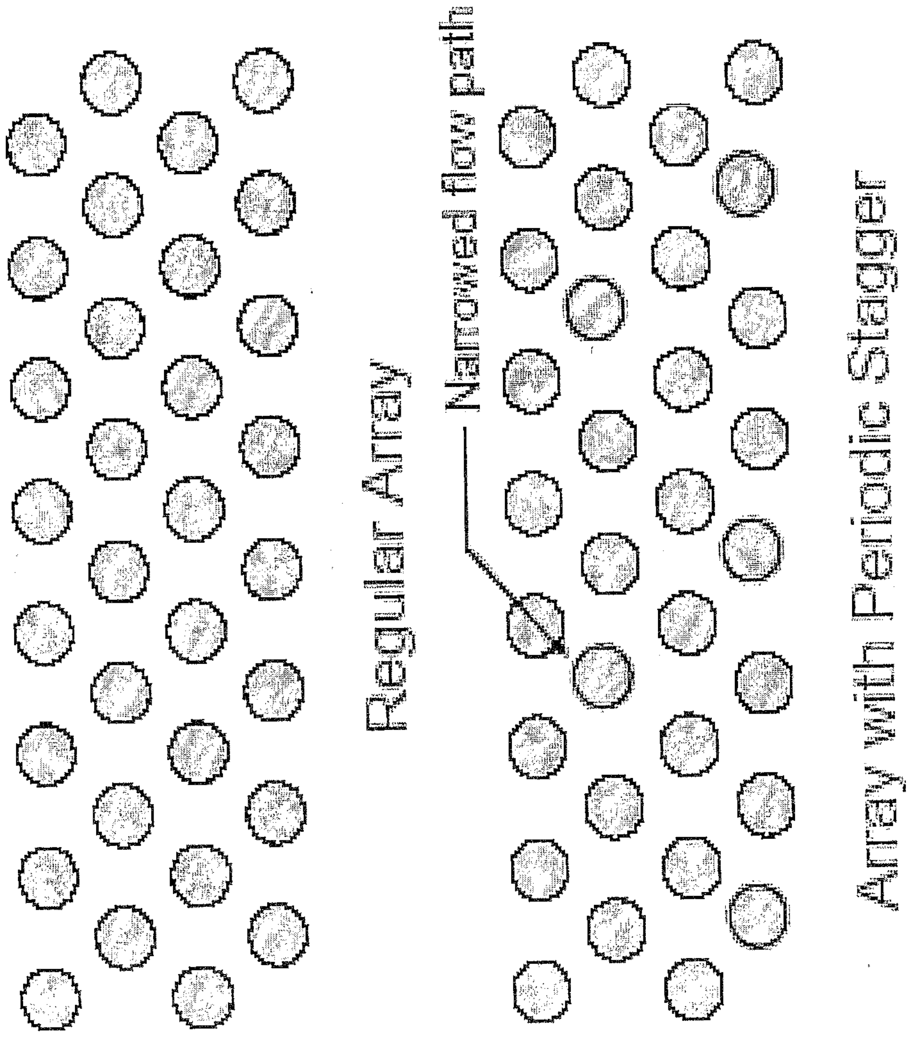
Regular Array
Narrowed flow path
Array with Periodic Stagger

Fig. 66B

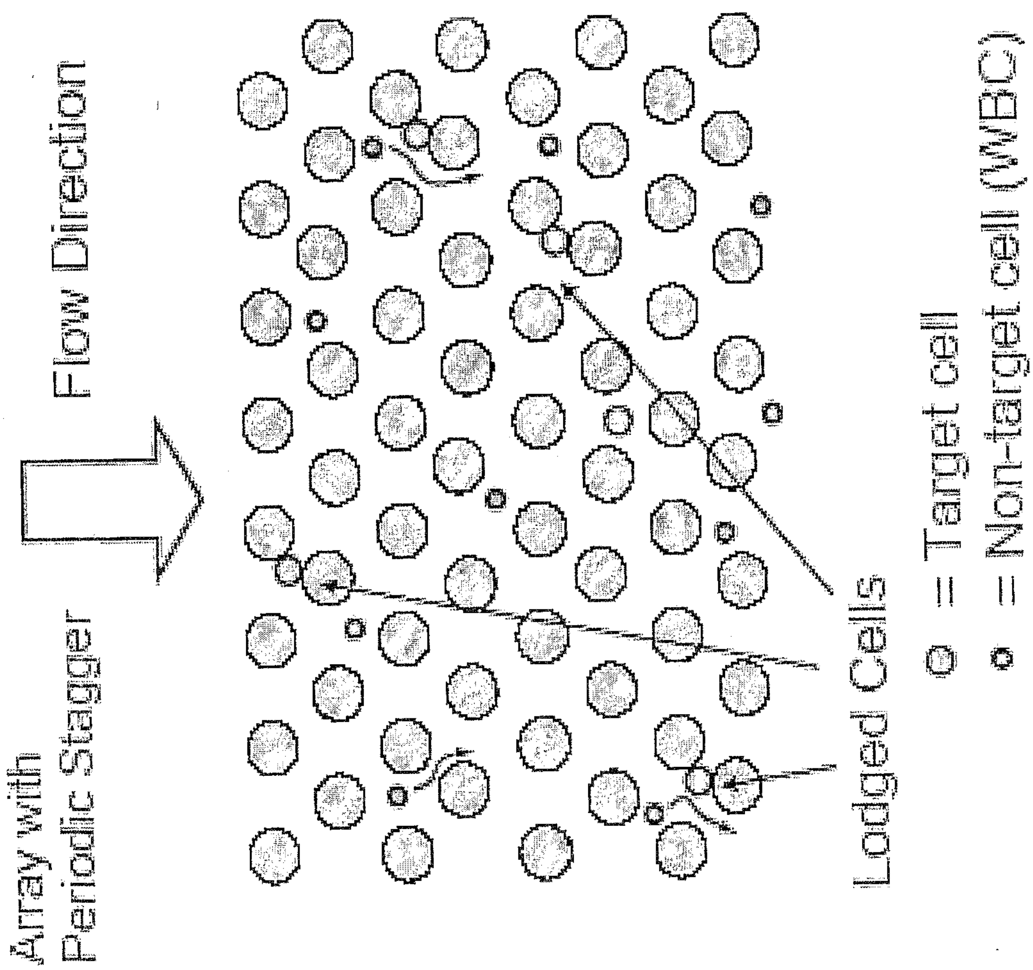
Array with
Periodic Stagger
Flow Direction
Lodged Cells
= Target cell
= Non-target cell (WBC)

Fig. 66C

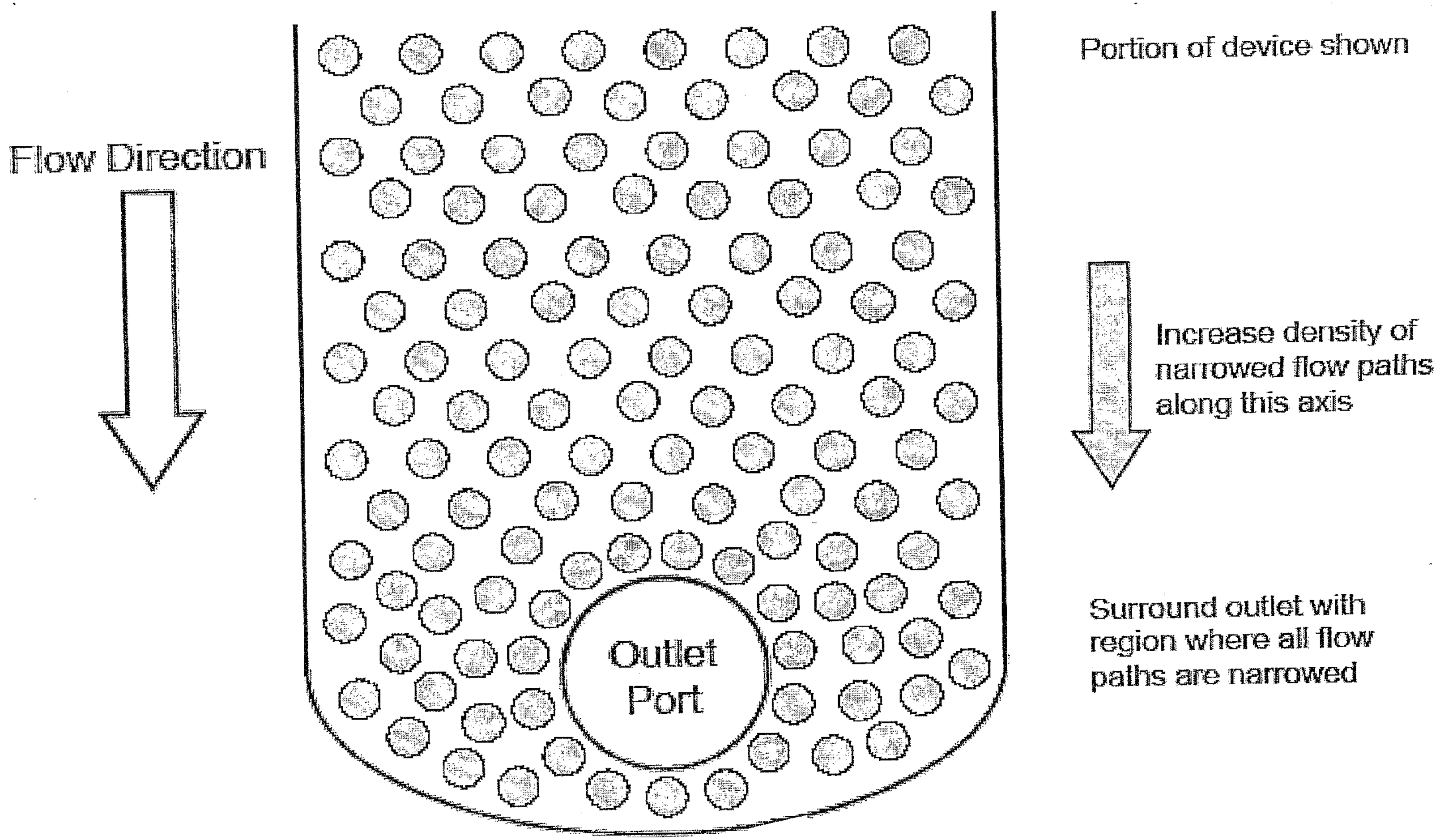
Flow Direction
Portion of device shown
Increase density of narrowed flow paths along this axis
Outlet Port
Surround outlet with region where all flow paths are narrowed

Fig. 66D

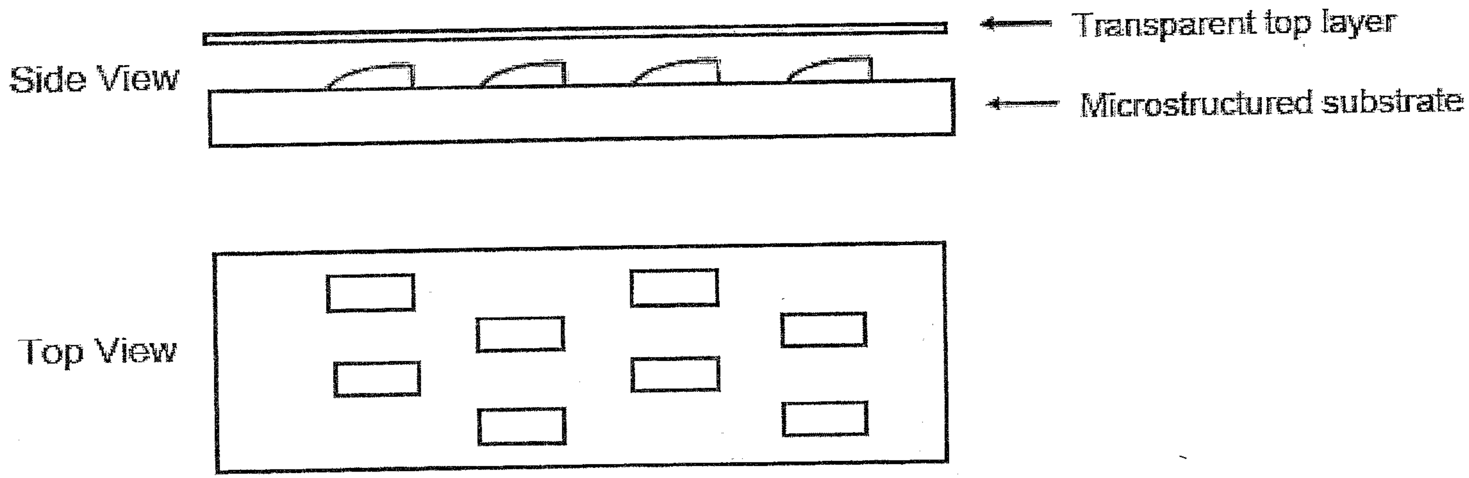


This device is structured in the depth dimension to create narrowed flow paths

Fig. 66E

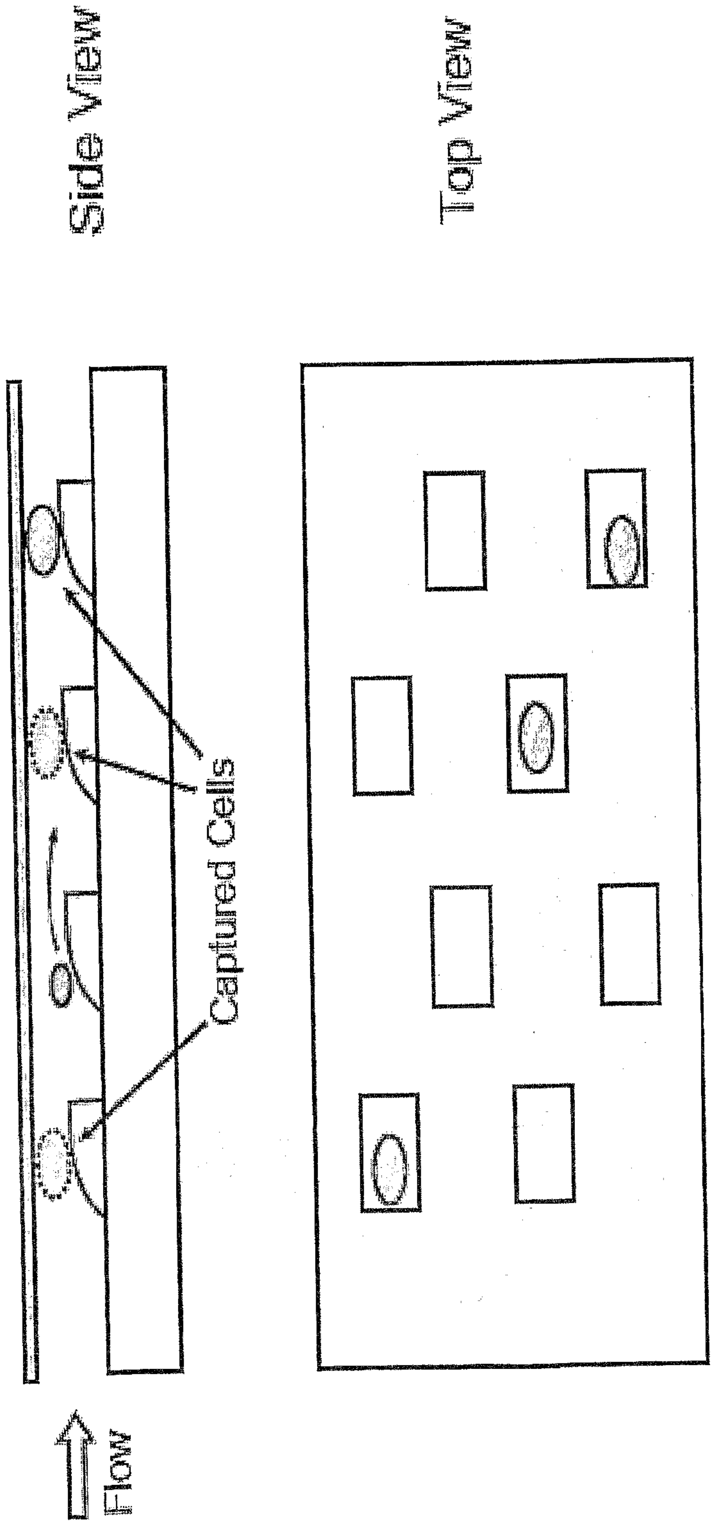


Fig. 66F

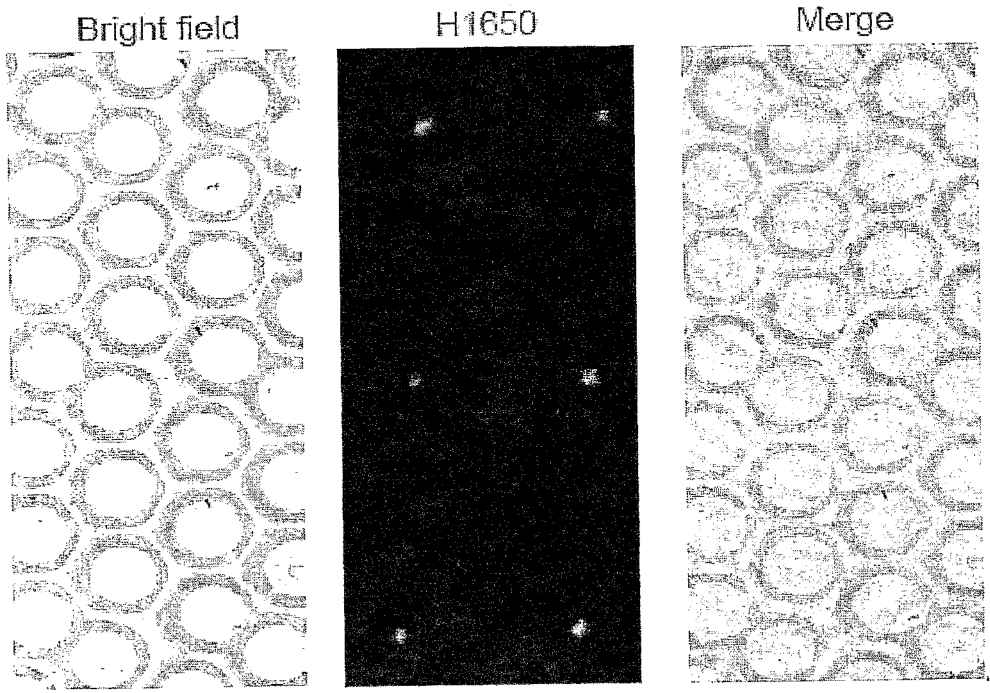


CM#7168 (type 7), H1650/ WBC, captufed cell# 1,079 x 2= 2,158

Each image is a composite of created from three adjacent microscope fields of view

Fig. 66G

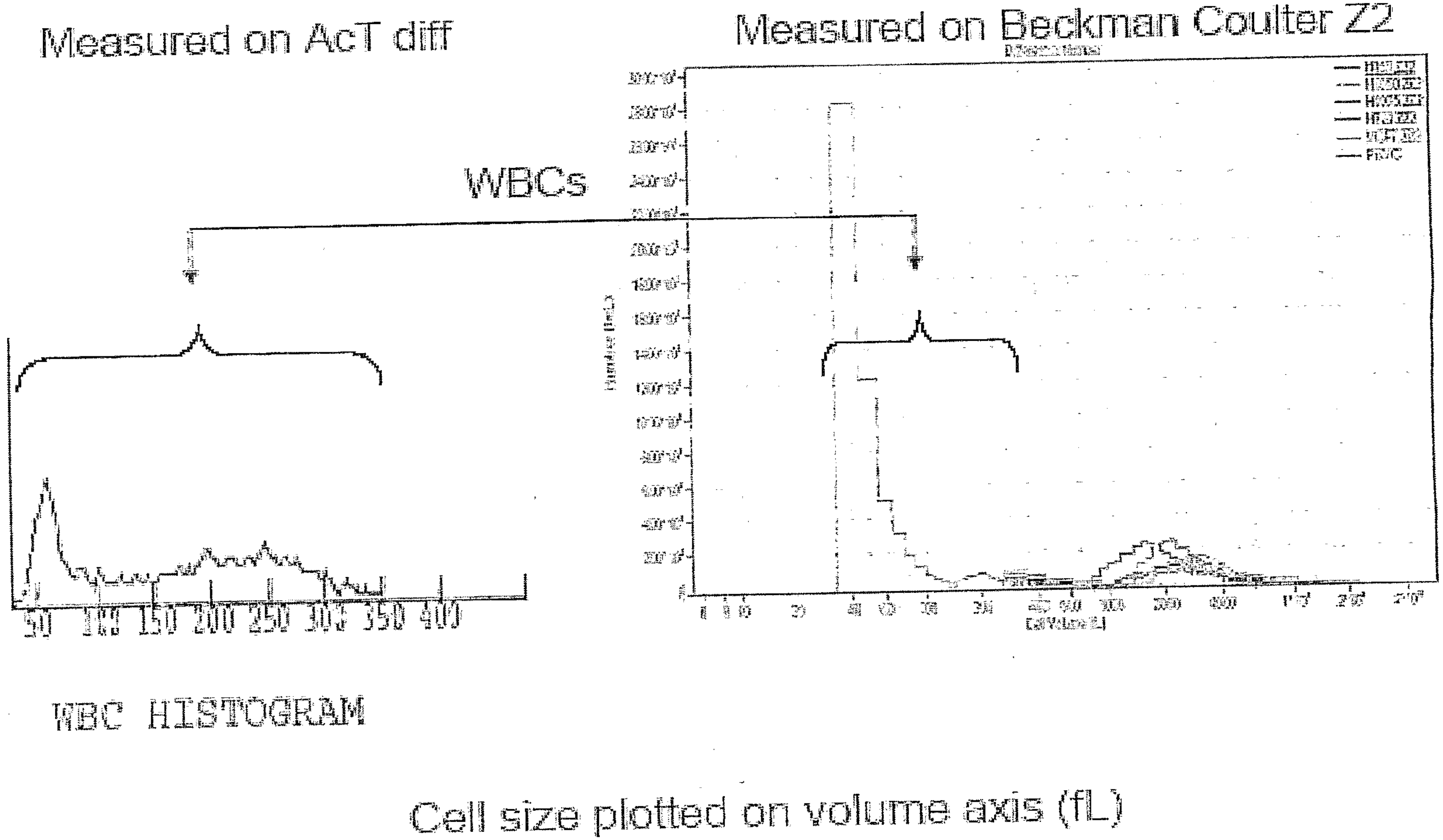
Measured on AcT diff
Measured on Beckman Coulter Z2
WBCs
50 100 150 200 250 300 350 400
WBC HISTOGRAM
Cell size plotted on volume axis (fL)

Fig. 67A

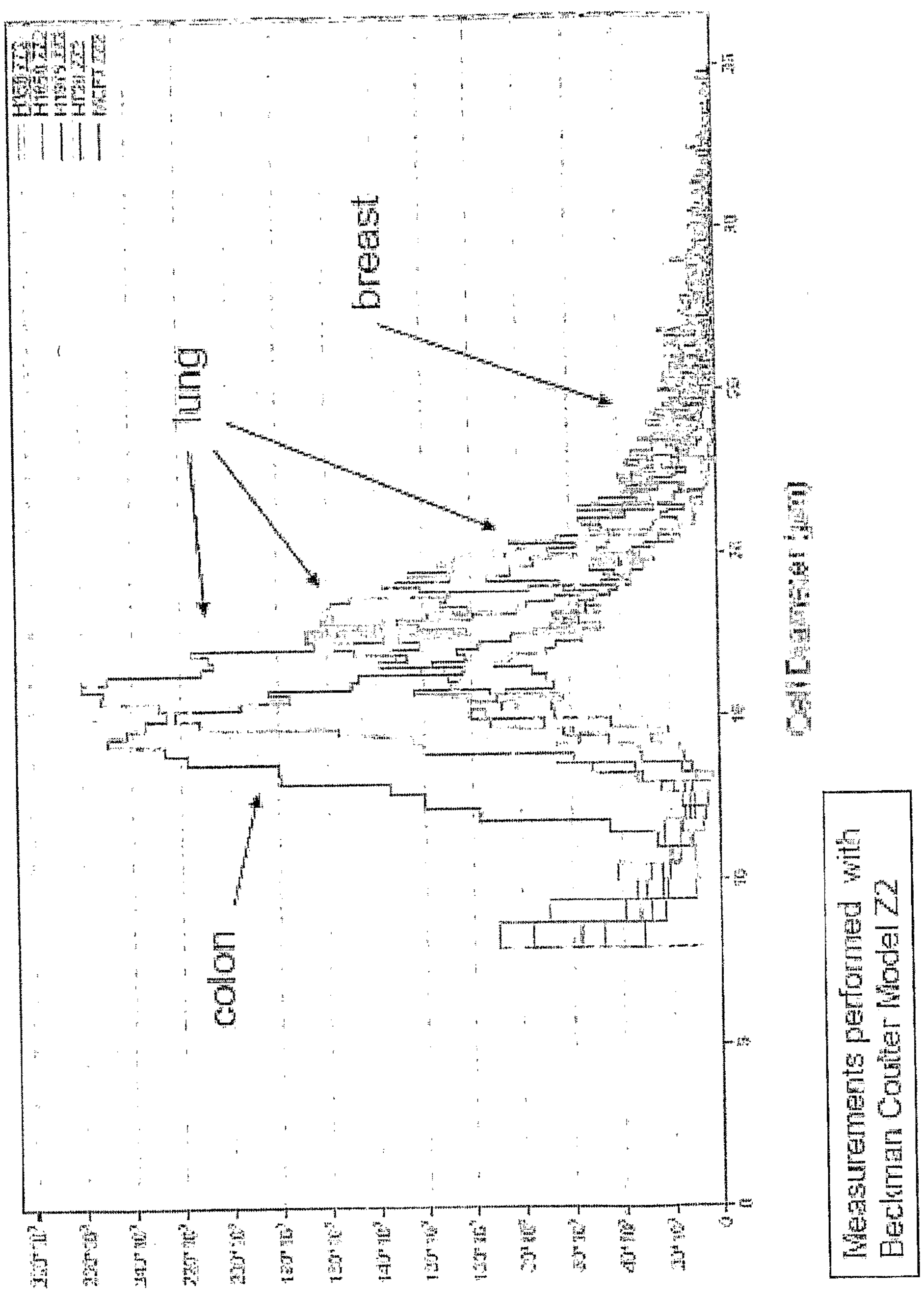
colon
lung
breast
Measurements performed with
Beckman Coulter Model Z2

Fig. 67B

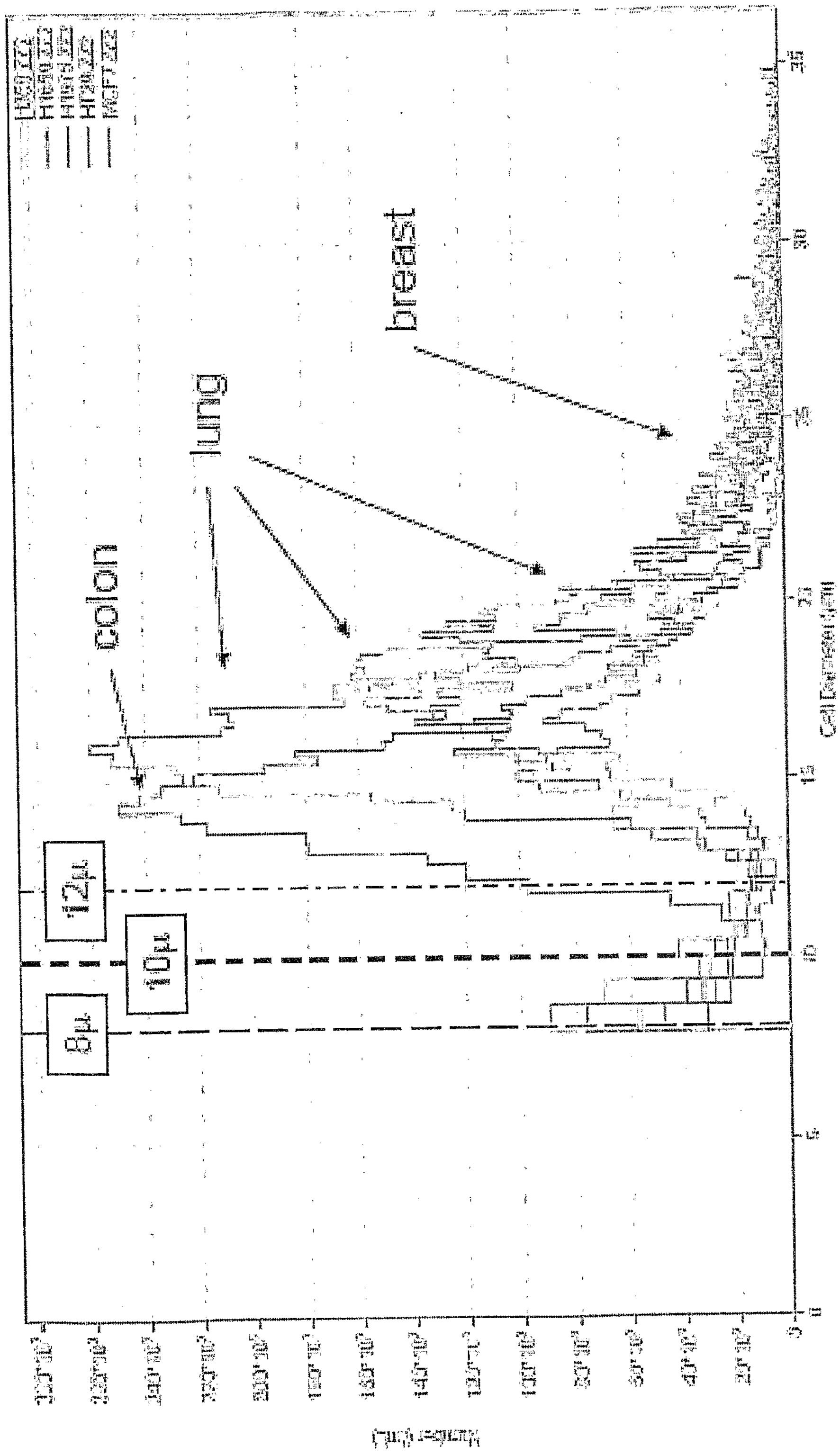


Fig. 67C

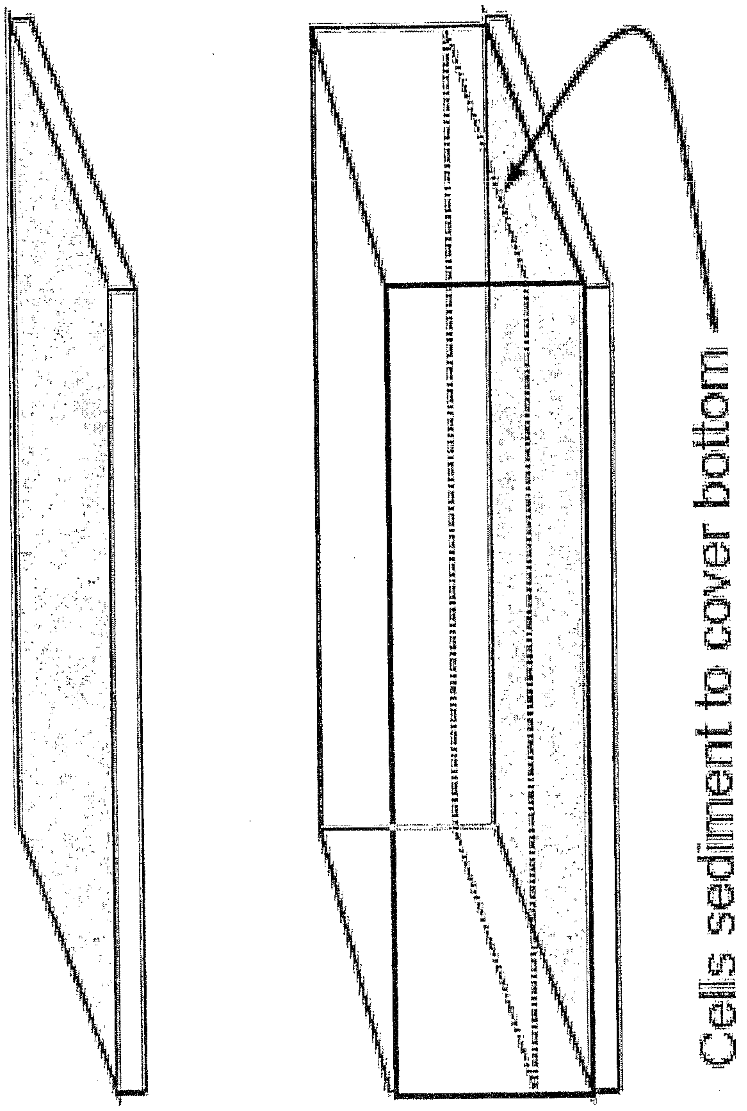


Fig. 68A

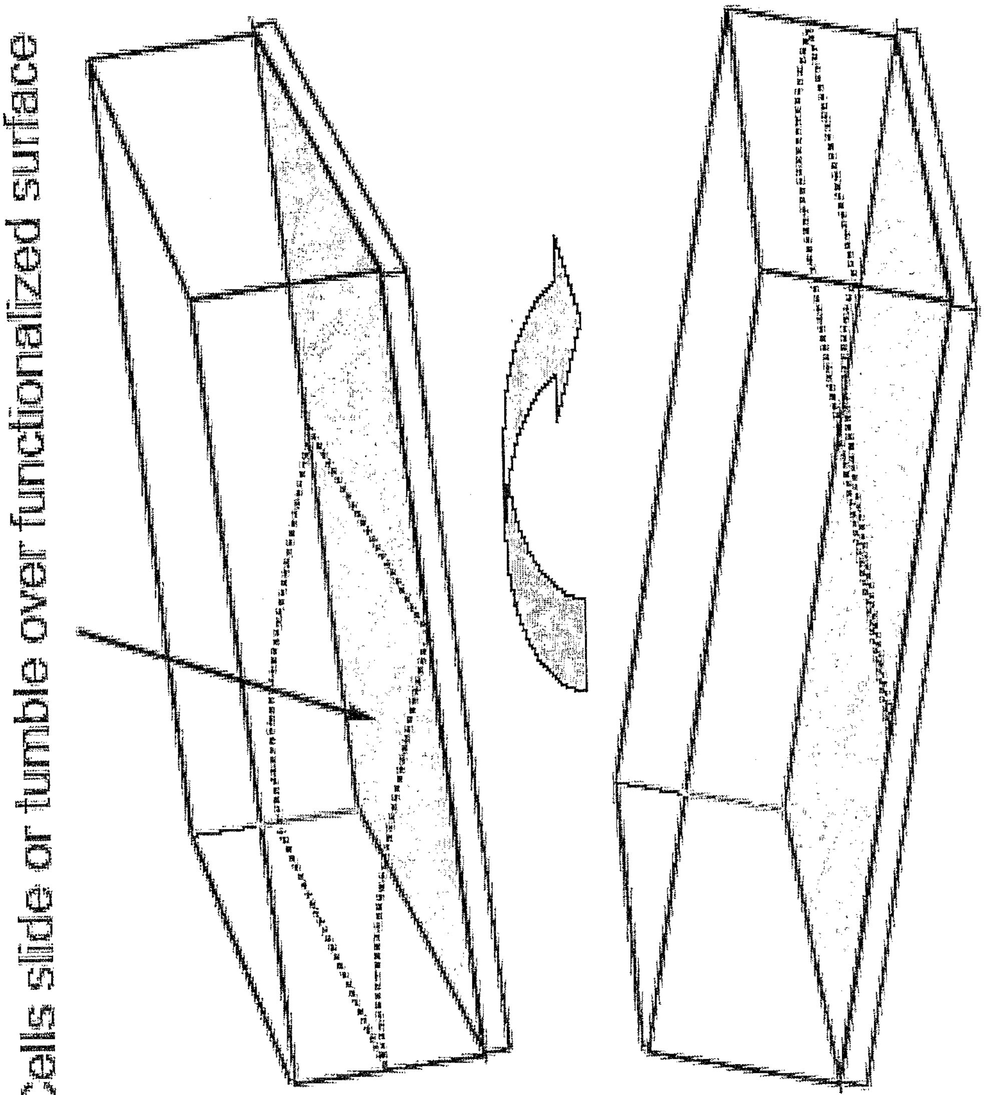


Fig. 68B

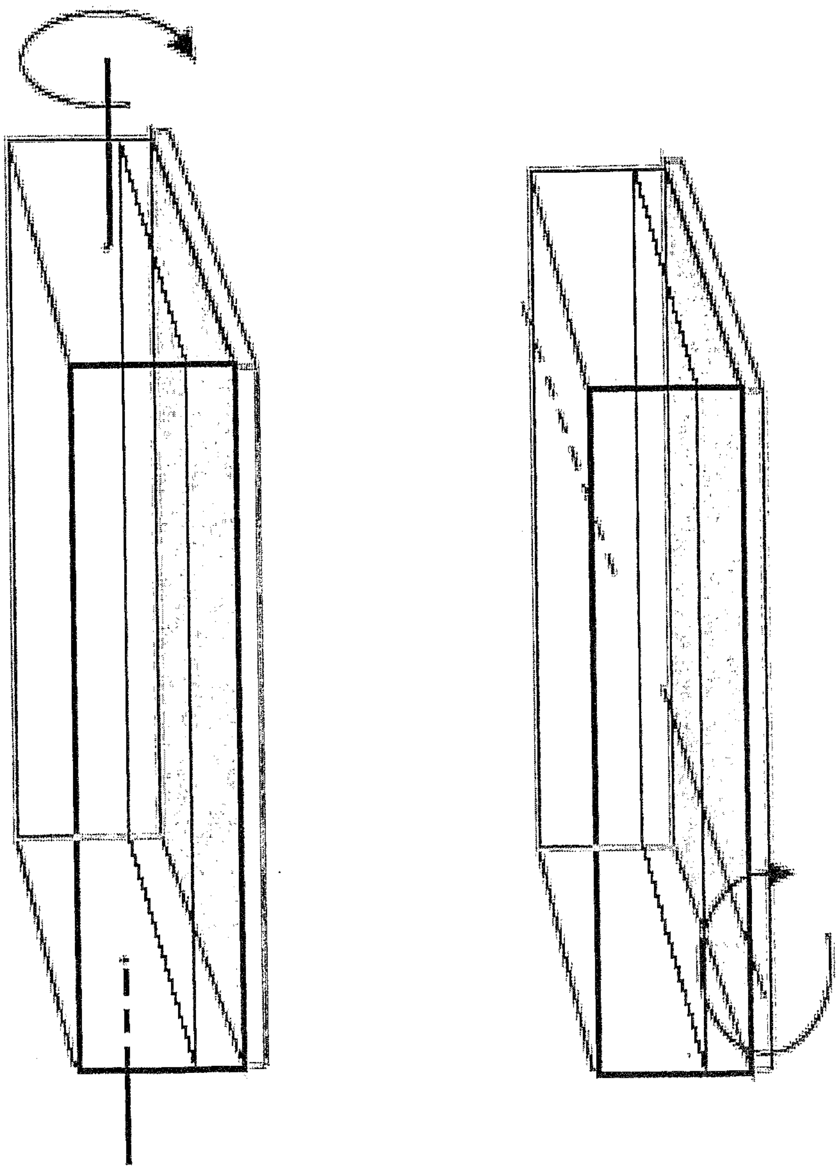

Fig. 68C

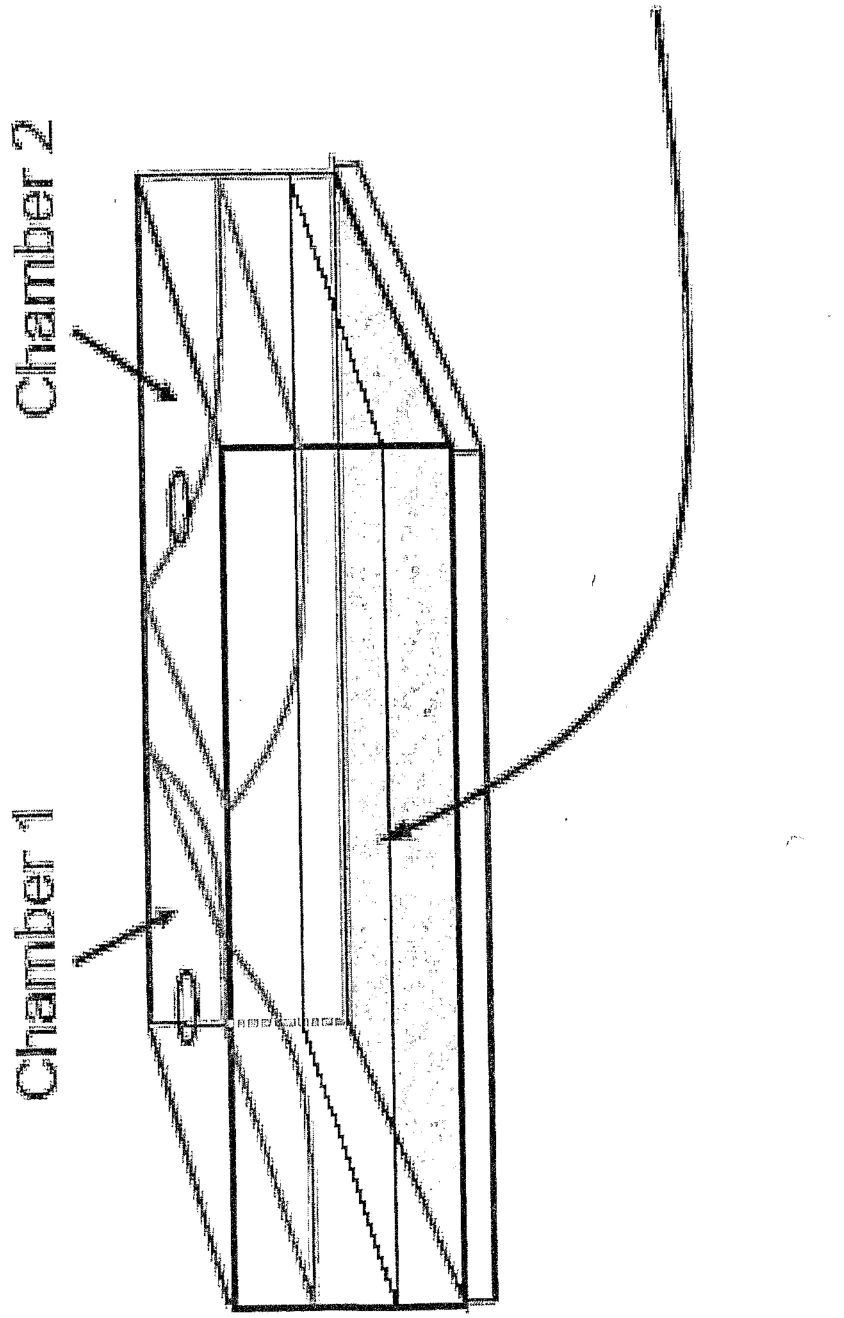


Fig. 68D

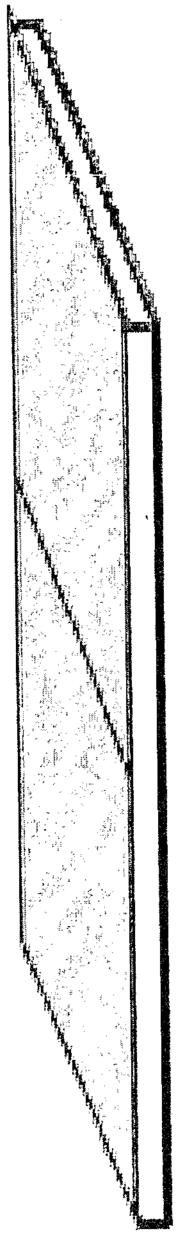

Fig. 68E

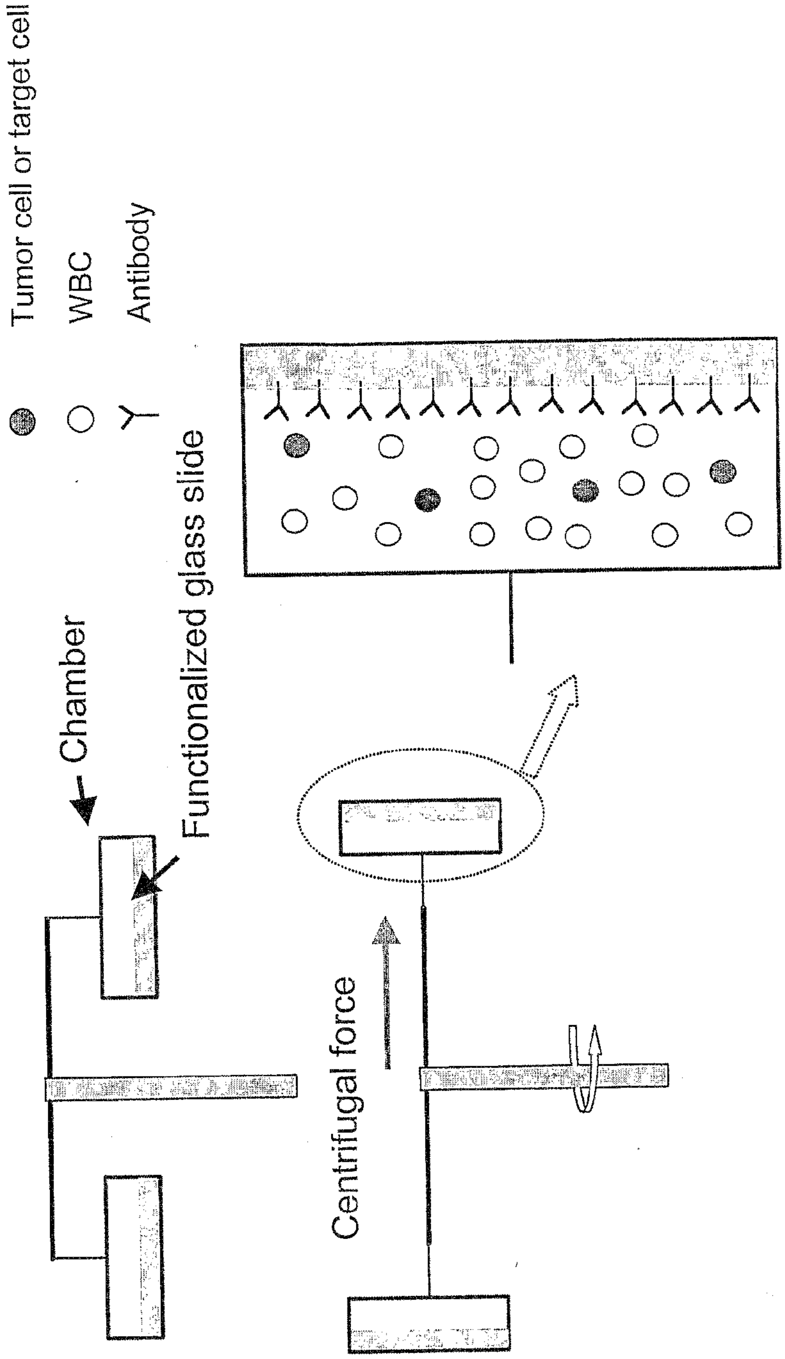
Tumor cell or target cell
WBC
Antibody
Chamber
Functionalized glass slide
Centrifugal force

Fig. 69A

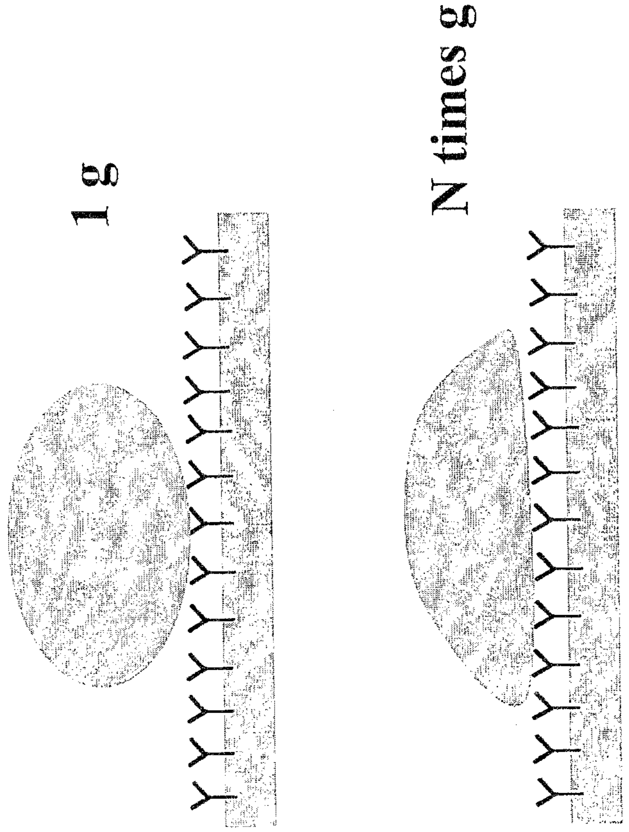


Fig. 69B

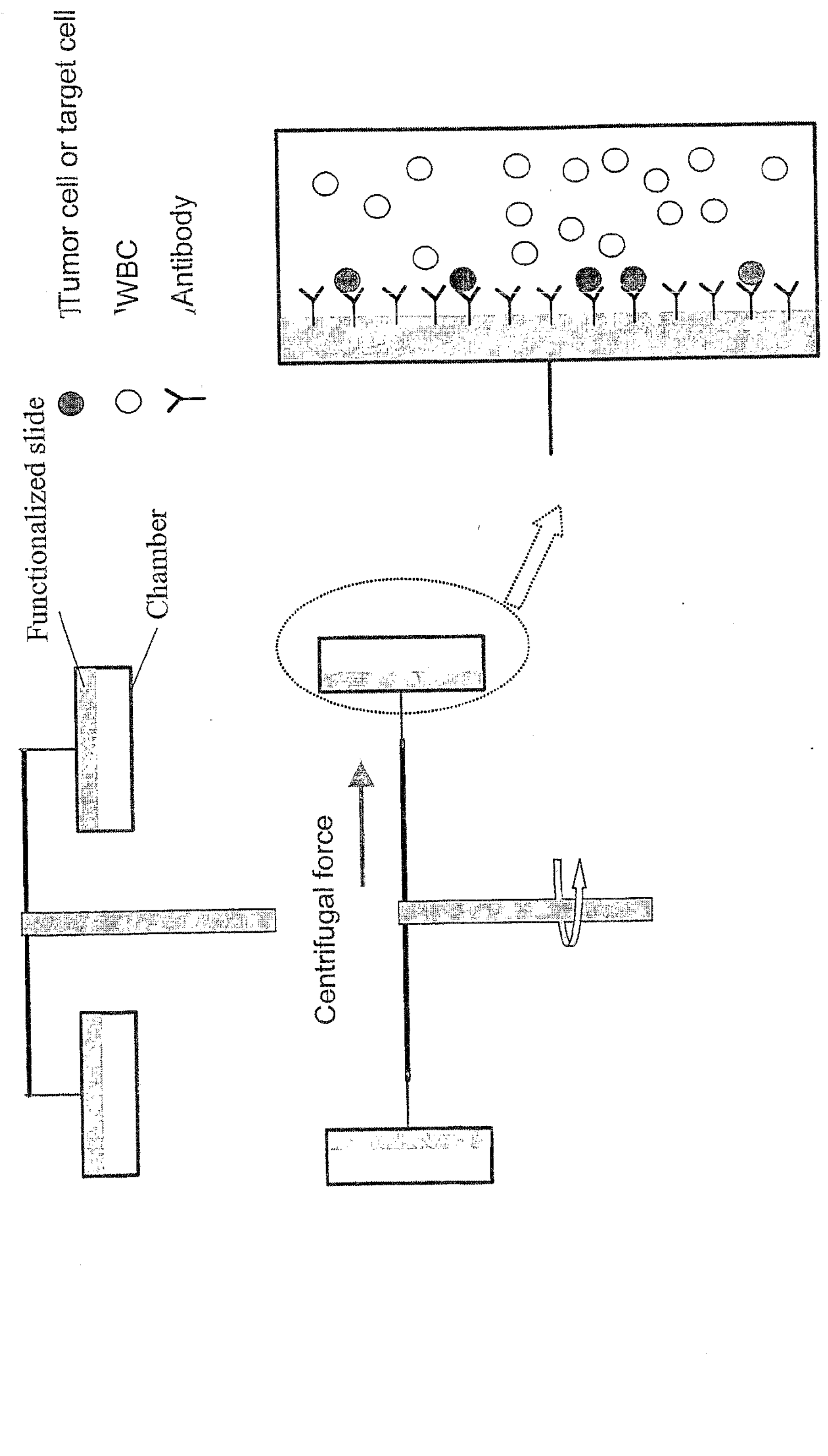
Functionalized slide
Chamber
Tumor cell or target cell
WBC
Antibody
Centrifugal force

Fig. 69C

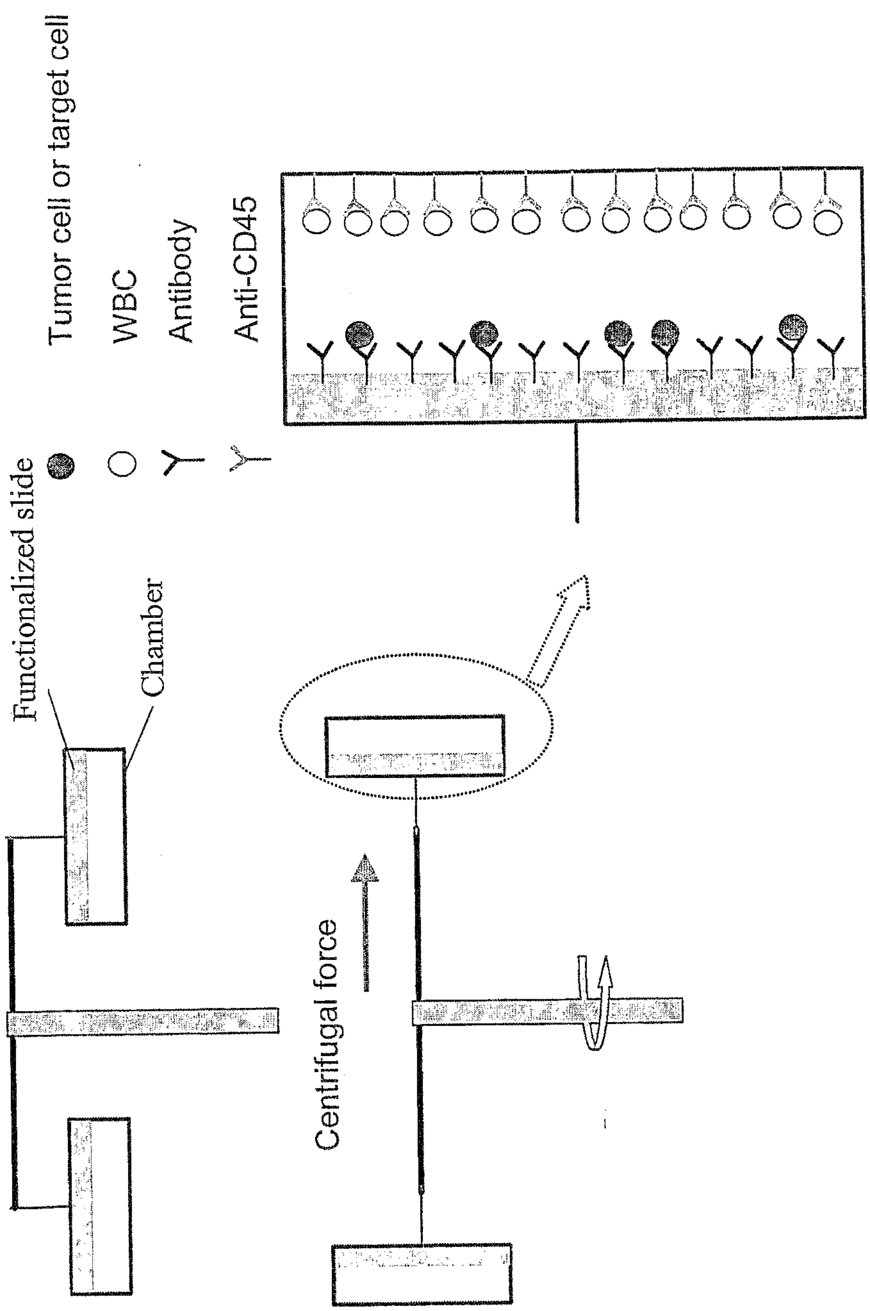
Functionalized slide
Chamber
Centrifugal force
Tumor cell or target cell
WBC
Antibody
Anti-CD45

Fig. 69D

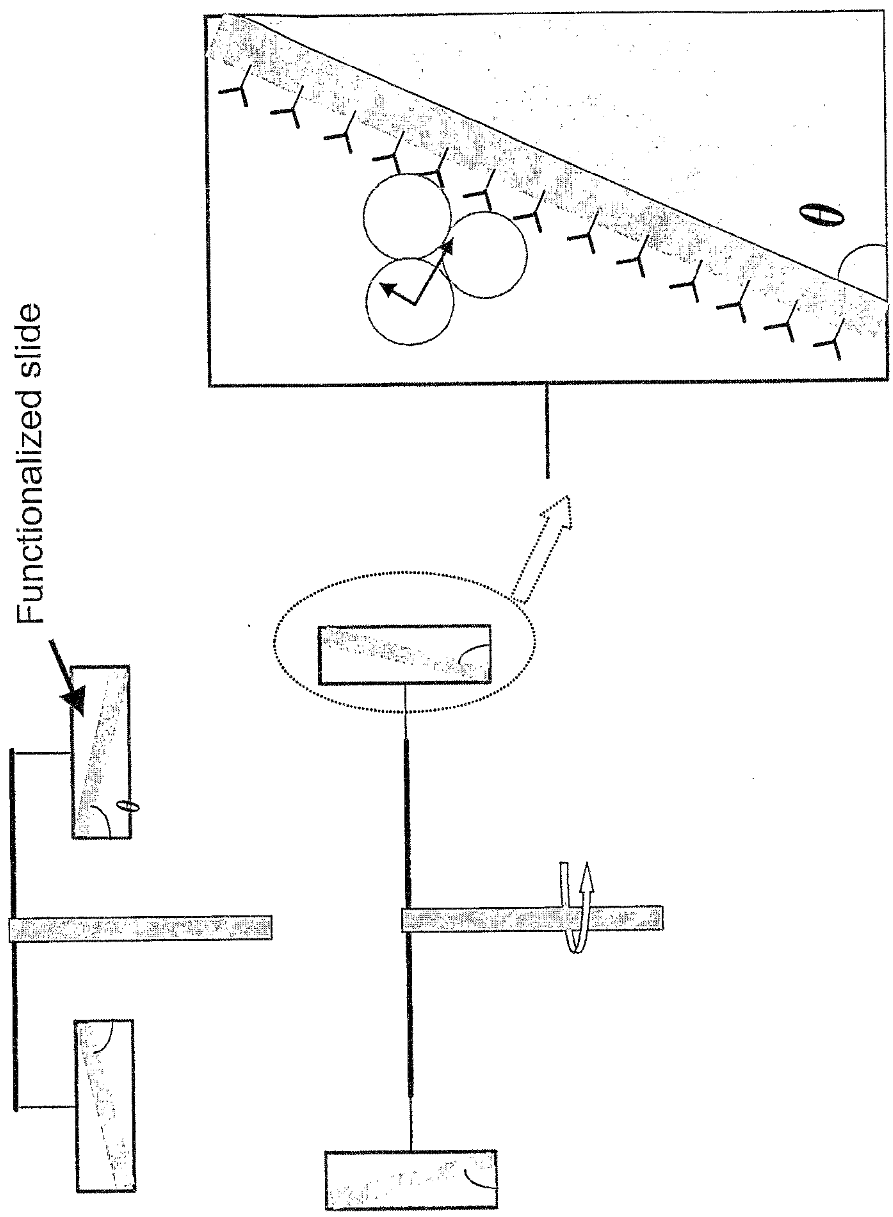


Fig. 69E

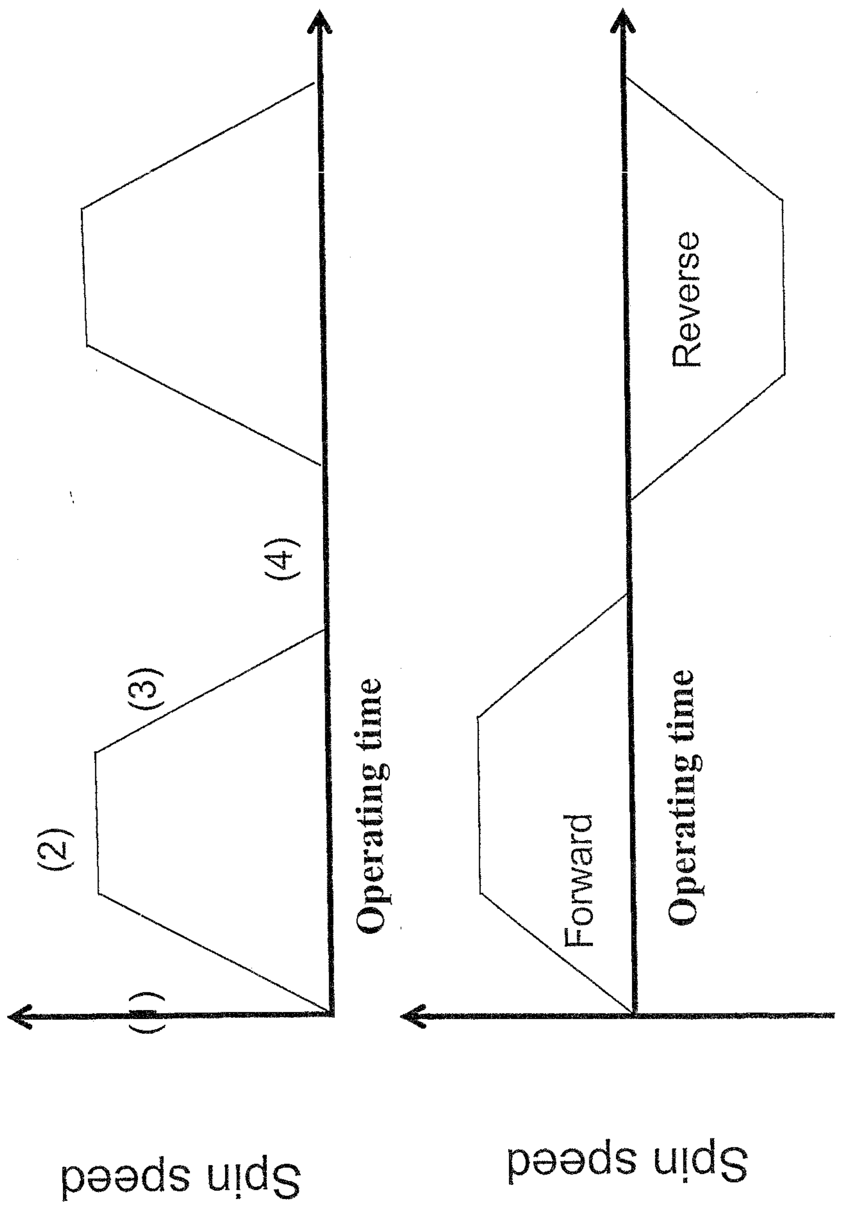
Spin speed
Operating time
(1)
(2)
(3)
(4)
Spin speed
Operating time
Forward
Reverse

Fig. 69F

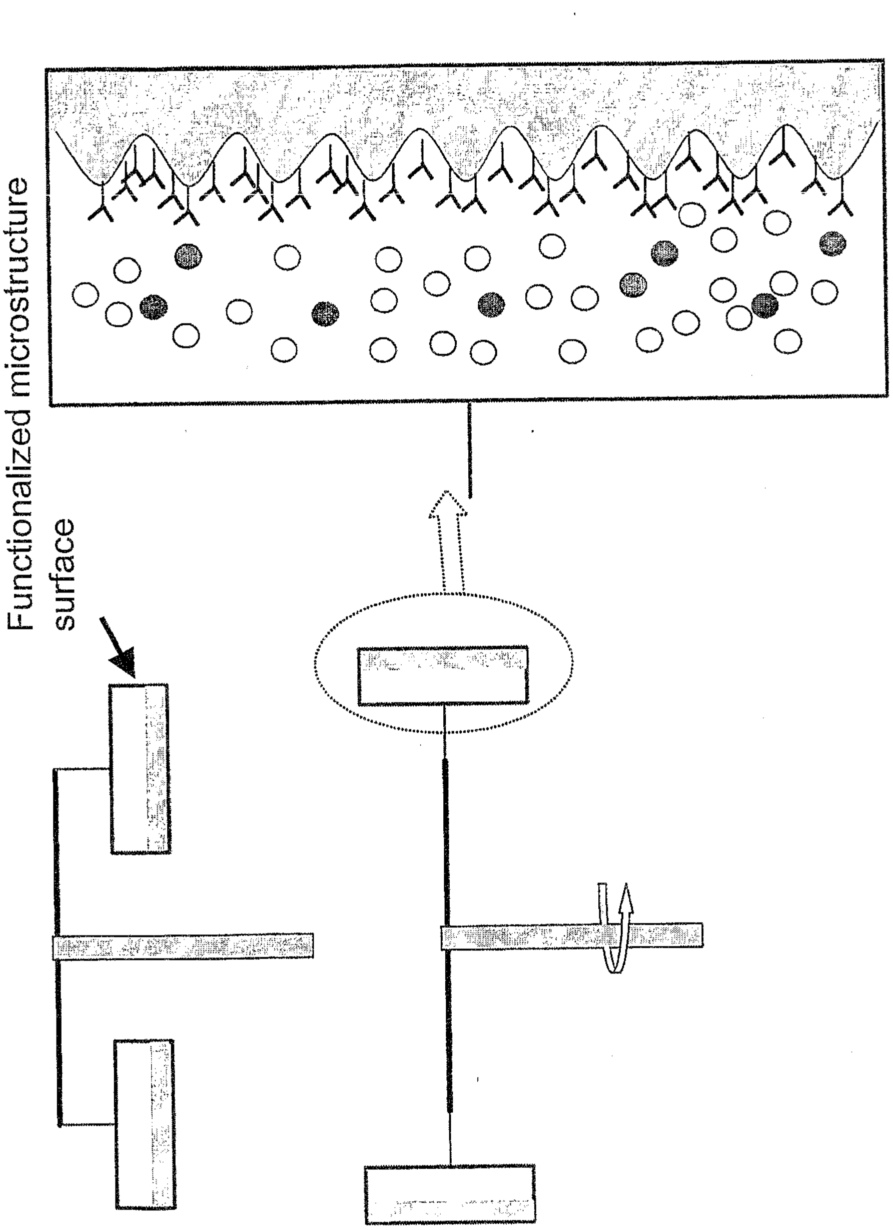


Fig. 69G

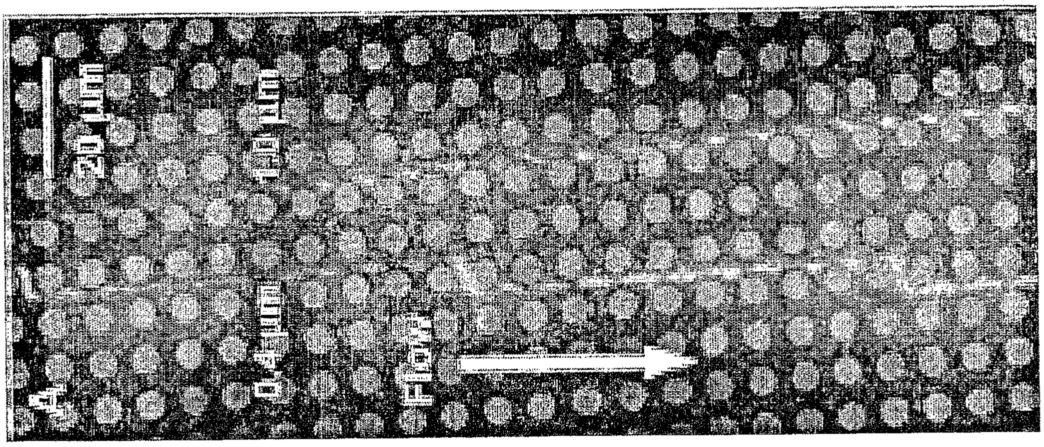


Fig. 70A

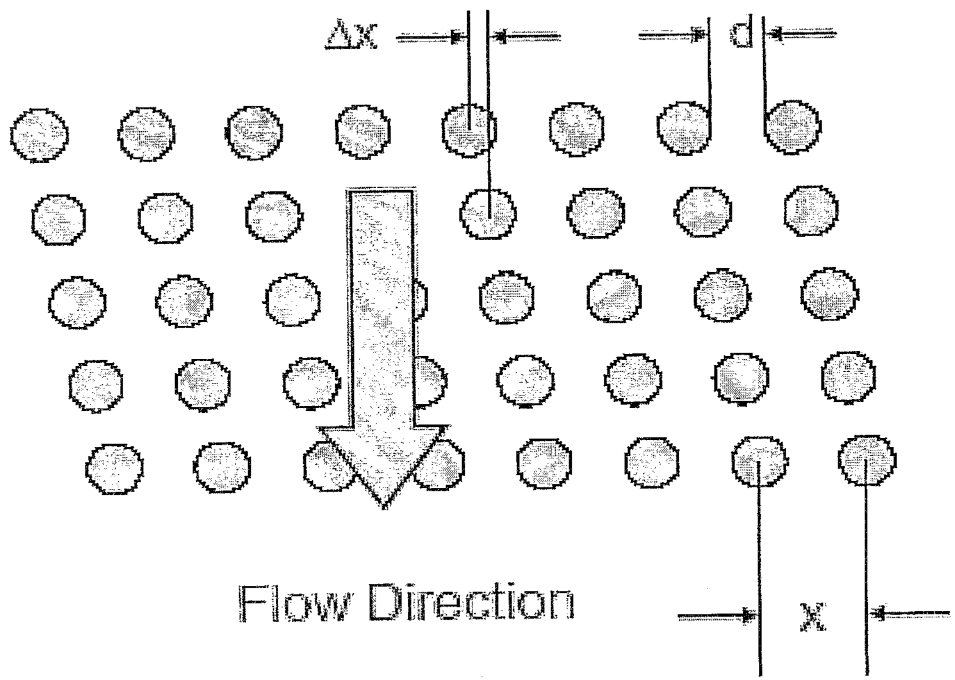


$\varepsilon = \Delta x / x$ $D_c = 2 f d \varepsilon$ $D_c$ is the critical diameter

f is a factor to account for parabolic velocity distribution and can be calculated from a fluids model

Fig. 70B

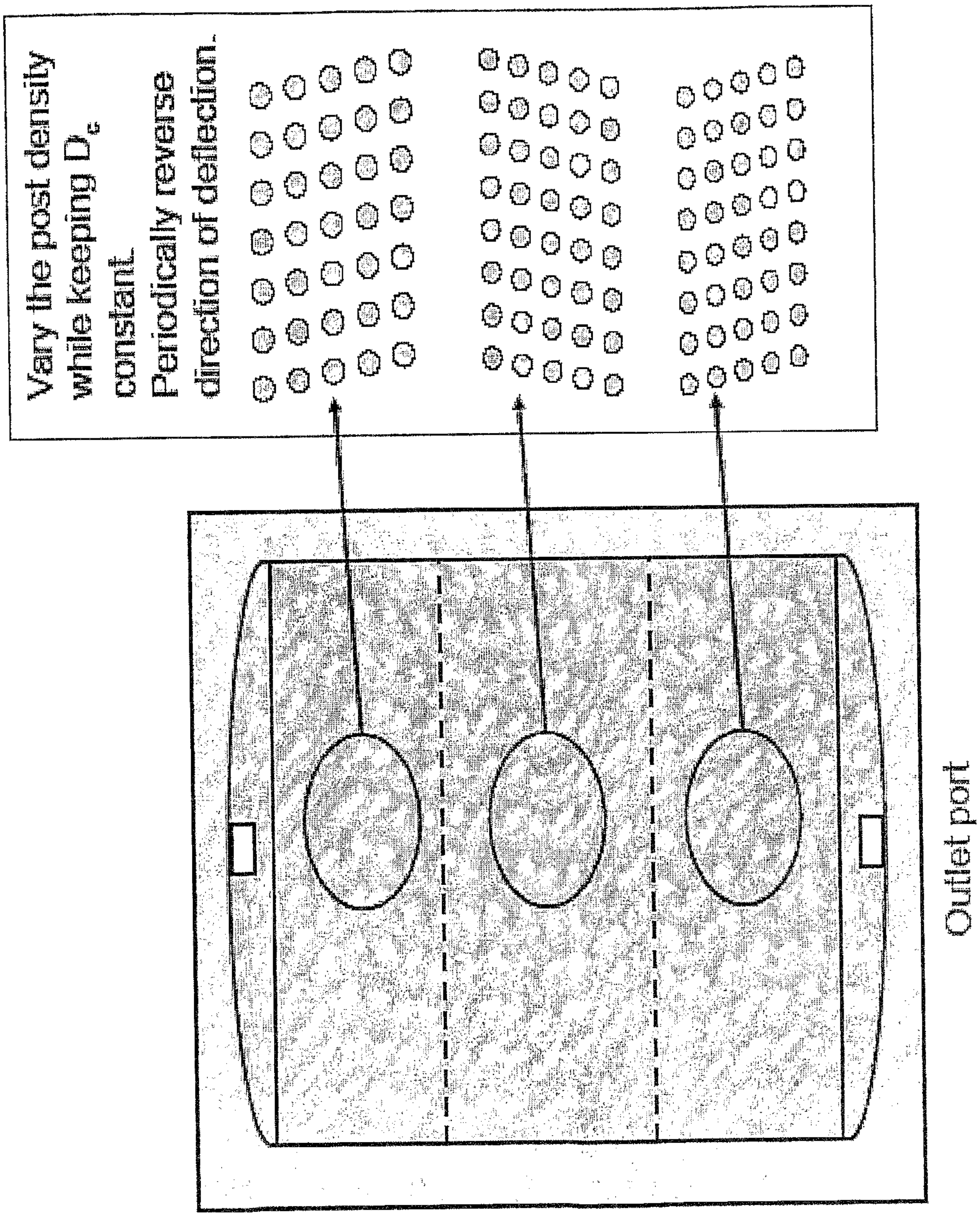
Vary the post density
while keeping $D_c$
constant.
Periodically reverse
direction of deflection.
Outlet port

Fig. 70C

Fig. 71 (1/4)

```
CCCGCCGCAGCGCGGCCGCAGCAGCCTCCGCCCCCCGCACGGTCTCAGCGCCCGACGCCG -185
............................................................
CCGAGGCGGCCGGAGTCCCGAGCTAGCCCCGGCGGCCGCCGCCGCCCAGACCGGACGACA -125
............................................................
GGCCACCTCGTCGGCGTCCGCCCGAGTCCCCGCCTCGCCGCCAACGCCACAACCACCGCG -65
............................................................
CACGGCCCCCTGACTCCGTCCAGTATTGATCGGGAGAGCCGGAGCGAGCTCTTCGGGGAG -5
............................................................
CAGCGATGCGACCCTCCGGGACGGCCGGGGCAGCGCTCCTGGCGCTGCTGGCTGCGCTCT 55
......-M--R--P--S--G--T--A--G--A--A--L--L--A--L--L--A--A--L-- 18
GCCCGGCGAGTCGGGCTCTGGAGGAAAAGAAAGTTTGCCAAGGCACGAGTAACAAGCTCA 115
C--P--A--S--R--A--L--E--E--K--K--V--C--Q--G--T--S--N--K--L-- 38
CGCAGTTGGGCACTTTTGAAGATCATTTTCTCAGCCTCCAGAGGATGTTCAATAACTGTG 175
T--Q--L--G--T--F--E--D--H--F--L--S--L--Q--R--M--F--N--N--C-- 58
AGGTGGTCCTTGGGAATTTGGAAATTACCTATGTGCAGAGGAATTATGATCTTTCCTTCT 235
E--V--V--L--G--N--L--E--I--T--Y--V--Q--R--N--Y--D--L--S--F-- 78
TAAAGACCATCCAGGAGGTGGCTGGTTATGTCCTCATTGCCCTCAACACAGTGGAGCGAA 295
L--K--T--I--Q--E--V--A--G--Y--V--L--I--A--L--N--T--V--E--R-- 98
TTCCTTTGGAAAACCTGCAGATCATCAGAGGAAATATGTACTACGAAAATTCCTATGCCT 355
I--P--L--E--N--L--Q--I--I--R--G--N--M--Y--Y--E--N--S--Y--A-- 118
TAGCAGTCTTATCTAACTATGATGCAAATAAAACCGGACTGAAGGAGCTGCCCATGAGAA 415
L--A--V--L--S--N--Y--D--A--N--K--T--G--L--K--E--L--P--M--R-- 138
ATTTACAGGAAATCCTGCATGGCGCCGTGCGGTTCAGCAACAACCCTGCCCTGTGCAACG 475
N--L--Q--E--I--L--H--G--A--V--R--F--S--N--N--P--A--L--C--N-- 158
TGGAGAGCATCCAGTGGCGGGACATAGTCAGCAGTGACTTTCTCAGCAACATGTCGATGG 535
V--E--S--I--Q--W--R--D--I--V--S--S--D--F--L--S--N--M--S--M-- 178
ACTTCCAGAACCACCTGGGCAGCTGCCAAAAGTGTGATCCAAGCTGTCCCAATGGGAGCT 595
D--F--Q--N--H--L--G--S--C--Q--K--C--D--P--S--C--P--N--G--S-- 198
GCTGGGGTGCAGGAGAGGAGAACTGCCAGAAACTGACCAAAATCATCTGTGCCCAGCAGT 655
C--W--G--A--G--E--E--N--C--Q--K--L--T--K--I--I--C--A--Q--Q-- 218
GCTCCGGGCGCTGCCGTGGCAAGTCCCCCAGTGACTGCTGCCACAACCAGTGTGCTGCAG 715
C--S--G  -R--C--R--G--K--S--P--S--D--C--C--H--N--Q--C--A--A-- 238
GCTGCACAGGCCCCCGGGAGAGCGACTGCCTGGTCTGCCGCAAATTCCGAGACGAAGCCA 775
G--C--T--G--P--R--E--S--D--C--L--V--C--R--K--F--R--D--E--A-- 258
CGTGCAAGGACACCTGCCCCCCACTCATGCTCTACAACCCCACCACGTACCAGATGGATG 835
T--C--K--D--T--C--P--P--L--M--L--Y--N--P--T--T--Y--Q--M--D-- 278
TGAACCCCGAGGGCAAATACAGCTTTGGTGCCACCTGCGTGAAGAAGTGTCCCCGTAATT 895
V--N--P--E--G--K--Y--S--F--G--A--T--C--V--K--K--C--P--R--N-- 298
```

ATGTGGTGACAGATCACGGCTCGTGCGTCCGAGCCTGTGGGGCCGACAGCTATGAGATGG 955
Y--V--V--T--D--H--G--S--C--V--R--A--C--G--A--D--S--Y--E--M-- 318

AGGAAGACGGCGTCCGCAAGTGTAAGAAGTGCGAAGGGCCTTGCCGCAAAGTGTGTAACG 1015
E--E--D--G--V--R--K--C--K--K--C--E--G--P--C--R--K--V--C--N-- 338

GAATAGGTATTGGTGAATTTAAAGACTCACTCTCCATAAATGCTACGAATATTAAACACT 1075
G--I--G--I--G--E--F--K--D--S--L--S--I--N--A--T--N--I--K--H-- 358

TCAAAAACTGCACCTCCATCAGTGGCGATCTCCACATCCTGCCGGTGGCATTTAGGGGTG 1135
F--K--N--C--T--S--I--S--G--D--L--H--I--L--P--V--A--F--R--G-- 378

ACTCCTTCACACATACTCCTCCTCTGGATCCACAGGAACTGGATATTCTGAAAACCGTAA 1195
D--S--F--T--H--T--P--P--L--D--P--Q--E--L--D--I--L--K--T--V-- 398

AGGAAATCACAGGGTTTTTGCTGATTCAGGCTTGGCCTGAAAACAGGACCGACCTCCATG 1255
K--E--I--T--G--F--L--L--I--Q--A--W--P--E--N--R--T--D--L--H-- 418

CCTTTGAGAACCTAGAAATCATACGCGGCAGGACCAAGCAACATGGTCAGTTTTCTCTTG 1315
A--F--E--N--L--E--I--I--R--G--R--T--K--Q--H--G--Q--F--S--L-- 438

CAGTCGTCAGCCTGAACATAACATCCTTGGGATTACGCTCCCTCAAGGAGATAAGTGATG 1375
A--V--V--S--L--N--I--T--S--L--G--L--R--S--L--K--E--I--S--D-- 458

GAGATGTGATAATTTCAGGAAACAAAAATTTGTGCTATGCAAATACAATAAACTGGAAAA 1435
G--D--V--I--I--S--G--N--K--N--L--C--Y--A--N--T--I--N--W--K-- 478

AACTGTTTGGGACCTCCGGTCAGAAAACCAAAATTATAAGCAACAGAGGTGAAAACAGCT 1495
K--L--F--G--T--S--G--Q--K--T--K--I--I--S--N--R--G--E--N--S-- 498

GCAAGGCCACAGGCCAGGTCTGCCATGCCTTGTGCTCCCCCGAGGGCTGCTGGGGCCCGG 1555
C--K--A--T--G--Q--V--C--H--A--L--C--S--P--E--G--C--W--G--P-- 518

AGCCCAGGGACTGCGTCTCTTGCCGGAATGTCAGCCGAGGCAGGGAATGCGTGGACAAGT 1615
E--P--R--D--C--V--S--C--R--N--V--S--R--G--R--E--C--V--D--K-- 538

GCAACCTTCTGGAGGGTGAGCCAAGGGAGTTTGTGGAGAACTCTGAGTGCATACAGTGCC 1675
C--N--L--L--E--G--E--P--R--E--F--V--E--N--S--E--C--I--Q--C-- 558

ACCCAGAGTGCCTGCCTCAGGCCATGAACATCACCTGCACAGGACGGGGACCAGACAACT 1735
H--P--E--C--L--P--Q--A--M--N--I--T--C--T--G--R--G--P--D--N-- 578

GTATCCAGTGTGCCCACTACATTGACGGCCCCCACTGCGTCAAGACCTGCCCGGCAGGAG 1795
C--I--Q--C--A--H--Y--I--D--G--P--H--C--V--K--T--C--P--A--G-- 598

TCATGGGAGAAAACAACACCCTGGTCTGGAAGTACGCAGACGCCGGCCATGTGTGCCACC 1855
V--M--G--E--N--N--T--L--V--W--K--Y--A--D--A--G--H--V--C--H-- 618

TGTGCCATCCAAACTGCACCTACGGATGCACTGGGCCAGGTCTTGAAGGCTGTCCAACGA 1915
L--C--H--P--N--C--T--Y--G--C--T--G--P--G--L--E--G--C--P--T-- 638

ATGGGCCTAAGATCCCGTCCATCGCCACTGGGATGGTGGGGGCCCTCCTCTTGCTGCTGG 1975
N--G--P--K--I--P--S--I--A--T--G--M--V--G--A--L--L--L--L--L-- 658

TGGTGGCCCTGGGGATCGGCCTCTTCATGCGAAGGCGCCACATCGTTCGGAAGCGCACGC 2035
V--V--A--L--G--I--G--L--F--M--R--R--R--H--I--V--R--K--R--T-- 678

Fig. 71 cont.
(2/4)

```
TGCGGAGGCTGCTGCAGGAGAGGGAGCTTGTGGAGCCTCTTACACCCAGTGGAGAAGCTC 2095
L--R--R--L--L--Q--E--R--E--L--V--E--P--L--T--P--S--G--E--A-- 698

CCAACCAAGCTCTCTTGAGGATCTTGAAGGAAACTGAATTCAAAAAGATCAAAGTGCTGG 2155
P--N--Q--A--L--L--R--I--L--K--E--T--E--F--K--K--I--K--V--L-- 718

GCTCCGGTGCGTTCGGCACGGTGTATAAGGGACTCTGGATCCCAGAAGGTGAGAAAGTTA 2215
G--S--G--A--F--G--T--V--Y--K--G--L--W--I--P--E--G--E--K--V-- 738

AAATTCCCGTCGCTATCAAGGAATTAAGAGAAGCAACATCTCCGAAAGCCAACAAGGAAA 2275
K--I--P--V--A--I--K--E--L--R--E--A--T--S--P--K--A--N--K--E-- 758

TCCTCGATGAAGCCTACGTGATGGCCAGCGTGGACAACCCCCACGTGTGCCGCCTGCTGG 2335
I--L--D--E--A--Y--V--M--A--S--V--D--N--P--H--V--C--R--L--L-- 778

GCATCTGCCTCACCTCCACCGTGCAGCTCATCACGCAGCTCATGCCCTTCGGCTGCCTCC 2395
G--I--C--L--T--S--T--V--Q--L--I--T--Q--L--M--P--F--G--C--L-- 798

TGGACTATGTCCGGGAACACAAAGACAATATTGGCTCCCAGTACCTGCTCAACTGGTGTG 2455
L--D--Y--V--R--E--H--K--D--N--I--G--S--Q--Y--L--L--N--W--C-- 818

TGCAGATCGCAAAGGGCATGAACTACTTGGAGGACCGTCGCTTGGTGCACCGCGACCTGG 2515
V--Q--I--A--K--G--M--N--Y--L--E--D--R--R--L--V--H--R--D--L-- 838

CAGCCAGGAACGTACTGGTGAAAACACCGCAGCATGTCAAGATCACAGATTTTGGGCTGG 2575
A--A--R--N--V--L--V--K--T--P--Q--H--V--K--I--T--D--F--G--L-- 858

CCAAACTGCTGGGTGCGGAAGAGAAAGAATACCATGCAGAAGGAGGCAAAGTGCCTATCA 2635
A--K--L--L--G--A--E--E--K--E--Y--H--A--E--G--G--K--V--P--I-- 878

AGTGGATGGCATTGGAATCAATTTTACACAGAATCTATACCCACCAGAGTGATGTCTGGA 2695
K--W--M--A--L--E--S--I--L--H--R--I--Y--T--H--Q--S--D--V--W-- 898

GCTACGGGGTGACTGTTTGGGAGTTGATGACCTTTGGATCCAAGCCATATGACGGAATCC 2755
S--Y--G--V--T--V--W--E--L--M--T--F--G--S--K--P--Y--D--G--I-- 918

CTGCCAGCGAGATCTCCTCCATCCTGGAGAAAGGAGAACGCCTCCCTCAGCCACCCATAT 2815
P--A--S--E--I--S--S--I--L--E--K--G--E--R--L--P--Q--P--P--I-- 938

GTACCATCGATGTCTACATGATCATGGTCAAGTGCTGGATGATAGACGCAGATAGTCGCC 2875
C--T--I--D--V--Y--M--I--M--V--K--C--W--M--I--D--A--D--S--R-- 958

CAAAGTTCCGTGAGTTGATCATCGAATTCTCCAAAATGGCCCGAGACCCCCAGCGCTACC 2935
P--K--F--R--E--L--I--I--E--F--S--K--M--A--R--D--P--Q--R--Y-- 978

TTGTCATTCAGGGGGATGAAAGAATGCATTTGCCAAGTCCTACAGACTCCAACTTCTACC 2995
L--V--I--Q--G--D--E--R--M--H--L--P--S--P--T--D--S--N--F--Y-- 998

GTGCCCTGATGGATGAAGAAGACATGGACGACGTGGTGGATGCCGACGAGTACCTCATCC 3055
R--A--L--M--D--E--E--D--M--D--D--V--V--D--A--D--E--Y--L--I-- 1018

CACAGCAGGGCTTCTTCAGCAGCCCCTCCACGTCACGGACTCCCCTCCTGAGCTCTCTGA 3115
P--Q--Q--G--F--F--S--S--P--S--T--S--R--T--P--L--L--S--S--L-- 1038

GTGCAACCAGCAACAATTCCACCGTGGCTTGCATTGATAGAAATGGGCTGCAAAGCTGTC 3175
S--A--T--S--N--N--S--T--V--A--C--I--D--R--N--G--L--Q--S--C-- 1058
```

Fig. 71 cont.
(3/4)

Fig. 71 cont. (4/4)

```
CCATCAAGGAAGACAGCTTCTTGCAGCGATACAGCTCAGACCCCACAGGCGCCTTGACTG 3235
P--I--K--E--D--S--F--L--Q--R--Y--S--S--D--P--T--G--A--L--T-  1078

AGGACAGCATAGACGACACCTTCCTCCCAGTGCCTGAATACATAAACCAGTCCGTTCCCA 3295
E--D--S--I--D--D--T--F--L--P--V--P--E--Y--I--N--Q--S--V--P-- 1098

AAAGGCCCGCTGGCTCTGTGCAGAATCCTGTCTATCACAATCAGCCTCTGAACCCCGCGC 3355
K--R--P--A--G--S--V--Q--N--P--V--Y--H--N--Q--P--L--N--P--A-  1118

CCAGCAGAGACCCACACTACCAGGACCCCCACAGCACTGCAGTGGGCAACCCCGAGTATC 3415
P--S--R--D--P--H--Y--Q--DT-P--H--S--T--A--V--G--N--P--E--Y-  1138

TCAACACTGTCCAGCCCACCTGTGTCAACAGCACATTCGACAGCCCTGCCCACTGGGCCC 3475
L--N--T--V--Q--P--T--C--V--N--S--T--F--D--S--P--A--H--W--A-  1158

AGAAAGGCAGCCACCAAATTAGCCTGGACAACCCTGACTACCAGCAGGACTTCTTTCCCA 3535
Q--K--G--S--H--Q--I--S--L--D--N--P--D--Y--Q--Q--D--F--F--P-  1178

AGGAAGCCAAGCCAAATGGCATCTTTAAGGGCTCCACAGCTGAAAATGCAGAATACCTAA 3595
K--E--A--K--P--N--G--I--F--K--G--S--T--A--E--N--A--E--Y--L-- 1198
GGGTCGCGCCACAAAGCAGTGAATTTATTGGAGCATGA 3633 (SEQ ID NO. 511)
R--V--A--P--Q--S--S--E--F--I--G--A--*- 1210 (SEQ ID NO. 512)
```

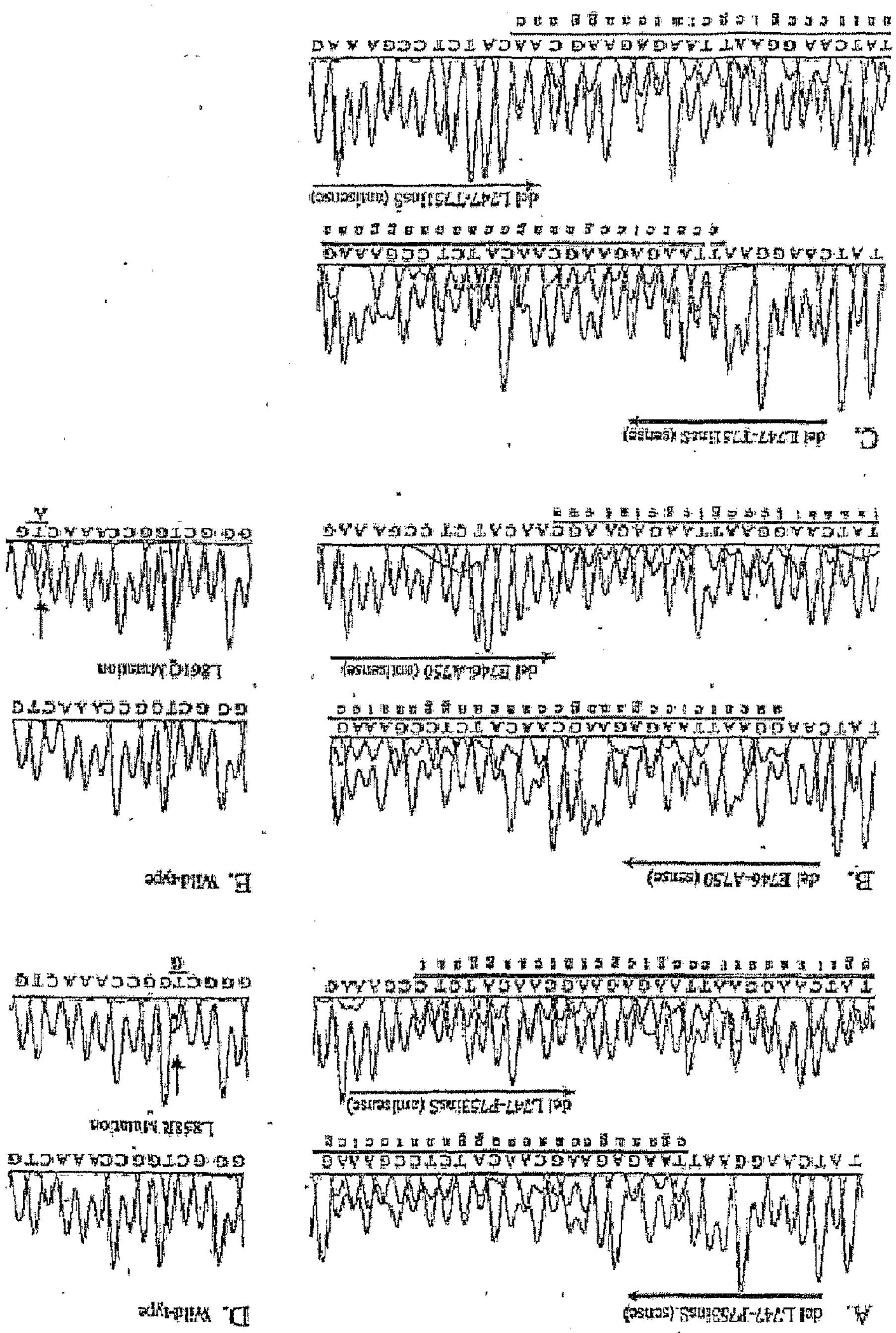
A. del L747-P753insS (sense)
del L747-P753insS (antisense)
B. del E746-A750 (sense)
del E746-A750 (antisense)
C. del L747-T751insS (sense)
del L747-T751insS (antisense)
D. Wild-type
L858R Mutation
E. Wild-type
L861Q Mutation

Figs. 72A-72E

Europäisches Patentamt
European Patent Office
Office européen des brevets

# EUROPEAN SEARCH REPORT

Application Number
EP 12 18 0052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CAGGANA M ET AL: "Development of a Rare Cell Fractionation Device: Application for Cancer Detection", IEEE TRANSACTIONS ON NANOBIOSCIENCE, IEEE SERVICE CENTER, PISCATAWAY, NY, US, vol. 3, no. 4, 1 December 2004 (2004-12-01), pages 251-256, XP011122944, ISSN: 1536-1241, DOI: 10.1109/TNB.2004.837903 * the whole document * ----- | 1-15 | INV. C12M1/00 C12M1/34 C12M3/00 C12Q1/68 G01N15/06 G01N33/00 G01N33/48 B82Y5/00 B82Y10/00 G01N33/543 G01N33/574 ADD. B01L3/00 |
| Y | WO 2004/029221 A2 (GEN HOSPITAL CORP [US]; GPB SCIENT LLC [US]; TONER MEHMET [US]; TRUSKE) 8 April 2004 (2004-04-08) * page 19, line 1 - page 35, line 29; claims; figures * ----- | 1-15 | |
| A | SCHOFIELD K ET AL: "THE USE OF EPCAM COATED IMMUNOMAGNETIC BEADS TO CAPTURE EPITHELIAL CELLS IN FLUID SPECIMENS", ACTA CYTOLOGICA, INTERNATIONAL ACADEMY OF CYTOLOGY, CHICAGO, IL, US, vol. 44, no. 5, 1 September 2000 (2000-09-01), page 919, XP008052884, ISSN: 0001-5547 * the whole document * ----- | 1-15 | TECHNICAL FIELDS SEARCHED (IPC) B01L G01N C12M |
| | -/-- | | |

The present search report has been drawn up for all claims

| Place of search | Date of completion of the search | Examiner |
|---|---|---|
| Munich | 16 April 2013 | Smith-Hewitt, Laura |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 0052

| Category | Citation of document with indication, where appropriate, of relevant passages | Relevant to claim | CLASSIFICATION OF THE APPLICATION (IPC) |
|---|---|---|---|
| A | WENT P TH ET AL: "Frequent EpCam Protein Expression in Human Carcinomas", HUMAN PATHOLOGY, SAUNDERS, PHILADELPHIA, PA, US, vol. 35, no. 1, 1 January 2004 (2004-01-01), pages 122-128, XP002592037, ISSN: 0046-8177, DOI: 10.1016/S0046-8177(03)00502-1 * the whole document * ----- | 11 | |
| A | Jianping Zheng ET AL: "ENHANCED DETECTION OF CIRCULATING TUMOR CELLS IN PERIPHERAL BLOOD SAMPLES FROM PATIENTS WITH ADVANCED BREAST CANCER", , 1 January 2004 (2004-01-01), XP055059675, American Association of Cancer Research Annual Meeting 2004 Retrieved from the Internet: URL:http://wavesense.com/reference literature/USC AACR.pdf [retrieved on 2013-04-15] * the whole document * ----- | 11 | TECHNICAL FIELDS SEARCHED (IPC) |

1

The present search report has been drawn up for all claims

| Place of search | Date of completion of the search | Examiner |
|---|---|---|
| Munich | 16 April 2013 | Smith-Hewitt, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 18 0052

This annex lists the patent family members relating to the patent documents cited in the above-mentioned European search report. The members are as contained in the European Patent Office EDP file on
The European Patent Office is in no way liable for these particulars which are merely given for the purpose of information.

16-04-2013

| Patent document cited in search report | Publication date | Patent family member(s) | Publication date |
|---|---|---|---|
| WO 2004029221 A2 | 08-04-2004 | AT 531257 T | 15-11-2011 |
| | | AU 2003277153 A1 | 19-04-2004 |
| | | AU 2010212376 A1 | 09-09-2010 |
| | | CA 2500392 A1 | 08-04-2004 |
| | | EP 1569510 A2 | 07-09-2005 |
| | | EP 2359689 A1 | 24-08-2011 |
| | | ES 2375724 T3 | 05-03-2012 |
| | | HK 1079960 A1 | 05-04-2012 |
| | | JP 2006501449 A | 12-01-2006 |
| | | JP 2010075191 A | 08-04-2010 |
| | | US 2006134599 A1 | 22-06-2006 |
| | | US 2007172903 A1 | 26-07-2007 |
| | | US 2007231851 A1 | 04-10-2007 |
| | | US 2007259424 A1 | 08-11-2007 |
| | | US 2007264675 A1 | 15-11-2007 |
| | | US 2012006760 A1 | 12-01-2012 |
| | | WO 2004029221 A2 | 08-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005018677 A
- US 20040144651 A
- WO 2004029221 A
- WO 2004113877 A
- US 5837115 A
- US 6692952 A
- US 703833 P
- US 60704067 B
- US 11227904 B
- US 20050266433 A
- US 5641628 A
- US 668415 P
- US 6692952 B
- US 20040166555 A
- US 146581 A
- US 20050282293 A
- US 227469 A
- WO 2005094357 A
- WO 0004194 A
- US 5493531 A
- US 5459039 A


### Non-patent literature cited in the description


- **TORRANCE et al.** *Nature Medicine,* September 2000, vol. 6 (9), 1024
- **GREENBERGER et al.** Proc. 11th NCI EORTC-AACR Symposium on New Drugs in Cancer Therapy. *Clinical Cancer Res.,* November 2000, vol. 6, ISSN 1078-0432
- **RABINDRAN et al.** *Cancer Res.,* 2004, vol. 64, 3958-3965
- **HOLBRO ; HYNES.** *Ann. Rev. Pharm. Tox.,* 2004, vol. 44, 195-217
- **TSOU et al.** *J. Med. Chem.,* 2005, vol. 48, 1107-1131
- **TEJPAR et al.** *J. Clin. Oncol. ASCO Annual Meeting Proc.,* 2004, vol. 22 (14S), 3579
- **HARLOW et al.** Antibodies: A Laboratory Manual. Cold Spring Harbor Laboratory Press, 1988
- **CARPENTER et al.** *Ann. Rev. Biochem.,* 1987, vol. 56, 881-914
- **HUMPHREY et al.** *PNAS (USA,* 1990, vol. 87, 4207-4211
- **HUANG et al.** *Science,* 2004, vol. 304, 987-990
- **GOURLEY et al.** *J. Phys. D: Appl. Phys.,* 2003, vol. 36, R228-R239
- **VO-DINH et al.** *IEEE Eng. Med. Biol. Mag.,* 2004, vol. 23, 40-49
- **PARRELLA et al.** *Cancer Res.,* 2001, vol. 61, 7623-7626
- **JONES et al.** *Cancer Res.,* 2001, vol. 61, 1299-1304
- **FLISS et al.** *Science,* 2000, vol. 287, 2017-2019
- **SORIA et al.** *Clin. Cancer Res.,* 1999, vol. 5, 971-975
- **CAREW et al.** *Mol. Cancer,* 2002, vol. 1, 9
- **WALLACE.** *Science,* 1999, vol. 283, 1482-1488
- **GIORDANO et al.** *Am. J.. Pathol.,* 2001, vol. 159, 1231-1238
- **BUCKHAULTS et al.** *Cancer Res.,* 2003, vol. 63, 4144-4149
- **DAS et al.** *J. Clin. Oncol.,* 2004, vol. 22, 4632-4642
- **HE et al.** *Nature,* 2005, vol. 435, 828-833
- **LU et al.** *Nature,* 2005, vol. 435, 834-838
- **O'DONNELL et al.** *Nature,* 2005, vol. 435, 839-843
- **CALIN et al.** *N. Engl. J. Med.,* 2005, vol. 353, 1793-1801
- **KAPLAN et al.** *Nature,* 2005, vol. 438, 820-827
- **BRUGGER et al.** *Blood,* 1994, vol. 83, 636-640
- **CARSON-WALTER et al.** *Cancer Res.,* 2001, vol. 61, 6649-6655
- **LANDEGRAN et al.** *Science,* 1988, vol. 241, 1077-1080
- **NAKAZAWA et al.** *Proc. Natl. Acad. Sci. USA,* 1994, vol. 91, 360-364
- **ABRAVAYA et al.** *Nucl. Acids Res.,* 1995, vol. 23, 675-682
- **GUATELLI et al.** *Proc. Natl. Acad. Sci. USA,* 1990, vol. 87, 1874-1 878
- **KWOH et al.** *Proc. Natl. Acad. Sci. USA,* 1989, vol. 86, 1173-1177
- **LIZARDI et al.** *BioTechnology,* 1988, vol. 6, 1197
- **MYERS et al.** *Science,* 1985, vol. 230, 1242

- **COTTON et al.** *Proc. Natl. Acad. Sci. USA,* 1988, vol. 85, 4397
- **SALEEBA et al.** *Methods Enzymol.,* 1992, vol. 2 (17), 286-295
- **HSU et al.** *Carcinogenesis,* 1994, vol. 15, 1657-1662
- **ORITA et al.** *Proc. Natl. Acad. Sci. USA,* 1989, vol. 86, 2766
- **COTTON.** *Mutat. Res.,* 1993, vol. 285, 125-144
- **HAYASHI.** *Genet. Anal. Tech. Appl.,* 1992, vol. 9, 73-79
- **KEEN et al.** *Trends Genet.,* 1991, vol. 7, 5
- **MYERS et al.** *Nature,* 1985, vol. 313, 495
- **ROSENBAUM ; REISSNER.** *Biophys. Chem.,* 1987, vol. 265, 12753
- **SAIKI et al.** *Nature,* 1986, vol. 324, 163
- **SAIKI et al.** *Proc. Natl. Acad. Sci. USA,* 1989, vol. 86, 6230
- **GIBBS et al.** *Nucl. Acids Res.,* 1989, vol. 17, 2437-2448
- **PROSSNER.** *Tibteeli,* 1993, vol. 11, 238
- **GASPARINI et al.** *Mol. Cell Probes,* 1992, vol. 6, 1
- **BARANY.** *Proc. Natl. Acad. Sci. USA,* 1991, vol. 88, 189
- **TAKAHASHI et al.** *Nature Med.,* 1999, vol. 5, 434-438
- **HEALY et al.** *Hum. Reprod. Update,* 1998, vol. 4, 736-740
- **GILL et al.** *Circ. Res.,* 2001, vol. 88, 167-174
- **J. SAMBROOK ; E.F. FRITCH ; T. MANIATIS.** Molecular Cloning - A Laboratory Manual. 1989, 7-24
- **PAEZ et al.** *Science,* 2004, vol. 304, 1497-1500